(19) **Europäisches Patentamt / European Patent Office / Office européen des brevets**

(11) **EP 3 133 398 B1**

(12) **EUROPEAN PATENT SPECIFICATION**

(45) Date of publication and mention of the grant of the patent:
**14.10.2020 Bulletin 2020/42**

(51) Int Cl.:
*G01N 33/53* (2006.01)    *G01N 33/68* (2006.01)

(21) Application number: **16001228.2**

(22) Date of filing: **08.01.2012**

(54) **METHODS AND COMPOSITIONS FOR DIAGNOSIS AND PROGNOSIS OF RENAL INJURY AND RENAL FAILURE**

VERFAHREN UND ZUSAMMENSETZUNGEN ZUR DIAGNOSE UND PROGNOSE VON NIERENLÄSION UND NIERENINSUFFIZIENZ

PROCÉDÉS ET COMPOSITIONS POUR LE DIAGNOSTIC ET LE PRONOSTIC DE LÉSION RÉNALE ET D'INSUFFISANCE RÉNALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 08.01.2011  US 201161430989 P
08.01.2011  US 201161430986 P
08.01.2011  US 201161430977 P
08.01.2011  US 201161430979 P
08.01.2011  US 201161430980 P
08.01.2011  US 201161430982 P
08.01.2011  US 201161430987 P
08.01.2011  US 201161430981 P
08.01.2011  US 201161430984 P
08.01.2011  US 201161430983 P
08.01.2011  US 201161430988 P
08.01.2011  US 201161430978 P
08.01.2011  US 201161430985 P

(43) Date of publication of application:
**22.02.2017 Bulletin 2017/08**

(62) Document number(s) of the earlier application(s) in accordance with Art. 76 EPC:
**12732048.9 / 2 661 626**

(73) Proprietor: **Astute Medical, Inc.**
**San Diego, CA 92121 (US)**

(72) Inventors:
• **Anderberg, Joseph**
 **Encinitas, CA 92024 (US)**
• **Gray, Jeff**
 **Solana Beach, CA 92075 (US)**
• **McPherson, Paul**
 **Encinitas, CA 92024 (US)**
• **Nakamura, Kevin**
 **Cardiff By The Sea, CA 92007 (US)**
• **Kampf, James, Patrick**
 **San Diego, CA 92130 (US)**

(74) Representative: **Wilding, James Roger et al**
**Mathys & Squire LLP**
**The Shard**
**32 London Bridge Street**
**London SE1 9SG (GB)**

(56) References cited:
**WO-A1-2010/059996    WO-A1-2010/091236**
**WO-A2-2010/149640**

• **KEIRA MELICAN ET AL: "Bacterial infection-mediated mucosal signalling induces local renal ischaemia as a defence against sepsis", CELLULAR MICROBIOLOGY, vol. 10, no. 10, 8 July 2008 (2008-07-08), pages 1987-1998, XP055333426, GB ISSN: 1462-5814, DOI: 10.1111/j.1462-5822.2008.01182.x**
• **JAN LE S ET AL: "Angiopoietin-like 4 is a proangiogenic factor produced during ischemia and in conventional renal cell carcinoma", AMERICAN JOURNAL OF PATHOLOGY; [10640], ELSEVIER INC, US, vol. 162, no. 5, 1 May 2003 (2003-05-01), pages 1521-1528, XP002377971, ISSN: 0002-9440**

**(Cont. next page)**

- JÉRÔME VERINE ET AL: "Determination of Angptl4 mRNA as a Diagnostic Marker of Primary and Metastatic Clear Cell Renal-Cell Carcinoma", PLOS ONE, vol. 5, no. 4, 29 April 2010 (2010-04-29), page e10421, XP55333343, DOI: 10.1371/journal.pone.0010421
- ITO Y ET AL: "Inhibition of angiogenesis and vascular leakiness by angiopoietin-related protein 4", CANCER RESEARCH, AMERICAN ASSOCIATION FOR CANCER RESEARCH, US, vol. 63, no. 20, 15 October 2003 (2003-10-15), pages 6651-6657, XP002373803, ISSN: 0008-5472
- ZHONG: "CR064, a fully human monoclonal antibody to angiopoietin related protein 4 (ARP4) inhibits ARP4-mediated angiogenesis and renal cell carcinoma survival in vitro", TUMOR BIOOGY 22: TRANSLATIONAL ANGIOGENESIS 1, vol. 65, 1 May 2005 (2005-05-01), page 706, XP055181293,
- YUE DU ET AL: "Urinary biomarkers to detect acute kidney injury in the pediatric emergency center", PEDIATRIC NEPHROLOGY ; JOURNAL OF THE INTERNATIONAL PEDIATRIC NEPHROLOGY ASSOCIATION, SPRINGER, BERLIN, DE, vol. 26, no. 2, 27 October 2010 (2010-10-27), pages 267-274, XP019856153, ISSN: 1432-198X, DOI: 10.1007/S00467-010-1673-0
- COCA STEVEN G ET AL: "Urinary biomarkers for acute kidney injury: perspectives on translation", CLINICAL JOURNAL OF THE AMERICAN SOCIETY OF NEPHROLOGY : CJASN, AMERICAN SOCIETY OF NEPHROLOGY, US, vol. 3, no. 2, 1 March 2008 (2008-03-01), pages 481-490, XP009108667, ISSN: 1555-905X, DOI: 10.2215/CJN.03520807
- LIANG X L ET AL: "Beyond Early Diagnosis: Prognostic Biomarkers for Monitoring Acute Kidney Injury", HONG KONG JOURNAL OF NEPHROLOGY, XX, HG, vol. 12, no. 2, 1 October 2010 (2010-10-01), pages 45-49, XP027547649, ISSN: 1561-5413 [retrieved on 2010-10-01]

SEQUENCE LISTING

<110> ASTUTE MEDICAL, INC.

<120> METHODS AND COMPOSITIONS FOR DIAGNOSIS AND PROGNOSIS OF RENAL INJURY AND RENAL FAILURE

<130> BLG14833PCTEPD1

<140> PCT/US2012/020572
<141> 2012-01-08

<150> 61/430,977
<151> 2011-01-08

<150> 61/430,978
<151> 2011-01-08

<150> 61/430,979
<151> 2011-01-08

<150> 61/430,980
<151> 2011-01-08

<150> 61/430,981
<151> 2011-01-08

<150> 61/430,982
<151> 2011-01-08

<150> 61/430,983
<151> 2011-01-08

<150> 61/430,984
<151> 2011-01-08

<150> 61/430,985
<151> 2011-01-08

<150> 61/430,986
<151> 2011-01-08

<150> 61/430,987
<151> 2011-01-08

<150> 61/430,988
<151> 2011-01-08

<150> 61/430,989
<151> 2011-01-08

<160> 17

<170> PatentIn version 3.5

<210> 1
<211> 208
<212> PRT
<213> Homo sapiens

<400> 1

```
        Met Lys Leu Leu Pro Ser Val Val Leu Lys Leu Phe Leu Ala Ala Val
        1                   5                   10                  15
```

```
Leu Ser Ala Leu Val Thr Gly Glu Ser Leu Glu Arg Leu Arg Arg Gly
         20                  25                  30

Leu Ala Ala Gly Thr Ser Asn Pro Asp Pro Pro Thr Val Ser Thr Asp
         35                  40                  45

Gln Leu Leu Pro Leu Gly Gly Gly Arg Asp Arg Lys Val Arg Asp Leu
    50                  55                  60

Gln Glu Ala Asp Leu Asp Leu Leu Arg Val Thr Leu Ser Ser Lys Pro
65                  70                  75                  80

Gln Ala Leu Ala Thr Pro Asn Lys Glu Glu His Gly Lys Arg Lys Lys
                85                  90                  95

Lys Gly Lys Gly Leu Gly Lys Lys Arg Asp Pro Cys Leu Arg Lys Tyr
        100                 105                 110

Lys Asp Phe Cys Ile His Gly Glu Cys Lys Tyr Val Lys Glu Leu Arg
        115                 120                 125

Ala Pro Ser Cys Ile Cys His Pro Gly Tyr His Gly Glu Arg Cys His
    130                 135                 140

Gly Leu Ser Leu Pro Val Glu Asn Arg Leu Tyr Thr Tyr Asp His Thr
145                 150                 155                 160

Thr Ile Leu Ala Val Val Ala Val Val Leu Ser Ser Val Cys Leu Leu
                165                 170                 175

Val Ile Val Gly Leu Leu Met Phe Arg Tyr His Arg Arg Gly Gly Tyr
        180                 185                 190

Asp Val Glu Asn Glu Glu Lys Val Lys Leu Gly Met Thr Asn Ser His
    195                 200                 205
```

<210> 2
<211> 2201
<212> PRT
<213> Homo sapiens

<400> 2

```
Met Gly Ala Met Thr Gln Leu Leu Ala Gly Val Phe Leu Ala Phe Leu
1               5                   10                  15

Ala Leu Ala Thr Glu Gly Gly Val Leu Lys Lys Val Ile Arg His Lys
         20                  25                  30
```

```
Arg Gln Ser Gly Val Asn Ala Thr Leu Pro Glu Glu Asn Gln Pro Val
        35              40              45

Val Phe Asn His Val Tyr Asn Ile Lys Leu Pro Val Gly Ser Gln Cys
        50              55              60

Ser Val Asp Leu Glu Ser Ala Ser Gly Glu Lys Asp Leu Ala Pro Pro
65              70              75              80

Ser Glu Pro Ser Glu Ser Phe Gln Glu His Thr Val Asp Gly Glu Asn
            85              90              95

Gln Ile Val Phe Thr His Arg Ile Asn Ile Pro Arg Arg Ala Cys Gly
        100             105             110

Cys Ala Ala Ala Pro Asp Val Lys Glu Leu Leu Ser Arg Leu Glu Glu
        115             120             125

Leu Glu Asn Leu Val Ser Ser Leu Arg Glu Gln Cys Thr Ala Gly Ala
        130             135             140

Gly Cys Cys Leu Gln Pro Ala Thr Gly Arg Leu Asp Thr Arg Pro Phe
145             150             155             160

Cys Ser Gly Arg Gly Asn Phe Ser Thr Glu Gly Cys Gly Cys Val Cys
            165             170             175

Glu Pro Gly Trp Lys Gly Pro Asn Cys Ser Glu Pro Glu Cys Pro Gly
            180             185             190

Asn Cys His Leu Arg Gly Arg Cys Ile Asp Gly Gln Cys Ile Cys Asp
        195             200             205

Asp Gly Phe Thr Gly Glu Asp Cys Ser Gln Leu Ala Cys Pro Ser Asp
        210             215             220

Cys Asn Asp Gln Gly Lys Cys Val Asn Gly Val Cys Ile Cys Phe Glu
225             230             235             240

Gly Tyr Ala Gly Ala Asp Cys Ser Arg Glu Ile Cys Pro Val Pro Cys
            245             250             255

Ser Glu Glu His Gly Thr Cys Val Asp Gly Leu Cys Val Cys His Asp
            260             265             270

Gly Phe Ala Gly Asp Asp Cys Asn Lys Pro Leu Cys Leu Asn Asn Cys
            275             280             285
```

```
Tyr Asn Arg Gly Arg Cys Val Glu Asn Glu Cys Val Cys Asp Glu Gly
    290             295             300

Phe Thr Gly Glu Asp Cys Ser Glu Leu Ile Cys Pro Asn Asp Cys Phe
305             310             315             320

Asp Arg Gly Arg Cys Ile Asn Gly Thr Cys Tyr Cys Glu Glu Gly Phe
                325             330             335

Thr Gly Glu Asp Cys Gly Lys Pro Thr Cys Pro His Ala Cys His Thr
            340             345             350

Gln Gly Arg Cys Glu Glu Gly Gln Cys Val Cys Asp Glu Gly Phe Ala
            355             360             365

Gly Val Asp Cys Ser Glu Lys Arg Cys Pro Ala Asp Cys His Asn Arg
    370             375             380

Gly Arg Cys Val Asp Gly Arg Cys Glu Cys Asp Asp Gly Phe Thr Gly
385             390             395             400

Ala Asp Cys Gly Glu Leu Lys Cys Pro Asn Gly Cys Ser Gly His Gly
            405             410             415

Arg Cys Val Asn Gly Gln Cys Val Cys Asp Glu Gly Tyr Thr Gly Glu
            420             425             430

Asp Cys Ser Gln Leu Arg Cys Pro Asn Asp Cys His Ser Arg Gly Arg
            435             440             445

Cys Val Glu Gly Lys Cys Val Cys Glu Gln Gly Phe Lys Gly Tyr Asp
    450             455             460

Cys Ser Asp Met Ser Cys Pro Asn Asp Cys His Gln His Gly Arg Cys
465             470             475             480

Val Asn Gly Met Cys Val Cys Asp Asp Gly Tyr Thr Gly Glu Asp Cys
            485             490             495

Arg Asp Arg Gln Cys Pro Arg Asp Cys Ser Asn Arg Gly Leu Cys Val
            500             505             510

Asp Gly Gln Cys Val Cys Glu Asp Gly Phe Thr Gly Pro Asp Cys Ala
            515             520             525

Glu Leu Ser Cys Pro Asn Asp Cys His Gly Gln Gly Arg Cys Val Asn
```

                530                        535                              540


Gly Gln Cys Val Cys His Glu Gly Phe Met Gly Lys Asp Cys Lys Glu
545                    550                    555                    560


Gln Arg Cys Pro Ser Asp Cys His Gly Gln Gly Arg Cys Val Asp Gly
                565                    570                    575


Gln Cys Ile Cys His Glu Gly Phe Thr Gly Leu Asp Cys Gly Gln His
            580                    585                    590


Ser Cys Pro Ser Asp Cys Asn Asn Leu Gly Gln Cys Val Ser Gly Arg
            595                    600                    605


Cys Ile Cys Asn Glu Gly Tyr Ser Gly Glu Asp Cys Ser Glu Val Ser
    610                    615                    620


Pro Pro Lys Asp Leu Val Val Thr Glu Val Thr Glu Glu Thr Val Asn
625                    630                    635                    640


Leu Ala Trp Asp Asn Glu Met Arg Val Thr Glu Tyr Leu Val Val Tyr
                645                    650                    655


Thr Pro Thr His Glu Gly Gly Leu Glu Met Gln Phe Arg Val Pro Gly
            660                    665                    670


Asp Gln Thr Ser Thr Ile Ile Gln Glu Leu Glu Pro Gly Val Glu Tyr
            675                    680                    685


Phe Ile Arg Val Phe Ala Ile Leu Glu Asn Lys Lys Ser Ile Pro Val
    690                    695                    700


Ser Ala Arg Val Ala Thr Tyr Leu Pro Ala Pro Glu Gly Leu Lys Phe
705                    710                    715                    720


Lys Ser Ile Lys Glu Thr Ser Val Glu Val Glu Trp Asp Pro Leu Asp
                725                    730                    735


Ile Ala Phe Glu Thr Trp Glu Ile Ile Phe Arg Asn Met Asn Lys Glu
            740                    745                    750


Asp Glu Gly Glu Ile Thr Lys Ser Leu Arg Arg Pro Glu Thr Ser Tyr
            755                    760                    765


Arg Gln Thr Gly Leu Ala Pro Gly Gln Glu Tyr Glu Ile Ser Leu His
    770                    775                    780

```
Ile Val Lys Asn Asn Thr Arg Gly Pro Gly Leu Lys Arg Val Thr Thr
785                 790             795                 800

Thr Arg Leu Asp Ala Pro Ser Gln Ile Glu Val Lys Asp Val Thr Asp
            805             810             815

Thr Thr Ala Leu Ile Thr Trp Phe Lys Pro Leu Ala Glu Ile Asp Gly
            820             825             830

Ile Glu Leu Thr Tyr Gly Ile Lys Asp Val Pro Gly Asp Arg Thr Thr
            835             840             845

Ile Asp Leu Thr Glu Asp Glu Asn Gln Tyr Ser Ile Gly Asn Leu Lys
    850             855             860

Pro Asp Thr Glu Tyr Glu Val Ser Leu Ile Ser Arg Arg Gly Asp Met
865             870             875                 880

Ser Ser Asn Pro Ala Lys Glu Thr Phe Thr Thr Gly Leu Asp Ala Pro
            885             890             895

Arg Asn Leu Arg Arg Val Ser Gln Thr Asp Asn Ser Ile Thr Leu Glu
            900             905             910

Trp Arg Asn Gly Lys Ala Ala Ile Asp Ser Tyr Arg Ile Lys Tyr Ala
            915             920             925

Pro Ile Ser Gly Gly Asp His Ala Glu Val Asp Val Pro Lys Ser Gln
    930             935             940

Gln Ala Thr Thr Lys Thr Thr Leu Thr Gly Leu Arg Pro Gly Thr Glu
945             950             955                 960

Tyr Gly Ile Gly Val Ser Ala Val Lys Glu Asp Lys Glu Ser Asn Pro
            965             970             975

Ala Thr Ile Asn Ala Ala Thr Glu Leu Asp Thr Pro Lys Asp Leu Gln
            980             985             990

Val Ser Glu Thr Ala Glu Thr Ser Leu Thr Leu Leu Trp Lys Thr Pro
    995             1000            1005

Leu Ala Lys Phe Asp Arg Tyr Arg Leu Asn Tyr Ser Leu Pro Thr
    1010            1015            1020

Gly Gln Trp Val Gly Val Gln Leu Pro Arg Asn Thr Thr Ser Tyr
    1025            1030            1035
```

```
Val Leu  Arg Gly Leu Glu Pro  Gly Gln Glu Tyr Asn  Val Leu Leu
    1040                1045                1050

Thr Ala  Glu Lys Gly Arg His  Lys Ser Lys Pro Ala  Arg Val Lys
    1055                1060                1065

Ala Ser  Thr Glu Gln Ala Pro  Glu Leu Glu Asn Leu  Thr Val Thr
    1070                1075                1080

Glu Val  Gly Trp Asp Gly Leu  Arg Leu Asn Trp Thr  Ala Ala Asp
    1085                1090                1095

Gln Ala  Tyr Glu His Phe Ile  Ile Gln Val Gln Glu  Ala Asn Lys
    1100                1105                1110

Val Glu  Ala Ala Arg Asn Leu  Thr Val Pro Gly Ser  Leu Arg Ala
    1115                1120                1125

Val Asp  Ile Pro Gly Leu Lys  Ala Ala Thr Pro Tyr  Thr Val Ser
    1130                1135                1140

Ile Tyr  Gly Val Ile Gln Gly  Tyr Arg Thr Pro Val  Leu Ser Ala
    1145                1150                1155

Glu Ala  Ser Thr Gly Glu Thr  Pro Asn Leu Gly Glu  Val Val Val
    1160                1165                1170

Ala Glu  Val Gly Trp Asp Ala  Leu Lys Leu Asn Trp  Thr Ala Pro
    1175                1180                1185

Glu Gly  Ala Tyr Glu Tyr Phe  Phe Ile Gln Val Gln  Glu Ala Asp
    1190                1195                1200

Thr Val  Glu Ala Ala Gln Asn  Leu Thr Val Pro Gly  Gly Leu Arg
    1205                1210                1215

Ser Thr  Asp Leu Pro Gly Leu  Lys Ala Ala Thr His  Tyr Thr Ile
    1220                1225                1230

Thr Ile  Arg Gly Val Thr Gln  Asp Phe Ser Thr Thr  Pro Leu Ser
    1235                1240                1245

Val Glu  Val Leu Thr Glu Glu  Val Pro Asp Met Gly  Asn Leu Thr
    1250                1255                1260

Val Thr  Glu Val Ser Trp Asp  Ala Leu Arg Leu Asn  Trp Thr Thr
    1265                1270                1275
```

```
Pro Asp  Gly Thr Tyr Asp Gln  Phe Thr Ile Gln Val  Gln Glu Ala
    1280             1285             1290

Asp Gln  Val Glu Glu Ala His  Asn Leu Thr Val Pro  Gly Ser Leu
    1295             1300             1305

Arg Ser  Met Glu Ile Pro Gly  Leu Arg Ala Gly Thr  Pro Tyr Thr
    1310             1315             1320

Val Thr  Leu His Gly Glu Val  Arg Gly His Ser Thr  Arg Pro Leu
    1325             1330             1335

Ala Val  Glu Val Val Thr Glu  Asp Leu Pro Gln Leu  Gly Asp Leu
    1340             1345             1350

Ala Val  Ser Glu Val Gly Trp  Asp Gly Leu Arg Leu  Asn Trp Thr
    1355             1360             1365

Ala Ala  Asp Asn Ala Tyr Glu  His Phe Val Ile Gln  Val Gln Glu
    1370             1375             1380

Val Asn  Lys Val Glu Ala Ala  Gln Asn Leu Thr Leu  Pro Gly Ser
    1385             1390             1395

Leu Arg  Ala Val Asp Ile Pro  Gly Leu Glu Ala Ala  Thr Pro Tyr
    1400             1405             1410

Arg Val  Ser Ile Tyr Gly Val  Ile Arg Gly Tyr Arg  Thr Pro Val
    1415             1420             1425

Leu Ser  Ala Glu Ala Ser Thr  Ala Lys Glu Pro Glu  Ile Gly Asn
    1430             1435             1440

Leu Asn  Val Ser Asp Ile Thr  Pro Glu Ser Phe Asn  Leu Ser Trp
    1445             1450             1455

Met Ala  Thr Asp Gly Ile Phe  Glu Thr Phe Thr Ile  Glu Ile Ile
    1460             1465             1470

Asp Ser  Asn Arg Leu Leu Glu  Thr Val Glu Tyr Asn  Ile Ser Gly
    1475             1480             1485

Ala Glu  Arg Thr Ala His Ile  Ser Gly Leu Pro Pro  Ser Thr Asp
    1490             1495             1500

Phe Ile  Val Tyr Leu Ser Gly  Leu Ala Pro Ser Ile  Arg Thr Lys
```

EP 3 133 398 B1

1505　　　　　　　1510　　　　　　　1515

Thr Ile Ser Ala Thr Ala Thr Thr Glu Ala Leu Pro Leu Leu Glu
　　　1520　　　　　　　1525　　　　　　　1530

Asn Leu Thr Ile Ser Asp Ile Asn Pro Tyr Gly Phe Thr Val Ser
　　　1535　　　　　　　1540　　　　　　　1545

Trp Met Ala Ser Glu Asn Ala Phe Asp Ser Phe Leu Val Thr Val
　　　1550　　　　　　　1555　　　　　　　1560

Val Asp Ser Gly Lys Leu Leu Asp Pro Gln Glu Phe Thr Leu Ser
　　　1565　　　　　　　1570　　　　　　　1575

Gly Thr Gln Arg Lys Leu Glu Leu Arg Gly Leu Ile Thr Gly Ile
　　　1580　　　　　　　1585　　　　　　　1590

Gly Tyr Glu Val Met Val Ser Gly Phe Thr Gln Gly His Gln Thr
　　　1595　　　　　　　1600　　　　　　　1605

Lys Pro Leu Arg Ala Glu Ile Val Thr Glu Ala Glu Pro Glu Val
　　　1610　　　　　　　1615　　　　　　　1620

Asp Asn Leu Leu Val Ser Asp Ala Thr Pro Asp Gly Phe Arg Leu
　　　1625　　　　　　　1630　　　　　　　1635

Ser Trp Thr Ala Asp Glu Gly Val Phe Asp Asn Phe Val Leu Lys
　　　1640　　　　　　　1645　　　　　　　1650

Ile Arg Asp Thr Lys Lys Gln Ser Glu Pro Leu Glu Ile Thr Leu
　　　1655　　　　　　　1660　　　　　　　1665

Leu Ala Pro Glu Arg Thr Arg Asp Ile Thr Gly Leu Arg Glu Ala
　　　1670　　　　　　　1675　　　　　　　1680

Thr Glu Tyr Glu Ile Glu Leu Tyr Gly Ile Ser Lys Gly Arg Arg
　　　1685　　　　　　　1690　　　　　　　1695

Ser Gln Thr Val Ser Ala Ile Ala Thr Thr Ala Met Gly Ser Pro
　　　1700　　　　　　　1705　　　　　　　1710

Lys Glu Val Ile Phe Ser Asp Ile Thr Glu Asn Ser Ala Thr Val
　　　1715　　　　　　　1720　　　　　　　1725

Ser Trp Arg Ala Pro Thr Ala Gln Val Glu Ser Phe Arg Ile Thr
　　　1730　　　　　　　1735　　　　　　　1740

256

```
Tyr Val Pro Ile Thr Gly Gly  Thr Pro Ser Met Val  Thr Val Asp
    1745              1750              1755

Gly Thr Lys Thr Gln Thr Arg  Leu Val Lys Leu Ile  Pro Gly Val
    1760              1765              1770

Glu Tyr Leu Val Ser Ile Ile  Ala Met Lys Gly Phe  Glu Glu Ser
    1775              1780              1785

Glu Pro Val Ser Gly Ser Phe  Thr Thr Ala Leu Asp  Gly Pro Ser
    1790              1795              1800

Gly Leu Val Thr Ala Asn Ile  Thr Asp Ser Glu Ala  Leu Ala Arg
    1805              1810              1815

Trp Gln Pro Ala Ile Ala Thr  Val Asp Ser Tyr Val  Ile Ser Tyr
    1820              1825              1830

Thr Gly Glu Lys Val Pro Glu  Ile Thr Arg Thr Val  Ser Gly Asn
    1835              1840              1845

Thr Val Glu Tyr Ala Leu Thr  Asp Leu Glu Pro Ala  Thr Glu Tyr
    1850              1855              1860

Thr Leu Arg Ile Phe Ala Glu  Lys Gly Pro Gln Lys  Ser Ser Thr
    1865              1870              1875

Ile Thr Ala Lys Phe Thr Thr  Asp Leu Asp Ser Pro  Arg Asp Leu
    1880              1885              1890

Thr Ala Thr Glu Val Gln Ser  Glu Thr Ala Leu Leu  Thr Trp Arg
    1895              1900              1905

Pro Pro Arg Ala Ser Val Thr  Gly Tyr Leu Leu Val  Tyr Glu Ser
    1910              1915              1920

Val Asp Gly Thr Val Lys Glu  Val Ile Val Gly Pro  Asp Thr Thr
    1925              1930              1935

Ser Tyr Ser Leu Ala Asp Leu  Ser Pro Ser Thr His  Tyr Thr Ala
    1940              1945              1950

Lys Ile Gln Ala Leu Asn Gly  Pro Leu Arg Ser Asn  Met Ile Gln
    1955              1960              1965

Thr Ile Phe Thr Thr Ile Gly  Leu Leu Tyr Pro Phe  Pro Lys Asp
    1970              1975              1980
```

```
Cys Ser Gln Ala Met Leu Asn Gly Asp Thr Thr Ser Gly Leu Tyr
    1985            1990            1995

Thr Ile Tyr Leu Asn Gly Asp Lys Ala Glu Ala Leu Glu Val Phe
    2000            2005            2010

Cys Asp Met Thr Ser Asp Gly Gly Gly Trp Ile Val Phe Leu Arg
    2015            2020            2025

Arg Lys Asn Gly Arg Glu Asn Phe Tyr Gln Asn Trp Lys Ala Tyr
    2030            2035            2040

Ala Ala Gly Phe Gly Asp Arg Arg Glu Glu Phe Trp Leu Gly Leu
    2045            2050            2055

Asp Asn Leu Asn Lys Ile Thr Ala Gln Gly Gln Tyr Glu Leu Arg
    2060            2065            2070

Val Asp Leu Arg Asp His Gly Glu Thr Ala Phe Ala Val Tyr Asp
    2075            2080            2085

Lys Phe Ser Val Gly Asp Ala Lys Thr Arg Tyr Lys Leu Lys Val
    2090            2095            2100

Glu Gly Tyr Ser Gly Thr Ala Gly Asp Ser Met Ala Tyr His Asn
    2105            2110            2115

Gly Arg Ser Phe Ser Thr Phe Asp Lys Asp Thr Asp Ser Ala Ile
    2120            2125            2130

Thr Asn Cys Ala Leu Ser Tyr Lys Gly Ala Phe Trp Tyr Arg Asn
    2135            2140            2145

Cys His Arg Val Asn Leu Met Gly Arg Tyr Gly Asp Asn Asn His
    2150            2155            2160

Ser Gln Gly Val Asn Trp Phe His Trp Lys Gly His Glu His Ser
    2165            2170            2175

Ile Gln Phe Ala Glu Met Lys Leu Arg Pro Ser Asn Phe Arg Asn
    2180            2185            2190

Leu Glu Gly Arg Arg Lys Arg Ala
    2195            2200
```

<210> 3
<211> 406

<212> PRT
<213> Homo sapiens

<400> 3

```
Met Ser Gly Ala Pro Thr Ala Gly Ala Ala Leu Met Leu Cys Ala Ala
1             5                   10              15

Thr Ala Val Leu Leu Ser Ala Gln Gly Gly Pro Val Gln Ser Lys Ser
             20                  25              30

Pro Arg Phe Ala Ser Trp Asp Glu Met Asn Val Leu Ala His Gly Leu
         35                  40              45

Leu Gln Leu Gly Gln Gly Leu Arg Glu His Ala Glu Arg Thr Arg Ser
    50                  55              60

Gln Leu Ser Ala Leu Glu Arg Arg Leu Ser Ala Cys Gly Ser Ala Cys
65                  70              75              80

Gln Gly Thr Glu Gly Ser Thr Asp Leu Pro Leu Ala Pro Glu Ser Arg
             85                  90              95

Val Asp Pro Glu Val Leu His Ser Leu Gln Thr Gln Leu Lys Ala Gln
         100                 105             110

Asn Ser Arg Ile Gln Gln Leu Phe His Lys Val Ala Gln Gln Gln Arg
         115                 120             125

His Leu Glu Lys Gln His Leu Arg Ile Gln His Leu Gln Ser Gln Phe
    130                 135             140

Gly Leu Leu Asp His Lys His Leu Asp His Glu Val Ala Lys Pro Ala
145             150             155             160

Arg Arg Lys Arg Leu Pro Glu Met Ala Gln Pro Val Asp Pro Ala His
             165             170             175

Asn Val Ser Arg Leu His Arg Leu Pro Arg Asp Cys Gln Glu Leu Phe
         180             185             190

Gln Val Gly Glu Arg Gln Ser Gly Leu Phe Glu Ile Gln Pro Gln Gly
         195             200             205

Ser Pro Pro Phe Leu Val Asn Cys Lys Met Thr Ser Asp Gly Gly Trp
    210             215             220

Thr Val Ile Gln Arg Arg His Asp Gly Ser Val Asp Phe Asn Arg Pro
225             230             235             240
```

260

```
Trp Glu Ala Tyr Lys Ala Gly Phe Gly Asp Pro His Gly Glu Phe Trp
                245             250             255

Leu Gly Leu Glu Lys Val His Ser Ile Thr Gly Asp Arg Asn Ser Arg
            260             265             270

Leu Ala Val Gln Leu Arg Asp Trp Asp Gly Asn Ala Glu Leu Leu Gln
        275             280             285

Phe Ser Val His Leu Gly Gly Glu Asp Thr Ala Tyr Ser Leu Gln Leu
    290             295             300

Thr Ala Pro Val Ala Gly Gln Leu Gly Ala Thr Thr Val Pro Pro Ser
305             310             315             320

Gly Leu Ser Val Pro Phe Ser Thr Trp Asp Gln Asp His Asp Leu Arg
            325             330             335

Arg Asp Lys Asn Cys Ala Lys Ser Leu Ser Gly Gly Trp Trp Phe Gly
        340             345             350

Thr Cys Ser His Ser Asn Leu Asn Gly Gln Tyr Phe Arg Ser Ile Pro
    355             360             365

Gln Gln Arg Gln Lys Leu Lys Lys Gly Ile Phe Trp Lys Thr Trp Arg
    370             375             380

Gly Arg Tyr Tyr Pro Leu Gln Ala Thr Thr Met Leu Ile Gln Pro Met
385             390             395             400

Ala Ala Glu Ala Ala Ser
                405
```

<210> 4
<211> 216
<212> PRT
<213> Homo sapiens

<400> 4

```
Met Arg Ser Gly Cys Val Val Val His Val Trp Ile Leu Ala Gly Leu
1               5               10              15

Trp Leu Ala Val Ala Gly Arg Pro Leu Ala Phe Ser Asp Ala Gly Pro
            20              25              30

His Val His Tyr Gly Trp Gly Asp Pro Ile Arg Leu Arg His Leu Tyr
            35              40              45
```

261

```
Thr Ser Gly Pro His Gly Leu Ser Ser Cys Phe Leu Arg Ile Arg Ala
    50              55              60

Asp Gly Val Val Asp Cys Ala Arg Gly Gln Ser Ala His Ser Leu Leu
65              70              75              80

Glu Ile Lys Ala Val Ala Leu Arg Thr Val Ala Ile Lys Gly Val His
                85              90              95

Ser Val Arg Tyr Leu Cys Met Gly Ala Asp Gly Lys Met Gln Gly Leu
            100             105             110

Leu Gln Tyr Ser Glu Glu Asp Cys Ala Phe Glu Glu Glu Ile Arg Pro
            115             120             125

Asp Gly Tyr Asn Val Tyr Arg Ser Glu Lys His Arg Leu Pro Val Ser
    130             135             140

Leu Ser Ser Ala Lys Gln Arg Gln Leu Tyr Lys Asn Arg Gly Phe Leu
145             150             155             160

Pro Leu Ser His Phe Leu Pro Met Leu Pro Met Val Pro Glu Glu Pro
            165             170             175

Glu Asp Leu Arg Gly His Leu Glu Ser Asp Met Phe Ser Ser Pro Leu
            180             185             190

Glu Thr Asp Ser Met Asp Pro Phe Gly Leu Val Thr Gly Leu Glu Ala
    195             200             205

Val Arg Ser Pro Ser Phe Glu Lys
    210             215
```

<210> 5
<211> 209
<212> PRT
<213> Homo sapiens

<400> 5

```
Met Asp Ser Asp Glu Thr Gly Phe Glu His Ser Gly Leu Trp Val Ser
1               5               10              15

Val Leu Ala Gly Leu Leu Leu Gly Ala Cys Gln Ala His Pro Ile Pro
            20              25              30

Asp Ser Ser Pro Leu Leu Gln Phe Gly Gly Gln Val Arg Gln Arg Tyr
        35              40              45

Leu Tyr Thr Asp Asp Ala Gln Gln Thr Glu Ala His Leu Glu Ile Arg

    50              55              60

Glu Asp Gly Thr Val Gly Gly Ala Ala Asp Gln Ser Pro Glu Ser Leu
65              70              75              80

Leu Gln Leu Lys Ala Leu Lys Pro Gly Val Ile Gln Ile Leu Gly Val
            85              90              95

Lys Thr Ser Arg Phe Leu Cys Gln Arg Pro Asp Gly Ala Leu Tyr Gly
            100             105             110

Ser Leu His Phe Asp Pro Glu Ala Cys Ser Phe Arg Glu Leu Leu Leu
            115             120             125

Glu Asp Gly Tyr Asn Val Tyr Gln Ser Glu Ala His Gly Leu Pro Leu
        130             135             140

His Leu Pro Gly Asn Lys Ser Pro His Arg Asp Pro Ala Pro Arg Gly
145             150             155             160

Pro Ala Arg Phe Leu Pro Leu Pro Gly Leu Pro Pro Ala Leu Pro Glu
            165             170             175

Pro Pro Gly Ile Leu Ala Pro Gln Pro Pro Asp Val Gly Ser Ser Asp
            180             185             190

Pro Leu Ser Met Val Gly Pro Ser Gln Gly Arg Ser Pro Ser Tyr Ala
            195             200             205

Ser
```

<210> 6
<211> 155
<212> PRT
<213> Homo sapiens

263

<400> 6

```
Met Ala Glu Gly Glu Ile Thr Thr Phe Thr Ala Leu Thr Glu Lys Phe
1               5               10              15

Asn Leu Pro Pro Gly Asn Tyr Lys Lys Pro Lys Leu Leu Tyr Cys Ser
            20              25              30

Asn Gly Gly His Phe Leu Arg Ile Leu Pro Asp Gly Thr Val Asp Gly
        35              40              45

Thr Arg Asp Arg Ser Asp Gln His Ile Gln Leu Gln Leu Ser Ala Glu
    50              55              60

Ser Val Gly Glu Val Tyr Ile Lys Ser Thr Glu Thr Gly Gln Tyr Leu
65              70              75              80

Ala Met Asp Thr Asp Gly Leu Leu Tyr Gly Ser Gln Thr Pro Asn Glu
            85              90              95

Glu Cys Leu Phe Leu Glu Arg Leu Glu Glu Asn His Tyr Asn Thr Tyr
            100             105             110

Ile Ser Lys Lys His Ala Glu Lys Asn Trp Phe Val Gly Leu Lys Lys
        115             120             125

Asn Gly Ser Cys Lys Arg Gly Pro Arg Thr His Tyr Gly Gln Lys Ala
    130             135             140

Ile Leu Phe Leu Pro Leu Pro Val Ser Ser Asp
145             150             155
```

<210> 7
<211> 470
<212> PRT
<213> Homo sapiens

<400> 7

```
Met Gly Lys Pro Trp Leu Arg Ala Leu Gln Leu Leu Leu Leu Leu Gly
1               5                   10                  15

Ala Ser Trp Ala Arg Ala Gly Ala Pro Arg Cys Thr Tyr Thr Phe Val
            20                  25                  30

Leu Pro Pro Gln Lys Phe Thr Gly Ala Val Cys Trp Ser Gly Pro Ala
        35                  40                  45

Ser Thr Arg Ala Thr Pro Glu Ala Ala Asn Ala Ser Glu Leu Ala Ala
    50                  55                  60

Leu Arg Met Arg Val Gly Arg His Glu Glu Leu Leu Arg Glu Leu Gln
65                  70                  75                  80

Arg Leu Ala Ala Ala Asp Gly Ala Val Ala Gly Glu Val Arg Ala Leu
            85                  90                  95

Arg Lys Glu Ser Arg Gly Leu Ser Ala Arg Leu Gly Gln Leu Arg Ala
            100                 105                 110

Gln Leu Gln His Glu Ala Gly Pro Gly Ala Gly Pro Gly Ala Asp Leu
        115                 120                 125
```

```
Gly Ala Glu Pro Ala Ala Ala Leu Ala Leu Leu Gly Glu Arg Val Leu
    130             135             140

Asn Ala Ser Ala Glu Ala Gln Arg Ala Ala Ala Arg Phe His Gln Leu
145             150             155             160

Asp Val Lys Phe Arg Glu Leu Ala Gln Leu Val Thr Gln Gln Ser Ser
            165             170             175

Leu Ile Ala Arg Leu Glu Arg Leu Cys Pro Gly Gly Ala Gly Gly Gln
            180             185             190

Gln Gln Val Leu Pro Pro Pro Pro Leu Val Pro Val Val Pro Val Arg
        195             200             205

Leu Val Gly Ser Thr Ser Asp Thr Ser Arg Met Leu Asp Pro Ala Pro
    210             215             220

Glu Pro Gln Arg Asp Gln Thr Gln Arg Gln Gln Glu Pro Met Ala Ser
225             230             235             240

Pro Met Pro Ala Gly His Pro Ala Val Pro Thr Lys Pro Val Gly Pro
            245             250             255

Trp Gln Asp Cys Ala Glu Ala Arg Gln Ala Gly His Glu Gln Ser Gly
            260             265             270

Val Tyr Glu Leu Arg Val Gly Arg His Val Val Ser Val Trp Cys Glu
        275             280             285

Gln Gln Leu Glu Gly Gly Gly Trp Thr Val Ile Gln Arg Arg Gln Asp
    290             295             300

Gly Ser Val Asn Phe Phe Thr Thr Trp Gln His Tyr Lys Ala Gly Phe
305             310             315             320

Gly Arg Pro Asp Gly Glu Tyr Trp Leu Gly Leu Glu Pro Val Tyr Gln
            325             330             335

Leu Thr Ser Arg Gly Asp His Glu Leu Leu Val Leu Leu Glu Asp Trp
            340             345             350

Gly Gly Arg Gly Ala Arg Ala His Tyr Asp Gly Phe Ser Leu Glu Pro
        355             360             365

Glu Ser Asp His Tyr Arg Leu Arg Leu Gly Gln Tyr His Gly Asp Ala
370             375             380
```

266

Gly Asp Ser Leu Ser Trp His Asn Asp Lys Pro Phe Ser Thr Val Asp
385             390             395             400

Arg Asp Arg Asp Ser Tyr Ser Gly Asn Cys Ala Leu Tyr Gln Arg Gly
            405             410             415

Gly Trp Trp Tyr His Ala Cys Ala His Ser Asn Leu Asn Gly Val Trp
        420             425             430

His His Gly Gly His Tyr Arg Ser Arg Tyr Gln Asp Gly Val Tyr Trp
        435             440             445

Ala Glu Phe Arg Gly Gly Ala Tyr Ser Leu Arg Lys Ala Ala Met Leu
    450             455             460

Ile Arg Pro Leu Lys Leu
465             470

<210> 8
<211> 169
<212> PRT
<213> Homo sapiens

<400> 8

Met Thr Ala Gly Arg Arg Met Glu Met Leu Cys Ala Gly Arg Val Pro
1           5               10              15

Ala Leu Leu Leu Cys Leu Gly Phe His Leu Leu Gln Ala Val Leu Ser
        20              25              30

Thr Thr Val Ile Pro Ser Cys Ile Pro Gly Glu Ser Ser Asp Asn Cys
        35              40              45

Thr Ala Leu Val Gln Thr Glu Asp Asn Pro Arg Val Ala Gln Val Ser
    50              55              60

Ile Thr Lys Cys Ser Ser Asp Met Asn Gly Tyr Cys Leu His Gly Gln
65              70              75              80

Cys Ile Tyr Leu Val Asp Met Ser Gln Asn Tyr Cys Arg Cys Glu Val
            85              90              95

Gly Tyr Thr Gly Val Arg Cys Glu His Phe Phe Leu Thr Val His Gln
        100             105             110

Pro Leu Ser Lys Glu Tyr Val Ala Leu Thr Val Ile Leu Ile Ile Leu
        115             120             125

267

```
Phe Leu Ile Thr Val Val Gly Ser Thr Tyr Tyr Phe Cys Arg Trp Tyr
    130                 135                 140

Arg Asn Arg Lys Ser Lys Glu Pro Lys Lys Glu Tyr Glu Arg Val Thr
145                 150                 155                 160

Ser Gly Asp Pro Glu Leu Pro Gln Val
                165
```

<210> 9
<211> 178
<212> PRT
<213> Homo sapiens

<400> 9

```
Met Asp Arg Ala Ala Arg Cys Ser Gly Ala Ser Ser Leu Pro Leu Leu
1               5                   10                  15

Leu Ala Leu Ala Leu Gly Leu Val Ile Leu His Cys Val Val Ala Asp
            20                  25                  30

Gly Asn Ser Thr Arg Ser Pro Glu Thr Asn Gly Leu Leu Cys Gly Asp
        35                  40                  45

Pro Glu Glu Asn Cys Ala Ala Thr Thr Thr Gln Ser Lys Arg Lys Gly
    50                  55                  60

His Phe Ser Arg Cys Pro Lys Gln Tyr Lys His Tyr Cys Ile Lys Gly
65                  70                  75                  80

Arg Cys Arg Phe Val Val Ala Glu Gln Thr Pro Ser Cys Val Cys Asp
                85                  90                  95

Glu Gly Tyr Ile Gly Ala Arg Cys Glu Arg Val Asp Leu Phe Tyr Leu
            100                 105                 110

Arg Gly Asp Arg Gly Gln Ile Leu Val Ile Cys Leu Ile Ala Val Met
        115                 120                 125

Val Val Phe Ile Ile Leu Val Ile Gly Val Cys Thr Cys Cys His Pro
    130                 135                 140

Leu Arg Lys Arg Arg Lys Arg Lys Lys Glu Glu Glu Met Glu Thr
145                 150                 155                 160

Leu Gly Lys Asp Ile Thr Pro Ile Asn Glu Asp Ile Glu Glu Thr Asn
                165                 170                 175
```

```
Ile Ala
```

<210> 10
<211> 252
<212> PRT
<213> Homo sapiens

<400> 10

```
Ile Ala
```

```
Met Arg Ala Pro Leu Leu Pro Pro Ala Pro Val Val Leu Ser Leu Leu
1               5               10              15

Ile Leu Gly Ser Gly His Tyr Ala Ala Gly Leu Asp Leu Asn Asp Thr
                20              25              30

Tyr Ser Gly Lys Arg Glu Pro Phe Ser Gly Asp His Ser Ala Asp Gly
        35              40              45

Phe Glu Val Thr Ser Arg Ser Glu Met Ser Ser Gly Ser Glu Ile Ser
    50              55              60

Pro Val Ser Glu Met Pro Ser Ser Ser Glu Pro Ser Ser Gly Ala Asp
65              70              75              80

Tyr Asp Tyr Ser Glu Glu Tyr Asp Asn Glu Pro Gln Ile Pro Gly Tyr
            85              90              95

Ile Val Asp Asp Ser Val Arg Val Glu Gln Val Val Lys Pro Pro Gln
        100             105             110

Asn Lys Thr Glu Ser Glu Asn Thr Ser Asp Lys Pro Lys Arg Lys Lys
        115             120             125

Lys Gly Gly Lys Asn Gly Lys Asn Arg Arg Asn Arg Lys Lys Lys Asn
    130             135             140

Pro Cys Asn Ala Glu Phe Gln Asn Phe Cys Ile His Gly Glu Cys Lys
145             150             155             160

Tyr Ile Glu His Leu Glu Ala Val Thr Cys Lys Cys Gln Gln Glu Tyr
            165             170             175

Phe Gly Glu Arg Cys Gly Glu Lys Ser Met Lys Thr His Ser Met Ile
        180             185             190

Asp Ser Ser Leu Ser Lys Ile Ala Leu Ala Ala Ile Ala Ala Phe Met
        195             200             205

Ser Ala Val Ile Leu Thr Ala Val Ala Val Ile Thr Val Gln Leu Arg
```

EP 3 133 398 B1

210                          215                          220

Arg Gln Tyr Val Arg Lys Tyr Glu Gly Glu Ala Glu Glu Arg Lys Lys
225                     230                235                     240

Leu Arg Gln Glu Asn Gly Asn Val His Ala Ile Ala
                    245                250

<210> 11
<211> 147
<212> PRT
<213> Homo sapiens

<400> 11

Met Val Met Gly Leu Gly Val Leu Leu Leu Val Phe Val Leu Gly Leu
1                5                    10                     15

Gly Leu Thr Pro Pro Thr Leu Ala Gln Asp Asn Ser Arg Tyr Thr His
                20                    25                     30

Phe Leu Thr Gln His Tyr Asp Ala Lys Pro Gln Gly Arg Asp Asp Arg
               35                    40                     45

Tyr Cys Glu Ser Ile Met Arg Arg Arg Gly Leu Thr Ser Pro Cys Lys
         50                    55                    60

Asp Ile Asn Thr Phe Ile His Gly Asn Lys Arg Ser Ile Lys Ala Ile
65                    70                    75                    80

Cys Glu Asn Lys Asn Gly Asn Pro His Arg Glu Asn Leu Arg Ile Ser
                    85                    90                    95

Lys Ser Ser Phe Gln Val Thr Thr Cys Lys Leu His Gly Gly Ser Pro
                    100                   105                   110

Trp Pro Pro Cys Gln Tyr Arg Ala Thr Ala Gly Phe Arg Asn Val Val
               115                   120                   125

Val Ala Cys Glu Asn Gly Leu Pro Val His Leu Asp Gln Ser Ile Phe
         130                   135                   140

Arg Arg Pro
145

<210> 12
<211> 1172
<212> PRT
<213> Homo sapiens

271

<400> 12

```
Met Val Trp Arg Leu Val Leu Leu Ala Leu Trp Val Trp Pro Ser Thr
1               5               10              15

Gln Ala Gly His Gln Asp Lys Asp Thr Thr Phe Asp Leu Phe Ser Ile
                20              25              30

Ser Asn Ile Asn Arg Lys Thr Ile Gly Ala Lys Gln Phe Arg Gly Pro
            35              40              45

Asp Pro Gly Val Pro Ala Tyr Arg Phe Val Arg Phe Asp Tyr Ile Pro
        50              55              60

Pro Val Asn Ala Asp Asp Leu Ser Lys Ile Thr Lys Ile Met Arg Gln
65              70              75              80

Lys Glu Gly Phe Phe Leu Thr Ala Gln Leu Lys Gln Asp Gly Lys Ser
                85              90              95

Arg Gly Thr Leu Leu Ala Leu Glu Gly Pro Gly Leu Ser Gln Arg Gln
            100             105             110

Phe Glu Ile Val Ser Asn Gly Pro Ala Asp Thr Leu Asp Leu Thr Tyr
            115             120             125

Trp Ile Asp Gly Thr Arg His Val Val Ser Leu Glu Asp Val Gly Leu
    130             135             140

Ala Asp Ser Gln Trp Lys Asn Val Thr Val Gln Val Ala Gly Glu Thr
145             150             155             160

Tyr Ser Leu His Val Gly Cys Asp Leu Ile Asp Ser Phe Ala Leu Asp
                165             170             175

Glu Pro Phe Tyr Glu His Leu Gln Ala Glu Lys Ser Arg Met Tyr Val
            180             185             190

Ala Lys Gly Ser Ala Arg Glu Ser His Phe Arg Gly Leu Leu Gln Asn
        195             200             205

Val His Leu Val Phe Glu Asn Ser Val Glu Asp Ile Leu Ser Lys Lys
    210             215             220

Gly Cys Gln Gln Gly Gln Gly Ala Glu Ile Asn Ala Ile Ser Glu Asn
225             230             235             240

Thr Glu Thr Leu Arg Leu Gly Pro His Val Thr Thr Glu Tyr Val Gly
            245             250             255
```

273

```
Pro Ser Ser Glu Arg Arg Pro Glu Val Cys Glu Arg Ser Cys Glu Glu
        260             265             270

Leu Gly Asn Met Val Gln Glu Leu Ser Gly Leu His Val Leu Val Asn
        275             280             285

Gln Leu Ser Glu Asn Leu Lys Arg Val Ser Asn Asp Asn Gln Phe Leu
        290             295             300

Trp Glu Leu Ile Gly Gly Pro Pro Lys Thr Arg Asn Met Ser Ala Cys
305             310             315             320

Trp Gln Asp Gly Arg Phe Phe Ala Glu Asn Glu Thr Trp Val Val Asp
        325             330             335

Ser Cys Thr Thr Cys Thr Cys Lys Lys Phe Lys Thr Ile Cys His Gln
        340             345             350

Ile Thr Cys Pro Pro Ala Thr Cys Ala Ser Pro Ser Phe Val Glu Gly
        355             360             365

Glu Cys Cys Pro Ser Cys Leu His Ser Val Asp Gly Glu Glu Gly Trp
        370             375             380

Ser Pro Trp Ala Glu Trp Thr Gln Cys Ser Val Thr Cys Gly Ser Gly
385             390             395             400

Thr Gln Gln Arg Gly Arg Ser Cys Asp Val Thr Ser Asn Thr Cys Leu
        405             410             415

Gly Pro Ser Ile Gln Thr Arg Ala Cys Ser Leu Ser Lys Cys Asp Thr
        420             425             430

Arg Ile Arg Gln Asp Gly Gly Trp Ser His Trp Ser Pro Trp Ser Ser
        435             440             445

Cys Ser Val Thr Cys Gly Val Gly Asn Ile Thr Arg Ile Arg Leu Cys
        450             455             460

Asn Ser Pro Val Pro Gln Met Gly Gly Lys Asn Cys Lys Gly Ser Gly
465             470             475             480

Arg Glu Thr Lys Ala Cys Gln Gly Ala Pro Cys Pro Ile Asp Gly Arg
        485             490             495

Trp Ser Pro Trp Ser Pro Trp Ser Ala Cys Thr Val Thr Cys Ala Gly
        500             505             510
```

274

```
Gly Ile Arg Glu Arg Thr Arg Val Cys Asn Ser Pro Glu Pro Gln Tyr
    515                 520                 525

Gly Gly Lys Ala Cys Val Gly Asp Val Gln Glu Arg Gln Met Cys Asn
    530                 535                 540

Lys Arg Ser Cys Pro Val Asp Gly Cys Leu Ser Asn Pro Cys Phe Pro
545                 550                 555                 560

Gly Ala Gln Cys Ser Ser Phe Pro Asp Gly Ser Trp Ser Cys Gly Ser
                565                 570                 575

Cys Pro Val Gly Phe Leu Gly Asn Gly Thr His Cys Glu Asp Leu Asp
                580                 585                 590

Glu Cys Ala Leu Val Pro Asp Ile Cys Phe Ser Thr Ser Lys Val Pro
    595                 600                 605

Arg Cys Val Asn Thr Gln Pro Gly Phe His Cys Leu Pro Cys Pro Pro
    610                 615                 620

Arg Tyr Arg Gly Asn Gln Pro Val Gly Val Gly Leu Glu Ala Ala Lys
625                 630                 635                 640

Thr Glu Lys Gln Val Cys Glu Pro Glu Asn Pro Cys Lys Asp Lys Thr
                645                 650                 655

His Asn Cys His Lys His Ala Glu Cys Ile Tyr Leu Gly His Phe Ser
                660                 665                 670

Asp Pro Met Tyr Lys Cys Glu Cys Gln Thr Gly Tyr Ala Gly Asp Gly
                675                 680                 685

Leu Ile Cys Gly Glu Asp Ser Asp Leu Asp Gly Trp Pro Asn Leu Asn
    690                 695                 700

Leu Val Cys Ala Thr Asn Ala Thr Tyr His Cys Ile Lys Asp Asn Cys
705                 710                 715                 720

Pro His Leu Pro Asn Ser Gly Gln Glu Asp Phe Asp Lys Asp Gly Ile
                725                 730                 735

Gly Asp Ala Cys Asp Asp Asp Asp Asp Asn Asp Gly Val Thr Asp Glu
                740                 745                 750

Lys Asp Asn Cys Gln Leu Leu Phe Asn Pro Arg Gln Ala Asp Tyr Asp
```

```
                755                      760                      765


        Lys Asp Glu Val Gly Asp Arg Cys Asp Asn Cys Pro Tyr Val His Asn
            770                      775                  780


        Pro Ala Gln Ile Asp Thr Asp Asn Asn Gly Glu Gly Asp Ala Cys Ser
        785                      790                  795                800


        Val Asp Ile Asp Gly Asp Asp Val Phe Asn Glu Arg Asp Asn Cys Pro
                         805                  810                      815


        Tyr Val Tyr Asn Thr Asp Gln Arg Asp Thr Asp Gly Asp Gly Val Gly
                     820                  825                      830


        Asp His Cys Asp Asn Cys Pro Leu Val His Asn Pro Asp Gln Thr Asp
                     835                      840                  845


        Val Asp Asn Asp Leu Val Gly Asp Gln Cys Asp Asn Asn Glu Asp Ile
        850                      855                  860


        Asp Asp Asp Gly His Gln Asn Asn Gln Asp Asn Cys Pro Tyr Ile Ser
        865                      870                  875                880


        Asn Ala Asn Gln Ala Asp His Asp Arg Asp Gly Gln Gly Asp Ala Cys
                     885                  890                      895


        Asp Pro Asp Asp Asp Asn Asp Gly Val Pro Asp Asp Arg Asp Asn Cys
                 900                  905                  910


        Arg Leu Val Phe Asn Pro Asp Gln Glu Asp Leu Asp Gly Asp Gly Arg
             915                      920                  925


        Gly Asp Ile Cys Lys Asp Asp Phe Asp Asn Asp Asn Ile Pro Asp Ile
             930                      935                  940


        Asp Asp Val Cys Pro Glu Asn Asn Ala Ile Ser Glu Thr Asp Phe Arg
        945                      950                  955                960


        Asn Phe Gln Met Val Pro Leu Asp Pro Lys Gly Thr Thr Gln Ile Asp
                         965                  970                  975


        Pro Asn Trp Val Ile Arg His Gln Gly Lys Glu Leu Val Gln Thr Ala
                     980                  985                  990


        Asn Ser Asp Pro Gly Ile Ala Val  Gly Phe Asp Glu Phe  Gly Ser Val
                 995                  1000                 1005
```

276

```
Asp Phe Ser Gly Thr Phe Tyr  Val Asn Thr Asp Arg  Asp Asp Asp
    1010            1015            1020


Tyr Ala Gly Phe Val Phe Gly  Tyr Gln Ser Ser Ser  Arg Phe Tyr
    1025            1030            1035


Val Val Met Trp Lys Gln Val  Thr Gln Thr Tyr Trp  Glu Asp Gln
    1040            1045            1050


Pro Thr Arg Ala Tyr Gly Tyr  Ser Gly Val Ser Leu  Lys Val Val
    1055            1060            1065


Asn Ser Thr Thr Gly Thr Gly  Glu His Leu Arg Asn  Ala Leu Trp
    1070            1075            1080


His Thr Gly Asn Thr Pro Gly  Gln Val Arg Thr Leu  Trp His Asp
    1085            1090            1095


Pro Arg Asn Ile Gly Trp Lys  Asp Tyr Thr Ala Tyr  Arg Trp His
    1100            1105            1110


Leu Thr His Arg Pro Lys Thr  Gly Tyr Ile Arg Val  Leu Val His
    1115            1120            1125


Glu Gly Lys Gln Val Met Ala  Asp Ser Gly Pro Ile  Tyr Asp Gln
    1130            1135            1140


Thr Tyr Ala Gly Gly Arg Leu  Gly Leu Phe Val Phe  Ser Gln Glu
    1145            1150            1155


Met Val Tyr Phe Ser Asp Leu  Lys Tyr Glu Cys Arg  Asp Ile
    1160            1165            1170
```

<210> 13
<211> 1754
<212> PRT
<213> Homo sapiens

<400> 13

277

Met Ala Pro Tyr Pro Cys Gly Cys His Ile Leu Leu Leu Leu Phe Cys
1                   5                   10                  15

Cys Leu Ala Ala Ala Arg Ala Asn Leu Leu Asn Leu Asn Trp Leu Trp
            20                  25                  30

Phe Asn Asn Glu Asp Thr Ser His Ala Ala Thr Thr Ile Pro Glu Pro
        35                  40                  45

Gln Gly Pro Leu Pro Val Gln Pro Thr Ala Asp Thr Thr Thr His Val

|  | 50 |  |  | 55 |  |  | 60 |  |
|---|---|---|---|---|---|---|---|---|

Thr Pro Arg Asn Gly Ser Thr Glu Pro Ala Thr Ala Pro Gly Ser Pro
65                 70              75                80

Glu Pro Pro Ser Glu Leu Leu Glu Asp Gly Gln Asp Thr Pro Thr Ser
             85              90               95

Ala Glu Ser Pro Asp Ala Pro Glu Glu Asn Ile Ala Gly Val Gly Ala
          100             105             110

Glu Ile Leu Asn Val Ala Lys Gly Ile Arg Ser Phe Val Gln Leu Trp
          115             120             125

Asn Asp Thr Val Pro Thr Glu Ser Leu Ala Arg Ala Glu Thr Leu Val
          130             135             140

Leu Glu Thr Pro Val Gly Pro Leu Ala Leu Ala Gly Pro Ser Ser Thr
145                 150              155                160

Pro Gln Glu Asn Gly Thr Thr Leu Trp Pro Ser Arg Gly Ile Pro Ser
          165             170             175

Ser Pro Gly Ala His Thr Thr Glu Ala Gly Thr Leu Pro Ala Pro Thr
          180             185             190

Pro Ser Pro Pro Ser Leu Gly Arg Pro Trp Ala Pro Leu Thr Gly Pro
          195             200             205

Ser Val Pro Pro Pro Ser Ser Gly Arg Ala Ser Leu Ser Ser Leu Leu
          210             215             220

Gly Gly Ala Pro Pro Trp Gly Ser Leu Gln Asp Pro Asp Ser Gln Gly
225                 230              235                240

Leu Ser Pro Ala Ala Ala Ala Pro Ser Gln Gln Leu Gln Arg Pro Asp
          245             250             255

Val Arg Leu Arg Thr Pro Leu Leu His Pro Leu Val Met Gly Ser Leu
          260             265             270

Gly Lys His Ala Ala Pro Ser Ala Phe Ser Ser Gly Leu Pro Gly Ala
          275             280             285

Leu Ser Gln Val Ala Val Thr Thr Leu Thr Arg Asp Ser Gly Ala Trp
          290             295             300

```
Val Ser His Val Ala Asn Ser Val Gly Pro Gly Leu Ala Asn Asn Ser
305             310             315                 320

Ala Leu Leu Gly Ala Asp Pro Glu Ala Pro Ala Gly Arg Cys Leu Pro
                325             330                 335

Leu Pro Pro Ser Leu Pro Val Cys Gly His Leu Gly Ile Ser Arg Phe
            340             345                 350

Trp Leu Pro Asn His Leu His His Glu Ser Gly Glu Gln Val Arg Ala
        355             360                 365

Gly Ala Arg Ala Trp Gly Gly Leu Leu Gln Thr His Cys His Pro Phe
    370             375                 380

Leu Ala Trp Phe Phe Cys Leu Leu Leu Val Pro Pro Cys Gly Ser Val
385             390                 395                 400

Pro Pro Pro Ala Pro Pro Pro Cys Cys Gln Phe Cys Glu Ala Leu Gln
            405             410                 415

Asp Ala Cys Trp Ser Arg Leu Gly Gly Gly Arg Leu Pro Val Ala Cys
            420             425                 430

Ala Ser Leu Pro Thr Gln Glu Asp Gly Tyr Cys Val Leu Ile Gly Pro
        435             440                 445

Ala Ala Glu Arg Ile Ser Glu Glu Val Gly Leu Leu Gln Leu Leu Gly
    450             455                 460

Asp Pro Pro Pro Gln Gln Val Thr Gln Thr Asp Asp Pro Asp Val Gly
465             470             475                 480

Leu Ala Tyr Val Phe Gly Pro Asp Ala Asn Ser Gly Gln Val Ala Arg
            485             490                 495

Tyr His Phe Pro Ser Leu Phe Phe Arg Asp Phe Ser Leu Leu Phe His
        500             505                 510

Ile Arg Pro Ala Thr Glu Gly Pro Gly Val Leu Phe Ala Ile Thr Asp
        515             520                 525

Ser Ala Gln Ala Met Val Leu Leu Gly Val Lys Leu Ser Gly Val Gln
    530             535                 540

Asp Gly His Gln Asp Ile Ser Leu Leu Tyr Thr Glu Pro Gly Ala Gly
545             550                 555                 560
```

280

Gln Thr His Thr Ala Ala Ser Phe Arg Leu Pro Ala Phe Val Gly Gln
                    565                 570                 575

Trp Thr His Leu Ala Leu Ser Val Ala Gly Gly Phe Val Ala Leu Tyr
                580                 585                 590

Val Asp Cys Glu Glu Phe Gln Arg Met Pro Leu Ala Arg Ser Ser Arg
            595                 600                 605

Gly Leu Glu Leu Glu Pro Gly Ala Gly Leu Phe Val Ala Gln Ala Gly
        610                 615                 620

Gly Ala Asp Pro Asp Lys Phe Gln Gly Val Ile Ala Glu Leu Lys Val
625                 630                 635                 640

Arg Arg Asp Pro Gln Val Ser Pro Met His Cys Leu Asp Glu Glu Gly
                645                 650                 655

Asp Asp Ser Asp Gly Ala Ser Gly Asp Ser Gly Ser Gly Leu Gly Asp
            660                 665                 670

Ala Arg Glu Leu Leu Arg Glu Glu Thr Gly Ala Ala Leu Lys Pro Arg
        675                 680                 685

Leu Pro Ala Pro Pro Pro Val Thr Thr Pro Pro Leu Ala Gly Gly Ser
    690                 695                 700

Ser Thr Glu Asp Ser Arg Ser Glu Glu Val Glu Glu Gln Thr Thr Val
705                 710                 715                 720

Ala Ser Leu Gly Ala Gln Thr Leu Pro Gly Ser Asp Ser Val Ser Thr
                725                 730                 735

Trp Asp Gly Ser Val Arg Thr Pro Gly Gly Arg Val Lys Glu Gly Gly
            740                 745                 750

Leu Lys Gly Gln Lys Gly Glu Pro Gly Val Pro Gly Pro Pro Gly Arg
        755                 760                 765

Ala Gly Pro Pro Gly Ser Pro Cys Leu Pro Gly Pro Pro Gly Leu Pro
    770                 775                 780

Cys Pro Val Ser Pro Leu Gly Pro Ala Gly Pro Ala Leu Gln Thr Val
785                 790                 795                 800

Pro Gly Pro Gln Gly Pro Pro Gly Pro Gly Arg Asp Gly Thr Pro
            805                 810                 815

281

Gly Arg Asp Gly Glu Pro Gly Asp Pro Gly Glu Asp Gly Lys Pro Gly
            820                 825                 830

Asp Thr Gly Pro Gln Gly Phe Pro Gly Thr Pro Gly Asp Val Gly Pro
            835                 840                 845

Lys Gly Asp Lys Gly Asp Pro Gly Val Gly Glu Arg Gly Pro Pro Gly
            850                 855                 860

Pro Gln Gly Pro Pro Gly Pro Pro Gly Pro Ser Phe Arg His Asp Lys
865                 870                 875                 880

Leu Thr Phe Ile Asp Met Glu Gly Ser Gly Phe Gly Gly Asp Leu Glu
                885                 890                 895

Ala Leu Arg Gly Pro Arg Gly Phe Pro Gly Pro Pro Gly Pro Pro Gly
            900                 905                 910

Val Pro Gly Leu Pro Gly Glu Pro Gly Arg Phe Gly Val Asn Ser Ser
            915                 920                 925

Asp Val Pro Gly Pro Ala Gly Leu Pro Gly Val Pro Gly Arg Glu Gly
            930                 935                 940

Pro Pro Gly Phe Pro Gly Leu Pro Gly Pro Pro Gly Pro Pro Gly Arg
945                 950                 955                 960

Glu Gly Pro Pro Gly Arg Thr Gly Gln Lys Gly Ser Leu Gly Glu Ala
                965                 970                 975

Gly Ala Pro Gly His Lys Gly Ser Lys Gly Ala Pro Gly Pro Ala Gly
            980                 985                 990

Ala Arg Gly Glu Ser Gly Leu Ala Gly Ala Pro Gly Pro Ala Gly Pro
            995                 1000                1005

Pro Gly Pro Pro Gly Pro Pro Gly Pro Pro Gly Pro Gly Leu Pro
    1010                1015                1020

Ala Gly Phe Asp Asp Met Glu Gly Ser Gly Gly Pro Phe Trp Ser
    1025                1030                1035

Thr Ala Arg Ser Ala Asp Gly Pro Gln Gly Pro Pro Gly Leu Pro
    1040                1045                1050

Gly Leu Lys Gly Asp Pro Gly Val Pro Gly Leu Pro Gly Ala Lys

| | 1055 | | | | | 1060 | | | | | 1065 | | |
|---|---|---|---|---|---|---|---|---|---|---|---|---|---|

Gly Glu Val Gly Ala Asp Gly Val Pro Gly Phe Pro Gly Leu Pro
        1070            1075            1080

Gly Arg Glu Gly Ile Ala Gly Pro Gln Gly Pro Lys Gly Asp Arg
        1085            1090            1095

Gly Ser Arg Gly Glu Lys Gly Asp Pro Gly Lys Asp Gly Val Gly
        1100            1105            1110

Gln Pro Gly Leu Pro Gly Pro Pro Gly Pro Pro Gly Pro Val Val
        1115            1120            1125

Tyr Val Ser Glu Gln Asp Gly Ser Val Leu Ser Val Pro Gly Pro
        1130            1135            1140

Glu Gly Arg Pro Gly Phe Ala Gly Phe Pro Gly Pro Ala Gly Pro
        1145            1150            1155

Lys Gly Asn Leu Gly Ser Lys Gly Glu Arg Gly Ser Pro Gly Pro
        1160            1165            1170

Lys Gly Glu Lys Gly Glu Pro Gly Ser Ile Phe Ser Pro Asp Gly
        1175            1180            1185

Gly Ala Leu Gly Pro Ala Gln Lys Gly Ala Lys Gly Glu Pro Gly
        1190            1195            1200

Phe Arg Gly Pro Pro Gly Pro Tyr Gly Arg Pro Gly Tyr Lys Gly
        1205            1210            1215

Glu Ile Gly Phe Pro Gly Arg Pro Gly Arg Pro Gly Met Asn Gly
        1220            1225            1230

Leu Lys Gly Glu Lys Gly Glu Pro Gly Asp Ala Ser Leu Gly Phe
        1235            1240            1245

Gly Met Arg Gly Met Pro Gly Pro Pro Gly Pro Pro Gly Pro Pro
        1250            1255            1260

Gly Pro Pro Gly Thr Pro Val Tyr Asp Ser Asn Val Phe Ala Glu
        1265            1270            1275

Ser Ser Arg Pro Gly Pro Pro Gly Leu Pro Gly Asn Gln Gly Pro
        1280            1285            1290

```
Pro Gly Pro Lys Gly Ala Lys  Gly Glu Val Gly Pro  Pro Gly Pro
    1295            1300                1305

Pro Gly Gln Phe Pro Phe Asp  Phe Leu Gln Leu Glu  Ala Glu Met
    1310            1315                1320

Lys Gly Glu Lys Gly Asp Arg  Gly Asp Ala Gly Gln  Lys Gly Glu
    1325            1330                1335

Arg Gly Glu Pro Gly Gly Gly  Gly Phe Phe Gly Ser  Ser Leu Pro
    1340            1345                1350

Gly Pro Pro Gly Pro Pro Gly  Pro Pro Gly Pro Arg  Gly Tyr Pro
    1355            1360                1365

Gly Ile Pro Gly Pro Lys Gly  Glu Ser Ile Arg Gly  Gln Pro Gly
    1370            1375                1380

Pro Pro Gly Pro Gln Gly Pro  Pro Gly Ile Gly Tyr  Glu Gly Arg
    1385            1390                1395

Gln Gly Pro Pro Gly Pro Pro  Gly Pro Pro Gly Pro  Pro Ser Phe
    1400            1405                1410

Pro Gly Pro His Arg Gln Thr  Ile Ser Val Pro Gly  Pro Pro Gly
    1415            1420                1425

Pro Pro Gly Pro Pro Gly Pro  Pro Gly Thr Met Gly  Ala Ser Ser
    1430            1435                1440

Gly Val Arg Leu Trp Ala Thr  Arg Gln Ala Met Leu  Gly Gln Val
    1445            1450                1455

His Glu Val Pro Glu Gly Trp  Leu Ile Phe Val Ala  Glu Gln Glu
    1460            1465                1470

Glu Leu Tyr Val Arg Val Gln  Asn Gly Phe Arg Lys  Val Gln Leu
    1475            1480                1485

Glu Ala Arg Thr Pro Leu Pro  Arg Gly Thr Asp Asn  Glu Val Ala
    1490            1495                1500

Ala Leu Gln Pro Pro Val Val  Gln Leu His Asp Ser  Asn Pro Tyr
    1505            1510                1515

Pro Arg Arg Glu His Pro His  Pro Thr Ala Arg Pro  Trp Arg Ala
    1520            1525                1530
```

284

```
        Asp Asp Ile Leu Ala Ser Pro  Pro Arg Leu Pro Glu  Pro Gln Pro
            1535                1540                1545

        Tyr Pro Gly Ala Pro His His  Ser Ser Tyr Val His  Leu Arg Pro
            1550                1555                1560

        Ala Arg Pro Thr Ser Pro Pro  Ala His Ser His Arg  Asp Phe Gln
            1565                1570                1575

        Pro Val Leu His Leu Val Ala  Leu Asn Ser Pro Leu  Ser Gly Gly
            1580                1585                1590

        Met Arg Gly Ile Arg Gly Ala  Asp Phe Gln Cys Phe  Gln Gln Ala
            1595                1600                1605

        Arg Ala Val Gly Leu Ala Gly  Thr Phe Arg Ala Phe  Leu Ser Ser
            1610                1615                1620

        Arg Leu Gln Asp Leu Tyr Ser  Ile Val Arg Arg Ala  Asp Arg Ala
            1625                1630                1635

        Ala Val Pro Ile Val Asn Leu  Lys Asp Glu Leu Leu  Phe Pro Ser
            1640                1645                1650

        Trp Glu Ala Leu Phe Ser Gly  Ser Glu Gly Pro Leu  Lys Pro Gly
            1655                1660                1665

        Ala Arg Ile Phe Ser Phe Asp  Gly Lys Asp Val Leu  Arg His Pro
            1670                1675                1680

        Thr Trp Pro Gln Lys Ser Val  Trp His Gly Ser Asp  Pro Asn Gly
            1685                1690                1695

        Arg Arg Leu Thr Glu Ser Tyr  Cys Glu Thr Trp Arg  Thr Glu Ala
            1700                1705                1710

        Pro Ser Ala Thr Gly Gln Ala  Ser Ser Leu Leu Gly  Gly Arg Leu
            1715                1720                1725

        Leu Gly Gln Ser Ala Ala Ser  Cys His His Ala Tyr  Ile Val Leu
            1730                1735                1740

        Cys Ile Glu Asn Ser Phe Met  Thr Ala Ser Lys
            1745                1750
```

<210> 14
<211> 35

<212> PRT
<213> Homo sapiens

<400> 14

```
Gln Asp Ala Cys Trp Ser Arg Leu Gly Gly Gly Arg Leu Pro Val Ala
1               5               10              15

Cys Ala Ser Leu Pro Thr Gln Glu Asp Gly Tyr Cys Val Leu Ile Gly
            20              25              30

Pro Ala Ala
        35
```

<210> 15
<211> 35
<212> PRT
<213> Homo sapiens

<400> 15

```
Met Ala Pro Arg Cys Pro Trp Pro Trp Pro Arg Arg Arg Arg Leu Leu
1               5               10              15

Asp Val Leu Ala Pro Leu Val Leu Leu Leu Gly Val Arg Ala Ala Ser
            20              25              30

Ala Glu Pro
        35
```

<210> 16
<211> 4
<212> PRT
<213> Homo sapiens

<400> 16

```
Ile Gln Leu Gln
1
```

<210> 17
<211> 4
<212> PRT
<213> Homo sapiens

<400> 17

```
Thr Asp Thr Lys
1
```

**Description**

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830.

Renal disease and/or injury may be acute or chronic. Acute and chronic kidney disease are described as follows (from Current Medical Diagnosis & Treatment 2008, 47th Ed, McGraw Hill, New York, pages 785-815):

"Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222 :

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; |
| **Type** | **Risk Factors** |
| | Immune-complex: Lupus glomerulonephritis, postinfectious glomerulonephritis, cryoglobulinemic glomerulonephritis |

(continued)

| Type | Risk Factors |
| --- | --- |
| Acute tubulointerstitial nephritis | Drug reaction (eg, β-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| **Postrenal** | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370,2005.

As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004,

proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, the RIFLE criteria provide a uniform definition of AKI which has been validated in numerous studies.
More recently, Mehta et al., Crit. Care 11:R31 (doi:10.1186.cc5713), 2007, proposes the following similar classifications for stratifying AKI patients, which have been modified from RIFLE:

"Stage I": increase in serum creatinine of more than or equal to 0.3 mg/dL ($\geq$26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006;7(4):177-197) uses a serum creatinine rise of 25% to define Contrast induced nephropathy (which is a type of AKI). Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.
Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

**BRIEF SUMMARY OF THE DISCLOSURE, INCLUDING THE INVENTION**

It is an object of the invention to provide methods and compositions for evaluating renal function in a subject. As described herein, measurement of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain (each referred to herein as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury). Based on the disclosure herein, the invention defined in the appended claims is provided.

The kidney injury markers of the present disclosure may be used, individually or in panels comprising a plurality of kidney injury markers, for risk stratification (that is, to identify subjects at risk for a future injury to renal function, for future progression to reduced renal function, for future progression to ARF, for future improvement in renal function, *etc.*); for diagnosis of existing disease (that is, to identify subjects who have suffered an injury to renal function, who have progressed to reduced renal function, who have progressed to ARF, *etc.*); for monitoring for deterioration or improvement of renal function; and for predicting a future medical outcome, such as improved or worsening renal function, a decreased or increased mortality risk, a decreased or increased risk that a subject will require renal replacement therapy (*i.e.,* hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present disclosure relates to methods for evaluating renal status in a subject. These methods comprise performing an assay method that is configured to detect one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are then correlated to the renal status of the subject. This correlation to renal status may include correlating the assay result(s) to one or more of risk stratification, diagnosis, prognosis, staging, classifying and monitoring of the subject as described herein. Thus, the present disclosure utilizes one or more kidney injury markers of the present disclosure for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In some instances, the assay result(s), for example measured concentration(s) of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In some instances, the assay result(s), for example measured concentration(s) of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In some instances, the assay result(s), for example measured concentration(s) of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, etc.), by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present disclosure must be compared to corresponding individual thresholds. Methods for combining assay results can comprise the use of multivariate logistical regression, loglinear modeling, neural network analysis, n-of-m analysis, decision tree analysis, calculating ratios of markers, etc. This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma. In the case of those kidney injury markers which are membrane proteins as described hereinafter, preferred assays detect soluble forms thereof.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score, risk scores of Thakar et al. (J. Am. Soc. Nephrol. 16: 162-68, 2005), Mehran et al. (J. Am. Coll. Cardiol. 44: 1393-99, 2004), Wijeysundera et al. (JAMA 297: 1801-9, 2007), Goldstein and Chawla (Clin. J. Am. Soc. Nephrol. 5: 943-49, 2010), or Chawla et al. (Kidney Intl. 68: 2274-80, 2005)), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present disclosure also relates to devices and kits for performing the methods described herein. Suitable kits comprise reagents sufficient for performing an assay for at least one of the described kidney injury markers, together with instructions for performing the described threshold comparisons.

In certain instances, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, etc.) as well as molecules that may be indirectly detected by production of a detectable reaction product (e.g., enzymes such as horseradish peroxidase, alkaline phosphatase, etc.) or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, etc. In certain of these methods, the solid phase antibody is coupled to a transducer (e.g., a diffraction grating, electrochemical sensor, etc) for generation of a signal, while in others, a signal is generated by a transducer that is spatially separate from the solid phase antibody (e.g., a fluorometer that employs an excitation light source and an optical detector). This list is not meant to be limiting. Antibody-based biosensors may also be employed to determine the presence or amount of analytes that optionally eliminate the need for a labeled molecule.

## DETAILED DESCRIPTION OF THE DISCLOSURE, INCLUDING THE INVENTION

The present disclosure relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various instances, a measured concentration of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain or one or more markers related thereto, are correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq 8.8$ $\mu$mol/L), a percentage increase in serum creatinine of greater than or equal to 20% (1.2-fold from baseline), or a reduction in urine output (documented oliguria of less than 0. 5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq 26.4$ $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "Proheparin-binding EGF-like growth factor" refers to one or more polypeptides present in a biological sample that are derived from the Proheparin-binding EGF-like growth factor precursor (Swiss-Prot Q99075 (SEQ ID NO: 1)):

```
          10         20         30         40         50         60
    MKLLPSVVLK LFLAAVLSAL VTGESLERLR RGLAAGTSNP DPPTVSTDQL LPLGGGRDRK

          70         80         90        100        110        120
    VRDLQEADLD LLRVTLSSKP QALATPNKEE HGKRKKKGKG LGKKRDPCLR KYKDFCIHGE

         130        140        150        160        170        180
    CKYVKELRAP SCICHPGYHG ERCHGLSLPV ENRLYTYDHT TILAVVAVVL SSVCLLVIVG

         190        200
    LLMFRYHRRG GYDVENEEKV KLGMTNSH
```

Most preferably, the Proheparin-binding EGF-like growth factor assay detects one or more soluble forms of Proheparin-binding EGF-like growth factor. Proheparin-binding EGF-like growth factor is a type I membrane protein having a large extracellular domain, most or all of which is present in soluble forms of Proheparin-binding EGF-like growth factor generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Proheparin-binding EGF-like growth factor:

| Residues | Length | Domain ID |
|---|---|---|
| 1-19 | 19 | Signal peptide |
| 20-208 | 189 | Proheparin-binding EGF-like growth factor |
| 63-148 | 86 | Heparin-binding EGF-like growth factor |
| 20-62 | 43 | Propeptide (alternative endings at 72, 73, 76, and 81) |
| 149-208 | 60 | Propeptide |
| 20-160 | 141 | Extracellular |
| 161-184 | 24 | Transmembrane |
| 185-208 | 24 | Cytoplasmic |

As used herein, the term "Tenascin" refers to one or more polypeptides present in a biological sample that are derived from the Tenascin precursor (Swiss-Prot P24821 (SEQ ID NO: 2))

```
            10         20         30         40         50         60
     MGAMTQLLAG VFLAFLALAT EGGVLKKVIR HKRQSGVNAT LPEENQPVVF NHVYNIKLPV

            70         80         90        100        110        120
     GSQCSVDLES ASGEKDLAPP SEPSESFQEH TVDGENQIVF THRINIPRRA CGCAAAPDVK

           130        140        150        160        170        180
     ELLSRLEELE NLVSSLREQC TAGAGCCLQP ATGRLDTRPF CSGRGNFSTE GCGCVCEPGW

           190        200        210        220        230        240
     KGPNCSEPEC PGNCHLRGRC IDGQCICDDG FTGEDCSQLA CPSDCNDQGK CVNGVCICFE

           250        260        270        280        290        300
     GYAGADCSRE ICPVPCSEEH GTCVDGLCVC HDGFAGDDCN KPLCLNNCYN RGRCVENECV

           310        320        330        340        350        360
     CDEGFTGEDC SELICPNDCF DRGRCINGTC YCEEGFTGED CGKPTCPHAC HTQGRCEEGQ

           370        380        390        400        410        420
     CVCDEGFAGV DCSEKRCPAD CHNRGRCVDG RCECDDGFTG ADCGELKCPN GCSGHGRCVN

           430        440        450        460        470        480
     GQCVCDEGYT GEDCSQLRCP NDCHSRGRCV EGKCVCEQGF KGYDCSDMSC PNDCHQHGRC

           490        500        510        520        530        540
```

VNGMCVCDDG YTGEDCRDRQ CPRDCSNRGL CVDGQCVCED GFTGPDCAEL SCPNDCHGQG

RCVNGQCVCH EGFMGKDCKE QRCPSDCHGQ GRCVDGQCIC HEGFTGLDCG QHSCPSDCNN

LGQCVSGRCI CNEGYSGEDC SEVSPPKDLV VTEVTEETVN LAWDNEMRVT EYLVVYTPTH

EGGLEMQFRV PGDQTSTIIQ ELEPGVEYFI RVFAILENKK SIPVSARVAT YLPAPEGLKF

KSIKETSVEV EWDPLDIAFE TWEIIFRNMN KEDEGEITKS LRRPETSYRQ TGLAPGQEYE

ISLHIVKNNT RGPGLKRVTT TRLDAPSQIE VKDVTDTTAL ITWFKPLAEI DGIELTYGIK

DVPGDRTTID LTEDENQYSI GNLKPDTEYE VSLISRRGDM SSNPAKETFT TGLDAPRNLR

RVSQTDNSIT LEWRNGKAAI DSYRIKYAPI SGGDHAEVDV PKSQQATTKT TLTGLRPGTE

YGIGVSAVKE DKESNPATIN AATELDTPKD LQVSETAETS LTLLWKTPLA KFDRYRLNYS

LPTGQWVGVQ LPRNTTSYVL RGLEPGQEYN VLLTAEKGRH KSKPARVKAS TEQAPELENL

TVTEVGWDGL RLNWTAADQA YEHFIIQVQE ANKVEAARNL TVPGSLRAVD IPGLKAATPY

TVSIYGVIQG YRTPVLSAEA STGETPNLGE VVVAEVGWDA LKLNWTAPEG AYEYFFIQVQ

EADTVEAAQN LTVPGGLRST DLPGLKAATH YTITIRGVTQ DFSTTPLSVE VLTEEVPDMG

NLTVTEVSWD ALRLNWTTPD GTYDQFTIQV QEADQVEEAH NLTVPGSLRS MEIPGLRAGT

PYTVTLHGEV RGHSTRPLAV EVVTEDLPQL GDLAVSEVGW DGLRLNWTAA DNAYEHFVIQ

VQEVNKVEAA QNLTLPGSLR AVDIPGLEAA TPYRVSIYGV IRGYRTPVLS AEASTAKEPE

IGNLNVSDIT PESFNLSWMA TDGIFETFTI EIIDSNRLLE TVEYNISGAE RTAHISGLPP

STDFIVYLSG LAPSIRTKTI SATATTEALP LLENLTISDI NPYGFTVSWM ASENAFDSFL

VTVVDSGKLL DPQEFTLSGT QRKLELRGLI TGIGYEVMVS GFTQGHQTKP LRAEIVTEAE

```
      1630       1640       1650       1660       1670       1680
 PEVDNLLVSD ATPDGFRLSW TADEGVFDNF VLKIRDTKKQ SEPLEITLLA PERTRDITGL

      1690       1700       1710       1720       1730       1740
 REATEYEIEL YGISKGRRSQ TVSAIATTAM GSPKEVIFSD ITENSATVSW RAPTAQVESF

      1750       1760       1770       1780       1790       1800
 RITYVPITGG TPSMVTVDGT KTQTRLVKLI PGVEYLVSII AMKGFEESEP VSGSFTTALD

      1810       1820       1830       1840       1850       1860
 GPSGLVTANI TDSEALARWQ PAIATVDSYV ISYTGEKVPE ITRTVSGNTV EYALTDLEPA

      1870       1880       1890       1900       1910       1920
 TEYTLRIFAE KGPQKSSTIT AKFTTDLDSP RDLTATEVQS ETALLTWRPP RASVTGYLLV

      1930       1940       1950       1960       1970       1980
 YESVDGTVKE VIVGPDTTSY SLADLSPSTH YTAKIQALNG PLRSNMIQTI FTTIGLLYPF

      1990       2000       2010       2020       2030       2040
 PKDCSQAMLN GDTTSGLYTI YLNGDKAEAL EVFCDMTSDG GGWIVFLRRK NGRENFYQNW

      2050       2060       2070       2080       2090       2100
 KAYAAGFGDR REEFWLGLDN LNKITAQGQY ELRVDLRDHG ETAFAVYDKF SVGDAKTRYK

      2110       2120       2130       2140       2150       2160
 LKVEGYSGTA GDSMAYHNGR SFSTFDKDTD SAITNCALSY KGAFWYRNCH RVNLMGRYGD

      2170       2180       2190       2200
 NNHSQGVNWF HWKGHEHSIQ FAEMKLRPSN FRNLEGRRKR A
```

The following domains have been identified in Tenascin:

| Residues | Length | Domain ID |
| --- | --- | --- |
| 1-22 | 22 | Signal peptide |
| 23-2201 | 2179 | Tenascin |
| 1072-1435 | | Missing in isoform 2 |
| 1527-1617 | | Missing in isoform 2 |
| 1072-1435 | | Missing in isoform 3 |
| 1527-1617 | | Missing in isoform 4 |
| 1072-1617 | | Missing in isoform 5 |
| 1072-1708 | | Missing in isoform 6 |

As used herein, the term "Angiopoietin-related protein 4" refers to one or more polypeptides present in a biological sample that are derived from the Angiopoietin-related protein 4 precursor (Swiss-Prot Q9BY76 (SEQ ID NO: 3))

```
        10         20         30         40         50         60
MSGAPTAGAA LMLCAATAVL LSAQGGPVQS KSPRFASWDE MNVLAHGLLQ LGQGLREHAE

        70         80         90        100        110        120
RTRSQLSALE RRLSACGSAC QGTEGSTDLP LAPESRVDPE VLHSLQTQLK AQNSRIQQLF

       130        140        150        160        170        180
HKVAQQQRHL EKQHLRIQHL QSQFGLLDHK HLDHEVAKPA RRKRLPEMAQ PVDPAHNVSR

       190        200        210        220        230        240
LHRLPRDCQE LFQVGERQSG LFEIQPQGSP PFLVNCKMTS DGGWTVIQRR HDGSVDFNRP

       250        260        270        280        290        300
WEAYKAGFGD PHGEFWLGLE KVHSITGDRN SRLAVQLRDW DGNAELLQFS VHLGGEDTAY

       310        320        330        340        350        360
SLQLTAPVAG QLGATTVPPS GLSVPFSTWD QDHDLRRDKN CAKSLSGGWW FGTCSHSNLN

       370        380        390        400
GQYFRSIPQQ RQKLKKGIFW KTWRGRYYPL QATTMLIQPM AAEAAS
```

The following domains have been identified in Angiopoietin-related protein 4:

| Residues | Length | Domain ID |
|----------|--------|-----------|
| 1-25 | 25 | Signal peptide |
| 26-406 | 381 | Angiopoietin-related protein 4 |

As used herein, the term "Fibroblast growth factor 19" refers to one or more polypeptides present in a biological sample that are derived from the Fibroblast growth factor 19 precursor (Swiss-Prot O95750 (SEQ ID NO: 4))

```
        10         20         30         40         50         60
MRSGCVVVHV WILAGLWLAV AGRPLAFSDA GPHVHYGWGD PIRLRHLYTS GPHGLSSCFL

        70         80         90        100        110        120
RIRADGVVDC ARGQSAHSLL EIKAVALRTV AIKGVHSVRY LCMGADGKMQ GLLQYSEEDC

       130        140        150        160        170        180
AFEEEIRPDG YNVYRSEKHR LPVSLSSAKQ RQLYKNRGFL PLSHFLPMLP MVPEEPEDLR

       190        200        210
GHLESDMFSS PLETDSMDPF GLVTGLEAVR SPSFEK
```

The following domains have been identified in Fibroblast growth factor 19:

| Residues | Length | Domain ID |
|----------|--------|-----------|
| 1-24 | 24 | Signal peptide |
| 25-216 | 192 | Fibroblast growth factor 19 |

As used herein, the term "Fibroblast growth factor 21" refers to one or more polypeptides present in a biological sample that are derived from the Fibroblast growth factor 21 precursor (Swiss-Prot Q9NSA1 (SEQ ID NO: 5))

```
         10          20          30          40          50          60
MDSDETGFEH  SGLWVSVLAG  LLLGACQAHP  IPDSSPLLQF  GGQVRQRYLY  TDDAQQTEAH

         70          80          90         100         110         120
LEIREDGTVG  GAADQSPESL  LQLKALKPGV  IQILGVKTSR  FLCQRPDGAL  YGSLHFDPEA

        130         140         150         160         170         180
CSFRELLLED  GYNVYQSEAH  GLPLHLPGNK  SPHRDPAPRG  PARFLPLPGL  PPALPEPPGI

        190         200
LAPQPPDVGS  SDPLSMVGPS  QGRSPSYAS
```

The following domains have been identified in Fibroblast growth factor 21:

| Residues | Length | Domain ID |
|----------|--------|-----------|
| 1-28 | 28 | Signal peptide |
| 29-209 | 181 | Fibroblast growth factor 21 |

As used herein, the term "Heparin-binding growth factor 1" refers to one or more polypeptides present in a biological sample that are derived from the Heparin-binding growth factor 1 precursor (Swiss-Prot P05230 (SEQ ID NO: 6))

```
         10          20          30          40          50          60
MAEGEITTFT  ALTEKFNLPP  GNYKKPKLLY  CSNGGHFLRI  LPDGTVDGTR  DRSDQHIQLQ

         70          80          90         100         110         120
LSAESVGEVY  IKSTETGQYL  AMDTDGLLYG  SQTPNEECLF  LERLEENHYN  TYISKKHAEK

        130         140         150
NWFVGLKKNG  SCKRGPRTHY  GQKAILFLPL  PVSSD
```

The following domains have been identified in Heparin-binding growth factor 1:

| Residues | Length | Domain ID |
|----------|--------|-----------|
| 1-15 | 15 | Signal peptide |
| 16-155 | 140 | Heparin-binding growth factor 1 |
| 57-60 | | IQLQ → TDTK in isoform 2 |
| 61-155 | | Missing in isoform 2 |

As used herein, the term "Angiopoietin-related protein 6" refers to one or more polypeptides present in a biological sample that are derived from the Angiopoietin-related protein 6 precursor (Swiss-Prot Q8NI99 (SEQ ID NO: 7))

```
           10         20         30         40         50         60
      MGKPWLRALQ LLLLLGASWA RAGAPRCTYT FVLPPQKFTG AVCWSGPAST RATPEAANAS

           70         80         90        100        110        120
      ELAALRMRVG RHEELLRELQ RLAAADGAVA GEVRALRKES RGLSARLGQL RAQLQHEAGP

          130        140        150        160        170        180
      GAGPGADLGA EPAAALALLG ERVLNASAEA QRAAARFHQL DVKFRELAQL VTQQSSLIAR

          190        200        210        220        230        240
      LERLCPGGAG GQQQVLPPPP LVPVVPVRLV GSTSDTSRML DPAPEPQRDQ TQRQQEPMAS

          250        260        270        280        290        300
      PMPAGHPAVP TKPVGPWQDC AEARQAGHEQ SGVYELRVGR HVVSVWCEQQ LEGGGWTVIQ

          310        320        330        340        350        360
      RRQDGSVNFF TTWQHYKAGF GRPDGEYWLG LEPVYQLTSR GDHELLVLLE DWGGRGARAH

          370        380        390        400        410        420
      YDGFSLEPES DHYRLRLGQY HGDAGDSLSW HNDKPFSTVD RDRDSYSGNC ALYQRGGWWY

          430        440        450        460        470
      HACAHSNLNG VWHHGGHYRS RYQDGVYWAE FRGGAYSLRK AAMLIRPLKL
```

The following domains have been identified in Angiopoietin-related protein 6:

| Residues | Length | Domain ID |
|----------|--------|-----------|
| 1-20 | 20 | Signal peptide |
| 21-470 | 450 | Angiopoietin-related protein 6 |

As used herein, the term "Proepiregulin" refers to one or more polypeptides present in a biological sample that are derived from the Proepiregulin precursor (Swiss-Prot 014944 (SEQ ID NO: 8)):

```
           10         20         30         40         50         60
      MTAGRRMEML CAGRVPALLL CLGFHLLQAV LSTTVIPSCI PGESSDNCTA LVQTEDNPRV

           70         80         90        100        110        120
      AQVSITKCSS DMNGYCLHGQ CIYLVDMSQN YCRCEVGYTG VRCEHFFLTV HQPLSKEYVA

                   130        140        150        160
           LTVILIILFL ITVVGSTYYF CRWYRNRKSK EPKKEYERVT SGDPELPQV
```

Most preferably, the Proepiregulin assay detects one or more soluble forms of Proepiregulin. Proepiregulin is a type I membrane protein having a large extracellular domain, most or all of which is present in soluble forms of Proepiregulin generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). Preferred assays detect Proepiregulin. The following domains have been identified in Proepiregulin:

| Residues | Length | Domain ID |
|----------|--------|-----------|
| 1-29 | 29 | Signal peptide |
| 30-169 | 140 | Proepiregulin |
| 60-108 | 49 | Proepiregulin |

(continued)

| Residues | Length | Domain ID |
|---|---|---|
| 109-169 | 61 | Propeptide |
| 60-119 | 60 | Extracellular |
| 120-140 | 21 | Transmembrane |
| 141-169 | 61 | Cytoplasmic |

As used herein, the term "Probetacellulin" refers to one or more polypeptides present in a biological sample that are derived from the Probetacellulin precursor (Swiss-Prot P35070 (SEQ ID NO: 9)):

```
          10         20         30         40         50         60
   MDRAARCSGA SSLPLLLALA LGLVILHCVV ADGNSTRSPE TNGLLCGDPE ENCAATTTQS

          70         80         90        100        110        120
   KRKGHFSRCP KQYKHYCIKG RCRFVVAEQT PSCVCDEGYI GARCERVDLF YLRGDRGQIL

         130        140        150        160        170
   VICLIAVMVV FIILVIGVCT CCHPLRKRRK RKKKEEEMET LGKDITPINE DIEETNIA
```

Most preferably, the Probetacellulin assay detects one or more soluble forms of Probetacellulin. Probetacellulin is a type I membrane protein having a large extracellular domain, most or all of which is present in soluble forms of Probetacellulin generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Probetacellulin:

| Residues | Length | Domain ID |
|---|---|---|
| 1-31 | 31 | Signal peptide |
| 32-178 | 147 | Probetacellulin |
| 32-111 | 80 | Betacellulin |
| 112-178 | 67 | Propeptide |
| 32-118 | 87 | Extracellular |
| 119-139 | 21 | Transmembrane |
| 140-178 | 39 | Cytoplasmic |

As used herein, the term "Amphiregulin" refers to one or more polypeptides present in a biological sample that are derived from the Amphiregulin precursor (Swiss-Prot P15514 (SEQ ID NO: 10)):

```
          10         20         30         40         50         60
   MRAPLLPPAP VVLSLLILGS GHYAAGLDLN DTYSGKREPF SGDHSADGFE VTSRSEMSSG

          70         80         90        100        110        120
   SEISPVSEMP SSSEPSSGAD YDYSEEYDNE PQIPGYIVDD SVRVEQVVKP PQNKTESENT

         130        140        150        160        170        180
   SDKPKRKKKG GKNGKNRRNR KKKNPCNAEF QNFCIHGECK YIEHLEAVTC KCQQEYFGER

         190        200        210        220        230        240
   CGEKSMKTHS MIDSSLSKIA LAAIAAFMSA VILTAVAVIT VQLRRQYVRK YEGEAEERKK

         250
   LRQENGNVHA IA
```

The following domains have been identified in Amphiregulin:

| Residues | Length | Domain ID |
|---|---|---|
| 1-19 | 19 | Signal peptide |
| 20-100 | 81 | Propeptide |
| 101-184 | 84 | Amphiregulin |
| 185-252 | 68 | Propeptide |
| 199-221 | 23 | Transmembrane |

As used herein, the term "Angiogenin" refers to one or more polypeptides present in a biological sample that are derived from the Angiogenin precursor (Swiss-Prot P03950 (SEQ ID NO: 11))

```
           10         20         30         40         50         60
    MVMGLGVLLL VFVLGLGLTP PTLAQDNSRY THFLTQHYDA KPQGRDDRYC ESIMRRRGLT

           70         80         90        100        110        120
    SPCKDINTFI HGNKRSIKAI CENKNGNPHR ENLRISKSSF QVTTCKLHGG SPWPPCQYRA

          130        140
    TAGFRNVVVA CENGLPVHLD QSIFRRP
```

The following domains have been identified in Angiogenin:

| Residues | Length | Domain ID |
|---|---|---|
| 1-24 | 24 | Signal peptide |
| 25-147 | 123 | Angiogenin |

As used herein, the term "Thrombospondin-2" refers to one or more polypeptides present in a biological sample that are derived from the Thrombospondin-2 precursor (Swiss-Prot P35442 (SEQ ID NO: 12))

```
           10         20         30         40         50         60
    MVWRLVLLAL WVWPSTQAGH QDKDTTFDLF SISNINRKTI GAKQFRGPDP GVPAYRFVRF

           70         80         90        100        110        120
    DYIPPVNADD LSKITKIMRQ KEGFFLTAQL KQDGKSRGTL LALEGPGLSQ RQFEIVSNGP

          130        140        150        160        170        180
    ADTLDLTYWI DGTRHVVSLE DVGLADSQWK NVTVQVAGET YSLHVGCDLI DSFALDEPFY

          190        200        210        220        230        240
    EHLQAEKSRM YVAKGSARES HFRGLLQNVH LVFENSVEDI LSKKGCQQGQ GAEINAISEN

          250        260        270        280        290        300
    TETLRLGPHV TTEYVGPSSE RRPEVCERSC EELGNMVQEL SGLHVLVNQL SENLKRVSND

          310        320        330        340        350        360
    NQFLWELIGG PPKTRNMSAC WQDGRFFAEN ETWVVDSCTT CTCKKFKTIC HQITCPPATC

          370        380        390        400        410        420
    ASPSFVEGEC CPSCLHSVDG EEGWSPWAEW TQCSVTCGSG TQQRGRSCDV TSNTCLGPSI
```

```
        430        440        450        460        470        480
QTRACSLSKC DTRIRQDGGW SHWSPWSSCS VTCGVGNITR IRLCNSPVPQ MGGKNCKGSG

        490        500        510        520        530        540
RETKACQGAP CPIDGRWSPW SPWSACTVTC AGGIRERTRV CNSPEPQYGG KACVGDVQER

        550        560        570        580        590        600
QMCNKRSCPV DGCLSNPCFP GAQCSSFPDG SWSCGSCPVG FLGNGTHCED LDECALVPDI

        610        620        630        640        650        660
CFSTSKVPRC VNTQPGFHCL PCPPRYRGNQ PVGVGLEAAK TEKQVCEPEN PCKDKTHNCH

        670        680        690        700        710        720
KHAECIYLGH FSDPMYKCEC QTGYAGDGLI CGEDSDLDGW PNLNLVCATN ATYHCIKDNC

        730        740        750        760        770        780
PHLPNSGQED FDKDGIGDAC DDDDDNDGVT DEKDNCQLLF NPRQADYDKD EVGDRCDNCP

        790        800        810        820        830        840
YVHNPAQIDT DNNGEGDACS VDIDGDDVFN ERDNCPYVYN TDQRDTDGDG VGDHCDNCPL

        850        860        870        880        890        900
VHNPDQTDVD NDLVGDQCDN NEDIDDDGHQ NNQDNCPYIS NANQADHDRD GQGDACDPDD

        910        920        930        940        950        960
DNDGVPDDRD NCRLVFNPDQ EDLDGDGRGD ICKDDFDNDN IPDIDDVCPE NNAISETDFR

        970        980        990       1000       1010       1020
NFQMVPLDPK GTTQIDPNWV IRHQGKELVQ TANSDPGIAV GFDEFGSVDF SGTFYVNTDR

       1030       1040       1050       1060       1070       1080
DDDYAGFVFG YQSSSRFYVV MWKQVTQTYW EDQPTRAYGY SGVSLKVVNS TTGTGEHLRN

       1090       1100       1110       1120       1130       1140
ALWHTGNTPG QVRTLWHDPR NIGWKDYTAY RWHLTHRPKT GYIRVLVHEG KQVMADSGPI

       1150       1160       1170
YDQTYAGGRL GLFVFSQEMV YFSDLKYECR DI
```

The following domains have been identified in Thrombospondin-2:

| Residues | Length | Domain ID |
| --- | --- | --- |
| 1-18 | 18 | Signal peptide |
| 19-1172 | 1154 | Thrombospondin-2 |

As used herein, the term "Collagen alpha-1(XVIII) chain" refers to one or more polypeptides present in a biological sample that are derived from the Collagen alpha-1(XVIII) chain precursor (Swiss-Prot P39060 (SEQ ID NO: 13))

```
                10          20          30          40          50          60
        MAPYPCGCHI LLLLFCCLAA ARANLLNLNW LWFNNEDTSH AATTIPEPQG PLPVQPTADT

                70          80          90         100         110         120
        TTHVTPRNGS TEPATAPGSP EPPSELLEDG QDTPTSAESP DAPEENIAGV GAEILNVAKG

               130         140         150         160         170         180
        IRSFVQLWND TVPTESLARA ETLVLETPVG PLALAGPSST PQENGTTLWP SRGIPSSPGA

               190         200         210         220         230         240
        HTTEAGTLPA PTPSPPSLGR PWAPLTGPSV PPPSSGRASL SSLLGGAPPW GSLQDPDSQG

               250         260         270         280         290         300
        LSPAAAAPSQ QLQRPDVRLR TPLLHPLVMG SLGKHAAPSA FSSGLPGALS QVAVTTLTRD

               310         320         330         340         350         360
        SGAWVSHVAN SVGPGLANNS ALLGADPEAP AGRCLPLPPS LPVCGHLGIS RFWLPNHLHH

               370         380         390         400         410         420
        ESGEQVRAGA RAWGGLLQTH CHPFLAWFFC LLLVPPCGSV PPPAPPPCCQ FCEALQDACW

               430         440         450         460         470         480
        SRLGGGRLPV ACASLPTQED GYCVLIGPAA ERISEEVGLL QLLGDPPPQQ VTQTDDPDVG

               490         500         510         520         530         540
        LAYVFGPDAN SGQVARYHFP SLFFRDFSLL FHIRPATEGP GVLFAITDSA QAMVLLGVKL

               550         560         570         580         590         600
        SGVQDGHQDI SLLYTEPGAG QTHTAASFRL PAFVGQWTHL ALSVAGGFVA LYVDCEEFQR

               610         620         630         640         650         660
        MPLARSSRGL ELEPGAGLFV AQAGGADPDK FQGVIAELKV RRDPQVSPMH CLDEEGDDSD

               670         680         690         700         710         720
        GASGDSGSGL GDARELLREE TGAALKPRLP APPPVTTPPL AGGSSTEDSR SEEVEEQTTV

               730         740         750         760         770         780
        ASLGAQTLPG SDSVSTWDGS VRTPGGRVKE GGLKGQKGEP GVPGPPGRAG PPGSPCLPGP

               790         800         810         820         830         840
        PGLPCPVSPL GPAGPALQTV PGPQGPPGPP GRDGTPGRDG EPGDPGEDGK PGDTGPQGFP

               850         860         870         880         890         900
        GTPGDVGPKG DKGDPGVGER GPPGPQGPPG PPGPSFRHDK LTFIDMEGSG FGGDLEALRG

               910         920         930         940         950         960
        PRGFPGPPGP PGVPGLPGEP GRFGVNSSDV PGPAGLPGVP GREGPPGFPG LPGPPGPPGR

               970         980         990        1000        1010        1020
        EGPPGRTGQK GSLGEAGAPG HKGSKGAPGP AGARGESGLA GAPGPAGPPG PPGPPGPPGP

              1030        1040        1050        1060        1070        1080
        GLPAGFDDME GSGGPFWSTA RSADGPQGPP GLPGLKGDPG VPGLPGAKGE VGADGVPGFP

              1090        1100        1110        1120        1130        1140
        GLPGREGIAG PQGPKGDRGS RGEKGDPGKD GVGQPGLPGP PGPPGPVVYV SEQDGSVLSV
```

22

```
            1150       1160       1170       1180       1190       1200
       PGPEGRPGFA GFPGPAGPKG NLGSKGERGS PGPKGEKGEP GSIFSPDGGA LGPAQKGAKG

            1210       1220       1230       1240       1250       1260
       EPGFRGPPGP YGRPGYKGEI GFPGRPGRPG MNGLKGEKGE PGDASLGFGM RGMPGPPGPP

            1270       1280       1290       1300       1310       1320
       GPPGPPGTPV YDSNVFAESS RPGPPGLPGN QGPPGPKGAK GEVGPPGPPG QFPFDFLQLE

            1330       1340       1350       1360       1370       1380
       AEMKGEKGDR GDAGQKGERG EPGGGGFFGS SLPGPPGPPG PPGPRGYPGI PGPKGESIRG

            1390       1400       1410       1420       1430       1440
       QPGPPGPQGP PGIGYEGRQG PPGPPGPPGP PSFPGPHRQT ISVPGPPGPP GPPGPPGTMG

            1450       1460       1470       1480       1490       1500
       ASSGVRLWAT RQAMLGQVHE VPEGWLIFVA EQEELYVRVQ NGFRKVQLEA RTPLPRGTDN

            1510       1520       1530       1540       1550       1560
       EVAALQPPVV QLHDSNPYPR REHPHPTARP WRADDILASP PRLPEPQPYP GAPHHSSYVH

            1570       1580       1590       1600       1610       1620
       LRPARPTSPP AHSHRDFQPV LHLVALNSPL SGGMRGIRGA DFQCFQQARA VGLAGTFRAF

            1630       1640       1650       1660       1670       1680
       LSSRLQDLYS IVRRADRAAV PIVNLKDELL FPSWEALFSG SEGPLKPGAR IFSFDGKDVL

            1690       1700       1710       1720       1730       1740
       RHPTWPQKSV WHGSDPNGRR LTESYCETWR TEAPSATGQA SSLLGGRLLG QSAASCHHAY

            1750
       IVLCIENSFM TASK
```

The following domains have been identified in Collagen alpha-1(XVIII) chain. Preferred assays detect Endostatin:

| Residues | Length | Domain ID |
| --- | --- | --- |
| 1-23 | 23 | Signal peptide |
| 24-1754 | 1731 | Collagen alpha-1(XVIII) chain |
| 1572-1754 | 183 | Endostatin |
| 216-450 | | Missing in isoform 2 |
| 1-415 | | Missing in isoform 3 |
| 416-450 | | QDACWSRLGGGRLPVACASLPTQEDGYCVLIGPAA (SEQ ID NO: 14) → MAPRCPWPWPRRRRLLDVLAPLVLLLGVRAASAEP (SEQ ID NO: 15) in isoform 3 |

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, etc.

In this regard, the skilled artisan will understand that the signals obtained from an immunoassay are a direct result of complexes formed between one or more antibodies and the target biomolecule (*i.e.,* the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.*) and nucleic acid measurements (mRNA quatitation). This list is not meant to be limiting.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994, including all tables, figures and claims.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377, including all tables, figures and claims. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.*, fluorescent moieties, electrochemical labels, metal chelates, *etc.*) as well as molecules that may be indirectly detected by production of a detectable reaction product (*e.g.*, enzymes such as horseradish peroxidase, alkaline phosphatase, *etc.*) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present disclosure provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present disclosure. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule, than its affinity for a non-target molecule. In preferred embodiments, Preferred antibodies bind with affinities of at least about $10^7$ $M^{-1}$, and preferably between about $10^8$ $M^{-1}$ to about $10^9$ $M^{-1}$, about $10^9$ $M^{-1}$ to about $10^{10}$ $M^{-1}$, or about $10^{10}$ $M^{-1}$ to about $10^{12}$ $M^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection theory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (P10909); Complement C3 (P01024); Cysteine-rich protein (CYR61, O00622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-lalpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (O95631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Betal (P01137); Transferrin (P02787); Trefoil factor 3 (TFF3, Q07654); Toll-Like protein 4 (O00206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, O00458); Isovaleryl-CoA dehydrogenase (IVD, P26440); I-TAC/CXCL11 (014625); Keratin 19 (P08727); Kim-1 (Hepatitis A virus cellular receptor 1, O43656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (O60356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present disclosure may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria

males and females 18 years of age or older;

undergoing a radiographic / angiographic procedure (such as a CT scan or coronary intervention) involving the intravascular administration of contrast media;

expected to be hospitalized for at least 48 hours after contrast administration.

able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 m$^2$ = 2 points, 20-40 mL/min/1.73 m$^2$ = 4 points, < 20 mL/min/1.73 m$^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria

males and females 18 years of age or older;

undergoing cardiovascular surgery;

Toronto/Ottawa Predictive Risk Index for Renal Replacement risk score of at least 2 (Wijeysundera et al., JAMA 297: 1801-9, 2007); and

able and willing to provide written informed consent for study participation and to comply with all study procedures.

Exclusion Criteria

known pregnancy;

previous renal transplantation;

acutely worsening renal function prior to enrollment (e.g., any category of RIFLE criteria);

already receiving dialysis (either acute or chronic) or in imminent need of dialysis at enrollment;

currently enrolled in another clinical study or expected to be enrolled in another clinical study within 7 days of cardiac surgery that involves drug infusion or a therapeutic intervention for AKI;

known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 1900 adults expected to be in the ICU for at least 48 hours will be enrolled. To be enrolled in the study, each patient must meet all of the following inclusion criteria and none of the following exclusion criteria:

Inclusion Criteria
males and females 18 years of age or older;

Study population 1: approximately 300 patients that have at least one of:

shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of:

IV antibiotics ordered in computerized physician order entry (CPOE) within 24 hours of enrollment;

contrast media exposure within 24 hours of enrollment;

increased Intra-Abdominal Pressure with acute decompensated heart failure; and

severe trauma as the primary reason for ICU admission and likely to be hospitalized in the ICU for 48 hours after enrollment;

Study population 3: approximately 300 patients expected to be hospitalized through acute care setting (ICU or ED) with a known risk factor for acute renal injury (*e.g.* sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment;

Study population 4: approximately 1000 patients that are 21 years of age or older, within 24 hours of being admitted into the ICU, expected to have an indwelling urinary catheter for at least 48 hours after enrollment, and have at least one of the following acute conditions within 24 hours prior to enrollment:
(i) respiratory SOFA score of $\geq 2$ (PaO2/FiO2 <300), (ii) cardiovascular SOFA score of $\geq 1$ (MAP < 70 mm Hg and/or any vasopressor required).

Exclusion Criteria

known pregnancy;

institutionalized individuals;

previous renal transplantation;

known acutely worsening renal function prior to enrollment (e.g., any category of RIFLE criteria);

received dialysis (either acute or chronic) within 5 days prior to enrollment or in imminent need of dialysis at the time of enrollment;

known infection with human immunodeficiency virus (HIV) or a hepatitis virus;

meets any of the following:

(i) active bleeding with an anticipated need for > 4 units PRBC in a day;

(ii) hemoglobin < 7 g/dL;

(iii) any other condition that in the physician's opinion would contraindicate drawing serial blood samples for clinical study purposes;

meets only the SBP < 90 mmHg inclusion criterion set forth above, and does not have shock in the attending physician's or principal investigator's opinion;

After obtaining informed consent, an EDTA anti-coagulated blood sample (10 mL) and a urine sample (25-50 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), 36 ($\pm$ 2), 48 ($\pm$ 2), 60 ($\pm$ 2), 72 ($\pm$ 2), and 84 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Units for the concentrations reported in the following data tables are as follows: Proheparin-binding EGF-like growth factor - pg/mL, Tenascin C - pg/mL, Angiopoietin-related protein 4 - ng/mL, Fibroblast growth factor 19 - ng/mL, Fibroblast growth factor 21 - ng/mL, Heparin-binding growth factor 1 - pg/mL, Angiopoietin-related protein 6 - ng/mL, Proepiregulin - pg/mL, Probetacellulin - pg/mL, Amphiregulin - pg/mL, Angiogenin - pg/mL, Thrombospondin-2 - pg/mL, and Collagen alpha-1(XVIII) chain (endostatin domain) - pg/mL,. In the case of those kidney injury markers which are membrane proteins as described herein, the assays used in these examples detect soluble forms thereof.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Markers were each measured by standard immunoassay methods using commercially available assay reagents in the urine samples and the plasma component of the blood samples collected.

Two cohorts were defined to represent a "diseased" and a "normal" population. While these terms are used for convenience, "diseased" and "normal" simply represent two cohorts for comparison (say RIFLE 0 vs RIFLE R, I and F; RIFLE 0 vs RIFLE R; RIFLE 0 and R vs RIFLE I and F; etc.). The time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) is determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may include patients adjudicated to stage R, I, or F on the basis of urine output; for those patients adjudicated to stage R, I, or F on the basis of urine output alone, the stage 0 cohort may include patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage is used.

The ability to distinguish cohort 1 from Cohort 2 was determined using ROC analysis. SE is the standard error of the AUC, n is the number of sample or individual patients ("pts," as indicated). Standard errors are calculated as described in Hanley, J. A., and McNeil, B.J., The meaning and use of the area under a receiver operating characteristic (ROC) curve. Radiology (1982) 143: 29-36; p values are calculated with a two-tailed Z-test. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 are determined. OR is the odds ratio calculated for the particular cutoff concentration, and 95% CI is the confidence interval for the odds ratio.

Fig. 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

**Angiogenin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4070 | 8980 | 4070 | 7030 | 4070 | 5170 |
| Average | 7410 | 12000 | 7410 | 9920 | 7410 | 7950 |
| Stdev | 7830 | 9230 | 7830 | 8050 | 7830 | 7320 |
| p(t-test) | | 2.1E-10 | | 0.0014 | | 0.65 |
| Min | 55.7 | 132 | 55.7 | 97.6 | 55.7 | 650 |
| Max | 30600 | 30600 | 30600 | 30600 | 30600 | 24000 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5850 | 7630 | 5850 | 9740 | 5850 | 7540 |
| Average | 9700 | 11300 | 9700 | 11000 | 9700 | 10100 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 8780 | 9140 | 8780 | 8280 | 8780 | 8050 |
| p(t-test) | | 0.19 | | 0.30 | | 0.76 |
| Min | 0.00873 | 677 | 0.00873 | 97.6 | 0.00873 | 426 |
| Max | 30600 | 30600 | 30600 | 30600 | 30600 | 30600 |
| n (Samp) | 1121 | 58 | 1121 | 51 | 1121 | 36 |
| n (Patient) | 395 | 58 | 395 | 51 | 395 | 36 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4280 | 11300 | 4280 | 6950 | 4280 | 4040 |
| Average | 8070 | 12700 | 8070 | 9800 | 8070 | 8380 |
| Stdev | 8270 | 9210 | 8270 | 7750 | 8270 | 7700 |
| p(t-test) | | 6.8E-10 | | 0.033 | | 0.81 |
| Min | 55.7 | 132 | 55.7 | 311 | 55.7 | 650 |
| Max | 30600 | 30600 | 30600 | 30600 | 30600 | 22100 |
| n (Samp) | 506 | 182 | 506 | 129 | 506 | 43 |
| n (Patient) | 215 | 182 | 215 | 129 | 215 | 43 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.57 | 0.66 | 0.61 | 0.55 | 0.59 | 0.55 | 0.54 | 0.52 |
| SE | 0.024 | 0.040 | 0.025 | 0.029 | 0.042 | 0.029 | 0.046 | 0.050 | 0.046 |
| p | 1.7E-10 | 0.10 | 3.1E-10 | 2.5E-4 | 0.25 | 0.0013 | 0.32 | 0.39 | 0.60 |
| nCohort 1 | 430 | 1121 | 506 | 430 | 1121 | 506 | 430 | 1121 | 506 |
| nCohort 2 | 195 | 58 | 182 | 132 | 51 | 129 | 46 | 36 | 43 |
| Cutoff 1 Sens 1 Spec 1 | 4060 | 4060 | 4350 | 3490 | 4300 | 3700 | 2680 | 4330 | 2620 |
| | 70% | 71% | 70% | 70% | 71% | 71% | 72% | 72% | 72% |
| | 50% | 39% | 51% | 44% | 41% | 43% | 36% | 41% | 34% |
| Cutoff 2 Sens 2 Spec 2 | 2910 | 3420 | 3350 | 2480 | 2230 | 2770 | 2090 | 2850 | 1880 |
| | 80% | 81% | 80% | 80% | 80% | 81% | 80% | 81% | 81% |
| | 38% | 33% | 40% | 35% | 23% | 35% | 30% | 28% | 26% |
| Cutoff 3 Sens 3 Spec 3 | 1680 | 1810 | 1820 | 1390 | 1440 | 1840 | 1510 | 1680 | 1510 |
| | 90% | 91% | 90% | 90% | 90% | 91% | 91% | 92% | 91% |
| | 23% | 18% | 24% | 18% | 14% | 25% | 19% | 16% | 19% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 Sens 4 Spec 4 | 8010 | 14500 | 9520 | 8010 | 14500 | 9520 | 8010 | 14500 | 9520 |
| | 53% | 36% | 53% | 45% | 39% | 41% | 37% | 25% | 33% |
| | 70% | 70% | 70% | 70% | 70% | 70% | 70% | 70% | 70% |
| Cutoff 5 Sens 5 Spec 5 | 14000 | 19700 | 16100 | 14000 | 19700 | 16100 | 14000 | 19700 | 16100 |
| | 42% | 29% | 42% | 35% | 16% | 28% | 24% | 22% | 26% |
| | 80% | 80% | 80% | 80% | 80% | 80% | 80% | 80% | 80% |
| Cutoff 6 Sens 6 Spec 6 | 20400 | 22700 | 20400 | 20400 | 22700 | 20400 | 20400 | 22700 | 20400 |
| | 16% | 12% | 18% | 8% | 4% | 7% | 4% | 8% | 2% |
| | 92% | 90% | 91% | 92% | 90% | 91% | 92% | 90% | 91% |
| OR Quart 2 p Value | 1.8 | 1.8 | 1.8 | 1.7 | 0.72 | 2.3 | 2.3 | 1.2 | 2.3 |
| | 0.031 | 0.16 | 0.048 | 0.096 | 0.49 | 0.011 | 0.080 | 0.78 | 0.082 |
| | 1.1 | 0.79 | 1.0 | 0.91 | 0.29 | 1.2 | 0.91 | 0.39 | 0.90 |
| 95% CI of OR Quart2 | 3.1 | 4.2 | 3.1 | 3.1 | 1.8 | 4.2 | 5.9 | 3.5 | 5.8 |
| OR Quart 3 p Value 95% CI of OR Quart3 | 2.2 | 1.8 | 2.5 | 1.9 | 1.5 | 2.4 | 1.8 | 2.4 | 1.0 |
| | 0.0041 | 0.16 | 0.0011 | 0.036 | 0.33 | 0.0054 | 0.24 | 0.077 | 1.0 |
| | 1.3 | 0.79 | 1.4 | 1.0 | 0.68 | 1.3 | 0.68 | 0.91 | 0.34 |
| | 3.7 | 4.2 | 4.4 | 3.5 | 3.2 | 4.5 | 4.7 | 6.3 | 2.9 |
| OR Quart 4 p Value 95% CI of OR Quart4 | 4.5 | 1.9 | 4.9 | 3.0 | 1.5 | 3.0 | 1.8 | 1.5 | 2.1 |
| | 1.5E-8 | 0.12 | 5.2E-9 | 2.6E-4 | 0.33 | 5.0E-4 | 0.24 | 0.44 | 0.12 |
| | 2.7 | 0.85 | 2.9 | 1.7 | 0.68 | 1.6 | 0.68 | 0.53 | 0.82 |
| | 7.5 | 4.4 | 8.3 | 5.4 | 3.2 | 5.5 | 4.7 | 4.3 | 5.4 |

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11.7 | 11.6 | 11.7 | 11.8 | 11.7 | 11.5 |
| Average | 38.9 | 38.7 | 38.9 | 65.8 | 38.9 | 55.3 |
| Stdev | 79.2 | 92.2 | 79.2 | 172 | 79.2 | 122 |
| p(t-test) | | 0.98 | | 0.025 | | 0.37 |
| Min | 0.000949 | 0.000466 | 0.000949 | 0.000981 | 0.000949 | 0.633 |
| Max | 538 | 789 | 538 | 1370 | 538 | 453 |
| n (Samp) | 363 | 168 | 363 | 103 | 363 | 22 |
| n (Patient) | 151 | 168 | 151 | 103 | 151 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.4 | 18.6 | 12.4 | 17.2 | 12.4 | 9.37 |
| Average | 44.1 | 50.5 | 44.1 | 79.9 | 44.1 | 63.1 |
| Stdev | 108 | 70.1 | 108 | 145 | 108 | 135 |
| p(t-test) | | 0.68 | | 0.046 | | 0.39 |
| Min | 0.000734 | 0.577 | 0.000734 | 1.82 | 0.000734 | 2.38 |
| Max | 1370 | 276 | 1370 | 645 | 1370 | 453 |
| n (Samp) | 960 | 49 | 960 | 39 | 960 | 25 |
| n (Patient) | 317 | 49 | 317 | 39 | 317 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.3 | 11.2 | 12.3 | 12.0 | 12.3 | 11.5 |
| Average | 45.8 | 40.8 | 45.8 | 56.2 | 45.8 | 35.1 |
| Stdev | 91.7 | 100 | 91.7 | 164 | 91.7 | 78.1 |
| p(t-test) | | 0.57 | | 0.38 | | 0.58 |
| Min | 0.000466 | 0.000466 | 0.000466 | 0.000981 | 0.000466 | 0.633 |
| Max | 624 | 789 | 624 | 1370 | 624 | 388 |
| n (Samp) | 444 | 155 | 444 | 104 | 444 | 24 |
| n (Patient) | 172 | 155 | 172 | 104 | 172 | 24 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.58 | 0.48 | 0.50 | 0.58 | 0.47 | 0.47 | 0.46 | 0.46 |
| SE | 0.027 | 0.044 | 0.027 | 0.032 | 0.049 | 0.032 | 0.065 | 0.060 | 0.062 |
| p | 0.66 | 0.076 | 0.53 | 0.97 | 0.11 | 0.32 | 0.59 | 0.53 | 0.49 |
| nCohort 1 | 363 | 960 | 444 | 363 | 960 | 444 | 363 | 960 | 444 |
| nCohort 2 | 168 | 49 | 155 | 103 | 39 | 104 | 22 | 25 | 24 |
| Cutoff 1 Sens 1 Spec 1 | 7.08 | 10.1 | 6.96 | 5.73 | 10.2 | 5.39 | 5.10 | 7.26 | 5.20 |
| | 70% | 71% | 70% | 71% | 72% | 70% | 73% | 72% | 71% |
| | 32% | 42% | 29% | 26% | 42% | 23% | 25% | 30% | 23% |
| Cutoff 2 Sens 2 Spec 2 | 4.53 | 5.80 | 4.61 | 4.21 | 7.47 | 3.76 | 2.96 | 5.34 | 2.60 |
| | 80% | 82% | 80% | 81% | 82% | 81% | 82% | 80% | 83% |
| | 23% | 23% | 20% | 20% | 31% | 15% | 13% | 21% | 9% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 Sens 3 Spec 3 | 2.96 | 3.15 | 3.08 | 2.53 | 3.85 | 2.52 | 1.52 | 4.48 | 1.52 |
| | 90% | 92% | 90% | 90% | 92% | 90% | 91% | 92% | 92% |
| | 13% | 10% | 11% | 10% | 14% | 9% | 5% | 18% | 4% |
| Cutoff 4 Sens 4 Spec 4 | 22.2 | 24.2 | 24.5 | 22.2 | 24.2 | 24.5 | 22.2 | 24.2 | 24.5 |
| | 32% | 45% | 26% | 33% | 31% | 27% | 27% | 16% | 33% |
| | 70% | 70% | 70% | 70% | 70% | 70% | 70% | 70% | 70% |
| Cutoff 5 Sens 5 Spec 5 | 36.8 | 42.2 | 44.4 | 36.8 | 42.2 | 44.4 | 36.8 | 42.2 | 44.4 |
| | 21% | 31% | 20% | 22% | 28% | 17% | 18% | 16% | 17% |
| | 80% | 80% | 80% | 80% | 80% | 80% | 80% | 80% | 80% |
| Cutoff 6 Sens 6 Spec 6 | 96.1 | 96.1 | 107 | 96.1 | 96.1 | 107 | 96.1 | 96.1 | 107 |
| | 8% | 14% | 7% | 14% | 21% | 11% | 14% | 16% | 4% |
| | 90% | 90% | 90% | 90% | 90% | 90% | 90% | 90% | 90% |
| OR Quart 2 p Value 95% CI of OR Quart2 | 1.3 | 0.90 | 1.2 | 0.85 | 1.5 | 1.2 | 1.0 | 1.5 | 0.24 |
| | 0.31 | 0.82 | 0.51 | 0.61 | 0.44 | 0.64 | 0.99 | 0.52 | 0.073 |
| | 0.78 | 0.36 | 0.71 | 0.46 | 0.53 | 0.63 | 0.28 | 0.42 | 0.049 |
| | 2.2 | 2.2 | 2.0 | 1.6 | 4.3 | 2.1 | 3.6 | 5.4 | 1.1 |
| OR Quart 3 p Value 95% CI of OR Quart3 | 1.0 | 1.1 | 1.3 | 0.86 | 2.0 | 0.85 | 1.2 | 2.0 | 0.61 |
| | 0.92 | 0.82 | 0.29 | 0.63 | 0.16 | 0.62 | 0.74 | 0.25 | 0.40 |
| | 0.60 | 0.46 | 0.79 | 0.46 | 0.75 | 0.45 | 0.36 | 0.61 | 0.19 |
| | 1.7 | 2.6 | 2.2 | 1.6 | 5.5 | 1.6 | 4.2 | 6.9 | 1.9 |
| OR Quart 4 | 1.3 | 2.0 | 1.1 | 1.0 | 2.0 | 1.4 | 1.2 | 1.8 | 1.1 |
| | 0.38 | 0.092 | 0.76 | 0.91 | 0.16 | 0.23 | 0.74 | 0.36 | 0.80 |
| p Value 95% CI of OR Quart4 | 0.75 | 0.89 | 0.64 | 0.57 | 0.75 | 0.79 | 0.36 | 0.51 | 0.42 |
| | 2.1 | 4.3 | 1.8 | 1.9 | 5.5 | 2.6 | 4.2 | 6.2 | 3.1 |

**Angiopoietin-related protein 6**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.105 | 0.0450 | 0.105 | 0.109 | 0.105 | 0.000350 |
| Average | 1.04 | 1.27 | 1.04 | 1.52 | 1.04 | 0.471 |
| Stdev | 2.57 | 7.27 | 2.57 | 3.99 | 2.57 | 1.02 |
| p(t-test) | | 0.60 | | 0.15 | | 0.33 |
| Min | 1.00E-4 | 1.00E-4 | 1.00E-4 | 0.000110 | 1.00E-4 | 0.000110 |
| Max | 25.9 | 89.0 | 25.9 | 22.4 | 25.9 | 4.24 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 333 | 156 | 333 | 97 | 333 | 20 |
| n (Patient) | 141 | 156 | 141 | 97 | 141 | 20 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0677 | 0.214 | 0.0677 | 0.0456 | 0.0677 | 0.000364 |
| Average | 1.10 | 0.704 | 1.10 | 3.20 | 1.10 | 0.501 |
| Stdev | 3.58 | 1.44 | 3.58 | 14.6 | 3.58 | 1.05 |
| p(t-test) | | 0.45 | | 0.0063 | | 0.42 |
| Min | 1.00E-4 | 1.00E-4 | 1.00E-4 | 0.000120 | 1.00E-4 | 1.00E-4 |
| Max | 70.5 | 8.76 | 70.5 | 89.0 | 70.5 | 4.24 |
| n (Samp) | 864 | 47 | 864 | 37 | 864 | 23 |
| n (Patient) | 292 | 47 | 292 | 37 | 292 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.151 | 0.0456 | 0.151 | 0.0635 | 0.151 | 0.000566 |
| Average | 1.06 | 1.38 | 1.06 | 1.64 | 1.06 | 0.329 |
| Stdev | 2.44 | 7.63 | 2.44 | 4.46 | 2.44 | 0.615 |
| p(t-test) | | 0.45 | | 0.081 | | 0.17 |
| Min | 1.00E-4 | 1.00E-4 | 1.00E-4 | 0.000110 | 1.00E-4 | 0.000110 |
| Max | 25.9 | 89.0 | 25.9 | 22.5 | 25.9 | 2.35 |
| n (Samp) | 414 | 143 | 414 | 97 | 414 | 21 |
| n (Patient) | 162 | 143 | 162 | 97 | 162 | 21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | 0.55 | 0.45 | 0.49 | 0.51 | 0.48 | 0.37 | 0.41 | 0.37 |
| SE | 0.028 | 0.044 | 0.028 | 0.033 | 0.049 | 0.033 | 0.069 | 0.063 | 0.067 |
| p | 0.18 | 0.25 | 0.088 | 0.69 | 0.81 | 0.52 | 0.061 | 0.16 | 0.047 |
| nCohort 1 | 333 | 864 | 414 | 333 | 864 | 414 | 333 | 864 | 414 |
| nCohort 2 | 156 | 47 | 143 | 97 | 37 | 97 | 20 | 23 | 21 |
| Cutoff 1 | 0.000336 | 0.00640 | 0.000278 | 0.000336 | 0.000336 | 0.000336 | 0.000206 | 0.000244 | 0.000206 |
| Sens 1 | 72% | 70% | 74% | 73% | 70% | 73% | 75% | 78% | 76% |
| Spec 1 | 23% | 45% | 23% | 23% | 30% | 24% | 11% | 18% | 12% |
| Cutoff 2 | 0.000249 | 0.000469 | 0.000249 | 0.000249 | 0.000249 | 0.000249 | 0.000203 | 0.000213 | 0.000203 |
| Sens 2 | 83% | 81% | 80% | 81% | 81% | 84% | 80% | 83% | 81% |
| Spec 2 | 16% | 33% | 17% | 16% | 25% | 17% | 9% | 17% | 10% |
| Cutoff 3 | 0.000203 | 0.000244 | 0.000123 | 0.000123 | 0.000157 | 0.000203 | 0.000122 | 0.000122 | 0.000122 |
| Sens 3 | 90% | 91% | 94% | 93% | 92% | 91% | 90% | 96% | 90% |
| Spec 3 | 9% | 18% | 7% | 6% | 11% | 10% | 6% | 6% | 7% |
| Cutoff 4 | 0.610 | 0.548 | 0.708 | 0.610 | 0.548 | 0.708 | 0.610 | 0.548 | 0.708 |
| Sens 4 | 27% | 38% | 24% | 32% | 32% | 28% | 30% | 22% | 19% |
| Spec 4 | 70% | 70% | 70% | 70% | 70% | 70% | 70% | 70% | 70% |
| Cutoff 5 | 1.28 | 1.17 | 1.54 | 1.28 | 1.17 | 1.54 | 1.28 | 1.17 | 1.54 |
| Sens 5 | 15% | 17% | 13% | 20% | 24% | 15% | 10% | 17% | 5% |
| Spec 5 | 80% | 80% | 80% | 80% | 80% | 80% | 80% | 80% | 80% |
| Cutoff 6 | 2.70 | 2.51 | 2.99 | 2.70 | 2.51 | 2.99 | 2.70 | 2.51 | 2.99 |
| Sens 6 | 6% | 4% | 6% | 9% | 14% | 10% | 5% | 4% | 0% |
| Spec 6 | 90% | 90% | 90% | 90% | 90% | 90% | 90% | 90% | 90% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 1.1 | 1.4 | 1.3 | 1.3 | 1.9 | 1.3 | 1.4 | 0.59 | 2.0 |
| | 0.86 | 0.49 | 0.31 | 0.40 | 0.18 | 0.41 | 0.69 | 0.48 | 0.42 |
| | 0.61 | 0.55 | 0.77 | 0.70 | 0.75 | 0.69 | 0.30 | 0.14 | 0.37 |
| | 1.8 | 3.5 | 2.3 | 2.5 | 4.9 | 2.5 | 6.3 | 2.5 | 11 |
| OR Quart 3 p Value 95% Clot OR Quart3 | 1.3 | 2.1 | 1.3 | 0.94 | 0.85 | 1.4 | 1.0 | 1.6 | 3.7 |
| | 0.31 | 0.10 | 0.31 | 0.86 | 0.78 | 0.33 | 0.99 | 0.40 | 0.11 |
| | 0.77 | 0.87 | 0.77 | 0.48 | 0.28 | 0.72 | 0.20 | 0.52 | 0.75 |
| | 2.3 | 4.9 | 2.3 | 1.8 | 2.6 | 2.6 | 5.2 | 5.0 | 18 |
| OR Quart 4 p Value 95% CI of OR Quart4 | 1.4 | 1.5 | 1.7 | 1.3 | 1.6 | 1.4 | 3.7 | 1.4 | 4.3 |
| | 0.25 | 0.37 | 0.070 | 0.40 | 0.34 | 0.32 | 0.054 | 0.55 | 0.070 |
| | 0.80 | 0.61 | 0.96 | 0.70 | 0.61 | 0.73 | 0.98 | 0.44 | 0.89 |
| | 2.3 | 3.8 | 2.9 | 2.5 | 4.2 | 2.6 | 14 | 4.5 | 21 |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 20.6 | 27.6 | 20.6 | 32.6 | 20.6 | 36.8 |
| Average | 46.4 | 39.6 | 46.4 | 101 | 46.4 | 103 |
| Stdev | 99.6 | 48.0 | 99.6 | 289 | 99.6 | 271 |
| p(t-test) | | 0.45 | | 0.0066 | | 0.017 |
| Min | 0.00401 | 0.00389 | 0.00401 | 0.00401 | 0.00401 | 3.84 |
| Max | 1310 | 300 | 1310 | 2190 | 1310 | 1270 |
| n (Samp) | 268 | 138 | 268 | 104 | 268 | 35 |
| n (Patient) | 148 | 138 | 148 | 104 | 148 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.5 | 31.6 | 23.5 | 46.4 | 23.5 | 40.2 |
| Average | 60.4 | 54.7 | 60.4 | 109 | 60.4 | 40.4 |
| Stdev | 177 | 65.1 | 177 | 172 | 177 | 28.4 |
| p(t-test) | | 0.84 | | 0.10 | | 0.59 |
| Min | 0.00389 | 6.31 | 0.00389 | 6.70 | 0.00389 | 4.90 |
| Max | 2190 | 289 | 2190 | 987 | 2190 | 124 |
| n (Samp) | 663 | 38 | 663 | 37 | 663 | 23 |
| n (Patient) | 288 | 38 | 288 | 37 | 288 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.9 | 29.8 | 23.9 | 37.2 | 23.9 | 39.6 |
| Average | 43.7 | 42.7 | 43.7 | 108 | 43.7 | 134 |
| Stdev | 64.1 | 49.9 | 64.1 | 320 | 64.1 | 289 |
| p(t-test) | | 0.87 | | 8.3E-4 | | 6.8E-6 |
| Min | 0.00131 | 0.00389 | 0.00131 | 0.00401 | 0.00131 | 3.84 |
| Max | 480 | 300 | 480 | 2190 | 480 | 1270 |
| n (Samp) | 313 | 127 | 313 | 102 | 313 | 32 |
| n (Patient) | 152 | 127 | 152 | 102 | 152 | 32 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.54 | 0.58 | 0.54 | 0.60 | 0.68 | 0.60 | 0.57 | 0.59 | 0.60 |
| SE | 0.030 | 0.050 | 0.031 | 0.034 | 0.050 | 0.033 | 0.053 | 0.063 | 0.055 |
| p | 0.24 | 0.12 | 0.19 | 0.0031 | 3.0E-4 | 0.0032 | 0.18 | 0.17 | 0.069 |
| nCohort 1 | 268 | 663 | 313 | 268 | 663 | 313 | 268 | 663 | 313 |
| nCohort 2 | 138 | 38 | 127 | 104 | 37 | 102 | 35 | 23 | 32 |
| Cutoff 1 Sens 1 Spec 1 | 16.0 | 16.8 | 16.3 | 18.3 | 24.0 | 20.9 | 16.3 | 22.1 | 18.5 |
|  | 70% | 71% | 70% | 70% | 70% | 71% | 71% | 74% | 72% |
|  | 40% | 38% | 37% | 45% | 51% | 46% | 40% | 48% | 41% |
| Cutoff 2 Sens 2 Spec 2 | 11.2 | 12.1 | 13.2 | 14.2 | 16.8 | 15.1 | 9.15 | 9.15 | 12.7 |
|  | 80% | 82% | 80% | 81% | 81% | 80% | 83% | 83% | 81% |
|  | 26% | 26% | 29% | 35% | 38% | 35% | 19% | 18% | 27% |
| Cutoff 3 Sens 3 Spec 3 | 7.44 | 9.02 | 6.50 | 8.80 | 14.5 | 9.55 | 5.56 | 6.50 | 5.56 |
|  | 91% | 92% | 91% | 90% | 92% | 90% | 91% | 91% | 91% |
|  | 15% | 18% | 13% | 18% | 32% | 20% | 11% | 13% | 11% |
| Cutoff 4 Sens 4 Spec 4 | 41.2 | 43.2 | 43.8 | 41.2 | 43.2 | 43.8 | 41.2 | 43.2 | 43.8 |
|  | 28% | 37% | 31% | 43% | 57% | 43% | 43% | 48% | 44% |
|  | 70% | 70% | 70% | 70% | 70% | 70% | 70% | 70% | 70% |
| Cutoff 5 Sens 5 Spec 5 | 54.4 | 54.7 | 56.0 | 54.4 | 54.7 | 56.0 | 54.4 | 54.7 | 56.0 |
|  | 18% | 29% | 20% | 29% | 46% | 27% | 20% | 22% | 25% |
|  | 80% | 80% | 81% | 80% | 80% | 81% | 80% | 80% | 81% |
| Cutoff 6 Sens 6 Spec 6 | 97.1 | 92.1 | 92.1 | 97.1 | 92.1 | 92.1 | 97.1 | 92.1 | 92.1 |
|  | 7% | 13% | 8% | 14% | 35% | 14% | 11% | 4% | 19% |
|  | 90% | 90% | 90% | 90% | 90% | 90% | 90% | 90% | 90% |
| OR Quart 2 p Value 95% CI of OR Quart2 | 1.2 | 1.3 | 1.5 | 1.7 | 5.8 | 1.4 | 0.47 | 0.39 | 0.65 |
|  | 0.57 | 0.61 | 0.22 | 0.12 | 0.024 | 0.31 | 0.23 | 0.27 | 0.52 |
|  | 0.65 | 0.47 | 0.80 | 0.87 | 1.3 | 0.72 | 0.13 | 0.075 | 0.18 |
|  | 2.2 | 3.6 | 2.7 | 3.5 | 27 | 2.9 | 1.6 | 2.0 | 2.4 |
| OR Quart 3 p Value 95% CI of OR Quart3 | 2.1 | 1.5 | 2.0 | 1.9 | 3.1 | 1.8 | 1.7 | 1.6 | 2.2 |
|  | 0.013 | 0.46 | 0.026 | 0.062 | 0.17 | 0.099 | 0.26 | 0.40 | 0.14 |
|  | 1.2 | 0.54 | 1.1 | 0.97 | 0.61 | 0.90 | 0.67 | 0.52 | 0.77 |
|  | 3.8 | 3.9 | 3.6 | 3.8 | 15 | 3.5 | 4.4 | 5.1 | 6.1 |
| OR Quart 4 p Value 95% CI of OR Quart4 | 1.2 | 1.8 | 1.5 | 2.5 | 9.9 | 2.6 | 1.3 | 1.6 | 1.7 |
|  | 0.47 | 0.25 | 0.22 | 0.0091 | 0.0023 | 0.0052 | 0.64 | 0.41 | 0.31 |
|  | 0.68 | 0.67 | 0.80 | 1.3 | 2.3 | 1.3 | 0.47 | 0.52 | 0.60 |
|  | 2.3 | 4.6 | 2.7 | 4.8 | 43 | 5.0 | 3.4 | 5.1 | 5.0 |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.752 | 0.909 | 0.752 | 1.07 | 0.752 | 0.909 |
| Average | 1.30 | 1.47 | 1.30 | 1.57 | 1.30 | 1.08 |
| Stdev | 2.01 | 1.98 | 2.01 | 2.12 | 2.01 | 1.06 |
| p(t-test) | | 0.42 | | 0.24 | | 0.53 |
| Min | 0.00179 | 0.00179 | 0.00179 | 0.00179 | 0.00179 | 0.00230 |
| Max | 23.1 | 14.9 | 23.1 | 11.6 | 23.1 | 4.42 |
| n (Samp) | 268 | 138 | 268 | 104 | 268 | 35 |
| n (Patient) | 148 | 138 | 148 | 104 | 148 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.761 | 1.65 | 0.761 | 1.62 | 0.761 | 1.70 |
| Average | 1.41 | 1.72 | 1.41 | 1.75 | 1.41 | 1.43 |
| Stdev | 2.42 | 1.77 | 2.42 | 1.92 | 2.42 | 1.33 |
| p(t-test) | | 0.44 | | 0.39 | | 0.97 |
| Min | 0.00179 | 0.00179 | 0.00179 | 0.00246 | 0.00179 | 0.00230 |
| Max | 28.3 | 6.72 | 28.3 | 8.64 | 28.3 | 4.42 |
| n (Samp) | 663 | 38 | 663 | 37 | 663 | 23 |
| n (Patient) | 288 | 38 | 288 | 37 | 288 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.354 | 1.11 | 0.354 | 1.40 | 0.354 | 1.11 |
| Average | 1.15 | 1.55 | 1.15 | 1.66 | 1.15 | 1.19 |
| Stdev | 1.55 | 1.99 | 1.55 | 2.03 | 1.55 | 0.989 |
| p(t-test) | | 0.022 | 0.0078 | 0.0078 | | 0.87 |
| Min | 0.00179 | 0.00179 | 0.00179 | 0.00179 | 0.00179 | 0.00240 |
| Max | 8.77 | 14.9 | 8.77 | 11.6 | 8.77 | 3.36 |
| n (Samp) | 313 | 127 | 313 | 102 | 313 | 32 |
| n (Patient) | 152 | 127 | 152 | 102 | 152 | 32 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.59 | 0.58 | 0.52 | 0.58 | 0.57 | 0.50 | 0.54 | 0.58 |
| SE | 0.030 | 0.050 | 0.031 | 0.034 | 0.050 | 0.033 | 0.052 | 0.063 | 0.055 |
| p | 0.31 | 0.064 | 0.012 | 0.61 | 0.11 | 0.051 | 0.93 | 0.47 | 0.12 |
| nCohort 1 | 268 | 663 | 313 | 268 | 663 | 313 | 268 | 663 | 313 |
| nCohort 2 | 138 | 38 | 127 | 104 | 37 | 102 | 35 | 23 | 32 |
| Cutoff 1 Sens 1 Spec 1 | 0.0522 | 0.108 | 0.0742 | 0.0305 | 0.354 | 0.0407 | 0.0435 | 0.0209 | 0.208 |
| | 71% | 71% | 72% | 71% | 70% | 71% | 77% | 74% | 72% |
| | 32% | 38% | 40% | 24% | 45% | 32% | 31% | 25% | 48% |
| Cutoff 2 Sens 2 Spec 2 | 0.00352 | 0.0378 | 0.00352 | 0.00342 | 0.0209 | 0.00342 | 0.0209 | 0.00342 | 0.0742 |
| | 81% | 84% | 81% | 83% | 81% | 81% | 80% | 83% | 81% |
| | 19% | 29% | 24% | 16% | 25% | 20% | 24% | 17% | 40% |
| Cutoff 3 Sens 3 Spec 3 | 0.00246 | 0.00342 | 0.00246 | 0.00230 | 0.00342 | 0.00230 | 0.00246 | 0.00230 | 0.00332 |
| | 92% | 92% | 93% | 93% | 92% | 92% | 91% | 96% | 91% |
| | 10% | 17% | 13% | 6% | 17% | 6% | 10% | 6% | 15% |
| Cutoff 4 Sens 4 Spec 4 | 1.74 | 1.76 | 1.58 | 1.74 | 1.76 | 1.58 | 1.74 | 1.76 | 1.58 |
| | 39% | 47% | 43% | 33% | 41% | 40% | 29% | 35% | 38% |
| | 72% | 70% | 70% | 72% | 70% | 70% | 72% | 70% | 70% |
| Cutoff 5 Sens 5 Spec 5 | 2.17 | 2.35 | 2.11 | 2.17 | 2.35 | 2.11 | 2.17 | 2.35 | 2.11 |
| | 27% | 29% | 31% | 20% | 22% | 29% | 11% | 30% | 22% |
| | 81% | 80% | 80% | 81% | 80% | 80% | 81% | 80% | 80% |
| Cutoff 6 | 3.12 | 3.36 | 3.12 | 3.12 | 3.36 | 3.12 | 3.12 | 3.36 | 3.12 |
| Sens 6 Spec 6 | 14% | 16% | 14% | 16% | 14% | 19% | 3% | 4% | 3% |
| | 90% | 91% | 90% | 90% | 91% | 90% | 90% | 91% | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 1.1 | 2.3 | 1.0 | 0.47 | 0.61 | 0.57 | 0.99 | 0.79 | 1.5 |
| | 0.69 | 0.13 | 0.88 | 0.030 | 0.40 | 0.11 | 0.98 | 0.73 | 0.52 |
| | 0.63 | 0.78 | 0.57 | 0.24 | 0.20 | 0.28 | 0.35 | 0.21 | 0.42 |
| | 2.0 | 6.7 | 1.9 | 0.93 | 1.9 | 1.1 | 2.8 | 3.0 | 5.7 |
| OR Quart 3 p Value 95% Clot OR Quart3 | 0.87 | 1.6 | 1.1 | 0.90 | 1.7 | 1.0 | 1.4 | 1.4 | 3.7 |
| | 0.65 | 0.40 | 0.76 | 0.75 | 0.26 | 0.90 | 0.48 | 0.56 | 0.029 |
| | 0.48 | 0.52 | 0.60 | 0.49 | 0.68 | 0.55 | 0.54 | 0.44 | 1.1 |
| | 1.6 | 5.1 | 2.0 | 1.7 | 4.1 | 2.0 | 3.7 | 4.6 | 12 |
| OR Quart 4 p Value 95% CI of OR Quart4 | 1.5 | 2.9 | 2.0 | 0.95 | 1.4 | 1.6 | 0.99 | 1.4 | 2.4 |
| | 0.16 | 0.043 | 0.021 | 0.87 | 0.48 | 0.14 | 0.98 | 0.57 | 0.17 |
| | 0.85 | 1.0 | 1.1 | 0.51 | 0.55 | 0.86 | 0.35 | 0.44 | 0.70 |
| | 2.7 | 8.3 | 3.5 | 1.8 | 3.6 | 2.9 | 2.8 | 4.5 | 8.0 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5040 | 6160 | 5040 | 5290 | 5040 | 4110 |
| Average | 18600 | 14200 | 18600 | 14600 | 18600 | 12700 |
| Stdev | 34200 | 23500 | 34200 | 22100 | 34200 | 23300 |
| p(t-test) | | 0.11 | | 0.21 | | 0.26 |
| Min | 323 | 300 | 323 | 281 | 323 | 485 |
| Max | 238000 | 173000 | 238000 | 133000 | 238000 | 132000 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5580 | 8680 | 5580 | 6800 | 5580 | 4300 |
| Average | 16400 | 28200 | 16400 | 19500 | 16400 | 18400 |
| Stdev | 28100 | 47400 | 28100 | 30200 | 28100 | 35800 |
| p(t-test) | | 0.0028 | | 0.43 | | 0.68 |
| Min | 0.0130 | 300 | 0.0130 | 281 | 0.0130 | 876 |
| Max | 238000 | 190000 | 238000 | 133000 | 238000 | 171000 |
| n (Samp) | 1121 | 58 | 1121 | 51 | 1121 | 36 |
| n (Patient) | 395 | 58 | 395 | 51 | 395 | 36 |

| UO only | 0hr prior to AKI stage | | 24hr prior to AKI stage | 48hr prior to AKI stage | | |
|---|---|---|---|---|---|---|
| | Cohort 1 | Cohort 2 | Cohort 1 | Cohort 2 | Cohort 1 | Cohort 2 |
| Median | 5490 | 6080 | 5490 | 5320 | 5490 | 4170 |
| Average | 19900 | 14100 | 19900 | 13200 | 19900 | 15700 |
| Stdev | 34400 | 24200 | 34400 | 17500 | 34400 | 29600 |
| p(t-test) | | 0.036 | | 0.033 | | 0.44 |
| Min | 323 | 389 | 323 | 531 | 323 | 485 |
| Max | 238000 | 180000 | 238000 | 84400 | 238000 | 173000 |
| n (Samp) | 506 | 182 | 506 | 129 | 506 | 43 |
| n (Patient) | 215 | 182 | 215 | 129 | 215 | 43 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.53 | 0.57 | 0.52 | 0.51 | 0.53 | 0.51 | 0.46 | 0.47 | 0.48 |
| SE | 0.025 | 0.040 | 0.025 | 0.029 | 0.042 | 0.029 | 0.046 | 0.050 | 0.046 |
| p | 0.19 | 0.070 | 0.32 | 0.66 | 0.55 | 0.82 | 0.33 | 0.53 | 0.65 |
| nCohort 1 | 430 | 1121 | 506 | 430 | 1121 | 506 | 430 | 1121 | 506 |
| nCohort 2 | 195 | 58 | 182 | 132 | 51 | 129 | 46 | 36 | 43 |
| Cutoff 1 Sens 1 Spec 1 | 3560 | 4110 | 3670 | 3370 | 3090 | 3480 | 2670 | 3160 | 2970 |
|  | 70% | 71% | 70% | 70% | 71% | 71% | 72% | 72% | 72% |
|  | 37% | 39% | 37% | 36% | 28% | 36% | 28% | 29% | 31% |
| Cutoff 2 Sens 2 Spec 2 | 2780 | 2380 | 3060 | 2420 | 2420 | 2740 | 1870 | 2400 | 2440 |
|  | 80% | 81% | 80% | 80% | 80% | 81% | 80% | 81% | 81% |
|  | 30% | 19% | 32% | 25% | 20% | 30% | 17% | 20% | 25% |
| Cutoff 3 Sens 3 Spec 3 | 1810 | 1670 | 2040 | 1760 | 1150 | 1880 | 904 | 1340 | 1290 |
|  | 90% | 91% | 90% | 90% | 90% | 91% | 91% | 92% | 91% |
|  | 16% | 12% | 19% | 16% | 6% | 17% | 6% | 8% | 9% |
| Cutoff 4 Sens 4 Spec 4 | 11600 | 11400 | 13400 | 11600 | 11400 | 13400 | 11600 | 11400 | 13400 |
|  | 29% | 41% | 25% | 29% | 33% | 28% | 22% | 22% | 26% |
|  | 70% | 70% | 70% | 70% | 70% | 70% | 70% | 70% | 70% |
| Cutoff 5 Sens 5 Spec 5 | 24300 | 21900 | 28700 | 24300 | 21900 | 28700 | 24300 | 21900 | 28700 |
|  | 14% | 29% | 12% | 20% | 24% | 14% | 15% | 22% | 16% |
|  | 80% | 80% | 80% | 80% | 80% | 80% | 80% | 80% | 80% |
| Cutoff 6 Sens 6 Spec 6 | 51700 | 44600 | 58300 | 51700 | 44600 | 58300 | 51700 | 44600 | 58300 |
|  | 6% | 17% | 4% | 10% | 14% | 5% | 7% | 11% | 7% |
|  | 90% | 90% | 90% | 90% | 90% | 90% | 90% | 90% | 90% |
| OR Quart2 p Value 95% CI of OR Quart2 | 1.8 | 0.91 | 2.8 | 1.3 | 0.68 | 1.9 | 1.2 | 1.0 | 1.1 |
|  | 0.025 | 0.83 | 9.4E-5 | 0.41 | 0.39 | 0.021 | 0.64 | 0.99 | 0.80 |
|  | 1.1 | 0.40 | 1.7 | 0.72 | 0.29 | 1.1 | 0.50 | 0.37 | 0.44 |
|  | 2.9 | 2.1 | 4.7 | 2.2 | 1.6 | 3.4 | 3.1 | 2.7 | 2.9 |
| OR Quart 3 | 2.0 | 1.3 | 2.7 | 1.2 | 1.0 | 1.7 | 1.8 | 1.4 | 1.8 |
|  | 0.0050 | 0.45 | 1.4E-4 | 0.47 | 1.0 | 0.070 | 0.20 | 0.48 | 0.20 |
| p Value 95% CI of OR Quart3 | 1.2 | 0.63 | 1.6 | 0.70 | 0.46 | 0.96 | 0.74 | 0.55 | 0.74 |
|  | 3.3 | 2.9 | 4.6 | 2.2 | 2.2 | 3.0 | 4.2 | 3.5 | 4.2 |
| OR Quart4 p Value 95% CI of OR Quart4 | 1.4 | 1.6 | 1.5 | 1.2 | 1.2 | 1.1 | 1.2 | 1.1 | 1.0 |
|  | 0.21 | 0.20 | 0.13 | 0.49 | 0.57 | 0.66 | 0.64 | 0.80 | 0.99 |
|  | 0.83 | 0.77 | 0.88 | 0.69 | 0.59 | 0.63 | 0.50 | 0.43 | 0.39 |
|  | 2.3 | 3.4 | 2.6 | 2.1 | 2.6 | 2.1 | 3.1 | 3.0 | 2.6 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.35 | 3.36 | 3.35 | 3.79 | 3.35 | 3.13 |
| Average | 4.65 | 5.50 | 4.65 | 6.97 | 4.65 | 6.32 |
| Stdev | 5.33 | 5.69 | 5.33 | 9.72 | 5.33 | 9.52 |
| p(t-test) | | 0.14 | | 0.0041 | | 0.13 |
| Min | 0.0801 | 0.0298 | 0.0801 | 0.0298 | 0.0801 | 0.376 |
| Max | 46.9 | 33.3 | 46.9 | 81.9 | 46.9 | 43.0 |
| n (Samp) | 264 | 133 | 264 | 99 | 264 | 33 |
| n (Patient) | 147 | 133 | 147 | 99 | 147 | 33 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.54 | 3.07 | 3.54 | 3.69 | 3.54 | 2.94 |
| Average | 6.54 | 4.01 | 6.54 | 5.23 | 6.54 | 5.23 |
| Stdev | 13.5 | 3.62 | 13.5 | 4.09 | 13.5 | 8.08 |
| p(t-test) | | 0.25 | | 0.56 | | 0.65 |
| Min | 0.000104 | 0.0298 | 0.000104 | 0.0298 | 0.000104 | 0.434 |
| Max | 279 | 16.5 | 279 | 15.8 | 279 | 37.5 |
| n (Samp) | 644 | 38 | 644 | 36 | 644 | 22 |
| n (Patient) | 285 | 38 | 285 | 36 | 285 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.36 | 3.82 | 3.36 | 3.88 | 3.36 | 3.13 |
| Average | 4.92 | 5.83 | 4.92 | 7.66 | 4.92 | 7.18 |
| Stdev | 6.15 | 5.78 | 6.15 | 11.2 | 6.15 | 9.67 |
| p(t-test) | | 0.16 | | 0.0024 | | 0.067 |
| Min | 0.000201 | 0.256 | 0.000201 | 0.235 | 0.000201 | 0.376 |
| Max | 49.4 | 33.3 | 49.4 | 81.9 | 49.4 | 43.0 |
| n (Samp) | 308 | 121 | 308 | 94 | 308 | 31 |
| n (Patient) | 151 | 121 | 151 | 94 | 151 | 31 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.55 | 0.44 | 0.57 | 0.57 | 0.53 | 0.57 | 0.51 | 0.44 | 0.52 |
| SE | 0.031 | 0.049 | 0.031 | 0.034 | 0.050 | 0.034 | 0.054 | 0.064 | 0.055 |
| p | 0.12 | 0.22 | 0.021 | 0.037 | 0.58 | 0.036 | 0.87 | 0.35 | 0.66 |
| nCohort 1 | 264 | 644 | 308 | 264 | 644 | 308 | 264 | 644 | 308 |
| nCohort 2 | 133 | 38 | 121 | 99 | 36 | 94 | 33 | 22 | 31 |
| Cutoff 1 Sens 1 Spec 1 | 1.95 | 1.66 | 2.23 | 2.10 | 2.43 | 2.12 | 1.02 | 1.56 | 1.21 |
|  | 71% | 71% | 70% | 71% | 72% | 70% | 73% | 73% | 71% |
|  | 33% | 25% | 34% | 35% | 37% | 33% | 18% | 24% | 20% |
| Cutoff 2 Sens 2 Spec 2 | 1.66 | 1.16 | 1.87 | 1.48 | 1.68 | 1.42 | 0.935 | 1.06 | 0.990 |
|  | 80% | 82% | 80% | 81% | 81% | 81% | 82% | 82% | 81% |
|  | 28% | 17% | 31% | 25% | 26% | 24% | 16% | 16% | 16% |
| Cutoff 3 Sens 3 Spec 3 | 1.06 | 0.477 | 1.36 | 0.733 | 0.899 | 0.733 | 0.717 | 0.795 | 0.795 |
|  | 90% | 92% | 90% | 91% | 92% | 90% | 91% | 91% | 90% |
|  | 19% | 6% | 22% | 12% | 12% | 12% | 11% | 11% | 13% |
| Cutoff 4 Sens 4 Spec 4 | 5.15 | 6.29 | 5.26 | 5.15 | 6.29 | 5.26 | 5.15 | 6.29 | 5.26 |
|  | 38% | 21% | 40% | 44% | 36% | 45% | 36% | 14% | 42% |
|  | 70% | 70% | 70% | 70% | 70% | 70% | 70% | 70% | 70% |
| Cutoff 5 Sens 5 Spec 5 | 6.60 | 9.07 | 6.57 | 6.60 | 9.07 | 6.57 | 6.60 | 9.07 | 6.57 |
|  | 30% | 11% | 31% | 38% | 19% | 40% | 27% | 14% | 35% |
|  | 80% | 80% | 80% | 80% | 80% | 80% | 80% | 80% | 80% |
| Cutoff 6 Sens 6 Spec 6 | 10.5 | 13.6 | 10.6 | 10.5 | 13.6 | 10.6 | 10.5 | 13.6 | 10.6 |
|  | 14% | 3% | 16% | 21% | 6% | 22% | 18% | 9% | 23% |
|  | 90% | 90% | 90% | 90% | 90% | 90% | 90% | 90% | 90% |
| OR Quart 2 p Value 95% CI of OR Quart2 | 1.9 | 1.2 | 2.4 | 1.1 | 1.5 | 0.99 | 0.51 | 2.4 | 0.56 |
|  | 0.036 | 0.78 | 0.0063 | 0.77 | 0.46 | 0.97 | 0.20 | 0.21 | 0.29 |
|  | 1.0 | 0.41 | 1.3 | 0.57 | 0.54 | 0.50 | 0.18 | 0.61 | 0.19 |
|  | 3.5 | 3.3 | 4.5 | 2.2 | 3.9 | 1.9 | 1.4 | 9.5 | 1.6 |
| OR Quart 3 p Value 95% CI of OR Quart3 | 0.95 | 1.8 | 1.3 | 0.61 | 1.6 | 0.61 | 0.51 | 1.7 | 0.27 |
|  | 0.87 | 0.24 | 0.50 | 0.19 | 0.34 | 0.20 | 0.20 | 0.48 | 0.054 |
|  | 0.50 | 0.68 | 0.65 | 0.29 | 0.61 | 0.29 | 0.18 | 0.40 | 0.072 |
|  | 1.8 | 4.6 | 2.4 | 1.3 | 4.3 | 1.3 | 1.4 | 7.2 | 1.0 |
| OR Quart 4 p Value | 2.0 | 1.6 | 2.5 | 2.2 | 1.1 | 2.3 | 0.88 | 2.4 | 1.2 |
|  | 0.020 | 0.33 | 0.0048 | 0.014 | 0.79 | 0.010 | 0.79 | 0.21 | 0.67 |
|  | 1.1 | 0.61 | 1.3 | 1.2 | 0.41 | 1.2 | 0.35 | 0.61 | 0.49 |
| 95% CI of OR Quart4 | 3.7 | 4.3 | 4.6 | 4.2 | 3.2 | 4.2 | 2.2 | 9.5 | 3.0 |

**Heparin-binding growth factor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 876 | 759 | 876 | 657 | 876 | 572 |
| Average | 1250 | 1120 | 1250 | 1250 | 1250 | 915 |
| Stdev | 1560 | 1350 | 1560 | 1570 | 1560 | 972 |
| p(t-test) | | 0.30 | | 0.98 | | 0.15 |
| Min | 0.609 | 12.9 | 0.609 | 8.16 | 0.609 | 0.779 |
| Max | 16700 | 13500 | 16700 | 7750 | 16700 | 3960 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 843 | 688 | 843 | 882 | 843 | 548 |
| Average | 1240 | 908 | 1240 | 917 | 1240 | 1000 |
| Stdev | 1480 | 889 | 1480 | 749 | 1480 | 1120 |
| p(t-test) | | 0.090 | | 0.12 | | 0.34 |
| Min | 0.00328 | 1.77 | 0.00328 | 29.5 | 0.00328 | 13.3 |
| Max | 16700 | 4350 | 16700 | 2920 | 16700 | 3960 |
| n (Samp) | 1121 | 58 | 1121 | 51 | 1121 | 36 |
| n (Patient) | 395 | 58 | 395 | 51 | 395 | 36 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 858 | 772 | 858 | 658 | 858 | 595 |
| Average | 1200 | 1170 | 1200 | 1270 | 1200 | 927 |
| Stdev | 1480 | 1490 | 1480 | 1580 | 1480 | 973 |
| p(t-test) | | 0.84 | | 0.65 | | 0.24 |
| Min | 0.609 | 12.9 | 0.609 | 8.16 | 0.609 | 0.779 |
| Max | 16700 | 13500 | 16700 | 7750 | 16700 | 3960 |
| n (Samp) | 506 | 182 | 506 | 129 | 506 | 43 |
| n (Patient) | 215 | 182 | 215 | 129 | 215 | 43 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.48 | 0.43 | 0.49 | 0.47 | 0.45 | 0.49 | 0.42 | 0.42 | 0.43 |
| SE | 0.025 | 0.040 | 0.025 | 0.029 | 0.042 | 0.029 | 0.046 | 0.050 | 0.047 |
| p | 0.52 | 0.086 | 0.84 | 0.33 | 0.26 | 0.60 | 0.082 | 0.13 | 0.16 |
| nCohort 1 | 430 | 1121 | 506 | 430 | 1121 | 506 | 430 | 1121 | 506 |
| nCohort 2 | 195 | 58 | 182 | 132 | 51 | 129 | 46 | 36 | 43 |
| Cutoff 1 Sens 1 Spec 1 | 477 | 358 | 466 | 318 | 262 | 360 | 359 | 262' | 359 |
|  | 70% | 71% | 70% | 70% | 71% | 71% | 72% | 72% | 72% |
|  | 31% | 24% | 31% | 23% | 18% | 26% | 25% | 18% | 26% |
| Cutoff 2 Sens 2 Spec 2 | 358 | 231 | 289 | 231 | 113 | 260 | 202 | 167 | 211 |
|  | 80% | 81% | 80% | 80% | 80% | 81% | 80% | 81% | 81% |
|  | 25% | 15% | 21% | 17% | 8% | 20% | 16% | 12% | 16% |
| Cutoff 3 Sens 3 Spec 3 | 170 | 122 | 159 | 103 | 87.4 | 121 | 41.6 | 54.1 | 51.2 |
|  | 90% | 91% | 90% | 90% | 90% | 91% | 91% | 92% | 91% |
|  | 13% | 9% | 13% | 10% | 7% | 10% | 5% | 4% | 6% |
| Cutoff 4 Sens 4 Spec 4 | 1440 | 1420 | 1400 | 1440 | 1420 | 1400 | 1440 | 1420 | 1400 |
|  | 25% | 21% | 26% | 30% | 27% | 29% | 22% | 31% | 21% |
|  | 70% | 70% | 70% | 70% | 70% | 70% | 70% | 70% | 70% |
| Cutoff 5 Sens 5 Spec 5 | 1750 | 1800 | 1720 | 1750 | 1800 | 1720 | 1750 | 1800 | 1720 |
|  | 15% | 12% | 16% | 23% | 10% | 24% | 17% | 19% | 14% |
|  | 80% | 80% | 80% | 80% | 80% | 80% | 80% | 80% | 80% |
| Cutoff 6 Sens 6 Spec 6 | 2430 | 2550 | 2410 | 2430 | 2550 | 2410 | 2430 | 2550 | 2410 |
|  | 9% | 9% | 10% | 11% | 4% | 12% | 9% | 11% | 9% |
|  | 90% | 90% | 90% | 90% | 90% | 90% | 90% | 90% | 90% |
| OR Quart2 p Value 95% CI OR Quart2 | 0.98 | 1.4 | 1.0 | 0.66 | 1.5 | 0.74 | 0.88 | 0.44 | 1.6 |
|  | 0.93 | 0.41 | 1.0 | 0.16 | 0.33 | 0.31 | 0.80 | 0.17 | 0.33 |
|  | 0.60 | 0.62 | 0.61 | 0.37 | 0.68 | 0.42 | 0.33 | 0.13 | 0.61 |
|  | 1.6 | 3.3 | 1.6 | 1.2 | 3.2 | 1.3 | 2.4 | 1.4 | 4.3 |
| OR Quart3 p Value 95% CI of OR Quart3 | 1.9 | 1.6 | 1.6 | 0.89 | 0.63 | 1.2 | 1.9 | 1.2 | 2.0 |
|  | 0.0085 | 0.23 | 0.058 | 0.67 | 0.34 | 0.42 | 0.14 | 0.64 | 0.16 |
|  | 1.2 | 0.73 | 0.98 | 0.51 | 0.24 | 0.73 | 0.80 | 0.50 | 0.76 |
|  | 3.0 | 3.7 | 2.5 | 1.5 | 1.6 | 2.1 | 4.5 | 3.0 | 5.1 |
| OR Quart4 p Value 95% CI ot OR Quart4 | 0.95 | 1.9 | 0.94 | 1.2 | 1.6 | 1.1 | 1.5 | 1.4 | 1.8 |
|  | 0.83 | 0.12 | 0.80 | 0.48 | 0.25 | 0.76 | 0.37 | 0.50 | 0.23 |
|  | 0.58 | 0.84 | 0.57 | 0.71 | 0.73 | 0.63 | 0.62 | 0.56 | 0.69 |
|  | 1.6 | 4.1 | 1.5 | 2.1 | 3.4 | 1.9 | 3.7 | 3.3 | 4.7 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.96E-5 | 2.68E-5 | 2.96E-5 | 3.44E-5 | 2.96E-5 | 2.66E-5 |
| Average | 0.00439 | 0.00352 | 0.00439 | 0.00561 | 0.00439 | 0.00939 |
| Stdev | 0.0252 | 0.0302 | 0.0252 | 0.0463 | 0.0252 | 0.0411 |
| p(t-test) | | 0.73 | | 0.73 | | 0.39 |
| Min | 5.62E-6 | 5.62E-6 | 5.62E-6 | 5.62E-6 | 5.62E-6 | 1.34E-5 |
| Max | 0.260 | 0.388 | 0.260 | 0.470 | 0.260 | 0.193 |
| n (Samp) | 363 | 168 | 363 | 103 | 363 | 22 |
| n (Patient) | 151 | 168 | 151 | 103 | 151 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.83E-5 | 2.83E-5 | 2.68E-5 | 2.83E-5 | 2.63E-5 |
| Average | 0.00440 | 0.00168 | 0.00440 | 0.0109 | 0.00440 | 0.000380 |
| Stdev | 0.0283 | 0.00444 | 0.0283 | 0.0620 | 0.0283 | 0.00166 |
| p(t-test) | | 0.50 | | 0.19 | | 0.48 |
| Min | 5.62E-6 | 5.62E-6 | 5.62E-6 | 5.62E-6 | 5.62E-6 | 5.62E-6 |
| Max | 0.470 | 0.0164 | 0.470 | 0.388 | 0.470 | 0.00836 |
| n (Samp) | 960 | 49 | 960 | 39 | 960 | 25 |
| n (Patient) | 317 | 49 | 317 | 39 | 317 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.68E-5 | 2.83E-5 | 4.13E-5 | 2.83E-5 | 3.20E-5 |
| Average | 0.00568 | 0.00415 | 0.00568 | 0.00569 | 0.00568 | 0.0300 |
| Stdev | 0.0323 | 0.0320 | 0.0323 | 0.0461 | 0.0323 | 0.106 |
| p(t-test) | | 0.61 | | 1.00 | | 0.0033 |
| Min | 5.62E-6 | 5.62E-6 | 5.62E-6 | 5.62E-6 | 5.62E-6 | 1.34E-5 |
| Max | 0.359 | 0.388 | 0.359 | 0.470 | 0.359 | 0.492 |
| n (Samp) | 444 | 155 | 444 | 104 | 444 | 24 |
| n (Patient) | 172 | 155 | 172 | 104 | 172 | 24 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.51 | 0.48 | 0.53 | 0.50 | 0.56 | 0.45 | 0.45 | 0.55 |
| SE | 0.027 | 0.042 | 0.027 | 0.033 | 0.047 | 0.032 | 0.065 | 0.060 | 0.062 |
| p | 0.20 | 0.87 | 0.49 | 0.39 | 0.92 | 0.048 | 0.47 | 0.40 | 0.39 |
| nCohort 1 | 363 | 960 | 444 | 363 | 960 | 444 | 363 | 960 | 444 |
| nCohort 2 | 168 | 49 | 155 | 103 | 39 | 104 | 22 | 25 | 24 |
| Cutoff 1 | 2.17E-5 | 1.92E-5 | 2.17E-5 | 2.46E-5 | 1.92E-5 | 2.46E-5 | 1.88E-5 | 2.17E-5 | 2.17E-5 |
| Sens 1 | 71% | 76% | 72% | 72% | 74% | 71% | 77% | 76% | 71% |
| Spec 1 | 25% | 25% | 27% | 33% | 25% | 35% | 19% | 28% | 27% |
| Cutoff 2 | 1.82E-5 | 1.82E-5 | 1.82E-5 | 1.92E-5 | 1.82E-5 | 1.92E-5 | 1.82E-5 | 1.88E-5 | 1.88E-5 |
| Sens 2 | 86% | 86% | 85% | 81% | 87% | 81% | 86% | 80% | 83% |
| Spec 2 | 16% | 18% | 16% | 23% | 18% | 23% | 16% | 22% | 18% |
| Cutoff 3 | 1.34E-5 | 7.53E-6 | 1.34E-5 | 1.82E-5 | 0 | 1.82E-5 | 1.34E-5 | 5.62E-6 | 1.34E-5 |
| Sens 3 | 93% | 94% | 93% | 91% | 100% | 92% | 91% | 92% | 92% |
| Spec 3 | 10% | 5% | 10% | 16% | 0% | 16% | 10% | 3% | 10% |
| Cutoff 4 | 8.63E-5 | 4.39E-5 | 4.39E-5 | 8.63E-5 | 4.39E-5 | 4.39E-5 | 8.63E-5 | 4.39E-5 | 4.39E-5 |
| Sens 4 | 24% | 31% | 26% | 35% | 36% | 43% | 27% | 20% | 42% |
| Spec 4 | 71% | 72% | 70% | 71% | 72% | 70% | 71% | 72% | 70% |
| Cutoff 5 | 0.000168 | 0.000114 | 0.000114 | 0.000168 | 0.000114 | 0.000114 | 0.000168 | 0.000114 | 0.000114 |
| Sens 5 | 11% | 18% | 17% | 19% | 31% | 27% | 18% | 16% | 33% |
| Spec 5 | 81% | 81% | 80% | 81% | 81% | 80% | 81% | 81% | 80% |
| Cutoff 6 | 0.00442 | 0.00364 | 0.00364 | 0.00442 | 0.00364 | 0.00364 | 0.00442 | 0.00364 | 0.00364 |
| Sens 6 | 8% | 14% | 8% | 8% | 10% | 10% | 9% | 4% | 25% |
| Spec 6 | 90% | 91% | 90% | 90% | 91% | 90% | 90% | 91% | 90% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 0.80 | 1.2 | 0.90 | 0.89 | 1.00 | 0.76 | 0.49 | 0.80 | 0.56 |
| | 0.42 | 0.69 | 0.68 | 0.72 | 0.99 | 0.41 | 0.32 | 0.74 | 0.36 |
| | 0.47 | 0.53 | 0.53 | 0.47 | 0.41 | 0.39 | 0.12 | 0.21 | 0.16 |
| | 1.4 | 2.6 | 1.5 | 1.7 | 2.4 | 1.5 | 2.0. | 3.0 | 2.0 |
| OR Quart 3 p Value 95% CI of OR Quart3 | 1.3 | 1.0 | 1.2 | 1.2 | 0.49 | 1.5 | 0.83 | 2.1 | 0.56 |
| | 0.36 | 1.0 | 0.43 | 0.64 | 0.20 | 0.18 | 0.77 | 0.20 | 0.36 |
| | 0.76 | 0.44 | 0.73 | 0.63 | 0.16 | 0.83 | 0.25 | 0.69 | 0.16 |
| | 2.1 | 2.3 | 2.0 | 2.1 | 1.4 | 2.7 | 2.8 | 6.1 | 2.0 |
| OR Quart 4 p Value 95% CI of OR Quart4 | 1.1 | 0.91 | 1.2 | 1.1 | 1.4 | 1.2 | 1.4 | 1.2 | 1.3 |
| | 0.66 | 0.82 | 0.57 | 0.78 | 0.41 | 0.54 | 0.57 | 0.76 | 0.61 |
| | 0.67 | 0.39 | 0.69 | 0.59 | 0.62 | 0.66 | 0.46 | 0.36 | 0.47 |
| | 1.9 | 2.1 | 1.9 | 2.0 | 3.3 | 2.2 | 4.1 | 4.0 | 3.6 |

EP 3 133 398 B1

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0153 | 0.0125 | 0.0153 | 0.0150 | 0.0153 | 0.00875 |
| Average | 0.113 | 0.157 | 0.113 | 0.196 | 0.113 | 0.234 |
| Stdev | 0.326 | 0.507 | 0.326 | 0.597 | 0.326 | 0.658 |
| p(t-test) | | 0.23 | | 0.064 | | 0.12 |
| Min | 1.14E-9 | 1.40E-9 | 1.14E-9 | 1.14E-9 | 1.14E-9 | 1.40E-9 |
| Max | 3.07 | 3.58 | 3.07 | 4.47 | 3.07 | 2.56 |
| n (Samp) | 363 | 168 | 363 | 103 | 363 | 22 |
| n (Patient) | 151 | 168 | 151 | 103 | 151 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0141 | 0.0212 | 0.0141 | 0.0165 | 0.0141 | 0.00900 |
| Average | 0.160 | 0.225 | 0.160 | 0.237 | 0.160 | 0.218 |
| Stdev | 0.532 | 0.660 | 0.532 | 0.626 | 0.532 | 0.618 |
| p(t-test) | | 0.41 | | 0.38 | | 0.59 |
| Min | 1.14E-9 | 4.16E-6 | 1.14E-9 | 0.000163 | 1.14E-9 | 6.78E-7 |
| Max | 8.92 | 3.42 | 8.92 | 3.58 | 8.92 | 2.56 |
| n (Samp) | 960 | 49 | 960 | 39 | 960 | 25 |
| n (Patient) | 317 | 49 | 317 | 39 | 317 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0157 | 0.0112 | 0.0157 | 0.0152 | 0.0157 | 0.00954 |
| Average | 0.132 | 0.159 | 0.132 | 0.183 | 0.132 | 0.271 |
| Stdev | 0.359 | 0.491 | 0.359 | 0.594 | 0.359 | 0.778 |
| p(t-test) | | 0.46 | | 0.26 | | 0.090 |
| Min | 1.14E-9 | 1.40E-9 | 1.14E-9 | 1.14E-9 | 1.14E-9 | 1.40E-9 |
| Max | 3.07 | 3.58 | 3.07 | 4.47 | 3.07 | 2.96 |
| n (Samp) | 444 | 155 | 444 | 104 | 444 | 24 |
| n (Patient) | 172 | 155 | 172 | 104 | 172 | 24 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.47 | 0.55 | 0.45 | 0.49 | 0.56 | 0.47 | 0.40 | 0.44 | 0.43 |
| SE | 0.027 | 0.043 | 0.027 | 0.032 | 0.049 | 0.032 | 0.066 | 0.060 | 0.062 |
| p | 0.33 | 0.26 | 0.083 | 0.76 | 0.18 | 0.32 | 0.11 | 0.30 | 0.23 |
| nCohort 1 | 363 | 960 | 444 | 363 | 960 | 444 | 363 | 960 | 444 |
| nCohort 2 | 168 | 49 | 155 | 103 | 39 | 104 | 22 | 25 | 24 |
| Cutoff 1 Sens 1 Spec 1 | 0.00513 | 0.00681 | 0.00479 | 0.00401 | 0.00811 | 0.00397 | 0.00605 | 0.00494 | 0.00605 |
|  | 70% | 71% | 70% | 71% | 72% | 70% | 73% | 72% | 71% |
|  | 26% | 32% | 23% | 22% | 35% | 21% | 29% | 25% | 28% |
| Cutoff 2 Sens 2 Spec 2 | 0.00273 | 0.00498 | 0.00273 | 0.00198 | 0.00544 | 0.00198 | 0.00115 | 0.00372 | 0.00115 |
|  | 80% | 82% | 80% | 81% | 82% | 81% | 82% | 80% | 83% |
|  | 17% | 25% | 15% | 12% | 27% | 11% | 8% | 20% | 8% |
| Cutoff 3 Sens 3 Spec 3 | 0.00105 | 0.00219 | 0.00105 | 0.000652 | 0.00108 | 0.000685 | 8.37E-7 | 0.000661 | 8.37E-7 |
|  | 90% | 92% | 90% | 90% | 92% | 90% | 91% | 92% | 92% |
|  | 8% | 13% | 7% | 7% | 7% | 6% | 2% | 6% | 2% |
| Cutoff 4 Sens 4 Spec 4 | 0.0369 | 0.0340 | 0.0438 | 0.0369 | 0.0340 | 0.0438 | 0.0369 | 0.0340 | 0.0438 |
|  | 27% | 37% | 25% | 34% | 44% | 28% | 18% | 24% | 25% |
|  | 70% | 70% | 70% | 70% | 70% | 70% | 70% | 70% | 70% |
| Cutoff 5 Sens 5 Spec 5 | 0.0727 | 0.0744 | 0.110 | 0.0727 | 0.0744 | 0.110 | 0.0727 | 0.0744 | 0.110 |
|  | 18% | 24% | 17% | 25% | 31% | 18% | 18% | 20% | 17% |
|  | 80% | 80% | 80% | 80% | 80% | 80% | 80% | 80% | 80% |
| Cutoff 6 | 0.291 | 0.361 | 0.347 | 0.291 | 0.361 | 0.347 | 0.291 | 0.361 | 0.347 |
| Sens 6 Spec 6 | 12% | 12% | 10% | 16% | 15% | 12% | 14% | 12% | 12% |
|  | 90% | 90% | 90% | 90% | 90% | 90% | 90% | 90% | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 0.83 | 1.1 | 0.93 | 0.71 | 1.4 | 0.95 | 0.24 | 0.60 | 0.32 |
| | 0.50 | 0.82 | 0.78 | 0.28 | 0.47 | 0.88 | 0.21 | 0.48 | 0.17 |
| | 0.49 | 0.46 | 0.54 | 0.38 | 0.54 | 0.52 | 0.027 | 0.14 | 0.064 |
| | 1.4 | 2.6 | 1.6 | 1.3 | 3.8 | 1.8 | 2.2 | 2.5 | 1.6 |
| OR Quart 3 p Value 95% CI of OR Quart3 | 1.2 | 1.3 | 1.2 | 0.59 | 1.00 | 0.65 | 3.0 | 2.1 | 1.5 |
| | 0.51 | 0.52 | 0.59 | 0.11 | 0.99 | 0.19 | 0.068 | 0.20 | 0.43 |
| | 0.71 | 0.57 | 0.68 | 0.31 | 0.34 | 0.34 | 0.92 | 0.69 | 0.53 |
| | 2.0 | 3.1 | 2.0 | 1.1 | 2.9 | 1.2 | 9.8 | 6.1 | 4.5 |
| OR Quart 4 p Value 95% CI of OR Quart4 | 1.2 | 1.5 | 1.6 | 1.2 | 2.2 | 1.5 | 1.6 | 1.4 | 1.2 |
| | 0.57 | 0.31 | 3.087 | 0.53 | 0.090 | 0.19 | 0.51 | 0.56 | 0.78 |
| | 0.69 | 0.67 | 0.94 | 0.67 | 0.88 | 0.82 | 0.42 | 0.44 | 0.38 |
| | 1.9 | 3.5 | 2.6 | 2.2 | 5.5 | 2.6 | 5.7 | 4.5 | 3.6 |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 134 | 130 | 134 | 159 | 134 | 141 |
| Average | 140 | 141 | 140 | 160 | 140 | 159 |
| Stdev | 52.5 | 60.1 | 52.5 | 71.7 | 52.5 | 64.0 |
| p(t-test) | | 0.81 | | 0.0056 | | 0.061 |
| Min | 31.0 | 40.9 | 31.0 | 52.1 | 31.0 | 67.2 |
| Max | 311 | 360 | 311 | 444 | 311 | 312 |
| n (Samp) | 250 | 131 | 250 | 95 | 250 | 32 |
| n (Patient) | 145 | 131 | 145 | 95 | 145 | 32 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 132 | 126 | 132 | 164 | 132 | 137 |
| Average | 142 | 137 | 142 | 155 | 142 | 142 |
| Stdev | 60.2 | 51.4 | 60.2 | 57.1 | 60.2 | 53.3 |
| p(t-test) | | 0.67 | | 0.19 | | 1.00 |
| Min | 31.0 | 47.9 | 31.0 | 55.2 | 31.0 | 64.6 |
| Max | 444 | 249 | 444 | 287 | 444 | 241 |
| n (Samp) | 619 | 37 | 619 | 35 | 619 | 21 |
| n (Patient) | 284 | 37 | 284 | 35 | 284 | 21 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 130 | 140 | 130 | 162 | 130 | 162 |
| Average | 136 | 146 | 136 | 162 | 136 | 168 |
| Stdev | 51.8 | 62.2 | 51.8 | 72.8 | 51.8 | 65.3 |
| p(t-test) | | 0.11 | | 2.7E-4 | | 0.0019 |
| Min | 31.0 | 40.9 | 31.0 | 52.1 | 31.0 | 67.2 |
| Max | 311 | 360 | 311 | 444 | 311 | 312 |
| n (Samp) | 291 | 119 | 291 | 91 | 291 | 29 |
| n (Patient) | 149 | 119 | 149 | 91 | 149 | 29 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | 0.49 | 0.53 | 0.57 | 0.58 | 0.60 | 0.58 | 0.51 | 0.64 |
| SE | 0.031 | 0.049 | 0.032 | 0.035 | 0.052 | 0.035 | 0.056 | 0.065 | 0.058 |
| p | 0.75 | 0.81 | 0.29 | 0.048 | 0.13 | 0.0065 | 0.17 | 0.84 | 0.015 |
| nCohort 1 | 250 | 619 | 291 | 250 | 619 | 291 | 250 | 619 | 291 |
| nCohort 2 | 131 | 37 | 119 | 95 | 35 | 91 | 32 | 21 | 29 |
| Cutoff 1 Sens 1 Spec 1 | 105 | 109 | 105 | 107 | 116 | 111 | 122 | 99.6 | 131 |
| | 70% | 70% | 71% | 71% | 71% | 70% | 72% | 71% | 72% |
| | 28% | 35% | 33% | 30% | 39% | 36% | 40% | 29% | 52% |
| Cutoff 2 Sens 2 Spec 2 | 86.4 | 95.7 | 85.9 | 88.0 | 99.6 | 87.8 | 97.1 | 97.5 | 90.2 |
| | 80% | 81% | 81% | 80% | 80% | 80% | 81% | 81% | 83% |
| | 16% | 26% | 17% | 18% | 29% | 19% | 23% | 28% | 21% |
| Cutoff 3 Sens 3 Spec 3 | 76.2 | 76.7 | 76.5 | 74.7 | 80.9 | 74.0 | 88.0 | 76.2 | 77.4 |
| | 90% | 92% | 91% | 91% | 91% | 90% | 91% | 90% | 93% |
| | 9% | 12% | 10% | 8% | 15% | 8% | 18% | 11% | 11% |
| Cutoff 4 Sens 4 Spec 4 | 162 | 166 | 161 | 162 | 166 | 161 | 162 | 166 | 161 |
| | 32% | 24% | 36% | 48% | 49% | 51% | 41% | 38% | 52% |
| | 70% | 70% | 70% | 70% | 70% | 70% | 70% | 70% | 70% |
| Cutoff 5 Sens 5 Spec 5 | 183 | 190 | 180 | 183 | 190 | 180 | 183 | 190 | 180 |
| | 21% | 16% | 25% | 33% | 29% | 35% | 31% | 24% | 45% |
| | 80% | 80% | 80% | 80% | 80% | 80% | 80% | 80% | 80% |
| Cutoff 6 Sens 6 Spec 6 | 203 | 225 | 203 | 203 | 225 | 203 | 203 | 225 | 203 |
| | 14% | 11% | 16% | 24% | 9% | 26% | 25% | 5% | 31% |
| | 90% | 90% | 90% | 90% | 90% | 90% | 90% | 90% | 90% |
| OR Quart 2 p Value 95% CI of OR Quart2 | 0.66 | 1.3 | 0.77 | 0.45 | 0.99 | 0.60 | 0.59 | 1.5 | 0.48 |
| | 0.18 | 0.61 | 0.41 | 0.032 | 0.99 | 0.18 | 0.37 | 0.52 | 0.31 |
| | 0.36 | 0.47 | 0.42 | 0.22 | 0.34 | 0.29 | 0.18 | 0.42 | 0.12 |
| | 1.2 | 3.6 | 1.4 | 0.94 | 2.9 | 1.3 | 1.9 | 5.5 | 2.0 |
| OR Quart 3 | 0.85 | 2.1 | 0.70 | 0.84 | 1.2 | 1.0 | 0.73 | 1.3 | 1.0 |
| | 0.58 | 0.12 | 0.27 | 0.61 | 0.79 | 1.0 | 0.57 | 0.74 | 1.0 |
| p Value 95% CI of OR Quart3 | 0.47 | 0.82 | 0.38 | 0.43 | 0.41 | 0.50 | 0.24 | 0.33 | 0.31 |
| | 1.5 | 5.3 | 1.3 | 1.6 | 3.2 | 2.0 | 2.2 | 4.8 | 3.2 |
| OR Quart 4 p Value 95% CI of OR Quart4 | 1.0 | 1.0 | 1.3 | 1.3 | 1.9 | 2.0 | 1.7 | 1.5 | 2.6 |
| | 0.96 | 1.0 | 0.33 | 0.36 | 0.18 | 0.031 | 0.25 | 0.52 | 0.063 |
| | 0.57 | 0.34 | 0.75 | 0.71 | 0.75 | 1.1 | 0.67 | 0.42 | 0.95 |
| | 1.8 | 2.9 | 2.4 | 2.5 | 4.9 | 3.8 | 4.5 | 5.5 | 7.2 |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1150 | 1450 | 1150 | 1130 | 1150 | 927 |
| Average | 1960 | 2200 | 1960 | 2870 | 1960 | 1940 |
| Stdev | 2780 | 2360 | 2780 | 7500 | 2780 | 3240 |
| p(t-test) | | 0.29 | | 0.035 | | 0.97 |
| Min | 33.0 | 47.5 | 33.0 | 23.5 | 33.0 | 105 |
| Max | 33000 | 21300 | 33000 | 68100 | 33000 | 19700 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1380 | 1460 | 1380 | 1250 | 1380 | 906 |
| Average | 2390 | 2200 | 2390 | 1670 | 2390 | 1470 |
| Stdev | 3860 | 2030 | 3860 | 1630 | 3860 | 1630 |
| p(t-test) | | 0.70 | | 0.18 | | 0.16 |
| Min | 0.0376 | 47.5 | 0.0376 | 23.5 | 0.0376 | 160 |
| Max | 68100 | 8500 | 68100 | 8210 | 68100 | 7210 |
| n (Samp) | 1121 | 58 | 1121 | 51 | 1121 | 36 |
| n (Patient) | 395 | 58 | 395 | 51 | 395 | 36 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1250 | 1470 | 1250 | 1170 | 1250 | 1080 |
| Average | 2040 | 2280 | 2040 | 3010 | 2040 | 2270 |
| Stdev | 2810 | 2400 | 2810 | 7590 | 2810 | 3520 |
| p(t-test) | | 0.29 | | 0.021 | | 0.61 |
| Min | 13.4 | 122 | 13.4 | 99.8 | 13.4 | 105 |
| Max | 33000 | 21300 | 33000 | 68100 | 33000 | 19700 |
| n (Samp) | 506 | 182 | 506 | 129 | 506 | 43 |
| n (Patient) | 215 | 182 | 215 | 129 | 215 | 43 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | 0.53 | 0.56 | 0.51 | 0.46 | 0.51 | 0.46 | 0.40 | 0.48 |
| SE | 0.025 | 0.039 | 0.025 | 0.029 | 0.042 | 0.029 | 0.046 | 0.051 | 0.046 |
| p | 0.015 | 0.47 | 0.017 | 0.81 | 0.35 | 0.83 | 0.40 | 0.041 | 0.67 |
| nCohort 1 | 430 | 1121 | 506 | 430 | 1121 | 506 | 430 | 1121 | 506 |
| nCohort 2 | 195 | 58 | 182 | 132 | 51 | 129 | 46 | 36 | 43 |
| Cutoff 1 Sens 1 Spec 1 | 843 | 999 | 891 | 655 | 914 | 671 | 525 | 589 | 575 |
| | 70% | 71% | 70% | 70% | 71% | 71% | 72% | 72% | 72% |
| | 37% | 38% | 37% | 29% | 35% | 27% | 23% | 23% | 22% |
| Cutoff 2 Sens 2 Spec 2 | 557 | 546 | 619 | 489 | 435 | 502 | 450 | 409 | 450 |
| | 80% | 81% | 80% | 80% | 80% | 81% | 80% | 81% | 81% |
| | 23% | 21% | 25% | 21% | 16% | 20% | 20% | 16% | 19% |
| Cutoff 3 Sens 3 Spec 3 | 314 | 217 | 348 | 362 | 171 | 401 | 275 | 272 | 277 |
| | 90% | 91% | 90% | 90% | 92% | 91% | 91% | 92% | 91% |
| | 13% | 6% | 14% | 16% | 5% | 18% | 12% | 8% | 11% |
| Cutoff 4 Sens 4 Spec 4 | 1870 | 2320 | 2010 | 1870 | 2320 | 2010 | 1870 | 2320 | 2010 |
| | 42% | 33% | 40% | 34% | 16% | 33% | 28% | 17% | 33% |
| | 70% | 70% | 70% | 70% | 70% | 70% | 70% | 70% | 70% |
| Cutoff 5 Sens 5 Spec 5 | 2630 | 3200 | 2780 | 2630 | 3200 | 2780 | 2630 | 3200 | 2780 |
| | 28% | 24% | 29% | 23% | 14% | 24% | 17% | 14% | 19% |
| | 80% | 80% | 80% | 80% | 80% | 80% | 80% | 80% | 80% |
| Cutoff 6 Sens 6 Spec 6 | 4290 | 5100 | 4310 | 4290 | 5100 | 4310 | 4290 | 5100 | 4310 |
| | 15% | 9% | 15% | 12% | 6% | 15% | 9% | 6% | 12% |
| | 90% | 90% | 90% | 90% | 90% | 90% | 90% | 90% | 90% |
| OR Quart 2 p Value 95% CI of OR Quart2 | 0.94 | 1.1 | 1.2 | 0.85 | 1.8 | 1.1 | 0.89 | 0.83 | 0.62 |
| | 0.80 | 0.84 | 0.38 | 0.55 | 0.20 | 0.70 | 0.81 | 0.77 | 0.34 |
| | 0.57 | 0.49 | 0.76 | 0.49 | 0.74 | 0.65 | 0.35 | 0.25 | 0.23 |
| | 1.5 | 2.4 | 2.1 | 1.5 | 4.3 | 1.9 | 2.3 | 2.8 | 1.7 |
| OR Quart 3 p Value 95% CI of OR Quart3 | 1.2 | 1.3 | 1.1 | 0.85 | 2.3 | 0.84 | 1.2 | 2.1 | 1.1 |
| | 0.39 | 0.45 | 0.70 | 0.57 | 0.051 | 0.55 | 0.65 | 0.16 | 0.81 |
| | 0.76 | 0.63 | 0.67 | 0.49 | 1.00 | 0.48 | 0.51 | 0.76 | 0.47 |
| | 2.0 | 2.9 | 1.8 | 1.5 | 5.5 | 1.5 | 2.9 | 5.5 | 2.6 |
| OR Quart 4 p Value | 1.7 | 1.4 | 1.8 | 0.99 | 1.4 | 1.1 | 1.6 | 2.2 | 1.2 |
| | 0.035 | 0.35 | 0.017 | 0.97 | 0.49 | 0.80 | 0.29 | 0.11 | 0.66 |
| | 1.0 | 0.67 | 1.1 | 0.58 | 0.55 | 0.62 | 0.68 | 0.84 | 0.52 |
| 95% CI of OR Quart4 | 2.7 | 3.1 | 2.9 | 1.7 | 3.5 | 1.8 | 3.7 | 5.9 | 2.8 |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.36 | 10.6 | 9.36 | 10.9 | 9.36 | 16.5 |
| Average | 14.9 | 17.8 | 14.9 | 99.7 | 14.9 | 24.7 |
| Stdev | 34.0 | 22.7 | 34.0 | 743 | 34.0 | 53.5 |
| p(t-test) | | 0.37 | | 0.063 | | 0.14 |
| Min | 0.00398 | 0.00398 | 0.00398 | 0.00398 | 0.00398 | 0.0184 |
| Max | 470 | 134 | 470 | 7540 | 470 | 317 |
| n (Samp) | 268 | 138 | 268 | 104 | 268 | 35 |
| n (Patient) | 148 | 138 | 148 | 104 | 148 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.7 | 6.88 | 10.7 | 11.2 | 10.7 | 4.09 |
| Average | 29.4 | 11.0 | 29.4 | 40.6 | 29.4 | 11.8 |
| Stdev | 295 | 12.7 | 295 | 154 | 295 | 13.6 |
| p(t-test) | | 0.70 | | 0.82 | | 0.77 |
| Min | 0.00398 | 0.0132 | 0.00398 | 0.00398 | 0.00398 | 0.0184 |
| Max | 7540 | 50.9 | 7540 | 945 | 7540 | 44.4 |
| n (Samp) | 663 | 38 | 663 | 37 | 663 | 23 |
| n (Patient) | 288 | 38 | 288 | 37 | 288 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.36 | 10.8 | 9.36 | 9.71 | 9.36 | 16.8 |
| Average | 17.9 | 19.0 | 17.9 | 93.1 | 17.9 | 31.5 |
| Stdev | 61.5 | 24.0 | 61.5 | 749 | 61.5 | 60.0 |
| p(t-test) | | 0.85 | | 0.079 | | 0.24 |
| Min | 0.00398 | 0.00398 | 0.00398 | 0.00398 | 0.00398 | 0.0184 |
| Max | 945 | 134 | 945 | 7540 | 945 | 317 |
| n (Samp) | 313 | 127 | 313 | 101 | 313 | 32 |
| n (Patient) | 152 | 127 | 152 | 101 | 152 | 32 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.47 | 0.55 | 0.56 | 0.55 | 0.54 | 0.62 | 0.47 | 0.62 |
| SE | 0.030 | 0.049 | 0.031 | 0.034 | 0.050 | 0.033 | 0.053 | 0.062 | 0.055 |
| p | 0.13 | 0.49 | 0.14 | 0.068 | 0.36 | 0.19 | 0.027 | 0.67 | 0.032 |
| nCohort 1 | 268 | 663 | 313 | 268 | 663 | 313 | 268 | 663 | 313 |
| nCohort 2 | 138 | 38 | 127 | 104 | 37 | 101 | 35 | 23 | 32 |
| Cutoff 1 Sens 1 Spec 1 | 0.533 | 0.338 | 0.338 | 1.39 | 0.338 | 0.338 | 0.452 | 0.178 | 3.21 |
| | 70% | 71% | 73% | 70% | 76% | 71% | 71% | 78% | 72% |
| | 32% | 28% | 29% | 35% | 28% | 29% | 31% | 26% | 37% |
| Cutoff 2 Sens 2 Spec 2 | 0.0840 | 0.117 | 0.0748 | 0.103 | 0.315 | 0.0840 | 0.338 | 0.167 | 0.315 |
| | 80% | 82% | 80% | 83% | 84% | 80% | 80% | 83% | 84% |
| | 22% | 23% | 19% | 24% | 26% | 22% | 29% | 24% | 28% |
| Cutoff 3 Sens 3 Spec 3 | 0.0184 | 0.0187 | 0.0179 | 0.0187 | 0.177 | 0.0187 | 0.103 | 0.0179 | 0.0840 |
| | 91% | 97% | 91% | 91% | 92% | 91% | 91% | 100% | 91% |
| | 7% | 11% | 7% | 9% | 24% | 10% | 24% | 9% | 22% |
| Cutoff 4 Sens 4 Spec 4 | 15.6 | 18.2 | 16.5 | 15.6 | 18.2 | 16.5 | 15.6 | 18.2 | 16.5 |
| | 40% | 21% | 39% | 42% | 46% | 42% | 51% | 35% | 50% |
| | 70% | 70% | 71% | 70% | 70% | 71% | 70% | 70% | 71% |
| Cutoff 5 Sens 5 Spec 5 | 20.7 | 25.9 | 21.9 | 20.7 | 25.9 | 21.9 | 20.7 | 25.9 | 21.9 |
| | 31% | 11% | 32% | 31% | 27% | 30% | 34% | 17% | 28% |
| | 80% | 80% | 80% | 80% | 80% | 80% | 80% | 80% | 80% |
| Cutoff 6 Sens 6 Spec 6 | 30.7 | 39.8 | 31.2 | 30.7 | 39.8 | 31.2 | 30.7 | 39.8 | 31.2 |
| | 22% | 5% | 24% | 18% | 11% | 21% | 14% | 4% | 22% |
| | 90% | 90% | 90% | 90% | 90% | 90% | 90% | 90% | 90% |
| OR Quart 2 p Value 95% CI of OR Quart2 | 0.86 | 1.2 | 1.0 | 1.7 | 2.7 | 1.2 | 3.2 | 0.83 | 1.2 |
| | 0.62 | 0.78 | 1.0 | 0.100 | 0.062 | 0.65 | 0.089 | 0.77 | 0.76 |
| | 0.47 | 0.41 | 0.55 | 0.90 | 0.95 | 0.61 | 0.84 | 0.25 | 0.36 |
| | 1.6 | 3.3 | 1.8 | 3.4 | 7.8 | 2.2 | 12 | 2.8 | 4.1 |
| OR Quart 3 p Value 95% CI of OR Quart3 | 0.79 | 1.9 | 0.95 | 0.76 | 1.4 | 0.74 | 2.8 | 1.2 | 1.9 |
| | 0.44 | 0.17 | 0.88 | 0.46 | 0.56 | 0.38 | 0.14 | 0.78 | 0.27 |
| | 0.43 | 0.75 | 0.52 | 0.37 | 0.44 | 0.37 | 0.72 | 0.39 | 0.61 |
| | 1.4 | 5.0 | 1.7 | 1.6 | 4.6 | 1.5 | 11 | 3.6 | 5.9 |
| OR Quart 4 p Value 95% CI of OR Quart4 | 1.8 | 1.5 | 1.7 | 2.5 | 2.5 | 1.7 | 5.9 | 0.83 | 2.6 |
| | 0.052 | 0.45 | 0.084 | 0.0049 | 0.091 | 0.097 | 0.0068 | 0.77 | 0.087 |
| | 1.00 | 0.54 | 0.94 | 1.3 | 0.86 | 0.91 | 1.6 | 0.25 | 0.87 |
| | 3.1 | 3.9 | 2.9 | 4.8 | 7.3 | 3.1 | 21 | 2.8 | 7.7 |

Fig. 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

**Angiogenin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5340 | 10900 | 5340 | 9120 | 5340 | 5200 |
| Average | 8920 | 12500 | 8920 | 11000 | 8920 | 9060 |
| Stdev | 8290 | 8910 | 8290 | 8520 | 8290 | 8710 |
| p(t-test) | | 6.5E-5 | | 0.018 | | 0.90 |
| Min | 0.00873 | 75.9 | 0.00873 | 303 | 0.00873 | 54.6 |
| Max | 30600 | 30600 | 30600 | 30600 | 30600 | 30600 |
| n (Samp) | 895 | 99 | 895 | 102 | 895 | 57 |
| n (Patient) | 374 | 99 | 374 | 102 | 374 | 57 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6050 | 12100 | 6050 | 14400 | 6050 | 11200 |
| Average | 9720 | 12000 | 9720 | 13300 | 9720 | 11700 |
| Stdev | 8640 | 8770 | 8640 | 9680 | 8640 | 8390 |
| p(t-test) | | 0.24 | | 0.035 | | 0.25 |
| Min | 0.00873 | 110 | 0.00873 | 772 | 0.00873 | 174 |
| Max | 30600 | 27800 | 30600 | 30600 | 30600 | 30600 |
| n (Samp) | 1357 | 20 | 1357 | 26 | 1357 | 25 |
| n (Patient) | 470 | 20 | 470 | 26 | 470 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5710 | 10900 | 5710 | 8180 | 5710 | 4400 |
| Average | 9470 | 12500 | 9470 | 10200 | 9470 | 8210 |
| Stdev | 8660 | 9100 | 8660 | 7920 | 8660 | 8360 |
| p(t-test) | | 0.0017 | | 0.46 | | 0.32 |
| Min | 0.00873 | 75.9 | 0.00873 | 303 | 0.00873 | 54.6 |
| Max | 30600 | 30600 | 30600 | 30600 | 30600 | 27300 |
| n (Samp) | 962 | 91 | 962 | 94 | 962 | 49 |
| n (Patient) | 367 | 91 | 367 | 94 | 367 | 49 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.62 | 0.57 | 0.60 | 0.59 | 0.60 | 0.55 | 0.50 | 0.58 | 0.45 |
| SE | 0.031 | 0.067 | 0.033 | 0.031 | 0.059 | 0.032 | 0.040 | 0.060 | 0.043 |
| p | 9.1E-5 | 0.28 | 0.0015 | 0.0051 | 0.078 | 0.12 | 0.95 | 0.19 | 0.28 |
| nCohort 1 | 895 | 1357 | 962 | 895 | 1357 | 962 | 895 | 1357 | 962 |
| nCohort 2 | 99 | 20 | 91 | 102 | 26 | 94 | 57 | 25 | 49 |
| Cutoff 1 | 5290 | 4780 | 4920 | 4180 | 4300 | 4180 | 2200 | 6020 | 2120 |
| Sens 1 | 71% | 70% | 70% | 71% | 73% | 70% | 70% | 72% | 71% |
| Spec 1 | 49% | 43% | 46% | 43% | 40% | 42% | 24% | 50% | 22% |
| Cutoff 2 | 3820 | 3870 | 4020 | 2940 | 3510 | 2820 | 1690 | 3240 | 1610 |
| Sens 2 | 81% | 80% | 80% | 80% | 81% | 81% | 81% | 80% | 82% |
| Spec 2 | 39% | 36% | 39% | 31% | 33% | 28% | 18% | 31% | 16% |
| Cutoff 3 | 1110 | 1110 | 1440 | 2230 | 1440 | 2000 | 989 | 1680 | 989 |
| Sens 3 | 91% | 90% | 90% | 90% | 92% | 90% | 91% | 92% | 92% |
| Spec 3 | 12% | 10% | 15% | 24% | 13% | 21% | 10% | 16% | 9% |
| Cutoff 4 | 12000 | 14500 | 14000 | 12000 | 14500 | 14000 | 12000 | 14500 | 14000 |
| Sens 4 | 47% | 45% | 42% | 38% | 50% | 28% | 32% | 40% | 24% |
| Spec 4 | 70% | 70% | 70% | 70% | 70% | 70% | 70% | 70% | 70% |
| Cutoff 5 | 18100 | 19700 | 19100 | 18100 | 19700 | 19100 | 18100 | 19700 | 19100 |
| Sens 5 | 36% | 25% | 36% | 23% | 35% | 16% | 21% | 28% | 20% |
| Spec 5 | 80% | 80% | 80% | 80% | 80% | 80% | 80% | 80% | 80% |
| Cutoff 6 | 20400 | 22100 | 22200 | 20400 | 22100 | 22200 | 20400 | 22100 | 22200 |
| Sens 6 | 17% | 10% | 13% | 11% | 15% | 7% | 11% | 8% | 8% |
| Spec 6 | 91% | 90% | 90% | 91% | 90% | 90% | 91% | 90% | 90% |
| OR Quart 2 | 1.1 | 0.75 | 1.2 | 2.3 | 1.00 | 1.5 | 0.86 | 1.00 | 0.72 |
|  | 0.87 | 0.70 | 0.59 | 0.017 | 1.00 | 0.25 | 0.70 | 1.00 | 0.48 |
| p Value 95% CI ot OR Quart2 | 0.52 | 0.17 | 0.60 | 1.2 | 0.29 | 0.76 | 0.40 | 0.25 | 0.28 |
|  | 2.2 | 3.4 | 2.5 | 4.6 | 3.5 | 2.9 | 1.8 | 4.0 | 1.8 |
| OR Quart 3 | 1.8 | 1.3 | 1.7 | 2.4 | 1.2 | 2.1 | 0.72 | 2.5 | 1.2 |
|  | 0.082 | 0.74 | 0.14 | 0.012 | 0.77 | 0.017 | 0.42 | 0.12 | 0.68 |
| p Value 95% CI of OR Quart3 | 0.93 | 0.33 | 0.85 | 1.2 | 0.36 | 1.1 | 0.32 | 0.79 | 0.52 |
|  | 3.4 | 4.7 | 3.2 | 4.7 | 4.0 | 4.0 | 1.6 | 8.2 | 2.7 |
| OR Quart 4 | 2.7 | 2.0 | 2.4 | 2.7 | 2.0 | 1.5 | 1.2 | 1.8 | 1.6 |
|  | 0.0015 | 0.26 | 0.0057 | 0.0042 | 0.20 | 0.25 | 0.59 | 0.37 | 0.24 |
| p Value 95% CI OR Quart4 | 1.5 | 0.60 | 1.3 | 1.4 | 0.68 | 0.76 | 0.60 | 0.51 | 0.73 |
|  | 5.0 | 6.8 | 4.6 | 5.2 | 6.0 | 2.9 | 2.5 | 6.1 | 3.5 |

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11.6 | 14.5 | 11.6 | 14.1 | 11.6 | 9.57 |
| Average | 41.8 | 50.7 | 41.8 | 76.3 | 41.8 | 55.9 |
| Stdev | 94.7 | 102 | 94.7 | 179 | 94.7 | 149 |
| p(t-test) | | 0.43 | | 0.0045 | | 0.36 |
| Min | 0.000466 | 1.68 | 0.000466 | 1.61 | 0.000466 | 0.794 |
| Max | 789 | 647 | 789 | 1370 | 789 | 878 |
| n (Samp) | 754 | 78 | 754 | 86 | 754 | 42 |
| n (Patient) | 298 | 78 | 298 | 86 | 298 | 42 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.6 | 27.8 | 12.6 | 18.2 | 12.6 | 13.5 |
| Average | 45.4 | 116 | 45.4 | 86.3 | 45.4 | 103 |
| Stdev | 107 | 182 | 107 | 117 | 107 | 218 |
| p(t-test) | | 0.0067 | | 0.072 | | 0.028 |
| Min | 0.000466 | 3.69 | 0.000466 | 2.98 | 0.000466 | 2.01 |
| Max | 1370 | 647 | 1370 | 413 | 1370 | 878 |
| n (Samp) | 1164 | 18 | 1164 | 23 | 1164 | 18 |
| n (Patient) | 379 | 18 | 379 | 23 | 379 | 18 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.0 | 13.8 | 12.0 | 13.4 | 12.0 | 9.31 |
| Average | 48.1 | 35.6 | 48.1 | 70.6 | 48.1 | 38.0 |
| Stdev | 104 | 51.2 | 104 | 182 | 104 | 76.2 |
| p(t-test) | | 0.32 | | 0.089 | | 0.57 |
| Min | 0.000466 | 1.68 | 0.000466 | 0.000466 | 0.000466 | 0.794 |
| Max | 878 | 301 | 878 | 1370 | 878 | 400 |
| n (Samp) | 838 | 70 | 838 | 80 | 838 | 36 |
| n (Patient) | 306 | 70 | 306 | 80 | 306 | 36 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.67 | 0.53 | 0.56 | 0.65 | 0.52 | 0.50 | 0.54 | 0.47 |
| SE | 0.035 | 0.071 | 0.036 | 0.034 | 0.063 | 0.034 | 0.046 | 0.070 | 0.050 |
| p | 0.048 | 0.018 | 0.39 | 0.078 | 0.019 | 0.51 | 0.92 | 0.60 | 0.50 |
| nCohort 1 | 754 | 1164 | 838 | 754 | 1164 | 838 | 754 | 1164 | 838 |
| nCohort 2 | 78 | 18 | 70 | 86 | 23 | 80 | 42 | 18 | 36 |
| Cutoff 1 | 8.30 | 14.8 | 8.19 | 7.57 | 14.0 | 7.57 | 7.08 | 7.57 | 6.86 |
| Sens 1 | 71% | 72% | 70% | 71% | 74% | 70% | 71% | 72% | 72% |
| Spec 1 | 38% | 55% | 36% | 35% | 53% | 34% | 32% | 31% | 31% |
| Cutoff 2 | 6.34 | 7.15 | 5.86 | 5.80 | 10.8 | 5.34 | 5.18 | 5.18 | 4.93 |
| Sens 2 | 81% | 83% | 80% | 80% | 83% | 80% | 81% | 83% | 81% |
| Spec 2 | 29% | 29% | 25% | 26% | 43% | 23% | 24% | 21% | 21% |
| Cutoff 3 | 3.72 | 4.54 | 3.66 | 3.39 | 6.96 | 3.28 | 3.66 | 2.62 | 3.49 |
| Sens 3 | 91% | 94% | 90% | 91% | 91% | 90% | 90% | 94% | 92% |
| Spec 3 | 15% | 18% | 14% | 14% | 28% | 12% | 15% | 8% | 13% |
| Cutoff 4 | 22.1 | 24.4 | 23.7 | 22.1 | 24.4 | 23.7 | 22.1 | 24.4 | 23.7 |
| Sens 4 | 38% | 56% | 33% | 35% | 39% | 32% | 26% | 33% | 28% |
| Spec 4 | 70% | 70% | 70% | 70% | 70% | 70% | 70% | 70% | 70% |
| Cutoff 5 | 37.2 | 44.0 | 43.1 | 37.2 | 44.0 | 43.1 | 37.2 | 44.0 | 43.1 |
| Sens 5 | 31% | 39% | 27% | 30% | 39% | 26% | 21% | 28% | 22% |
| Spec 5 | 80% | 80% | 80% | 80% | 80% | 80% | 80% | 80% | 80% |
| Cutoff 6 Sens 6 Spec 6 | 93.7 | 99.5 | 133 | 93.7 | 99.5 | 133 | 93.7 | 99.5 | 133 |
| | 14% | 28% | 7% | 19% | 30% | 15% | 12% | 28% | 8% |
| | 90% | 90% | 90% | 90% | 90% | 90% | 90% | 90% | 90% |
| OR Quart 2 | 1.5 | 1.00 | 1.4 | 1.3 | 2.0 | 0.94 | 0.79 | 1.2 | 0.55 |
| | 0.29 | 1.00 | 0.37 | 0.50 | 0.42 | 0.85 | 0.63 | 0.74 | 0.29 |
| p Value 95% CI of OR Quart2 | 0.72 | 0.14 | 0.68 | 0.65 | 0.36 | 0.48 | 0.31 | 0.33 | 0.18 |
| | 3.0 | 7.1 | 2.8 | 2.5 | 11 | 1.8 | 2.0 | 4.7 | 1.7 |
| OR Quart 3 | 1.4 | 3.6 | 1.1 | 1.3 | 4.1 | 1.1 | 1.3 | 0.75 | 1.4 |
| | 0.37 | 0.12 | 0.85 | 0.50 | 0.077 | 0.87 | 0.52 | 0.70 | 0.50 |
| p Value 95% CI of OR Quart3 | 0.68 | 0.73 | 0.51 | 0.65 | 0.86 | 0.55 | 0.57 | 0.17 | 0.56 |
| | 2.9 | 17 | 2.3 | 2.5 | 19 | 2.0 | 3.1 | 3.4 | 3.3 |
| OR Quart 4 | 1.9 | 3.5 | 1.6 | 1.7 | 4.6 | 1.2 | 1.1 | 1.5 | 1.1 |
| | 0.068 | 0.12 | 0.17 | 0.11 | 0.052 | 0.53 | 0.82 | 0.53 | 0.81 |
| p Value 95% CI of OR Quart4 | 0.95 | 0.73 | 0.81 | 0.88 | 0.98 | 0.65 | 0.46 | 0.42 | 0.45 |
| | 3.8 | 17 | 3.3 | 3.2 | 21 | 2.3 | 2.7 | 5.4 | 2.8 |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.2 | 27.7 | 23.2 | 35.1 | 23.2 | 31.2 |
| Average | 54.1 | 48.4 | 54.1 | 167 | 54.1 | 73.3 |
| Stdev | 141 | 61.9 | 141 | 527 | 141 | 176 |
| p(t-test) | | 0.74 | | 2.8E-5 | | 0.42 |
| Min | 0.00401 | 1.75 | 0.00401 | 1.75 | 0.00401 | 0.00413 |
| Max | 1640 | 300 | 1640 | 3480 | 1640 | 1070 |
| n (Samp) | 597 | 69 | 597 | 76 | 597 | 38 |
| n (Patient) | 279 | 69 | 279 | 76 | 279 | 38 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.7 | 64.2 | 24.7 | 42.6 | 24.7 | 66.6 |
| Average | 59.4 | 91.9 | 59.4 | 165 | 59.4 | 94.2 |
| Stdev | 165 | 88.9 | 165 | 403 | 165 | 80.8 |
| p(t-test) | | 0.55 | | 0.012 | | 0.43 |
| sCr only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Min | 0.00131 | 22.5 | 0.00131 | 6.70 | 0.00131 | 9.39 |
| Max | 2190 | 289 | 2190 | 1710 | 2190 | 246 |
| n (Samp) | 827 | 9 | 827 | 17 | 827 | 14 |
| n (Patient) | 352 | 9 | 352 | 17 | 352 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.6 | 27.7 | 24.6 | 33.2 | 24.6 | 26.6 |
| Average | 51.6 | 44.1 | 51.6 | 150 | 51.6 | 121 |
| Stdev | 125 | 55.6 | 125 | 510 | 125 | 331 |
| p(t-test) | | 0.63 | | 1.2E-4 | | 0.0059 |
| Min | 0.00131 | 1.75 | 0.00131 | 1.75 | 0.00131 | 0.00413 |
| Max | 1640 | 300 | 1640 | 3480 | 1640 | 1710 |
| n (Samp) | 604 | 66 | 604 | 72 | 604 | 35 |
| n (Patient) | 263 | 66 | 263 | 72 | 263 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.76 | 0.51 | 0.60 | 0.69 | 0.58 | 0.54 | 0.74 | 0.53 |
| SE | 0.037 | 0.093 | 0.038 | 0.036 | 0.072 | 0.037 | 0.049 | 0.077 | 0.051 |
| p | 0.24 | 0.0047 | 0.69 | 0.0080 | 0.0072 | 0.037 | 0.42 | 0.0014 | 0.60 |
| nCohort 1 | 597 | 827 | 604 | 597 | 827 | 604 | 597 | 827 | 604 |
| nCohort 2 | 69 | 9 | 66 | 76 | 17 | 72 | 38 | 14 | 35 |
| Cutoff 1 | 17.1 | 45.7 | 15.5 | 20.9 | 35.3 | 20.6 | 15.1 | 40.4 | 15.1 |
| Sens 1 | 71% | 78% | 71% | 71% | 71% | 71% | 71% | 71% | 71% |
| Spec 1 | 38% | 71% | 33% | 46% | 63% | 44% | 34% | 67% | 32% |
| Cutoff 2 | 12.3 | 24.3 | 11.6 | 15.9 | 32.4 | 15.9 | 10.6 | 25.8 | 11.5 |
| Sens 2 | 81% | 89% | 80% | 80% | 82% | 81% | 82% | 86% | 80% |
| Spec 2 | 26% | 49% | 23% | 35% | 60% | 34% | 21% | 52% | 23% |
| Cutoff 3 | 6.31 | 22.3 | 6.31 | 4.81 | 14.6 | 4.81 | 5.07 | 25.0 | 5.07 |
| Sens 3 | 91% | 100% | 91% | 91% | 94% | 90% | 92% | 93% | 91% |
| Spec 3 | 12% | 46% | 12% | 9% | 31% | 9% | 9% | 51% | 9% |
| Cutoff 4 | 42.8 | 43.9 | 43.9 | 42.8 | 43.9 | 43.9 | 42.8 | 43.9 | 43.9 |
| Sens 4 | 32% | 78% | 29% | 39% | 47% | 36% | 42% | 57% | 37% |
| Spec 4 | 70% | 70% | 70% | 70% | 70% | 70% | 70% | 70% | 70% |
| Cutoff 5 | 53.8 | 57.2 | 55.2 | 53.8 | 57.2 | 55.2 | 53.8 | 57.2 | 55.2 |
| Sens 5 | 25% | 56% | 21% | 32% | 29% | 29% | 32% | 57% | 23% |
| Spec 5 | 80% | 80% | 80% | 80% | 80% | 80% | 80% | 80% | 80% |
| Cutoff 6 | 88.4 | 96.9 | 87.2 | 88.4 | 96.9 | 87.2 | 88.4 | 96.9 | 87.2 |
| Sens 6 | 13% | 22% | 12% | 18% | 24% | 17% | 13% | 36% | 14% |
| Spec 6 | 90% | 90% | 90% | 90% | 90% | 90% | 90% | 90% | 90% |
| OR Quart 2 | 1.1 | >2.0 | 1.1 | 1.1 | 1.0 | 1.1 | 0.99 | 0 | 0.88 |
| | 0.85 | <0.57 | 0.86 | 0.84 | 1.0 | 0.84 | 0.99 | na | 0.79 |
| p Value | 0.49 | >0.18 | 0.50 | 0.49 | 0.062 | 0.49 | 0.38 | na | 0.33 |
| 95% CI OR Quart2 | 2.4 | na | 2.3 | 2.4 | 16 | 2.4 | 2.6 | na | 2.3 |
| OR Quart 3 | 1.8 | >2.0 | 1.5 | 2.0 | 9.4 | 1.7 | 0.88 | 5.1 | 0.76 |
| | 0.11 | <0.57 | 0.28 | 0.059 | 0.035 | 0.15 | 0.79 | 0.14 | 0.60 |
| p Value 95% CI of OR Quart3 | 0.87 | >0.18 | 0.72 | 0.97 | 1.2 | 0.82 | 0.33 | 0.59 | 0.28 |
| | 3.7 | na | 3.1 | 4.1 | 75 | 3.5 | 2.3 | 44 | 2.1 |
| OR Quart 4 | 1.6 | >5.1 | 1.2 | 2.1 | 6.1 | 2.0 | 1.4 | 8.2 | 1.2 |
| | 0.21 | <0.14 | 0.58 | 0.044 | 0.094 | 0.059 | 0.51 | 0.048 | 0.66 |
| p Value 95% CI of OR Quart4 | 0.77 | >0.59 | 0.59 | 1.0 | 0.73 | 0.97 | 0.55 | 1.0 | 0.50 |
| | 3.3 | na | 2.6 | 4.2 | 52 | 4.0 | 3.3 | 66 | 3.1 |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.793 | 1.51 | 0.793 | 1.46 | 0.793 | 1.46 |
| Average | 1.42 | 1.43 | 1.42 | 1.47 | 1.42 | 1.31 |
| Stdev | 2.26 | 1.58 | 2.26 | 1.91 | 2.26 | 1.22 |
| p(t-test) | | 0.95 | | 0.85 | | 0.77 |
| Min | 0.00179 | 0.00179 | 0.00179 | 0.00179 | 0.00179 | 0.00179 |
| Max | 27.4 | 9.73 | 27.4 | 11.6 | 27.4 | 4.74 |
| n (Samp) | 597 | 69 | 597 | 76 | 597 | 38 |
| n (Patient) | 279 | 69 | 279 | 76 | 279 | 38 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.761 | 2.17 | 0.761 | 1.56 | 0.761 | 1.72 |
| Average | 1.40 | 2.29 | 1.40 | 1.31 | 1.40 | 1.71 |
| Stdev | 2.30 | 1.19 | 2.30 | 1.13 | 2.30 | 1.74 |
| p(t-test) | | 0.25 | | 0.87 | | 0.63 |
| Min | 0.00179 | 0.00342 | 0.00179 | 0.00352 | 0.00179 | 0.00246 |
| Max | 28.3 | 3.74 | 28.3 | 3.65 | 28.3 | 5.61 |
| n (Samp) | 827 | 9 | 827 | 17 | 827 | 14 |
| n (Patient) | 352 | 9 | 352 | 17 | 352 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.793 | 1.11 | 0.793 | 1.46 | 0.793 | 1.43 |
| Average | 1.39 | 1.31 | 1.39 | 1.55 | 1.39 | 1.26 |
| Stdev | 2.08 | 1.57 | 2.08 | 1.98 | 2.08 | 1.24 |
| p(t-test) | | 0.76 | | 0.54 | | 0.71 |
| Min | 0.00179 | 0.00179 | 0.00179 | 0.00179 | 0.00179 | 0.00179 |
| Max | 27.4 | 9.73 | 27.4 | 11.6 | 27.4 | 4.74 |
| n (Samp) | 604 | 66 | 604 | 72 | 604 | 35 |
| n (Patient) | 263 | 66 | 263 | 72 | 263 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | 0.73 | 0.50 | 0.51 | 0.55 | 0.52 | 0.53 | 0.55 | 0.52 |
| SE | 0.037 | 0.097 | 0.038 | 0.035 | 0.073 | 0.036 | 0.049 | 0.080 | 0.051 |
| p | 0.62 | 0.018 | 0.93 | 0.80 | 0.48 | 0.53 | 0.61 | 0.52 | 0.76 |
| nCohort 1 | 597 | 827 | 604 | 597 | 827 | 604 | 597 | 827 | 604 |
| nCohort 2 | 69 | 9 | 66 | 76 | 17 | 72 | 38 | 14 | 35 |
| Cutoff 1 | 0.0407 | 1.97 | 0.0305 | 0.0407 | 0.0522 | 0.0435 | 0.0742 | 0.0209 | 0.0522 |
| Sens 1 | 71% | 78% | 71% | 71% | 71% | 71% | 71% | 71% | 71% |
| Spec 1 | 27% | 73% | 25% | 27% | 33% | 31% | 33% | 25% | 32% |
| Cutoff 2 | 0.00332 | 1.10 | 0.00332 | 0.00342 | 0.0178 | 0.00342 | 0.00342 | 0.00332 | 0.00342 |
| Sens 2 | 83% | 89% | 82% | 86% | 82% | 85% | 87% | 86% | 86% |
| Spec 2 | 12% | 54% | 14% | 16% | 23% | 18% | 16% | 14% | 18% |
| Cutoff 3 | 0.00184 | 0.00332 | 0.00184 | 0.00246 | 0.00342 | 0.00246 | 0.00240 | 0.00240 | 0.00240 |
| Sens 3 | 94% | 100% | 94% | 93% | 100% | 93% | 95% | 100% | 94% |
| Spec 3 | 3% | 14% | 3% | 9% | 18% | 11% | 7% | 8% | 9% |
| Cutoff 4 | 1.87 | 1.87 | 1.87 | 1.87 | 1.87 | 1.87 | 1.87 | 1.87 | 1.87 |
| Sens 4 | 36% | 78% | 30% | 29% | 35% | 32% | 29% | 29% | 31% |
| Spec 4 | 71% | 71% | 71% | 71% | 71% | 71% | 71% | 71% | 71% |
| Cutoff 5 | 2.41 | 2.41 | 2.41 | 2.41 | 2.41 | 2.41 | 2.41 | 2.41 | 2.41 |
| Sens 5 | 26% | 44% | 23% | 21% | 18% | 24% | 21% | 29% | 20% |
| Spec 5 | 80% | 80% | 80% | 80% | 80% | 80% | 80% | 80% | 80% |
| Cutoff 6 | 3.36 | 3.36 | 3.36 | 3.36 | 3.36 | 3.36 | 3.36 | 3.36 | 3.36 |
| Sens 6 | 7% | 11% | 6% | 12% | 6% | 14% | 5% | 14% | 6% |
| Spec 6 | 91% | 91% | 90% | 91% | 91% | 90% | 91% | 91% | 90% |
| OR Quart 2 | 0.55 | 0 | 0.83 | 0.69 | 0.50 | 1.0 | 0.99 | 0 | 0.99 |
| | 0.13 | na | 0.61 | 0.29 | 0.42 | 1.0 | 0.99 | na | 0.99 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value 95% CI of OR Quart2 | 0.25 | na | 0.41 | 0.34 | 0.090 | 0.49 | 0.36 | na | 0.36 |
| | 1.2 | na | 1.7 | 1.4 | 2.7 | 2.0 | 2.7 | na | 2.7 |
| OR Quart 3 | 0.88 | 3.0 | 0.60 | 1.1 | 1.8 | 1.1 | 1.8 | 1.0 | 1.4 |
| | 0.72 | 0.34 | 0.19 | 0.87 | 0.37 | 0.72 | 0.20 | 1.0 | 0.49 |
| p Value | 0.44 | 0.31 | 0.28 | 0.56 | 0.51 | 0.57 | 0.74 | 0.29 | 0.55 |
| 95% CI of OR Quart3 | 1.8 | 29 | 1.3 | 2.0 | 6.2 | 2.3 | 4.4 | 3.5 | 3.6 |
| OR Quart 4 | 1.2 | 5.1 | 1.0 | 0.83 | 1.0 | 1.1 | 0.99 | 0.79 | 0.99 |
| | 0.63 | 0.14 | 0.98 | 0.60 | 1.0 | 0.72 | 0.99 | 0.73 | 0.99 |
| p Value 95% CI of OR Quart4 | 0.61 | 0.59 | 0.51 | 0.43 | 0.25 | 0.57 | 0.36 | 0.21 | 0.36 |
| | 2.3 | 44 | 2.0 | 1.6 | 4.1 | 2.3 | 2.7 | 3.0 | 2.7 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5250 | 6510 | 5250 | 6350 | 5250 | 4000 |
| Average | 17300 | 18400 | 17300 | 18000 | 17300 | 12000 |
| Stdev | 31000 | 35100 | 31000 | 27900 | 31000 | 22800 |
| p(t-test) | | 0.74 | | 0.83 | | 0.20 |
| Min | 0.0130 | 444 | 0.0130 | 957 | 0.0130 | 261 |
| Max | 238000 | 227000 | 238000 | 148000 | 238000 | 149000 |
| n (Samp) | 895 | 99 | 895 | 102 | 895 | 57 |
| n (Patient) | 374 | 99 | 374 | 102 | 374 | 57 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5530 | 11500 | 5530 | 7760 | 5530 | 6440 |
| Average | 16600 | 39800 | 16600 | 22500 | 16600 | 22000 |
| Stdev | 28500 | 63800 | 28500 | 27900 | 28500 | 35100 |
| p(t-test) | 4.4E-4 | 4.4E-4 | | 0.30 | | 0.35 |
| Min | 0.0130 | 104 | 0.0130 | 930 | 0.0130 | 876 |
| Max | 238000 | 227000 | 238000 | 110000 | 238000 | 149000 |
| n (Samp) | 1357 | 20 | 1357 | 26 | 1357 | 25 |
| n (Patient) | 470 | 20 | 470 | 26 | 470 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5760 | 6270 | 5760 | 6310 | 5760 | 4060 |
| Average | 18700 | 15800 | 18700 | 16800 | 18700 | 10300 |
| Stdev | 32100 | 28700 | 32100 | 28100 | 32100 | 14800 |
| p(t-test) | | 0.41 | | 0.57 | | 0.066 |
| Min | 0.0130 | 444 | 0.0130 | 957 | 0.0130 | 261 |
| Max | 238000 | 190000 | 238000 | 148000 | 238000 | 62900 |
| n (Samp) | 962 | 91 | 962 | 94 | 962 | 49 |
| n (Patient) | 367 | 91 | 367 | 94 | 367 | 49 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.57 | 0.64 | 0.53 | 0.57 | 0.60 | 0.53 | 0.45 | 0.53 | 0.43 |
| SE | 0.031 | 0.067 | 0.032 | 0.031 | 0.059 | 0.032 | 0.040 | 0.059 | 0.044 |
| p | 0.025 | 0.031 | 0.31 | 0.024 | 0.11 | 0.32 | 0.24 | 0.56 | 0.12 |
| nCohort 1 | 895 | 1357 | 962 | 895 | 1357 | 962 | 895 | 1357 | 962 |
| nCohort 2 | 99 | 20 | 91 | 102 | 26 | 94 | 57 | 25 | 49 |
| Cutoff 1 | 4740 | 6440 | 4710 | 4190 | 4220 | 4410 | 2670 | 3180 | 2670 |
| Sens 1 | 71% | 70% | 70% | 71% | 73% | 70% | 70% | 72% | 71% |
| Spec 1 | 47% | 56% | 44% | 43% | 40% | 42% | 27% | 29% | 25% |
| Cutoff 2 | 3990 | 4110 | 3970 | 3180 | 3340 | 3100 | 2090 | 2550 | 1890 |
| Sens 2 | 81% | 80% | 80% | 80% | 81% | 81% | 81% | 80% | 82% |
| Spec 2 | 41% | 39% | 38% | 32% | 31% | 29% | 19% | 22% | 15% |
| Cutoff 3 | 2150 | 3120 | 2150 | 2480 | 2420 | 2120 | 1290 | 1290 | 1290 |
| Sens 3 | 91% | 90% | 90% | 90% | 92% | 90% | 91% | 92% | 92% |
| Spec 3 | 19% | 29% | 18% | 24% | 21% | 17% | 9% | 8% | 7% |
| Cutoff 4 | 10700 | 11500 | 12800 | 10700 | 11500 | 12800 | 10700 | 11500 | 12800 |
| Sens 4 | 31% | 50% | 27% | 36% | 38% | 27% | 23% | 36% | 20% |
| Spec 4 | 70% | 70% | 70% | 70% | 70% | 70% | 70% | 70% | 70% |
| Cutoff 5 | 22400 | 22100 | 26200 | 22400 | 22100 | 26200 | 22400 | 22100 | 26200 |
| Sens 5 | 19% | 35% | 14% | 20% | 38% | 14% | 12% | 24% | 12% |
| Spec 5 | 80% | 80% | 80% | 80% | 80% | 80% | 80% | 80% | 80% |
| Cutoff 6 | 48800 | 45900 | 51900 | 48800 | 45900 | 51900 | 48800 | 45900 | 51900 |
| Sens 6 | 10% | 25% | 8% | 12% | 15% | 10% | 4% | 16% | 4% |
| Spec 6 | 90% | 90% | 90% | 90% | 90% | 90% | 90% | 90% | 90% |
| OR Quart 2 | 2.6 | 2.0 | 2.0 | 3.4 | 2.4 | 2.8 | 1.4 | 0.83 | 1.4 |
|  | 0.0074 | 0.42 | 0.038 | 6.2E-4 | 0.22 | 0.0035 | 0.40 | 0.76 | 0.48 |
| p Value 95% CI of OR Quart2 | 1.3 | 0.37 | 1.0 | 1.7 | 0.60 | 1.4 | 0.62 | 0.25 | 0.55 |
|  | 5.2 | 11 | 4.0 | 6.9 | 9.2 | 5.6 | 3.3 | 2.7 | 3.5 |
| OR Quart 3 | 2.8 | 2.5 | 2.3 | 2.7 | 2.0 | 2.7 | 1.9 | 0.83 | 2.1 |
|  | 0.0034 | 0.27 | 0.014 | 0.0061 | 0.33 | 0.0051 | 0.12 | 0.76 | 0.10 |
| p Value 95% CI of OR Quart3 | 1.4 | 0.49 | 1.2 | 1.3 | 0.50 | 1.3 | 0.84 | 0.25 | 0.87 |
|  | 5.6 | 13 | 4.4 | 5.6 | 8.1 | 5.4 | 4.1 | 2.7 | 4.9 |
| OR Quart 4 | 2.4 | 4.6 | 1.5 | 2.8 | 3.4 | 1.8 | 1.5 | 1.5 | 1.8 |
|  | 0.015 | 0.053 | 0.29 | 0.0044 | 0.065 | 0.11 | 0.31 | 0.44 | 0.19 |
| p Value 95% CI of OR Quart4 | 1.2 | 0.98 | 0.72 | 1.4 | 0.93 | 0.87 | 0.67 | 0.53 | 0.74 |
|  | 4.8 | 21 | 3.0 | 5.8 | 12 | 3.8 | 3.5 | 4.3 | 4.4 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.20 | 4.21 | 3.20 | 5.03 | 3.20 | 2.79 |
| Average | 5.72 | 5.80 | 5.72 | 8.53 | 5.72 | 5.54 |
| Stdev | 13.2 | 5.24 | 13.2 | 12.4 | 13.2 | 8.89 |
| p(t-test) | | 0.96 | | 0.088 | | 0.94 |
| Min | 0.0298 | 0.235 | 0.0298 | 0.00189 | 0.0298 | 0.000104 |
| Max | 279 | 31.4 | 279 | 81.9 | 279 | 43.0 |
| n (Samp) | 581 | 67 | 581 | 72 | 581 | 35 |
| n (Patient) | 276 | 67 | 276 | 72 | 276 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.45 | 4.45 | 3.45 | 5.98 | 3.45 | 4.31 |
| Average | 6.39 | 6.33 | 6.39 | 8.93 | 6.39 | 5.53 |
| Stdev | 13.2 | 5.02 | 13.2 | 12.7 | 13.2 | 3.77 |
| p(t-test) | | 0.99 | | 0.43 | | 0.81 |
| Min | 0.000104 | 0.847 | 0.000104 | 1.11 | 0.000104 | 0.667 |
| Max | 279 | 16.5 | 279 | 57.1 | 279 | 15.2 |
| n (Samp) | 801 | 9 | 801 | 17 | 801 | 13 |
| n (Patient) | 349 | 9 | 349 | 17 | 349 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.35 | 3.67 | 3.35 | 4.63 | 3.35 | 2.79 |
| Average | 5.95 | 5.55 | 5.95 | 7.97 | 5.95 | 7.04 |
| Stdev | 13.4 | 5.21 | 13.4 | 11.4 | 13.4 | 12.7 |
| p(t-test) | | 0.81 | | 0.23 | | 0.65 |
| Min | 0.000201 | 0.235 | 0.000201 | 0.00189 | 0.000201 | 0.000104 |
| Max | 279 | 31.4 | 279 | 81.9 | 279 | 57.1 |
| n (Samp) | 585 | 64 | 585 | 68 | 585 | 33 |
| n (Patient) | 260 | 64 | 260 | 68 | 260 | 33 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.60 | 0.56 | 0.61 | 0.65 | 0.58 | 0.45 | 0.59 | 0.44 |
| SE | 0.038 | 0.10 | 0.039 | 0.037 | 0.073 | 0.038 | 0.052 | 0.084 | 0.053 |
| p | 0.021 | 0.31 | 0.16 | 0.0029 | 0.041 | 0.026 | 0.29 | 0.27 | 0.28 |
| nCohort 1 | 581 | 801 | 585 | 581 | 801 | 585 | 581 | 801 | 585 |
| nCohort 2 | 67 | 9 | 64 | 72 | 17 | 68 | 35 | 13 | 33 |
| Cutoff 1 Sens 1 Spec 1 | 2.56 | 3.18 | 2.37 | 2.85 | 5.23 | 2.64 | 1.37 | 3.39 | 1.37 |
| | 70% | 78% | 70% | 71% | 71% | 71% | 71% | 77% | 73% |
| | 42% | 47% | 37% | 46% | 66% | 41% | 22% | 49% | 21% |
| Cutoff 2 Sens 2 | 2.17 | 3.15 | 1.88 | 1.86 | 2.56 | 1.80 | 0.806 | 2.95 | 0.806 |
| | 81% | 89% | 81% | 81% | 82% | 81% | 80% | 85% | 82% |
| Spec 2 | 37% | 47% | 31% | 32% | 39% | 29% | 12% | 44% | 11% |
| Cutoff 3 Sens 3 Spec 3 | 1.76 | 0.840 | 1.57 | 0.750 | 1.68 | 0.709 | 0.367 | 2.22 | 0.367 |
| | 91% | 100% | 91% | 90% | 94% | 91% | 91% | 92% | 91% |
| | 30% | 12% | 25% | 11% | 26% | 9% | 4% | 35% | 3% |
| Cutoff 4 Sens 4 Spec 4 | 5.47 | 6.00 | 5.71 | 5.47 | 6.00 | 5.71 | 5.47 | 6.00 | 5.71 |
| | 37% | 33% | 36% | 46% | 47% | 41% | 26% | 38% | 27% |
| | 70% | 70% | 70% | 70% | 70% | 70% | 70% | 70% | 70% |
| Cutoff 5 Sens 5 Spec 5 | 7.36 | 8.53 | 7.77 | 7.36 | 8.53 | 7.77 | 7.36 | 8.53 | 7.77 |
| | 24% | 33% | 22% | 33% | 29% | 32% | 23% | 15% | 21% |
| | 80% | 80% | 80% | 80% | 80% | 80% | 80% | 80% | 80% |
| Cutoff 6 Sens 6 Spec 6 | 12.1 | 13.6 | 12.5 | 12.1 | 13.6 | 12.5 | 12.1 | 13.6 | 12.5 |
| | 10% | 11% | 6% | 19% | 6% | 21% | 9% | 8% | 9% |
| | 90% | 90% | 90% | 90% | 90% | 90% | 90% | 90% | 90% |
| OR Quart2 p Value 95% CI of OR Quart2 | 3.9 | 2.0 | 3.3 | 1.0 | 3.0 | 0.84 | 0.65 | 3.0 | 0.43 |
| | 0.0046 | 0.57 | 0.0082 | 1.0 | 0.34 | 0.67 | 0.43 | 0.34 | 0.17 |
| | 1.5 | 0.18 | 1.4 | 0.42 | 0.31 | 0.36 | 0.23 | 0.31 | 0.13 |
| | 9.9 | 22 | 8.0 | 2.4 | 29 | 1.9 | 1.9 | 29 | 1.4 |
| OR Quart3 p Value 95% CI of OR Quart3 | 3.5 | 3.0 | 2.9 | 2.0 | 8.3 | 1.4 | 0.88 | 5.1 | 1.0 |
| | 0.010 | 0.34 | 0.018 | 0.067 | 0.047 | 0.35 | 0.80 | 0.14 | 1.0 |
| | 1.3 | 0.31 | 1.2 | 0.95 | 1.0 | 0.68 | 0.33 | 0.59 | 0.39 |
| | 8.9 | 29 | 7.2 | 4.4 | 67 | 3.0 | 2.4 | 44 | 2.6 |
| OR Quart4 p Value 95% CI ot OR Quart4 | 3.9 | 3.0 | 2.6 | 3.0 | 5.1 | 2.2 | 1.4 | 4.0 | 1.2 |
| | 0.0046 | 0.34 | 0.041 | 0.0036 | 0.14 | 0.031 | 0.50 | 0.21 | 0.63 |
| | 1.5 | 0.31 | 1.0 | 1.4 | 0.59 | 1.1 | 0.56 | 0.45 | 0.50 |
| | 9.9 | 29 | 6.4 | 6.2 | 44 | 4.4 | 3.3 | 36 | 3.1 |

**Heparin-binding growth factor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 854 | 754 | 854 | 729 | 854 | 595 |
| Average | 1230 | 1000 | 1230 | 1140 | 1230 | 1060 |
| Stdev | 1510 | 922 | 1510 | 1170 | 1510 | 1420 |
| p(t-test) | | 0.14 | | 0.59 | | 0.41 |
| Min | 0.00328 | 28.3 | 0.00328 | 8.50 | 0.00328 | 13.2 |
| Max | 16700 | 5510 | 16700 | 6000 | 16700 | 7750 |
| n (Samp) | 895 | 99 | 895 | 101 | 895 | 57 |
| n (Patient) | 374 | 99 | 374 | 101 | 374 | 57 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 847 | 536 | 847 | 545 | 847 | 289 |
| Average | 1220 | 631 | 1220 | 851 | 1220 | 664 |
| Stdev | 1460 | 445 | 1460 | 753 | 1460 | 802 |
| p(t-test) | | 0.069 | | 0.20 | | 0.055 |
| Min | 0.00328 | 70.1 | 0.00328 | 90.5 | 0.00328 | 1.77 |
| Max | 16700 | 1870 | 16700 | 2920 | 16700 | 3380 |
| n (Samp) | 1357 | 20 | 1357 | 25 | 1357 | 25 |
| n (Patient) | 470 | 20 | 470 | 25 | 470 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 834 | 846 | 834 | 797 | 834 | 670 |
| Average | 1200 | 1040 | 1200 | 1170 | 1200 | 1140 |
| Stdev | 1490 | 941 | 1490 | 1190 | 1490 | 1470 |
| p(t-test) | | 0.32 | | 0.81 | | 0.78 |
| Min | 0.00328 | 28.3 | 0.00328 | 8.50 | 0.00328 | 13.2 |
| Max | 16700 | 5510 | 16700 | 6000 | 16700 | 7750 |
| n (Samp) | 961 | 91 | 961 | 94 | 961 | 49 |
| n (Patient) | 367 | 91 | 367 | 94 | 367 | 49 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.37 | 0.49 | 0.49 | 0.43 | 0.50 | 0.43 | 0.33 | 0.47 |
| SE | 0.031 | 0.067 | 0.032 | 0.030 | 0.060 | 0.031 | 0.041 | 0.060 | 0.043 |
| p | 0.35 | 0.055 | 0.79 | 0.75 | 0.24 | 0.98 | 0.10 | 0.0043 | 0.49 |
| nCohort 1 | 895 | 1357 | 961 | 895 | 1357 | 961 | 895 | 1357 | 961 |
| nCohort 2 | 99 | 20 | 91 | 101 | 25 | 94 | 57 | 25 | 49 |
| Cutoff 1 | 444 | 403 | 454 | 467 | 294 | 467 | 318 | 169 | 401 |
| Sens 1 | 71% | 70% | 70% | 70% | 72% | 70% | 70% | 72% | 71% |
| Spec 1 | 29% | 27% | 30% | 31% | 20% | 31% | 22% | 12% | 27% |
| Cutoff 2 | 249 | 242 | 280 | 359 | 247 | 359 | 192 | 77.7 | 287 |
| Sens 2 | 81% | 80% | 80% | 80% | 80% | 81% | 81% | 80% | 82% |
| Spec 2 | 17% | 17% | 19% | 24% | 17% | 24% | 13% | 6% | 19% |
| Cutoff 3 | 161 | 187 | 173 | 169 | 110 | 152 | 61.5 | 34.6 | 77.7 |
| Sens 3 | 91% | 90% | 90% | 90% | 92% | 90% | 91% | 92% | 92% |
| Spec 3 | 11% | 13% | 12% | 12% | 8% | 11% | 6% | 3% | 6% |
| Cutoff 4 | 1390 | 1400 | 1340 | 1390 | 1400 | 1340 | 1390 | 1400 | 1340 |
| Sens 4 | 26% | 5% | 29% | 27% | 20% | 27% | 25% | 20% | 27% |
| Spec 4 | 70% | 70% | 70% | 70% | 70% | 70% | 70% | 70% | 70% |
| Cutoff 5 | 1750 | 1780 | 1720 | 1750 | 1780 | 1720 | 1750 | 1780 | 1720 |
| Sens 5 | 16% | 5% | 16% | 21% | 12% | 21% | 12% | 8% | 14% |
| Spec 5 | 80% | 80% | 80% | 80% | 80% | 80% | 80% | 80% | 80% |
| Cutoff 6 | 2490 | 2520 | 2450 | 2490 | 2520 | 2450 | 2490 | 2520 | 2450 |
| Sens 6 | 6% | 0% | 8% | 11% | 4% | 12% | 9% | 4% | 8% |
| Spec 6 | 90% | 90% | 90% | 90% | 90% | 90% | 90% | 90% | 90% |
| OR Quart 2 | 1.4 | 5.1 | 1.5 | 1.0 | 1.0 | 1.4 | 1.3 | 2.5 | 1.1 |
| | 0.27 | 0.14 | 0.17 | 1.0 | 1.00 | 0.29 | 0.52 | 0.27 | 0.82 |
| p Value 95% CI of OR Quart2 | 0.76 | 0.59 | 0.83 | 0.55 | 0.29 | 0.76 | 0.57 | 0.49 | 0.48 |
| | 2.6 | 44 | 2.8 | 1.8 | 3.5 | 2.5 | 3.1 | 13 | 2.5 |
| OR Quart 3 | 1.5 | 8.2 | 1.1 | 1.3 | 1.4 | 1.3 | 1.4 | 2.5 | 1.1 |
| | 0.17 | 0.048 | 0.87 | 0.32 | 0.56 | 0.44 | 0.40 | 0.27 | 0.83 |
| p Value 95% CI of OR Quart3 | 0.83 | 1.0 | 0.55 | 0.76 | 0.44 | 0.69 | 0.62 | 0.49 | 0.47 |
| | 2.8 | 66 | 2.0 | 2.3 | 4.5 | 2.3 | 3.3 | 13 | 2.5 |
| OR Quart 4 | 1.4 | 6.1 | 1.3 | 0.91 | 1.6 | 1.1 | 2.1 | 6.7 | 1.3 |
| | 0.27 | 0.095 | 0.43 | 0.76 | 0.40 | 0.76 | 0.064 | 0.012 | 0.53 |
| p Value 95% CI of OR Quart4 | 0.76 | 0.73 | 0.69 | 0.50 | 0.52 | 0.59 | 0.96 | 1.5 | 0.58 |
| | 2.6 | 51 | 2.4 | 1.7 | 5.0 | 2.1 | 4.6 | 30 | 2.9 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.96E-5 | 2.75E-5 | 2.96E-5 | 3.44E-5 | 2.96E-5 | 2.58E-5 |
| Average | 0.00393 | 0.0150 | 0.00393 | 0.0190 | 0.00393 | 0.00519 |
| Stdev | 0.0267 | 0.116 | 0.0267 | 0.0885 | 0.0267 | 0.0298 |
| p(t-test) | | 0.033 | | 4.9E-4 | | 0.77 |
| Min | 5.62E-6 | 5.62E-6 | 5.62E-6 | 5.62E-6 | 5.62E-6 | 5.62E-6 |
| Max | 0.476 | 1.02 | 0.476 | 0.560 | 0.476 | 0.193 |
| n (Samp) | 754 | 78 | 754 | 86 | 754 | 42 |
| n (Patient) | 298 | 78 | 298 | 86 | 298 | 42 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.96E-5 | 3.28E-5 | 2.96E-5 | 2.63E-5 | 2.96E-5 | 2.66E-5 |
| Average | 0.00514 | 0.0622 | 0.00514 | 0.0258 | 0.00514 | 0.000233 |
| Stdev | 0.0328 | 0.240 | 0.0328 | 0.116 | 0.0328 | 0.000850 |
| p(t-test) | | 3.9E-8 | | 0.0068 | | 0.53 |
| Min | 5.62E-6 | 7.53E-6 | 5.62E-6 | 5.62E-6 | 5.62E-6 | 5.62E-6 |
| Max | 3.476 | 1.02 | 0.476 | 0.560 | 0.476 | 0.00364 |
| n (Samp) | 1164 | 18 | 1164 | 23 | 1164 | 18 |
| n (Patient) | 379 | 18 | 379 | 23 | 379 | 18 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.68E-5 | 2.83E-5 | 3.44E-5 | 2.83E-5 | 2.58E-5 |
| Average | 0.00941 | 0.0153 | 0.00941 | 0.0138 | 0.00941 | 0.00595 |
| Stdev | 0.0925 | 0.122 | 0.0925 | 0.0684 | 0.0925 | 0.0321 |
| p(t-test) | | 0.62 | | 0.68 | | 0.82 |
| Min | 5.62E-6 | 5.62E-6 | 5.62E-6 | 5.62E-6 | 5.62E-6 | 5.62E-6 |
| Max | 2.28 | 1.02 | 2.28 | 0.470 | 2.28 | 0.193 |
| n (Samp) | 838 | 70 | 838 | 80 | 838 | 36 |
| n (Patient) | 306 | 70 | 306 | 80 | 306 | 36 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | 0.54 | 0.47 | 0.55 | 0.50 | 0.57 | 0.43 | 0.41 | 0.46 |
| SE | 0.035 | 0.070 | 0.036 | 0.034 | 0.061 | 0.035 | 0.047 | 0.071 | 0.050 |
| p | 0.53 | 0.54 | 0.41 | 0.12 | 0.98 | 0.055 | 0.12 | 0.22 | 0.39 |
| nCohort 1 | 754 | 1164 | 838 | 754 | 1164 | 838 | 754 | 1164 | 838 |
| nCohort 2 | 78 | 18 | 70 | 86 | 23 | 80 | 42 | 18 | 36 |
| Cutoff 1 Sens 1 Spec 1 | 1.92E-5 | 1.88E-5 | 2.17E-5 | 2.46E-5 | 1.88E-5 | 2.46E-5 | 1.92E-5 | 2.17E-5 | 1.92E-5 |
| | 74% | 78% | 70% | 73% | 78% | 72% | 76% | 72% | 75% |
| | 23% | 20% | 28% | 34% | 20% | 35% | 23% | 28% | 24% |
| Cutoff 2 Sens 2 Spec 2 | 1.82E-5 | 1.82E-5 | 1.88E-5 | 1.92E-5 | 1.82E-5 | 1.92E-5 | 1.82E-5 | 1.92E-5 | 1.82E-5 |
| | 86% | 89% | 80% | 81% | 87% | 81% | 86% | 83% | 83% |
| | 15% | 17% | 20% | 23% | 17% | 24% | 15% | 24% | 16% |
| Cutoff 3 Sens 3 Spec 3 | 1.34E-5 | 7.53E-6 | 1.34E-5 | 1.82E-5 | 1.66E-5 | 1.82E-5 | 1.34E-5 | 1.82E-5 | 1.34E-5 |
| | 94% | 94% | 93% | 91% | 91% | 90% | 95% | 94% | 94% |
| | 9% | 5% | 9% | 15% | 13% | 16% | 9% | 17% | 9% |
| Cutoff 4 Sens 4 Spec 4 | 8.63E-5 | 4.39E-5 | 4.39E-5 | 8.63E-5 | 4.39E-5 | 4.39E-5 | 8.63E-5 | 4.39E-5 | 4.39E-5 |
| | 22% | 39% | 21% | 35% | 35% | 41% | 14% | 11% | 22% |
| | 73% | 72% | 70% | 73% | 72% | 70% | 73% | 72% | 70% |
| Cutoff 5 Sens 5 Spec 5 | 0.000168 | 0.000114 | 0.000114 | 0.000168 | 0.000114 | 0.000114 | 0.000168 | 0.000114 | 0.000114 |
| | 15% | 33% | 16% | 27% | 26% | 31% | 12% | 11% | 14% |
| | 84% | 80% | 80% | 84% | 80% | 80% | 84% | 80% | 80% |
| Cutoff 6 Sens 6 Spec 6 | 0.00325 | 0.00364 | 0.00364 | 0.00325 | 0.00364 | 0.00364 | 0.00325 | 0.00364 | 0.00364 |
| | 13% | 28% | 9% | 14% | 17% | 11% | 10% | 0% | 8% |
| | 90% | 90% | 90% | 90% | 90% | 90% | 90% | 90% | 90% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 1.1 | 0.49 | 1.6 | 1.1 | 0.28 | 1.1 | 1.3 | 1.5 | 1.7 |
| | 0.86 | 0.32 | 0.20 | 0.74 | 0.12 | 0.73 | 0.59 | 0.65 | 0.31 |
| | 0.53 | 0.12 | 0.78 | 0.58 | 0.058 | 0.56 | 0.46 | 0.25 | 0.61 |
| | 2.1 | 2.0 | 3.5 | 2.1 | 1.4 | 2.3 | 4.0 | 9.1 | 4.8 |
| OR Quart 3 | 1.4 | 0.33 | 1.8 | 1.1 | 1.0 | 1.1 | 2.6 | 5.1 | 1.9 |
| | 0.32 | 0.18 | 0.11 | 0.87 | 1.0 | 0.86 | 0.051 | 0.036 | 0.22 |
| p Value 95% CI of OR Quart3 | 0.72 | 0.066 | 0.88 | 0.55 | 0.35 | 0.53 | 1.00 | 1.1 | 0.68 |
| | 2.7 | 1.6 | 3.8 | 2.0 | 2.9 | 2.2 | 6.9 | 24 | 5.2 |
| OR Quart 4 p Value 95% CI of OR Quart4 | 1.2 | 1.2 | 1.5 | 1.4 | 1.0 | 1.9 | 2.2 | 1.5 | 1.5 |
| | 0.61 | 0.78 | 0.26 | 0.27 | 0.99 | 0.046 | 0.11 | 0.65 | 0.43 |
| | 0.61 | 0.39 | 0.73 | 0.76 | 0.35 | 1.0 | 0.84 | 0.25 | 0.53 |
| | 2.4 | 3.5 | 3.3 | 2.7 | 2.9 | 3.6 | 6.0 | 9.1 | 4.4 |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0141 | 0.0142 | 0.0141 | 0.0159 | 0.0141 | 0.0141 |
| Average | 0.134 | 0.189 | 0.134 | 0.264 | 0.134 | 0.113 |
| Stdev | 0.490 | 0.614 | 0.490 | 0.778 | 0.490 | 0.249 |
| p(t-test) | | 0.36 | | 0.031 | | 0.78 |
| Min | 1.14E-9 | 1.14E-9 | 1.14E-9 | 1.40E-9 | 1.14E-9 | 0.000186 |
| Max | 8.92 | 4.66 | 8.92 | 4.47 | 8.92 | 1.05 |
| n (Samp) | 754 | 78 | 754 | 86 | 754 | 42 |
| n (Patient) | 298 | 78 | 298 | 86 | 298 | 42 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0143 | 0.0158 | 0.0143 | 0.0371 | 0.0143 | 0.0137 |
| Average | 0.157 | 0.352 | 0.157 | 0.217 | 0.157 | 0.133 |
| Stdev | 0.510 | 1.09 | 0.510 | 0.706 | 0.510 | 0.209 |
| p(t-test) | | 0.12 | | 0.58 | | 0.85 |
| Min | 1.14E-9 | 0.00163 | 1.14E-9 | 7.54E-8 | 1.14E-9 | 0.000952 |
| Max | 8.92 | 4.66 | 8.92 | 3.42 | 8.92 | 0.635 |
| n (Samp) | 1164 | 18 | 1164 | 23 | 1164 | 18 |
| n (Patient) | 379 | 18 | 379 | 23 | 379 | 18 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0145 | 0.0134 | 0.0145 | 0.0146 | 0.0145 | 0.0141 |
| Average | 0.183 | 0.131 | 0.183 | 0.235 | 0.183 | 0.0928 |
| Stdev | 0.639 | 0.352 | 0.639 | 0.722 | 0.639 | 0.237 |
| p(t-test) | | 0.50 | | 0.50 | | 0.40 |
| Min | 1.14E-9 | 1.14E-9 | 1.14E-9 | 1.40E-9 | 1.14E-9 | 0.000186 |
| Max | 8.92 | 2.40 | 8.92 | 4.47 | 8.92 | 1.05 |
| n (Samp) | 838 | 70 | 838 | 80 | 838 | 36 |
| n (Patient) | 306 | 70 | 306 | 80 | 306 | 36 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.55 | 0.49 | 0.53 | 0.61 | 0.51 | 0.49 | 0.54 | 0.46 |
| SE | 0.035 | 0.070 | 0.036 | 0.033 | 0.063 | 0.034 | 0.046 | 0.070 | 0.050 |
| p | 0.71 | 0.48 | 0.79 | 0.31 | 0.086 | 0.77 | 0.81 | 0.60 | 0.43 |
| nCohort 1 | 754 | 1164 | 838 | 754 | 1164 | 838 | 754 | 1164 | 838 |
| nCohort 2 | 78 | 18 | 70 | 86 | 23 | 80 | 42 | 18 | 36 |
| Cutoff 1 Sens 1 Spec 1 | 0.00681 | 0.00693 | 0.00611 | 0.00657 | 0.0157 | 0.00657 | 0.00752 | 0.00639 | 0.00324 |
| | 71% | 72% | 70% | 71% | 74% | 70% | 71% | 72% | 72% |
| | 34% | 33% | 30% | 33% | 53% | 32% | 35% | 31% | 18% |
| Cutoff 2 Sens 2 Spec 2 | 0.00435 | 0.00618 | 0.00378 | 0.00479 | 0.00811 | 0.00481 | 0.00254 | 0.00583 | 0.00204 |
| | 81% | 83% | 80% | 80% | 83% | 80% | 81% | 83% | 81% |
| | 24% | 30% | 21% | 26% | 36% | 25% | 16% | 29% | 13% |
| Cutoff 3 Sens 3 Spec 3 | 0.00151 | 0.00338 | 0.00151 | 0.00123 | 0.00124 | 0.00198 | 0.00105 | 0.00254 | 0.000965 |
| | 91% | 94% | 90% | 91% | 91% | 90% | 90% | 94% | 92% |
| | 10% | 19% | 9% | 9% | 8% | 12% | 8% | 15% | 7% |
| Cutoff 4 Sens 4 Spec 4 | 0.0343 | 0.0360 | 0.0369 | 0.0343 | 0.0360 | 0.0369 | 0.0343 | 0.0360 | 0.0369 |
| | 29% | 33% | 29% | 38% | 57% | 34% | 29% | 39% | 31% |
| | 70% | 70% | 70% | 70% | 70% | 70% | 70% | 70% | 70% |
| Cutoff 5 Sens 5 Spec 5 | 0.0695 | 0.0887 | 0.0945 | 0.0695 | 0.0887 | 0.0945 | 0.0695 | 0.0887 | 0.0945 |
| | 26% | 33% | 21% | 24% | 22% | 24% | 19% | 28% | 14% |
| | 80% | 80% | 80% | 80% | 80% | 80% | 80% | 80% | 80% |
| Cutoff 6 Sens 6 Spec 6 | 0.295 | 0.363 | 0.416 | 0.295 | 0.363 | 0.416 | 0.295 | 0.363 | 0.416 |
| | 17% | 17% | 10% | 15% | 9% | 12% | 14% | 17% | 8% |
| | 90% | 90% | 90% | 90% | 90% | 90% | 90% | 90% | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 1.5 | 1.7 | 0.88 | 1.4 | 0.49 | 1.6 | 1.1 | 2.0 | 1.6 |
| | 0.24 | 0.48 | 0.72 | 0.32 | 0.42 | 0.19 | 0.82 | 0.33 | 0.33 |
| | 0.76 | 0.40 | 0.44 | 0.72 | 0.090 | 0.80 | 0.46 | 0.50 | 0.61 |
| | 2.9 | 7.1 | 1.8 | 2.7 | 2.7 | 3.0 | 2.7 | 8.1 | 4.2 |
| OR Quart 3 p Value 95% CI of OR Quart3 | 1.2 | 1.3 | 1.1 | 1.1 | 2.0 | 0.93 | 1.0 | 1.3 | 1.0 |
| | 0.60 | 0.70 | 0.86 | 0.73 | 0.25 | 0.85 | 1.0 | 0.70 | 1.0 |
| | 0.60 | 0.30 | 0.54 | 0.57 | 0.60 | 0.45 | 0.41 | 0.30 | 0.34 |
| | 2.4 | 6.0 | 2.1 | 2.2 | 6.8 | 1.9 | 2.5 | 6.0 | 2.9 |
| OR Quart 4 p Value | 1.3 | 2.0 | 0.94 | 1.7 | 2.3 | 1.6 | 1.1 | 1.7 | 1.6 |
| | 0.49 | 0.33 | 0.86 | 0.11 | 0.17 | 0.15 | 0.82 | 0.48 | 0.33 |
| | 0.64 | 0.50 | 0.47 | 0.88 | 0.69 | 0.84 | 0.46 | 0.40 | 0.61 |
| 95% CI of OR Quart4 | 2.5 | 8.1 | 1.9 | 3.2 | 7.5 | 3.1 | 2.7 | 7.1 | 4.2 |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1240 | 1920 | 1240 | 1530 | 1240 | 1120 |
| Average | 1930 | 2680 | 1930 | 4030 | 1930 | 1970 |
| Stdev | 2780 | 2580 | 2780 | 10600 | 2780 | 3040 |
| p(t-test) | | 0.011 | | 2.9E-6 | | 0.92 |
| Min | 0.0376 | 66.6 | 0.0376 | 108 | 0.0376 | 14.6 |
| Max | 45800 | 11900 | 45800 | 80100 | 45800 | 19700 |
| n (Samp) | 895 | 99 | 895 | 102 | 895 | 57 |
| n (Patient) | 374 | 99 | 374 | 102 | 374 | 57 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1370 | 1810 | 1370 | 1520 | 1370 | 1280 |
| Average | 2310 | 3240 | 2310 | 5550 | 2310 | 1900 |
| Stdev | 3600 | 2910 | 3600 | 15400 | 3600 | 1810 |
| p(t-test) | | 0.25 | | 7.9E-5 | | 0.57 |
| Min | 0.0376 | 227 | 0.0376 | 174 | 0.0376 | 201 |
| Max | 58100 | 8700 | 68100 | 80100 | 68100 | 7000 |
| n (Samp) | 1357 | 20 | 1357 | 26 | 1357 | 25 |
| n (Patient) | 470 | 20 | 470 | 26 | 470 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1280 | 1860 | 1280 | 1570 | 1280 | 1160 |
| Average | 2140 | 2550 | 2140 | 3340 | 2140 | 2160 |
| Stdev | 3830 | 2460 | 3830 | 7570 | 3830 | 3300 |
| p(t-test) | | 0.32 | | 0.010 | | 0.98 |
| Min | 0.0376 | 66.6 | 0.0376 | 108 | 0.0376 | 14.6 |
| Max | 80100 | 11900 | 80100 | 68100 | 80100 | 19700 |
| n (Samp) | 962 | 91 | 962 | 94 | 962 | 49 |
| n (Patient) | 367 | 91 | 367 | 94 | 367 | 49 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.59 | 0.62 | 0.57 | 0.57 | 0.57 | 0.56 | 0.47 | 0.49 | 0.46 |
| SE | 0.031 | 0.068 | 0.032 | 0.031 | 0.059 | 0.032 | 0.040 | 0.059 | 0.043 |
| p | 0.0029 | 0.064 | 0.032 | 0.030 | 0.21 | 0.083 | 0.40 | 0.87 | 0.41 |
| nCohort 1 | 895 | 1357 | 962 | 895 | 1357 | 962 | 895 | 1357 | 962 |
| nCohort 2 | 99 | 20 | 91 | 102 | 26 | 94 | 57 | 25 | 49 |
| Cutoff 1 Sens 1 Spec 1 | 899 | 1380 | 899 | 799 | 1020 | 799 | 575 | 679 | 541 |
|  | 71% | 70% | 70% | 71% | 73% | 70% | 70% | 72% | 71% |
|  | 37% | 51% | 35% | 34% | 39% | 32% | 23% | 26% | 21% |
| Cutoff 2 Sens 2 Spec 2 | 612 | 878 | 612 | 501 | 686 | 498 | 450 | 589 | 450 |
|  | 81% | 80% | 80% | 80% | 81% | 81% | 81% | 80% | 82% |
|  | 25% | 34% | 24% | 20% | 27% | 19% | 19% | 23% | 17% |
| Cutoff 3 Sens 3 Spec 3 | 293 | 831 | 332 | 348 | 275 | 353 | 214 | 354 | 191 |
|  | 91% | 90% | 90% | 90% | 92% | 90% | 91% | 92% | 92% |
|  | 10% | 32% | 11% | 14% | 9% | 13% | 7% | 13% | 5% |
| Cutoff 4 Sens 4 Spec 4 | 2000 | 2300 | 2070 | 2000 | 2300 | 2070 | 2000 | 2300 | 2070 |
|  | 48% | 40% | 48% | 41% | 35% | 44% | 25% | 24% | 27% |
|  | 70% | 70% | 70% | 70% | 70% | 70% | 70% | 70% | 70% |
| Cutoff 5 Sens 5 Spec 5 | 2680 | 3160 | 2840 | 2680 | 3160 | 2840 | 2680 | 3160 | 2840 |
|  | 36% | 35% | 33% | 33% | 35% | 32% | 21% | 24% | 18% |
|  | 80% | 80% | 80% | 80% | 80% | 80% | 80% | 80% | 80% |
| Cutoff 6 Sens 6 Spec 6 | 4110 | 4870 | 4360 | 4110 | 4870 | 4360 | 4110 | 4870 | 4360 |
|  | 21% | 25% | 16% | 20% | 19% | 17% | 11% | 8% | 12% |
|  | 90% | 90% | 90% | 90% | 90% | 90% | 90% | 90% | 90% |
| OR Quart 2 p Value 95% CI of OR Quart2 | 1.00 | 2.0 | 1.0 | 0.55 | 1.8 | 0.62 | 0.92 | 0.83 | 1.0 |
|  | 0.99 | 0.42 | 1.0 | 0.076 | 0.37 | 0.15 | 0.84 | 0.77 | 1.0 |
|  | 0.52 | 0.37 | 0.52 | 0.28 | 0.51 | 0.32 | 0.41 | 0.25 | 0.43 |
|  | 1.9 | 11 | 1.9 | 1.1 | 6.1 | 1.2 | 2.1 | 2.8 | 2.4 |
| OR Quart 3 p Value 95% CI of OR Quart3 | 0.95 | 3.6 | 0.84 | 0.96 | 1.5 | 0.74 | 1.0 | 1.2 | 1.0 |
|  | 0.87 | 0.12 | 0.61 | 0.88 | 0.53 | 0.35 | 1.0 | 0.78 | 1.0 |
|  | 0.49 | 0.73 | 0.43 | 0.54 | 0.42 | 0.40 | 0.45 | 0.39 | 0.43 |
|  | 1.8 | 17 | 1.6 | 1.7 | 5.4 | 1.4 | 2.2 | 3.5 | 2.4 |
| OR Quart 4 p Value 95% CI of OR Quart4 | 2.2 | 3.5 | 1.8 | 1.4 | 2.3 | 1.4 | 1.5 | 1.2 | 1.5 |
|  | 0.0072 | 0.12 | 0.048 | 0.18 | 0.17 | 0.22 | 0.27 | 0.78 | 0.32 |
|  | 1.2 | 0.73 | 1.0 | 0.84 | 0.69 | 0.82 | 0.72 | 0.39 | 0.68 |
|  | 3.9 | 17 | 3.2 | 2.5 | 7.5 | 2.4 | 3.1 | 3.5 | 3.3 |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.3 | 12.5 | 10.3 | 10.6 | 10.3 | 0.452 |
| Average | 17.8 | 18.7 | 17.8 | 126 | 17.8 | 22.1 |
| Stdev | 47.1 | 20.2 | 47.1 | 866 | 47.1 | 56.8 |
| p(t-test) | | 0.87 | | 0.0025 | | 0.59 |
| Min | 0.00398 | 0.00398 | 0.00398 | 0.00398 | 0.00398 | 0.00398 |
| Max | 945 | 91.5 | 945 | 7540 | 945 | 317 |
| n (Samp) | 597 | 69 | 597 | 76 | 597 | 37 |
| n (Patient) | 279 69 | | 279 | 76 | 279 | 37 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.6 | 0.452 | 10.6 | 20.6 | 10.6 | 0.397 |
| Average | 28.3 | 5.80 | 28.3 | 21.0 | 28.3 | 12.3 |
| Stdev | 266 | 10.5 | 266 | 18.3 | 266 | 17.3 |
| p(t-test) | | 0.80 | | 0.91 | | 0.82 |
| Min | 0.00398 | 0.0132 | 0.00398 | 0.00398 | 0.00398 | 0.0184 |
| Max | 7540 | 31.7 | 7540 | 72.0 | 7540 | 50.9 |
| n (Samp) | 826 | 9 | 826 | 17 | 826 | 14 |
| n (Patient) | 352 | 9 | 352 | 17 | 352 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.6 | 12.5 | 10.6 | 9.71 | 10.6 | 2.31 |
| Average | 17.7 | 19.1 | 17.7 | 131 | 17.7 | 25.1 |
| Stdev | 46.7 | 20.3 | 46.7 | 890 | 46.7 | 58.7 |
| p(t-test) | | 0.81 | | 0.0019 | | 0.38 |
| Min | 0.00398 | 0.00398 | 0.00398 | 0.00398 | 0.00398 | 0.00398 |
| Max | 945 | 91.5 | 945 | 7540 | 945 | 317 |
| n (Samp) | 604 | 66 | 604 | 72 | 604 | 34 |
| n (Patient) | 263 | 66 | 263 | 72 | 263 | 34 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.55 | 0.31 | 0.56 | 0.54 | 0.62 | 0.52 | 0.43 | 0.43 | 0.48 |
| SE | 0.037 | 0.099 | 0.038 | 0.036 | 0.073 | 0.036 | 0.050 | 0.080 | 0.051 |
| p | 0.20 | 0.054 | 0.12 | 0.33 | 0.10 | 0.63 | 0.19 | 0.41 | 0.71 |
| nCohort 1 | 597 | 826 | 604 | 597 | 826 | 604 | 597 | 826 | 604 |
| nCohort 2 | 69 | 9 | 66 | 76 | 17 | 72 | 37 | 14 | 34 |
| Cutoff 1 Sens 1 Spec 1 | 0.533 | 0.0187 | 4.89 | 1.39 | 10.0 | 0.338 | 0.117 | 0.315 | 0.315 |
|  | 71% | 78% | 71% | 71% | 71% | 74% | 70% | 71% | 71% |
|  | 31% | 10% | 38% | 32% | 49% | 28% | 23% | 27% | 27% |
| Cutoff 2 Sens 2 Spec 2 | 0.117 | 0.00398 | 0.315 | 0.117 | 5.43 | 0.0748 | 0.0208 | 0.0187 | 0.103 |
|  | 81% | 100% | 82% | 80% | 82% | 83% | 81% | 86% | 82% |
|  | 23% | 2% | 27% | 23% | 40% | 18% | 14% | 10% | 23% |
| Cutoff 3 Sens 3 Spec 3 | 0.0132 | 0.00398 | 0.0132 | 0.0190 | 0.338 | 0.0187 | 0.0179 | 0.0184 | 0.0179 |
|  | 94% | 100% | 95% | 91% | 94% | 92% | 92% | 93% | 91% |
|  | 3% | 2% | 4% | 12% | 29% | 11% | 7% | 9% | 8% |
| Cutoff 4 Sens 4 Spec 4 | 17.2 | 19.3 | 18.0 | 17.2 | 19.3 | 18.0 | 17.2 | 19.3 | 18.0 |
|  | 42% | 11% | 41% | 37% | 53% | 35% | 32% | 29% | 38% |
|  | 70% | 70% | 70% | 70% | 70% | 70% | 70% | 70% | 70% |
| Cutoff 5 Sens 5 Spec 5 | 25.6 | 28.1 | 25.8 | 25.6 | 28.1 | 25.8 | 25.6 | 28.1 | 25.8 |
|  | 29% | 11% | 29% | 26% | 24% | 25% | 19% | 21% | 24% |
|  | 80% | 81% | 80% | 80% | 81% | 80% | 80% | 81% | 80% |
| Cutoff 6 Sens 6 Spec 6 | 39.2 | 43.5 | 38.4 | 39.2 | 43.5 | 38.4 | 39.2 | 43.5 | 38.4 |
|  | 13% | 0% | 14% | 16% | 6% | 17% | 14% | 7% | 15% |
|  | 90% | 90% | 90% | 90% | 90% | 90% | 90% | 90% | 90% |
| OR Quart 2 p Value 95% CI of OR Quart2 | 1.1 | 0 | 1.2 | 1.5 | 5.1 | 1.1 | 0.75 | 0.25 | 0.66 |
|  | 0.86 | na | 0.69 | 0.23 | 0.14 | 0.86 | 0.59 | 0.21 | 0.44 |
|  | 0.50 | na | 0.53 | 0.77 | 0.59 | 0.54 | 0.25 | 0.027 | 0.23 |
|  | 2.3 | na | 2.6 | 3.1 | 44 | 2.1 | 2.2 | 2.2 | 1.9 |
| OR Quart 3 p Value 95% CI of OR Quart3 | 1.1 | 4.1 | 1.4 | 1.2 | 5.1 | 0.88 . | 1.3 | 1.3 | 1.1 |
|  | 0.85 | 0.21 | 0.43 | 0.58 | 0.14 | 0.72 | 0.63 | 0.74 | 0.81 |
|  | 0.50 | 0.45 | 0.63 | 0.60 | 0.59 | 0.43 | 0.49 | 0.33 | 0.44 |
|  | 2.3 | 37 | 3.0 | 2.5 | 44 | 1.8 | 3.3 | 4.7 | 2.8 |
| OR Quart 4 p Value 95% CI of OR Quart4 | 1.9 | 4.1 | 2.2 | 1.4 | 6.1 | 1.1 | 1.7 | 1.0 | 1.0 |
|  | 0.067 | 0.21 | 0.039 | 0.30 | 0.095 | 0.86 | 0.26 | 1.0 | 0.99 |
|  | 0.96 | 0.45 | 1.0 | 0.72 | 0.73 | 0.54 | 0.68 | 0.25 | 0.39 |
|  | 3.8 | 37 | 4.5 | 2.9 | 51 | 2.1 | 4.2 | 4.1 | 2.6 |

Fig. 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

**Angiopoietin-related protein 4**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 11.6 | 10.1 | 15.0 | 40.4 | 10.9 | 9.76 |
| Average | 39.6 | 39.3 | 49.2 | 70.6 | 36.1 | 43.8 |
| Stdev | 103 | 85.6 | 104 | 83.2 | 93.4 | 101 |
| p(t-test) |  | 0.99 |  | 0.44 |  | 0.59 |
| Min | 0.000466 | 1.12 | 0.577 | 5.74 | 0.000466 | 0.00194 |
| Max | 789 | 645 | 624 | 276 | 789 | 645 |
| n (Samp) | 128 | 85 | 41 | 18 | 115 | 73 |
| n (Patient) | 128 | 85 | 41 | 18 | 115 | 73 |

|  | At Enrollment | | |
|---|---|---|---|
|  | sCr or UO | sCr only | UO only |
| AUC | 0.51 | 0.66 | 0.52 |
| SE | 0.041 | 0.080 | 0.043 |
| p | 0.72 | 0.045 | 0.65 |
| nCohort 1 | 128 | 41 | 115 |
| nCohort 2 | 85 | 18 | 73 |
| Cutoff 1 | 6.86 | 12.5 | 6.65 |
| Sens 1 | 71% | 72% | 71% |
| Spec 1 | 31% | 46% | 32% |
| Cutoff 2 | 4.68 | 10.1 | 4.43 |
| Sens 2 | 80% | 83% | 81% |
| Spec 2 | 23% | 39% | 17% |
| Cutoff 3 | 3.17 | 5.81 | 2.85 |
| Sens 3 | 91% | 94% | 90% |
| Spec 3 | 12% | 24% | 9% |
| Cutoff 4 | 21.9 | 35.2 | 17.4 |
| Sens 4 | 34% | 61% | 42% |
| Spec 4 | 70% | 71% | 70% |
| Cutoff 5 | 35.2 | 55.4 | 28.9 |
| Sens 5 | 25% | 39% | 25% |
| Spec 5 | 80% | 80% | 80% |
| Cutoff 6 | 77.4 | 83.9 | 65.2 |
| Sens 6 | 11% | 22% | 16% |

(continued)

|  | At Enrollment | | |
|---|---|---|---|
|  | sCr or UO | sCr only | UO only |
| Spec 6 | 91% | 90% | 90% |
| OR Quart 2 | 0.93 | 1.3 | 0.84 |
| p Value | 0.84 | 0.74 | 0.67 |
| 95% CI of | 0.43 | 0.24 | 0.37 |
| OR Quart2 | 2.0 | 7.4 | 1.9 |
| OR Quart 3 | 0.62 | 1.3 | 0.57 |
| p Value | 0.23 | 0.74 | 0.20 |
| 95% CI of | 0.28 | 0.24 | 0.24 |
| OR Quart3 | 1.4 | 7.4 | 1.3 |
| OR Quart 4 | 0.97 | 3.2 | 1.1 |
| p Value | 0.93 | 0.16 | 0.84 |
| 95% CI of | 0.45 | 0.63 | 0.48 |
| OR Quart4 | 2.1 | 16 | 2.5 |

**Amphiregulin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 25.7 | 30.6 | 32.9 | 80.6 | 24.8 | 30.2 |
| Average | 60.4 | 121 | 69.1 | 481 | 54.7 | 39.6 |
| Stdev | 164 | 467 | 93.8 | 1010 | 170 | 40.2 |
| p(t-test) |  | 0.21 |  | 0.011 |  | 0.52 |
| Min | 1.21 | 0.00389 | 6.31 | 9.15 | 1.21 | 0.00389 |
| Max | 1640 | 3480 | 426 | 3480 | 1640 | 211 |
| n (Samp) | 113 | 67 | 41 | 13 | 96 | 54 |
| n (Patient) | 113 | 67 | 41 | 13 | 96 | 54 |

|  | At Enrollment | | |
|---|---|---|---|
|  | sCr or UO | sCr only | UO only |
| AUC | 0.52 | 0.69 | 0.53 |
| SE | 0.045 | 0.090 | 0.049 |
| p | 0.59 | 0.031 | 0.60 |
| nCohort 1 | 113 | 41 | 96 |
| nCohort 2 | 67 | 13 | 54 |

(continued)

| | At Enrollment | | |
|---|---|---|---|
| | sCr or UO | sCr only | UO only |
| Cutoff 1 Sens 1 Spec 1 | 16.3 | 37.8 | 16.8 |
| | 70% | 77% | 70% |
| | 33% | 61% | 34% |
| Cutoff 2 Sens 2 Spec 2 | 12.2 | 28.1 | 14.0 |
| | 81% | 85% | 81% |
| | 21% | 46% | 25% |
| Cutoff 3 Sens 3 Spec 3 | 9.94 | 9.15 | 10.5 |
| | 91% | 92% | 91% |
| | 16% | 12% | 17% |
| Cutoff 4 Sens 4 Spec 4 | 42.2 | 59.5 | 42.2 |
| | 31% | 62% | 30% |
| | 71% | 71% | 71% |
| Cutoff 5 Sens 5 Spec 5 | 56.0 | 82.1 | 50.9 |
| | 22% | 46% | 19% |
| | 81% | 80% | 80% |
| Cutoff 6 Sens 6 Spec 6 | 96.9 | 189 | 72.6 |
| | 12% | 31% | 13% |
| | 90% | 90% | 91% |
| OR Quart 2 p Value 95% CI of OR Quart2 | 0.91 | 0.42 | 0.96 |
| | 0.82 | 0.51 | 0.94 |
| | 0.38 | 0.034 | 0.36 |
| | 2.2 | 5.3 | 2.5 |
| OR Quart 3 p Value 95% CI of OR Quart3 | 1.4 | 2.4 | 2.0 |
| | 0.39 | 0.36 | 0.16 |
| | 0.62 | 0.36 | 0.77 |
| | 3.4 | 17 | 5.1 |
| OR Quart 4 | 1.0 | 4.1 | 0.96 |
| p Value | 1.0 | 0.13 | 0.94 |
| 95% CI of | 0.42 | 0.65 | 0.36 |
| OR Quart4 | 2.4 | 26 | 2.5 |

**Endostatin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 5200 | 5720 | 5200 | 31100 | 5620 | 5490 |

(continued)

|  | sCr or UO | | sCr only | | UO only | |
| --- | --- | --- | --- | --- | --- | --- |
|  | Cohort 1 | Cohort 2 | Cohort 1 | Cohort 2 | Cohort 1 | Cohort 2 |
| Average | 14300 | 15400 | 16300 | 58800 | 14100 | 14600 |
| Stdev | 24200 | 28400 | 25000 | 67700 | 25600 | 27700 |
| p(t-test) |  | 0.75 |  | 2.6E-4 |  | 0.89 |
| Min | 0.0130 | 389 | 300 | 860 | 0.0130 | 389 |
| Max | 145000 | 173000 | 116000 | 190000 | 180000 | 148000 |
| n (Samp) | 155 | 96 | 51 | 19 | 138 | 83 |
| n (Patient) | 155 | 96 | 51 | 19 | 138 | 83 |

|  | At Enrollment | | |
| --- | --- | --- | --- |
|  | sCr or UO | sCr only | UO only |
| AUC | 0.53 | 0.73 | 0.51 |
| SE | 0.038 | 0.072 | 0.040 |
| p | 0.41 | 0.0012 | 0.74 |
| nCohort 1 | 155 | 51 | 138 |
| nCohort 2 | 96 | 19 | 83 |
| Cutoff 1 | 3630 | 9260 | 4360 |
| Sens 1 | 71% | 74% | 71% |
| Spec 1 | 39% | 65% | 42% |
| Cutoff 2 | 2820 | 4640 | 3080 |
| Sens 2 | 80% | 84% | 81% |
| Spec 2 | 28% | 45% | 27% |
| Cutoff 3 | 1600 | 2300 | 1850 |
| Sens 3 | 91% | 95% | 90% |
| Spec 3 | 11% | 29% | 12% |
| Cutoff 4 | 9260 | 12800 | 10500 |
| Sens 4 | 31% | 63% | 24% |
| Spec 4 | 70% | 71% | 70% |
| Cutoff 5 | 19200 | 21900 | 18200 |
| Sens 5 | 19% | 58% | 17% |
| Spec 5 | 80% | 80% | 80% |
| Cutoff 6 | 41300 | 57800 | 35800 |
| Sens 6 | 8% | 32% | 10% |
| Spec 6 | 90% | 90% | 91% |
| OR Quart 2 | 1.7 | 1.5 | 1.6 |
| p Value | 0.16 | 0.68 | 0.24 |
| 95% CI of | 0.81 | 0.22 | 0.73 |

(continued)

|  | At Enrollment | | |
|---|---|---|---|
|  | sCr or UO | sCr only | UO only |
| OR Quart2 | 3.5 | 10 | 3.5 |
| OR Quart 3 | 1.6 | 2.3 | 1.5 |
| p Value | 0.22 | 0.38 | 0.33 |
| 95% CI of | 0.76 | 0.36 | 0.68 |
| OR Quart3 | 3.3 | 15 | 3.2 |
| OR Quart 4 | 1.4 | 9.4 | 0.97 |
| p Value | 0.38 | 0.012 | 0.95 |
| 95% CI of | 0.66 | 1.6 | 0.44 |
| OR Quart4 | 2.9 | 54 | 2.2 |

**Fibroblast growth factor 19**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 3.21E-5 | 2.68E-5 | 4.39E-5 | 2.68E-5 | 2.83E-5 | 2.83E-5 |
| Average | 0.00453 | 0.00701 | 0.000785 | 0.00265 | 0.00506 | 0.00880 |
| Stdev | 0.0421 | 0.0426 | 0.00286 | 0.00580 | 0.0445 | 0.0466 |
| p(t-test) |  | 0.68 |  | 0.10 |  | 0.58 |
| Min | 5.62E-6 | 5.62E-6 | 5.62E-6 | 1.66E-5 | 5.62E-6 | 5.62E-6 |
| Max | 0.476 | 0.388 | 0.0164 | 0.0157 | 0.476 | 0.388 |
| n (Samp) | 128 | 85 | 41 | 18 | 115 | 73 |
| n (Patient) | 128 | 85 | 41 | 18 | 115 | 73 |

|  | At Enrollment | | |
|---|---|---|---|
|  | sCr or UO | sCr only | UO only |
| AUC | 0.48 | 0.55 | 0.49 |
| SE | 0.041 | 0.083 | 0.043 |
| p | 0.54 | 0.51 | 0.84 |
| nCohort 1 | 128 | 41 | 115 |
| nCohort 2 | 85 | 18 | 73 |
| Cutoff 1 | 1.92E-5 | 2.53E-5 | 2.17E-5 |
| Sens 1 | 75% | 72% | 71% |
| Spec 1 | 23% | 37% | 29% |
| Cutoff 2 | 1.82E-5 | 2.17E-5 | 1.82E-5 |
| Sens 2 | 86% | 83% | 85% |

(continued)

|  | At Enrollment | | |
|---|---|---|---|
|  | sCr or UO | sCr only | UO only |
| Spec 2 | 13% | 32% | 15% |
| Cutoff 3 | 1.66E-5 | 1.82E-5 | 1.66E-5 |
| Sens 3 | 91% | 94% | 90% |
| Spec 3 | 10% | 10% | 11% |
| Cutoff 4 | 0.000114 | 8.63E-5 | 8.63E-5 |
| Sens 4 | 21% | 39% | 23% |
| Spec 4 | 82% | 71% | 72% |
| Cutoff 5 | 0.000114 | 0.000114 | 0.000114 |
| Sens 5 | 21% | 33% | 19% |
| Spec 5 | 82% | 83% | 83% |
| Cutoff 6 | 0.00127 | 0.000197 | 0.000197 |
| Sens 6 | 15% | 22% | 18% |
| Spec 6 | 91% | 90% | 90% |
| OR Quart 2 | 0.69 | 6.9 | 1.6 |
| p Value | 0.35 | 0.037 | 0.29 |
| 95% CI of | 0.31 | 1.1 | 0.68 |
| OR Quart2 | 1.5 | 42 | 3.6 |
| OR Quart 3 | 1.2 | 0.92 | 1.2 |
| p Value | 0.64 | 0.94 | 0.67 |
| 95% CI of | 0.56 | 0.11 | 0.52 |
| OR Quart3 | 2.6 | 7.6 | 2.8 |
| OR Quart 4 | 1.0 | 4.0 | 1.2 |
| p Value | 0.94 | 0.14 | 0.67 |
| 95% CI of | 0.48 | 0.65 | 0.52 |
| OR Quart4 | 2.2 | 25 | 2.8 |

**Fibroblast growth factor 21**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.0125 | 0.0121 | 0.0255 | 0.0979 | 0.0100 | 0.0112 |
| Average | 0.122 | 0.193 | 0.147 | 0.432 | 0.132 | 0.180 |
| Stdev | 0.413 | 0.614 | 0.477 | 0.837 | 0.443 | 0.580 |
| p(t-test) |  | 0.31 |  | 0.10 |  | 0.52 |
| Min | 1.40E-9 | 1.40E-9 | 4.16E-6 | 0.00126 | 1.40E-9 | 1.40E-9 |
| Max | 2.88 | 3.58 | 2.88 | 3.42 | 3.07 | 3.58 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| n (Samp) | 128 | 85 | 41 | 18 | 115 | 73 |
| n (Patient) | 128 | 85 | 41 | 18 | 115 | 73 |

|  | At Enrollment | | |
|---|---|---|---|
|  | sCr or UO | sCr only | UO only |
| AUC | 0.52 | 0.70 | 0.52 |
| SE | 0.041 | 0.078 | 0.043 |
| p | 0.58 | 0.012 | 0.70 |
| nCohort 1 | 128 | 41 | 115 |
| nCohort 2 | 85 | 18 | 73 |
| Cutoff 1 | 0.00522 | 0.0330 | 0.00432 |
| Sens 1 | 71% | 72% | 71% |
| Spec 1 | 30% | 63% | 28% |
| Cutoff 2 | 0.00355 | 0.0192 | 0.00311 |
| Sens 2 | 80% | 83% | 81% |
| Spec 2 | 24% | 46% | 23% |
| Cutoff 3 | 0.00187 | 0.00324 | 0.00124 |
| Sens 3 | 91% | 94% | 90% |
| Spec 3 | 16% | 12% | 10% |
| Cutoff 4 | 0.0297 | 0.0419 | 0.0263 |
| Sens 4 | 34% | 61% | 34% |
| Spec 4 | 70% | 71% | 70% |
| Cutoff 5 | 0.0509 | 0.0696 | 0.0386 |
| Sens 5 | 24% | 50% | 26% |
| Spec 5 | 80% | 80% | 80% |
| Cutoff 6 | 0.145 | 0.135 | 0.256 |
| Sens 6 | 16% | 44% | 14% |
| Spec 6 | 91% | 90% | 90% |
| OR Quart 2 | 1.0 | 1.5 | 0.84 |
| p Value | 1.0 | 0.69 | 0.67 |
| 95% CI of | 0.46 | 0.21 | 0.36 |
| OR Quart2 | 2.2 | 11 | 1.9 |
| OR Quart 3 | 0.92 | 2.2 | 0.84 |
| p Value | 0.84 | 0.42 | 0.67 |
| 95% CI of | 0.42 | 0.33 | 0.36 |
| OR Quart3 | 2.0 | 14 | 1.9 |

(continued)

|  | At Enrollment | | |
|---|---|---|---|
|  | sCr or UO | sCr only | UO only |
| OR Quart 4 | 1.1 | 9.0 | 1.1 |
| p Value | 0.75 | 0.018 | 0.83 |
| 95% CI of | 0.52 | 1.5 | 0.48 |
| OR Quart4 | 2.4 | 55 | 2.5 |

Fig. 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

**Angiogenin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 6570 | 20400 | 6570 | 20400 | 6570 | 20400 |
| Average | 9580 | 18900 | 9580 | 17700 | 9580 | 17200 |
| Stdev | 8320 | 9570 | 8320 | 10100 | 8320 | 9450 |
| p(t-test) |  | 5.9E-9 |  | 6.4E-7 |  | 8.5E-5 |
| Min | 77.7 | 647 | 77.7 | 647 | 77.7 | 1090 |
| Max | 30600 | 30600 | 30600 | 30600 | 30600 | 30600 |
| n (Samp) | 203 | 36 | 203 | 34 | 203 | 22 |
| n (Patient) | 203 | 36 | 203 | 34 | 203 | 22 |
| sCr only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 11200 | 22300 | 11200 | 21100 | 11200 | 20400 |
| Average | 13000 | 21900 | 13000 | 20600 | 13000 | 19400 |
| Stdev | 9540 | 8480 | 9540 | 9460 | 9540 | 8650 |
| p(t-test) |  | 5.9E-5 |  | 6.3E-4 |  | 0.011 |
| Min | 0.00873 | 647 | 0.00873 | 647 | 0.00873 | 1820 |
| Max | 30600 | 30600 | 30600 | 30600 | 30600 | 30600 |
| n (Samp) | 395 | 20 | 395 | 20 | 395 | 15 |
| n (Patient) | 395 | 20 | 395 | 20 | 395 | 15 |
| UO only | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
| Median | 8170 | 20400 | 8170 | 20400 | 8170 | 20400 |
| Average | 10900 | 18000 | 10900 | 17200 | 10900 | 17600 |
| Stdev | 8900 | 9830 | 8900 | 10200 | 8900 | 10400 |
| p(t-test) |  | 5.0E-4 |  | 0.0029 |  | 0.0090 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 77.7 | 2260 | 77.7 | 1060 | 77.7 | 1090 |
| Max | 30600 | 30600 | 30600 | 30600 | 30600 | 30600 |
| n (Samp) | 215 | 22 | 215 | 20 | 215 | 13 |
| n (Patient) | 215 | 22 | 215 | 20 | 215 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | 0.76 | 0.71 | 0.72 | 0.72 | 0.68 | 0.73 | 0.70 | 0.69 |
| SE | 0.048 | 0.064 | 0.064 | 0.052 | 0.066 | 0.068 | 0.064 | 0.077 | 0.084 |
| p | 4.7E-8 | 6.2E-5 | 0.0013 | 1.9E-5 | 0.0011 | 0.010 | 3.8E-4 | 0.011 | 0.023 |
| nCohort 1 | 203 | 395 | 215 | 203 | 395 | 215 | 203 | 395 | 215 |
| nCohort 2 | 36 | 20 | 22 | 34 | 20 | 20 | 22 | 15 | 13 |
| Cutoff 1 | 14600 | 20300 | 10900 | 11000 | 20300 | 10900 | 10000 | 15600 | 10000 |
| Sens 1 | 72% | 80% | 73% | 71% | 75% | 70% | 73% | 73% | 77% |
| Spec 1 | 74% | 68% | 60% | 65% | 68% | 60% | 63% | 59% | 58% |
| Cutoff 2 | 9130 | 20300 | 5040 | 5010 | 15900 | 5040 | 7710 | 11700 | 5040 |
| Sens 2 | 81% | 80% | 82% | 82% | 80% | 80% | 82% | 80% | 85% |
| Spec 2 | 61% | 68% | 39% | 44% | 60% | 39% | 56% | 52% | 39% |
| Cutoff 3 | 3680 | 9330 | 3680 | 2230 | 6050 | 3010 | 3010 | 7710 | 3010 |
| Sens 3 | 92% | 90% | 91% | 91% | 90% | 90% | 91% | 93% | 92% |
| Spec 3 | 33% | 46% | 29% | 19% | 35% | 21% | 25% | 41% | 21% |
| Cutoff 4 | 12900 | 20400 | 16200 | 12900 | 20400 | 16200 | 12900 | 20400 | 16200 |
| Sens 4 | 72% | 55% | 59% | 68% | 50% | 60% | 64% | 40% | 62% |
| Spec 4 | 70% | 79% | 70% | 70% | 79% | 70% | 70% | 79% | 70% |
| Cutoff 5 | 20000 | 20900 | 20400 | 20000 | 20900 | 20400 | 20000 | 20900 | 20400 |
| Sens 5 | 64% | 55% | 36% | 59% | 50% | 35% | 59% | 40% | 31% |
| Spec 5 | 80% | 80% | 87% | 80% | 80% | 87% | 80% | 80% | 87% |
| Cutoff 6 | 20400 | 27700 | 24000 | 20400 | 27700 | 24000 | 20400 | 27700 | 24000 |
| Sens 6 Spec 6 | 42% | 35% | 27% | 38% | 30% | 25% | 32% | 20% | 31% |
| | 90% | 90% | 90% | 90% | 90% | 90% | 90% | 90% | 90% |
| OR Quart 2 p Value 95% CI of OR Quart2 | 1.3 | 2.0 | 1.5 | 0.58 | 0.99 | 0.23 | 0.32 | 2.0 | 0.49 |
| | 0.71 | 0.57 | 0.65 | 0.47 | 0.99 | 0.20 | 0.33 | 0.57 | 0.57 |
| | 0.29 | 0.18 | 0.25 | 0.13 | 0.14 | 0.025 | 0.032 | 0.18 | 0.043 |
| | 6.2 | 22 | 9.5 | 2.5 | 7.2 | 2.1 | 3.2 | 22 | 5.6 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 3 p Value 95% CI of OR Quart3 | 2.9 | 6.2 | 2.6 | 1.2 | 3.1 | 0.98 | 1.7 | 6.3 | 1.0 |
| | 0.13 | 0.093 | 0.26 | 0.75 | 0.17 | 0.98 | 0.47 | 0.091 | 1.0 |
| | 0.72 | 0.74 | 0.49 | 0.35 | 0.61 | 0.23 | 0.39 | 0.75 | 0.14 |
| | 11 | 53 | 14 | 4.2 | 16 | 4.1 | 7.6 | 53 | 7.4 |
| OR Quart p Value 95% CI of OR Quart4 | 10 | 12 | 7.1 | 5.4 | 5.4 | 3.1 | 5.2 | 6.2 | 4.5 |
| | 4.0E-4 | 0.018 | 0.013 | 0.0019 | 0.033 | 0.067 | 0.014 | 0.093 | 0.065 |
| | 2.8 | 1.5 | 1.5 | 1.9 | 1.1 | 0.92 | 1.4 | 0.74 | 0.91 |
| | 36 | 95 | 33 | 16 | 25 | 10 | 19 | 53 | 22 |

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 16.9 | 49.1 | 16.9 | 29.8 | 16.9 | 18.9 |
| Average | 56.0 | 143 | 56.0 | 139 | 56.0 | 108 |
| Stdev | 99.2 | 195 | 99.2 | 202 | 99.2 | 221 |
| p(t-test) | | 3.0E-4 | | 7.6E-4 | | 0.079 |
| Min | 0.00423 | 4.54 | 0.00423 | 4.54 | 0.00423 | 6.66 |
| Max | 538 | 878 | 538 | 878 | 538 | 878 |
| n (Samp) | 151 | 32 | 151 | 30 | 151 | 18 |
| n (Patient) | 151 | 32 | 151 | 30 | 151 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.3 | 37.6 | 21.3 | 30.7 | 21.3 | 21.5 |
| Average | 74.3 | 160 | 74.3 | 155 | 74.3 | 137 |
| Stdev | 157 | 230 | 157 | 229 | 157 | 255 |
| p(t-test) | | 0.030 | | 0.041 | | 0.17 |
| Min | 0.00423 | 14.1 | 0.00423 | 14.1 | 0.00423 | 9.39 |
| Max | 1370 | 878 | 1370 | 878 | 1370 | 878 |
| n (Samp) | 317 | 18 | 317 | 18 | 317 | 13 |
| n (Patient) | 317 | 18 | 317 | 18 | 317 | 13 |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 19.2 | 36.5 | 19.2 | 19.8 | 19.2 | 12.0 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 64.1 | 96.0 | 64.1 | 89.8 | 64.1 | 25.4 |
| Stdev | 109 | 130 | 109 | 139 | 109 | 33.3 |
| p(t-test) | | 0.24 | | 0.37 | | 0.27 |
| Min | 0.00423 | 4.54 | 0.00423 | 4,54 | 0.00423 | 6.66 |
| Max | 624 | 400 | 624 | 400 | 624 | 116 |
| n (Samp) | 172 | 19 | 172 | 17 | 172 | 10 |
| n (Patient) | 172 | 19 | 172 | 17 | 172 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.68 | 0.63 | 0.67 | 0.67 | 0.57 | 0.57 | 0.58 | 0.42 |
| SE | 0.055 | 0.071 | 0.072 | 0.058 | 0.072 | 0.075 | 0.074 | 0.084 | 0.097 |
| p | 1.4E-4 | 0.011 | 0.076 | 0.0027 | 0.021 | 0.35 | 0.35 | 0.34 | 0.41 |
| nCohort 1 | 151 | 317 | 172 | 151 | 317 | 172 | 151 | 317 | 172 |
| nCohort 2 | 32 | 18 | 19 | 30 | 18 | 17 | 18 | 13 | 10 |
| Cutoff 1 | 21.0 | 21.5 | 16.6 | 17.8 | 20.7 | 16.2 | 13.7 | 16.6 | 10.6 |
| Sens 1 | 72% | 72% | 74% | 70% | 72% | 71% | 72% | 77% | 70% |
| Spec 1 | 56% | 50% | 46% | 52% | 48% | 44% | 44% | 42% | 28% |
| Cutoff 2 | 16.9 | 17.8 | 12.5 | 16.6 | 16.9 | 11.4 | 11.2 | 16.2 | 9.29 |
| Sens 2 | 81% | 83% | 84% | 80% | 83% | 82% | 83% | 85% | 80% |
| Spec 2 | 50% | 44% | 38% | 49% | 43% | 33% | 34% | 41% | 26% |
| Cutoff 3 | 13.7 | 16.2 | 10.6 | 12.5 | 16.2 | 10.6 | 9.06 | 14.0 | 9.06 |
| Sens 3 | 91% | 94% | 95% | 90% | 94% | 94% | 94% | 92% | 90% |
| Spec 3 | 44% | 41% | 28% | 42% | 41% | 28% | 29% | 37% | 26% |
| Cutoff 4 | 35.6 | 39.5 | 48.2 | 35.6 | 39.5 | 48.2 | 35.6 | 39.5 | 48.2 |
| Sens 4 | 56% | 50% | 47% | 50% | 44% | 29% | 28% | 31% | 10% |
| Spec 4 | 70% | 70% | 70% | 70% | 70% | 70% | 70% | 70% | 70% |
| Cutoff 5 | 76.7 | 76.7 | 84.1 | 76.7 | 76.7 | 84.1 | 76.7 | 76.7 | 84.1 |
| Sens 5 | 41% | 33% | 32% | 33% | 33% | 24% | 22% | 23% | 10% |
| Spec 5 | 80% | 80% | 80% | 80% | 80% | 80% | 80% | 80% | 80% |
| Cutoff 6 | 151 | 207 | 170 | 151 | 207 | 170 | 151 | 207 | 170 |
| Sens 6 | 28% | 33% | 16% | 30% | 28% | 18% | 17% | 23% | 0% |
| Spec 6 | 90% | 90% | 90% | 90% | 90% | 90% | 90% | 90% | 90% |
| OR Quart 2 | 7.9 0.058 | >6.4 <0.089 | 6.6 0.087 | 11 0.026 | >7.5 <0.061 | 8.0 0.056 | 8.2 0.054 | 5.2 0.14 | 2.1 0.55 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.93 | >0.75 | 0.76 | 1.3 | >0.91 | 0.95 | 0.96 | 0.59 | 0.18 |
| 95% CI ot OR Quart2 | 67 | na | 57 | 91 | na | 68 | 70 | 45 | 24 |
| OR Quart 3 | 14 | >4.2 | 5.3 | 13 | >4.2 | 5.5 | 6.8 | 4.2 | 6.8 |
| | 0.014 | <0.21 | 0.13 | 0.018 | <0.21 | 0.13 | 0.082 | 0.21 | 0.083 |
| p Value 95% CI of OR Quart3 | 1.7 | >0.45 | 0.60 | 1.5 | >0.45 | 0.61 | 0.79 | 0.45 | 0.78 |
| | 110 | na | 48 | 100 | na | 49 | 59 | 38 | 58 |
| OR Quart 4 p Value 95% CI of OR Quart4 | 17 | >8.7 | 7.9 | 12 | >7.5 | 4.2 | 4.2 | 3.0 | 1.0 |
| | 0.0073 | <0.043 | 0.059 | 0.020 | <0.061 | 0.21 | 0.21 | 0.34 | 0.99 |
| | 2.2 | >1.1 | 0.93 | 1.5 | >0.91 | 0.45 | 0.45 | 0.31 | 0.062 |
| | 140 | na | 67 | 100 | na | 39 | 39 | 30 | 17 |

**Angiopoietin-related protein 6**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.292 | 0.543 | 0.292 | 0.257 | 0.292 | 0.183 |
| Average | 1.72 | 2.40 | 1.72 | 2.14 | 1.72 | 1.06 |
| Stdev | 3.60 | 4.44 | 3.60 | 4.57 | 3.60 | 1.92 |
| p(t-test) | | 0.38 | | 0.60 | | 0.47 |
| Min | 0.000122 | 0.000364 | 0.000122 | 0.000157 | 0.000122 | 0.000206 |
| Max | 25.9 | 22.4 | 25.9 | 22.4 | 25.9 | 6.31 |
| n (Samp) | 141 | 29 | 141 | 27 | 141 | 16 |
| n (Patient) | 141 | 29 | 141 | 27 | 141 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.421 | 0.399 | 0.421 | 0.399 | 0.421 | 0.218 |
| Average | 2.19 | 1.75 | 2.19 | 1.55 | 2.19 | 1.39 |
| Stdev | 5.66 | 2.25 | 5.66 | 2.20 | 5.66 | 2.13 |
| p(t-test) | | 0.76 | | 0.66 | | 0.63 |
| Min | 0.000122 | 0.000364 | 0.000122 | 0.000364 | 0.000122 | 0.000364 |
| Max | 70.5 | 6.31 | 70.5 | 6.31 | 70.5 | 6.31 |
| n (Samp) | 292 | 16 | 292 | 16 | 292 | 12 |
| n (Patient) | 292 | 16 | 292 | 16 | 292 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.360 | 0.282 | 0.360 | 0.210 | 0.360 | 0.00357 |
| Average | 1.80 | 2.56 | 1.80 | 2.33 | 1.80 | 0.178 |
| Stdev | 3.47 | 5.39 | 3.47 | 5.68 | 3.47 | 0.269 |
| p(t-test) | | 0.41 | | 0.59 | | 0.16 |
| Min | 0.000122 | 0.000364 | 0.000122 | 0.000157 | 0.000122 | 0.000206 |
| Max | 25.9 | 22.4 | 25.9 | 22.4 | 25.9 | 0.802 |
| n (Samp) | 162 | 18 | 162 | 16 | 162 | 9 |
| n (Patient) | 162 | 18 | 162 | 16 | 162 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.55 | 0.52 | 0.53 | 0.52 | 0.45 | 0.45 | 0.48 | 0.30 |
| SE | 0.060 | 0.076 | 0.073 | 0.061 | 0.075 | 0.077 | 0.078 | 0.086 | 0.100 |
| p | 0.15 | 0.48 | 0.74 | 0.68 | 0.75 | 0.50 | 0.50 | 0.84 | 0.047 |
| nCohort 1 | 141 | 292 | 162 | 141 | 292 | 162 | 141 | 292 | 162 |
| nCohort 2 | 29 | 16 | 18 | 27 | 16 | 16 | 16 | 12 | 9 |
| Cutoff 1 | 0.153 | 0.153 | 0.114 | 0.111 | 0.153 | 0.00224 | 0.00224 | 0.0480 | 0.00224 |
| Sens 1 | 72% | 75% | 72% | 70% | 75% | 88% | 81% | 75% | 78% |
| Spec 1 | 41% | 36% | 32% | 38% | 36% | 19% | 19% | 31% | 19% |
| Cutoff 2 | 0.0628 | 0.119 | 0.00224 | 0.00224 | 0.0480 | 0.00224 | 0.00224 | 0.00224 | 0.000336 |
| Sens 2 | 83% | 81% | 94% | 89% | 81% | 88% | 81% | 83% | 89% |
| Spec 2 | 35% | 34% | 19% | 19% | 31% | 19% | 19% | 19% | 10% |
| Cutoff 3 | 0.00224 | 0.00224 | 0.00224 | 0.000336 | 0.000336 | 0.000336 | 0.000336 | 0.000336 | 0.000205 |
| Sens 3 | 97% | 94% | 94% | 96% | 100% | 94% | 94% | 100% | 100% |
| Spec 3 | 19% | 19% | 19% | 11% | 10% | 10% | 11% | 10% | 1% |
| Cutoff 4 | 1.28 | 1.25 | 1.45 | 1.28 | 1.25 | 1.45 | 1.28 | 1.25 | 1.45 |
| Sens 4 | 34% | 38% | 33% | 26% | 31% | 25% | 19% | 33% | 0% |
| Spec 4 | 70% | 70% | 70% | 70% | 70% | 70% | 70% | 70% | 70% |
| Cutoff 5 | 2.60 | 2.44 | 2.67 | 2.60 | 2.44 | 2.67 | 2.60 | 2.44 | 2.67 |
| Sens 5 | 31% | 31% | 28% | 26% | 25% | 25% | 19% | 25% | 0% |
| Spec 5 | 80% | 80% | 80% | 80% | 80% | 80% | 80% | 80% | 80% |
| Cutoff 6 | 4.06 | 5.79 | 4.30 | 4.06 | 5.79 | 4.30 | 4.06 | 5.79 | 4.30 |
| Sens 6 | 21% | 6% | 17% | 19% | 6% | 12% | 12% | 8% | 0% |
| Spec 6 | 90% | 90% | 90% | 90% | 90% | 90% | 90% | 90% | 90% |
| OR Quart 2 | 2.0 | 3.2 | 1.6 | 1.0 | 1.7 | 0.24 | 1.0 | 0.66 | >2.1 |
| | 0.27 | 0.17 | 0.50 | 1.0 | 0.47 | 0.21 | 0.97 | 0.65 | <0.55 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 0.60 | 0.62 | 0.41 | 0.32 | 0.39 | 0.026 | 0.19 | 0.11 | >0.18 |
| 95% CI of OR Quart2 | 6.4 | 16 | 6.0 | 3.2 | 7.4 | 2.2 | 5.4 | 4.1 | na |
| OR Quart 3 | 1.2 | 1.5 | 0.73 | 0.83 | 1.4 | 1.6 | 1.4 | 1.4 | >3.2 |
| | 0.75 | 0.65 | 0.69 | 0.76 | 0.70 | 0.50 | 0.67 | 0.70 | <0.32 |
| p Value | 0.35 | 0.25 | 0.15 | 0.25 | 0.29 | 0.41 | 0.29 | 0.29 | >0.32 |
| 95% CI OR Quart3 | 4.4 | 9.4 | 3.5 | 2.7 | 6.3 | 6.0 | 6.8 | 6.3 | na |
| OR Quart 4 | 2.0 | 2.6 | 1.3 | 1.0 | 1.4 | 1.3 | 2.2 | 1.0 | >4.5 |
| | 0.27 | 0.26 | 0.73 | 1.0 | 0.70 | 0.70 | 0.28 | 1.0 | <0.19 |
| p Value | 0.60 | 0.49 | 0.32 | 0.32 | 0.29 | 0.33 | 0.52 | 0.20 | >0.48 |
| 95% CI of OR Quart4 | 6.4 | 14 | 5.1 | 3.2 | 6.3 | 5.3 | 9.7 | 5.1 | na |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 26.7 | 87.0 | 26.7 | 75.5 | 26.7 | 56.9 |
| Average | 60.5 | 383 | 60.5 | 374 | 60.5 | 94.2 |
| Stdev | 127 | 783 | 127 | 799 | 127 | 92.9 |
| p(t-test) | 4.4E-6 | 4.4E-6 | | 1.1E-5 | | 0.29 |
| Min | 0.140 | 12.7 | 0.140 | 12.7 | 0.140 | 23.2 |
| Max | 1310 | 3480 | 1310 | 3480 | 1310 | 364 |
| n (Samp) | 148 | 28 | 148 | 27 | 148 | 17 |
| n (Patient) | 148 | 28 | 148 | 27 | 148 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 33.6 | 129 | 33.6 | 73.8 | 33.6 | 73.2 |
| Average | 85.0 | 254 | 85.0 | 239 | 85.0 | 114 |
| Stdev | 230 | 424 | 230 | 426 | 230 | 99.8 |
| p(t-test) | | 0.0090 | | 0.017 | | 0.66 |
| Min | 0.140 | 12.7 | 0.140 | 12.7 | 0.140 | 23.2 |
| Max | 2190 | 1710 | 2190 | 1710 | 2190 | 364 |
| n (Samp) | 288 | 15 | 288 | 15 | 288 | 12 |
| n (Patient) | 288 | 15 | 288 | 15 | 288 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 33.6 | 77.2 | 33.6 | 75.5 | 33.6 | 39.8 |
| Average | 59.7 | 422 | 59.7 | 424 | 59.7 | 67.7 |
| Stdev | 80.1 | 914 | 80.1 | 941 | 80.1 | 79.0 |
| p(t-test) | | 3.1E-6 | 4.8E-6 | 4.8E-6 | | 0.76 |
| Min | 0.140 | 13.4 | 0.140 | 13.4 | 0.140 | 13.4 |
| Max | 480 | 3480 | 480 | 3480 | 480 | 273 |
| n (Samp) | 152 | 18 | 152 | 17 | 152 | 10 |
| n (Patient) | 152 | 18 | 152 | 17 | 152 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | 0.77 | 0.73 | 0.79 | 0.76 | 0.72 | 0.74 | 0.76 | 0.57 |
| SE | 0.052 | 0.072 | 0.070 | 0.054 | 0.073 | 0.073 | 0.071 | 0.082 | 0.097 |
| p | 7.1E-9 | 1.5E-4 | 8.2E-4 | 8.8E-8 | 3.3E-4 | 0.0026 | 7.5E-4 | 0.0014 | 0.45 |
| nCohort 1 | 148 | 288 | 152 | 148 | 288 | 152 | 148 | 288 | 152 |
| nCohort 2 | 28 | 15 | 18 | 27 | 15 | 17 | 17 | 12 | 10 |
| Cutoff 1 | 54.4 | 56.0 | 42.9 | 54.4 | 56.0 | 42.9 | 45.3 | 56.0 | 25.6 |
| Sens 1 | 71% | 73% | 72% | 70% | 73% | 71% | 71% | 75% | 70% |
| Spec 1 | 75% | 72% | 57% | 75% | 72% | 57% | 66% | 72% | 41% |
| Cutoff 2 | 40.8 | 40.8 | 33.6 | 40.8 | 40.8 | 33.6 | 33.6 | 40.8 | 24.6 |
| Sens 2 | 82% | 80% | 83% | 81% | 80% | 82% | 82% | 83% | 80% |
| Spec 2 | 62% | 56% | 51% | 62% | 56% | 51% | 57% | 56% | 39% |
| Cutoff 3 | 25.6 | 33.6 | 24.6 | 25.6 | 33.6 | 24.6 | 24.6 | 33.6 | 22.0 |
| Sens 3 | 93% | 93% | 94% | 93% | 93% | 94% | 94% | 92% | 90% |
| Spec 3 | 49% | 50% | 39% | 49% | 50% | 39% | 46% | 50% | 36% |
| Cutoff 4 | 48.2 | 54.5 | 53.4 | 48.2 | 54.5 | 53.4 | 48.2 | 54.5 | 53.4 |
| Sens 4 | 75% | 73% | 61% | 74% | 73% | 59% | 65% | 75% | 30% |
| Spec 4 | 70% | 70% | 70% | 70% | 70% | 70% | 70% | 70% | 70% |
| Cutoff 5 | 64.2 | 72.7 | 74.9 | 64.2 | 72.7 | 74.9 | 64.2 | 72.7 | 74.9 |
| Sens 5 | 64% | 67% | 56% | 63% | 60% | 53% | 47% | 58% | 20% |
| Spec 5 | 80% | 80% | 80% | 80% | 80% | 80% | 80% | 80% | 80% |
| Cutoff 6 | 125 | 133 | 128 | 125 | 133 | 128 | 125 | 133 | 128 |
| Sens 6 | 46% | 47% | 39% | 41% | 40% | 35% | 24% | 25% | 20% |
| Spec 6 | 91% | 90% | 90% | 91% | 90% | 90% | 91% | 90% | 90% |
| OR Quart 2 | 3.1 | 2.0 | 4.2 | 2.0 | 2.0 | 4.3 | >3.2 | >2.1 | 3.1 |
| | 0.33 | 0.57 | 0.21 | 0.58 | 0.57 | 0.20 | <0.32 | <0.56 | 0.34 |
| p Value 95% CI of OR Quart2 | 0.31 | 0.18 | 0.45 | 0.17 | 0.18 | 0.46 | >0.32 | >0.18 | 0.31 |
| | 31 | 23 | 39 | 23 | 23 | 40 | na | na | 31 |
| OR Quart 3 | 8.1 | 2.0 | 3.2 | 9.3 | 2.0 | 3.2 | >7.0 | >2.1 | 4.3 |
| | 0.055 | 0.57 | 0.33 | 0.039 | 0.57 | 0.33 | <0.077 | <0.56 | 0.20 |
| p Value 95% CI of OR Quart3 | 0.96 | 0.18 | 0.31 | 1.1 | 0.18 | 0.31 | >0.81 | >0.18 | 0.46 |
| | 69 | 23 | 32 | 78 | 23 | 32 | na | na | 41 |
| OR Quart 4 | 27 | 11 | 12 | 24 | 11 | 11 | >9.6 | >9.0 | 2.0 |
| | 0.0018 | 0.023 | 0.019 | 0.0027 | 0.023 | 0.027 | <0.037 | <0.041 | 0.58 |
| p Value 95% CI of OR Quart4 | 3.4 | 1.4 | 1.5 | 3.0 | 1.4 | 1.3 | >1.1 | >1.1 | 0.17 |
| | 220 | 90 | 100 | 190 | 90 | 90 | na | na | 23 |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.56 | 2.45 | 1.56 | 2.48 | 1.56 | 2.48 |
| Average | 1.86 | 3.11 | 1.86 | 3.03 | 1.86 | 2.65 |
| Stdev | 2.43 | 2.60 | 2.43 | 2.72 | 2.43 | 1.27 |
| p(t-test) | | 0.015 | | 0.025 | | 0.19 |
| Min | 0.00230 | 0.00240 | 0.00230 | 0.00240 | 0.00230 | 0.00240 |
| Max | 23.1 | 11.6 | 23.1 | 11.6 | 23.1 | 4.42 |
| n (Samp) | 148 | 28 | 148 | 27 | 148 | 17 |
| n (Patient) | 148 | 28 | 148 | 27 | 148 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.72 | 2.48 | 1.72 | 2.48 | 1.72 | 2.48 |
| Average | 2.22 | 2.80 | 2.22 | 2.77 | 2.22 | 2.59 |
| Stdev | 3.19 | 1.30 | 3.19 | 1.33 | 3.19 | 1.55 |
| p(t-test) | | 0.49 | | 0.51 | | 0.70 |
| Min | 0.00230 | 0.00240 | 0.00230 | 0.00240 | 0.00230 | 0.00240 |
| Max | 28.3 | 4.42 | 28.3 | 4.42 | 28.3 | 4.42 |
| n (Samp) | 288 | 15 | 288 | 15 | 288 | 12 |
| n (Patient) | 288 | 15 | 288 | 15 | 288 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.56 | 2.09 | 1.56 | 2.17 | 1.56 | 2.29 |
| Average | 1.76 | 3.26 | 1.76 | 3.18 | 1.76 | 2.60 |
| Stdev | 1.81 | 3.09 | 1.81 | 3.28 | 1.81 | 1.09 |
| p(t-test) | | 0.0027 | | 0.0064 | | 0.15 |
| Min | 0.00230 | 0.00240 | 0.00230 | 0.00240 | 0.00230 | 1.11 |
| Max | 8.77 | 11.6 | 8.77 | 11.6 | 8.77 | 4.42 |
| n (Samp) | 152 | 18 | 152 | 17 | 152 | 10 |
| n (Patient) | 152 | 18 | 152 | 17 | 152 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.69 | 0.70 | 0.67 | 0.68 | 0.65 | 0.71 | 0.64 | 0.72 |
| SE | 0.058 | 0.078 | 0.072 | 0.061 | 0.078 | 0.075 | 0.073 | 0.088 | 0.094 |
| p | 5.3E-4 | 0.015 | 0.0064 | 0.0050 | 0.020 | 0.040 | 0.0047 | 0.11 | 0.018 |
| nCohort 1 | 148 | 288 | 152 | 148 | 288 | 152 | 148 | 288 | 152 |
| nCohort 2 | 28 | 15 | 18 | 27 | 15 | 17 | 17 | 12 | 10 |
| Cutoff 1 | 1.87 | 1.97 | 1.87 | 1.62 | 1.74 | 1.74 | 1.97 | 1.97 | 1.97 |
| Sens 1 | 71% | 73% | 72% | 70% | 73% | 71% | 71% | 75% | 70% |
| Spec 1 | 64% | 58% | 65% | 57% | 52% | 62% | 66% | 58% | 66% |
| Cutoff 2 | 1.70 | 1.74 | 1.74 | 1.51 | 1.63 | 0.909 | 1.74 | 1.63 | 1.87 |
| Sens 2 | 82% | 80% | 83% | 81% | 80% | 82% | 82% | 83% | 80% |
| Spec 2 | 57% | 52% | 62% | 49% | 49% | 40% | 61% | 49% | 65% |
| Cutoff 3 | 0.761 | 1.51 | 0.761 | 0.0209 | 1.51 | 0.0209 | 1.03 | 0.0209 | 1.74 |
| Sens 3 | 93% | 93% | 94% | 93% | 93% | 94% | 94% | 92% | 90% |
| Spec 3 | 35% | 42% | 36% | 9% | 42% | 13% | 39% | 9% | 62% |
| Cutoff 4 | 2.41 | 2.48 | 2.35 | 2.41 | 2.48 | 2.35 | 2.41 | 2.48 | 2.35 |
| Sens 4 | 54% | 47% | 44% | 56% | 47% | 47% | 59% | 42% | 50% |
| Spec 4 | 71% | 72% | 70% | 71% | 72% | 70% | 71% | 72% | 70% |
| Cutoff 5 | 2.79 | 3.06 | 3.05 | 2.79 | 3.06 | 3.05 | 2.79 | 3.06 | 3.05 |
| Sens 5 | 39% | 47% | 28% | 41% | 47% | 29% | 35% | 42% | 30% |
| Spec 5 | 80% | 80% | 80% | 80% | 80% | 80% | 80% | 80% | 80% |
| Cutoff 6 | 3.77 | 3.96 | 4.06 | 3.77 | 3.96 | 4.06 | 3.77 | 3.96 | 4.06 |
| Sens 6 | 29% | 27% | 22% | 30% | 27% | 24% | 29% | 33% | 20% |
| Spec 6 | 91% | 90% | 90% | 91% | 90% | 90% | 91% | 90% | 90% |
| OR Quart 2 | 1.0 | 2.0 | 0.98 | 0.98 | 3.0 | 1.0 | 1.0 | 0.49 | >1.0 |
| | 1.0 | 0.57 | 0.99 | 0.98 | 0.34 | 1.0 | 1.0 | 0.57 | <1.0 |
| p Value 95% CI of OR Quart2 | 0.13 | 0.18 | 0.059 | 0.19 | 0.31 | 0.13 | 0.060 | 0.044 | >0.060 |
| | 7.4 | 23 | 16 | 5.1 | 30 | 7.5 | 17 | 5.6 | na |
| OR Quart 3 | 8.8 | 5.2 | 11 | 3.9 | 4.1 | 3.3 | 9.7 | 2.1 | >5.7 |
| | 0.0062 | 0.14 | 0.025 | 0.050 | 0.21 | 0.16 | 0.037 | 0.41 | <0.12 |
| p Value 95% CI of OR Quart3 | 1.9 | 0.59 | 1.3 | 1.00 | 0.45 | 0.63 | 1.2 | 0.37 | >0.64 |
| | 42 | 46 | 93 | 15 | 38 | 18 | 82 | 12 | na |
| OR Quart 4 | 7.0 | 7.5 | 8.0 | 4.4 | 7.5 | 3.9 | 8.0 | 2.6 | >4.3 |
| | 0.015 | 0.062 | 0.058 | 0.031 | 0.062 | 0.10 | 0.057 | 0.26 | <0.20 |
| p Value 95% CI of OR Quart4 | 1.5 | 0.90 | 0.94 | 1.1 | 0.90 | 0.76 | 0.94 | 0.49 | >0.46 |
| | 34 | 63 | 68 | 17 | 63 | 20 | 68 | 14 | na |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6560 | 29400 | 6560 | 19200 | 6560 | 10700 |
| Average | 26500 | 53100 | 26500 | 45900 | 26500 | 38500 |
| Stdev | 42900 | 61100 | 42900 | 57700 | 42900 | 62400 |
| p(t-test) | | 0.0016 | | 0.021 | | 0.24 |
| Min | 362 | 1980 | 362 | 1290 | 362 | 1790 |
| Max | 238000 | 227000 | 238000 | 227000 | 238000 | 227000 |
| n (Samp) | 203 | 36 | 203 | 34 | 203 | 22 |
| n (Patient) | 203 | 36 | 203 | 34 | 203 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 8080 | 42800 | 8080 | 33800 | 8080 | 20200 |
| Average | 26200 | 62300 | 26200 | 56700 | 26200 | 45100 |
| Stdev | 39100 | 66000 | 39100 | 66100 | 39100 | 66500 |
| p(t-test) | | 1.3E-4 | | 0.0012 | | 0.077 |
| Min | 0.0130 | 1980 | 0.0130 | 1980 | 0.0130 | 3260 |
| Max | 238000 | 227000 | 238000 | 227000 | 238000 | 227000 |
| n (Samp) | 395 | 20 | 395 | 20 | 395 | 15 |
| n (Patient) | 395 | 20 | 395 | 20 | 395 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7540 | 22700 | 7540 | 16300 | 7540 | 7270 |
| Average | 28500 | 51300 | 28500 | 44500 | 28500 | 48600 |
| Stdev | 43600 | 62400 | 43600 | 57400 | 43600 | 70300 |
| p(t-test) | | 0.027 | | 0.13 | | 0.12 |
| Min | 362 | 2950 | 362 | 1290 | 362 | 1790 |
| Max | 238000 | 190000 | 238000 | 178000 | 238000 | 178000 |
| n (Samp) | 215 | 22 | 215 | 20 | 215 | 13 |
| n (Patient) | 215 | 22 | 215 | 20 | 215 | 13 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.69 | 0.70 | 0.66 | 0.66 | 0.67 | 0.62 | 0.58 | 0.63 | 0.55 |
| SE | 0.052 | 0.067 | 0.066 | 0.054 | 0.068 | 0.069 | 0.067 | 0.079 | 0.085 |
| p | 1.9E-4 | 0.0023 | 0.015 | 0.0041 | 0.0097 | 0.085 | 0.20 | 0.11 | 0.56 |
| nCohort 1 | 203 | 395 | 215 | 203 | 395 | 215 | 203 | 395 | 215 |
| nCohort 2 | 36 | 20 | 22 | 34 | 20 | 20 | 22 | 15 | 13 |
| Cutoff 1 | 10500 | 10700 | 7220 | 9350 | 10700 | 7220 | 5500 | 9440 | 4050 |
| Sens 1 | 72% | 70% | 73% | 71% | 70% | 70% | 73% | 73% | 77% |
| Spec 1 | 60% | 57% | 49% | 58% | 57% | 49% | 43% | 54% | 30% |
| Cutoff 2 | 5500 | 9440 | 4870 | 4480 | 5880 | 4480 | 4050 | 5880 | 3410 |
| Sens 2 | 81% | 80% | 82% | 82% | 80% | 80% | 82% | 80% | 85% |
| Spec 2 | 43% | 54% | 35% | 38% | 40% | 35% | 33% | 40% | 25% |
| Cutoff 3 | 4050 | 4410 | 4410 | 3120 | 4410 | 4050 | 3120 | 3410 | 2780 |
| Sens 3 | 92% | 90% | 91% | 91% | 90% | 90% | 91% | 93% | 92% |
| Spec 3 | 33% | 31% | 34% | 26% | 31% | 30% | 26% | 21% | 22% |
| Cutoff 4 | 22300 | 24500 | 26500 | 22300 | 24500 | 26500 | 22300 | 24500 | 26500 |
| Sens 4 | 56% | 60% | 45% | 47% | 55% | 40% | 32% | 40% | 31% |
| Spec 4 | 70% | 70% | 70% | 70% | 70% | 70% | 70% | 70% | 70% |
| Cutoff 5 | 43300 | 42100 | 45300 | 43300 | 42100 | 45300 | 43300 | 42100 | 45300 |
| Sens 5 | 42% | 50% | 36% | 38% | 45% | 35% | 23% | 27% | 31% |
| Spec 5 | 80% | 80% | 80% | 80% | 80% | 80% | 80% | 80% | 80% |
| Cutoff 6 | 73500 | 73500 | 89000 | 73500 | 73500 | 89000 | 73500 | 73500 | 89000 |
| Sens 6 | 22% | 30% | 18% | 18% | 25% | 15% | 18% | 20% | 23% |
| Spec 6 | 90% | 90% | 90% | 90% | 90% | 90% | 90% | 90% | 90% |
| OR Quart 2 | 2.1 | 0.99 | 6.6 | 1.6 | 1.5 | 2.6 | 1.7 | 0.99 | 1.4 |
|  | 0.32 | 0.99 | 0.086 | 0.51 | 0.66 | 0.27 | 0.47 | 0.99 | 0.70 |
| p Value 95% CI of OR Quart2 | 0.49 | 0.14 | 0.77 | 0.42 | 0.25 | 0.48 | 0.39 | 0.14 | 0.29 |
|  | 8.7 | 7.2 | 56 | 5.8 | 9.2 | 14 | 7.6 | 7.2 | 6.4 |
| OR Quart 3 | 4.7 | 2.6 | 7.8 | 2.8 | 2.6 | 3.2 | 2.9 | 3.7 | 0.65 |
|  | 0.022 | 0.27 | 0.058 | 0.098 | 0.27 | 0.17 | 0.13 | 0.11 | 0.65 |
| p Value 95% CI of OR Quart3 | 1.2 | 0.48 | 0.93 | 0.83 | 0.48 | 0.61 | 0.74 | 0.75 | 0.11 |
|  | 18 | 13 | 66 | 9.5 | 13 | 16 | 12 | 18 | 4.1 |
| OR Quart 4 | 6.2 | 6.0 | 8.9 | 4.2 | 5.4 | 3.8 | 2.1 | 2.0 | 1.4 |
|  | 0.0059 | 0.022 | 0.042 | 0.017 | 0.033 | 0.11 | 0.32 | 0.42 | 0.70 |
| p Value 95% CI of OR Quart4 | 1.7 | 1.3 | 1.1 | 1.3 | 1.1 | 0.75 | 0.49 | 0.36 | 0.29 |
|  | 23 | 28 | 74 | 14 | 25 | 19 | 8.8 | 11 | 6.4 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.70 | 10.5 | 3.70 | 9.92 | 3.70 | 5.43 |
| Average | 5.74 | 15.9 | 5.74 | 15.2 | 5.74 | 9.29 |
| Stdev | 6.51 | 18.4 | 6.51 | 18.7 | 6.51 | 11.5 |
| p(t-test) | 4.4E-7 | 4.4E-7 | | 3.7E-6 | | 0.054 |
| Min | 0.123 | 0.945 | 0.123 | 0.945 | 0.123 | 0.376 |
| Max | 46.9 | 81.9 | 46.9 | 81.9 | 46.9 | 49.4 |
| n (Samp) | 147 | 28 | 147 | 27 | 147 | 17 |
| n (Patient) | 147 | 28 | 147 | 27 | 147 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.85 | 9.92 | 4.85 | 9.33 | 4.85 | 7.09 |
| Average | 9.22 | 15.2 | 9.22 | 14.6 | 9.22 | 11.9 |
| Stdev | 19.0 | 16.3 | 19.0 | 16.4 | 19.0 | 12.8 |
| p(t-test) | | 0.23 | | 0.29 | | 0.62 |
| Min | 0.123 | 1.42 | 0.123 | 1.42 | 0.123 | 3.19 |
| Max | 279 | 57.1 | 279 | 57.1 | 279 | 49.4 |
| n (Samp) | 285 | 15 | 285 | 15 | 285 | 12 |
| n (Patient) | 285 | 15 | 285 | 15 | 285 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.04 | 9.86 | 4.04 | 9.33 | 4.04 | 3.50 |
| Average | 6.57 | 13.9 | 6.57 | 13.2 | 6.57 | 4.19 |
| Stdev | 7.96 | 18.5 | 7.96 | 18.8 | 7.96 | 2.96 |
| p(t-test) | | 0.0025 | | 0.0077 | | 0.35 |
| Min | 0.123 | 0.945 | 0.123 | 0.945 | 0.123 | 0.376 |
| Max | 49.4 | 81.9 | 49.4 | 81.9 | 49.4 | 10.5 |
| n (Samp) | 151 | 18 | 151 | 17 | 151 | 10 |
| n (Patient) | 151 | 18 | 151 | 17 | 151 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | 0.69 | 0.68 | 0.74 | 0.67 | 0.66 | 0.64 | 0.66 | 0.44 |
| SE | 0.056 | 0.078 | 0.073 | 0.058 | 0.078 | 0.075 | 0.076 | 0.087 | 0.097 |
| p | 5.5E-6 | 0.014 | 0.014 | 3.8E-5 | 0.026 | 0.030 | 0.070 | 0.062 | 0.54 |
| nCohort 1 | 147 | 285 | 151 | 147 | 285 | 151 | 147 | 285 | 151 |
| nCohort 2 | 28 | 15 | 18 | 27 | 15 | 17 | 17 | 12 | 10 |
| Cutoff 1 | 5.34 | 6.73 | 3.80 | 5.34 | 6.73 | 3.80 | 4.51 | 5.34 | 2.31 |
| Sens 1 | 71% | 73% | 72% | 70% | 73% | 71% | 71% | 75% | 70% |
| Spec 1 | 65% | 61% | 49% | 65% | 61% | 49% | 59% | 54% | 27% |
| Cutoff 2 | 3.80 | 5.34 | 2.97 | 3.80 | 5.34 | 2.97 | 3.15 | 4.91 | 2.16 |
| Sens 2 | 82% | 80% | 83% | 81% | 80% | 82% | 82% | 83% | 80% |
| Spec 2 | 54% | 54% | 38% | 54% | 54% | 38% | 41% | 51% | 26% |
| Cutoff 3 | 2.16 | 3.15 | 2.16 | 2.16 | 3.15 | 2.16 | 1.99 | 4.60 | 1.99 |
| Sens 3 | 93% | 93% | 94% | 93% | 93% | 94% | 94% | 92% | 90% |
| Spec 3 | 30% | 36% | 26% | 30% | 36% | 26% | 27% | 49% | 25% |
| Cutoff 4 | 5.99 | 9.07 | 6.30 | 5.99 | 9.07 | 6.30 | 5.99 | 9.07 | 6.30 |
| Sens 4 | 68% | 60% | 61% | 67% | 53% | 59% | 47% | 42% | 20% |
| Spec 4 | 70% | 70% | 70% | 70% | 70% | 70% | 70% | 70% | 70% |
| Cutoff 5 | 8.29 | 12.5 | 8.53 | 8.29 | 12.5 | 8.53 | 8.29 | 12.5 | 8.53 |
| Sens 5 | 61% | 33% | 56% | 56% | 27% | 53% | 29% | 25% | 10% |
| Spec 5 | 80% | 80% | 80% | 80% | 80% | 80% | 80% | 80% | 80% |
| Cutoff 6 | 13.0 | 19.4 | 13.6 | 13.0 | 19.4 | 13.6 | 13.0 | 19.4 | 13.6 |
| Sens 6 | 36% | 20% | 28% | 30% | 20% | 24% | 18% | 17% | 0% |
| Spec 6 | 90% | 90% | 91% | 90% | 90% | 91% | 90% | 90% | 91% |
| OR Quart 2 | 2.0 | 2.0 | 2.1 | 2.0 | 2.0 | 2.1 | 3.2 | >3.1 | 1.0 |
| | 0.42 | 0.57 | 0.41 | 0.42 | 0.57 | 0.41 | 0.33 | <0.33 | 0.98 |
| p Value 95% CI of OR Quart2 | 0.36 | 0.18 | 0.36 | 0.36 | 0.18 | 0.36 | 0.31 | >0.32 | 0.14 |
| | 12 | 23 | 12 | 12 | 23 | 12 | 32 | na | 7.7 |
| OR Quart 3 | 2.6 | 6.4 | 1.0 | 3.3 | 7.6 | 1.0 | 8.2 | >6.5 | 2.2 |
| | 0.26 | 0.089 | 1.0 | 0.16 | 0.061 | 1.0 | 0.054 | <0.086 | 0.39 |
| p Value | 0.48 | 0.76 | 0.13 | 0.63 | 0.91 | 0.13 | 0.96 | >0.77 | 0.37 |
| 95% CI of OR Quart3 | 14 | 55 | 7.5 | 17 | 64 | 7.5 | 70 | na | 13 |
| OR Quart 4 | 13 | 6.4 | 6.1 | 11 | 5.3 | 5.5 | 6.9 | >3.1 | 1.0 |
| | 0.0012 | 0.089 | 0.026 | 0.0028 | 0.13 | 0.038 | 0.081 | <0.33 | 0.98 |
| p Value 95% CI of OR Quart4 | 2.8 | 0.76 | 1.2 | 2.3 | 0.60 | 1.1 | 0.79 | >0.31 | 0.14 |
| | 60 | 55 | 30 | 50 | 46 | 27 | 60 | na | 7.7 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000114 | 0.000197 | 0.000114 | 0.000197 | 0.000114 | 0.000732 |
| Average | 0.0104 | 0.108 | 0.0104 | 0.0505 | 0.0104 | 0.0493 |
| Stdev | 0.0383 | 0.260 | 0.0383 | 0.143 | 0.0383 | 0.138 |
| p(t-test) | | 1.6E-5 | | 0.0032 | | 0.0070 |
| Min | 5.62E-6 | 1.88E-5 | 5.62E-6 | 1.66E-5 | 5.62E-6 | 1.66E-5 |
| Max | 0.260 | 1.02 | 0.260 | 0.560 | 0.260 | 0.560 |
| n (Samp) | 151 | 32 | 151 | 30 | 151 | 18 |
| n (Patient) | 151 | 32 | 151 | 30 | 151 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000114 | 0.00341 | 0.000114 | 0.000732 | 0.000114 | 0.00127 |
| Average | 0.0111 | 0.159 | 0.0111 | 0.0551 | 0.0111 | 0.0642 |
| Stdev | 0.0451 | 0.324 | 0.0451 | 0.151 | 0.0451 | 0.162 |
| p(t-test) | | 5.1E-12 | | 0.0012 | | 5.8E-4 |
| Min | 5.62E-6 | 2.96E-5 | 5.62E-6 | 1.66E-5 | 5.62E-6 | 2.96E-5 |
| Max | 0.470 | 1.02 | 0.470 | 0.560 | 0.470 | 0.560 |
| n (Samp) | 317 | 18 | 317 | 18 | 317 | 13 |
| n (Patient) | 317 | 18 | 317 | 18 | 317 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.51E-5 | 4.39E-5 | 6.51E-5 | 3.88E-5 | 6.51E-5 | 0.000118 |
| Average | 0.0127 | 0.0848 | 0.0127 | 0.0908 | 0.0127 | 0.00682 |
| Stdev | 0.0478 | 0.251 | 0.0478 | 0.265 | 0.0478 | 0.0142 |
| p(t-test) | | 0.0011 | | 7.8E-4 | | 0.70 |
| Min | 5.62E-6 | 1.88E-5 | 5.62E-6 | 1.88E-5 | 5.62E-6 | 1.66E-5 |
| Max | 0.359 | 1.02 | 0.359 | 1.02 | 0.359 | 0.0446 |
| n (Samp) | 172 | 19 | 172 | 17 | 172 | 10 |
| n (Patient) | 172 | 19 | 172 | 17 | 172 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.68 | 0.55 | 0.55 | 0.62 | 0.51 | 0.60 | 0.63 | 0.54 |
| SE | 0.057 | 0.071 | 0.071 | 0.059 | 0.072 | 0.074 | 0.074 | 0.085 | 0.096 |
| p | 0.052 | 0.012 | 0.47 | 0.36 | 0.10 | 0.94 | 0.18 | 0.14 | 0.70 |
| nCohort 1 | 151 | 317 | 172 | 151 | 317 | 172 | 151 | 317 | 172 |
| nCohort 2 | 32 | 18 | 19 | 30 | 18 | 17 | 18 | 13 | 10 |
| Cutoff 1 | 3.44E-5 | 8.63E-5 | 2.83E-5 | 3.21E-5 | 3.21E-5 | 2.83E-5 | 3.21E-5 | 2.83E-5 | 2.83E-5 |
| Sens 1 | 72% | 72% | 79% | 70% | 72% | 76% | 72% | 100% | 90% |
| Spec 1 | 36% | 49% | 29% | 34% | 35% | 29% | 34% | 26% | 29% |
| Cutoff 2 | 2.83E-5 | 2.83E-5 | 2.68E-5 | 2.83E-5 | 2.83E-5 | 2.53E-5 | 2.83E-5 | 2.83E-5 | 2.83E-5 |
| Sens 2 | 88% | 100% | 84% | 83% | 94% | 82% | 94% | 100% | 90% |
| Spec 2 | 25% | 26% | 26% | 25% | 26% | 21% | 25% | 26% | 29% |
| Cutoff 3 | 2.68E-5 | 2.83E-5 | 2.46E-5 | 2.46E-5 | 2.83E-5 | 2.38E-5 | 2.83E-5 | 2.83E-5 | 2.83E-5 |
| Sens 3 | 91% | 100% | 95% | 90% | 94% | 94% | 94% | 100% | 90% |
| Spec 3 | 22% | 26% | 16% | 13% | 26% | 14% | 25% | 26% | 29% |
| Cutoff 4 | 0.00146 | 0.00127 | 0.00121 | 0.00146 | 0.00127 | 0.00121 | 0.00146 | 0.00127 | 0.00121 |
| Sens 4 | 41% | 50% | 32% | 37% | 44% | 24% | 44% | 46% | 30% |
| Spec 4 | 70% | 70% | 70% | 70% | 70% | 70% | 70% | 70% | 70% |
| Cutoff 5 | 0.00836 | 0.00672 | 0.00590 | 0.00836 | 0.00672 | 0.00590 | 0.00836 | 0.00672 | 0.00590 |
| Sens 5 | 31% | 44% | 26% | 27% | 39% | 24% | 28% | 46% | 30% |
| Spec 5 | 80% | 80% | 80% | 80% | 80% | 80% | 80% | 80% | 80% |
| Cutoff 6 | 0.0157 | 0.0171 | 0.0157 | 0.0157 | 0.0171 | 0.0157 | 0.0157 | 0.0171 | 0.0157 |
| Sens 6 | 25% | 28% | 21% | 17% | 17% | 18% | 17% | 15% | 10% |
| Spec 6 | 90% | 91% | 90% | 90% | 91% | 90% | 90% | 91% | 90% |
| OR Quart 2 | 2.5 / 0.15 | >5.3 / <0.13 | 2.5 / 0.20 | 1.7 / 0.37 | 5.2 / 0.14 | 1.6 / 0.51 | 1.4 / 0.69 | >5.3 / <0.13 | 4.2 / 0.21 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value 95% CI of OR Quart2 | 0.71 | >0.60 | 0.61 | 0.52 | 0.59 | 0.41 | 0.29 | >0.60 | 0.45 |
| | 8.8 | na | 10 | 5.8 | 45 | 6.0 | 6.5 | na | 39 |
| OR Quart 3 | 1.8 | >4.2 | 1.3 | 1.2 | 4.1 | 0.73 | 1.0 | >2.0 | 2.0 |
| | 0.36 | <0.21 | 0.72 | 0.75 | 0.21 | 0.70 | 1.0 | <0.56 | 0.56 |
| p Value 95% CI OR Quart3 | 0.50 | >0.45 | 0.28 | 0.35 | 0.45 | 0.15 | 0.19 | >0.18 | 0.18 |
| | 6.8 | na | 6.3 | 4.4 | 37 | 3.5 | 5.3 | na | 23 |
| OR Quart 4 | 3.6 | >10.0 | 1.7 | 2.5 | 8.6 | 0.98 | 3.0 | >6.4 | 3.1 |
| | 0.039 | <0.031 | 0.48 | 0.12 | 0.044 | 0.98 | 0.13 | <0.089 | 0.34 |
| p Value | 1.1 | >1.2 | 0.38 | 0.80 | 1.1 | 0.23 | 0.73 | >0.75 | 0.31 |
| 95% CI of OR Quart4 | 12 | na | 7.6 | 7.9 | 71 | 4.2 | 12 | na | 31 |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0243 | 0.124 | 0.0243 | 0.0612 | 0.0243 | 0.0328 |
| Average | 0.188 | 0.546 | 0.188 | 0.331 | 0.188 | 0.176 |
| Stdev | 0.436 | 1.20 | 0.436 | 0.496 | 0.436 | 0.298 |
| p(t-test) | | 0.0044 | | 0.11 | | 0.91 |
| Min | 7.25E-6 | 0.00670 | 7.25E-6 | 0.00673 | 7.25E-6 | 0.00644 |
| Max | 3.07 | 6.31 | 3.07 | 1.59 | 3.07 | 1.07 |
| n (Samp) | 151 | 32 | 151 | 30 | 151 | 18 |
| n (Patient) | 151 | 32 | 151 | 30 | 151 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0253 | 0.0532 | 0.0253 | 0.0532 | 0.0253 | 0.0317 |
| Average | 0.284 | 0.556 | 0.284 | 0.290 | 0.284 | 0.139 |
| Stdev | 0.789 | 1.49 | 0.789 | 0.496 | 0.789 | 0.228 |
| p(t-test) | | 0.18 | | 0.98 | | 0.51 |
| Min | 6.78E-7 | 0.00928 | 6.78E-7 | 0.00804 | 6.78E-7 | 0.00804 |
| Max | 8.92 | 6.31 | 8.92 | 1.56 | 8.92 | 0.725 |
| n (Samp) | 317 | 18 | 317 | 18 | 317 | 13 |
| n (Patient) | 317 | 18 | 317 | 18 | 317 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0258 | 0.226 | 0.0258 | 0.0598 | 0.0258 | 0.0249 |
| Average | 0.235 | 0.481 | 0.235 | 0.377 | 0.235 | 0.299 |
| Stdev | 0.505 | 0.718 | 0.505 | 0.546 | 0.505 | 0.551 |
| p(t-test) | | 0.056 | | 0.28 | | 0.70 |
| Min | 7.25E-6 | 0.00670 | 7.25E-6 | 0.00673 | 7.25E-6 | 0.00644 |
| Max | 3.07 | 2.69 | 3.07 | 1.59 | 3.07 | 1.56 |
| n (Samp) | 172 | 19 | 172 | 17 | 172 | 10 |
| n (Patient) | 172 | 19 | 172 | 17 | 172 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | 0.64 | 0.67 | 0.66 | 0.63 | 0.63 | 0.57 | 0.54 | 0.54 |
| SE | 0.055 | 0.072 | 0.071 | 0.058 | 0.072 | 0.075 | 0.074 | 0.083 | 0.096 |
| p | 6.1E-4 | 0.048 | 0.016 | 0.0055 | 0.076 | 0.079 | 0.36 | 0.65 | 0.70 |
| nCohort 1 | 151 | 317 | 172 | 151 | 317 | 172 | 151 | 317 | 172 |
| nCohort 2 | 32 | 18 | 19 | 30 | 18 | 17 | 18 | 13 | 10 |
| Cutoff 1 | 0.0317 | 0.0317 | 0.0313 | 0.0317 | 0.0317 | 0.0313 | 0.0165 | 0.0148 | 0.0165 |
| Sens 1 | 72% | 72% | 74% | 70% | 72% | 71% | 72% | 77% | 70% |
| Spec 1 | 58% | 56% | 55% | 58% | 56% | 55% | 42% | 36% | 40% |
| Cutoff 2 | 0.0191 | 0.0191 | 0.0165 | 0.0171 | 0.0181 | 0.0165 | 0.0127 | 0.0134 | 0.0130 |
| Sens 2 | 81% | 83% | 84% | 80% | 83% | 82% | 83% | 85% | 80% |
| Spec 2 | 44% | 42% | 40% | 42% | 41% | 40% | 33% | 32% | 31% |
| Cutoff 3 | 0.0147 | 0.0148 | 0.00670 | 0.00912 | 0.00926 | 0.00766 | 0.00776 | 0.00926 | 0.00956 |
| Sens 3 | 91% | 94% | 95% | 90% | 94% | 94% | 94% | 92% | 90% |
| Spec 3 | 37% | 36% | 19% | 26% | 23% | 22% | 24% | 23% | 24% |
| Cutoff 4 | 0.0934 | 0.0850 | 0.144 | 0.0934 | 0.0850 | 0.144 | 0.0934 | 0.0850 | 0.144 |
| Sens 4 | 50% | 39% | 58% | 40% | 39% | 41% | 28% | 31% | 30% |
| Spec 4 | 70% | 70% | 70% | 70% | 70% | 70% | 70% | 70% | 70% |
| Cutoff 5 | 0.221 | 0.255 | 0.295 | 0.221 | 0.255 | 0.295 | 0.221 | 0.255 | 0.295 |
| Sens 5 | 44% | 28% | 42% | 33% | 28% | 35% | 22% | 23% | 20% |
| Spec 5 | 80% | 80% | 80% | 80% | 80% | 80% | 80% | 80% | 80% |
| Cutoff 6 | 0.514 | 0.740 | 0.719 | 0.514 | 0.740 | 0.719 | 0.514 | 0.740 | 0.719 |
| Sens 6 | 22% | 11% | 21% | 23% | 11% | 24% | 11% | 0% | 20% |
| Spec 6 | 90% | 90% | 90% | 90% | 90% | 90% | 90% | 90% | 90% |
| OR Quart 2 | 1.7 | 4.1 | 0.64 | 1.0 | 1.5 | 0.65 | 3.3 | 2.0 | 1.5 |
| | 0.48 | 0.21 | 0.63 | 1.0 | 0.66 | 0.65 | 0.16 | 0.42 | 0.67 |
| p Value | 0.38 | 0.45 | 0.10 | 0.23 | 0.24 | 0.10 | 0.63 | 0.36 | 0.24 |
| 95% CI of OR Quart2 | 7.6 | 37 | 4.0 | 4.3 | 9.2 | 4.1 | 18 | 11 | 9.4 |
| OR Quart 3 | 3.4 | 8.6 | 1.7 | 3.3 | 4.3 | 1.7 | 2.7 | 2.1 | 1.0 |
| | 0.082 | 0.044 | 0.48 | 0.056 | 0.072 | 0.46 | 0.25 | 0.41 | 1.0 |
| p Value | 0.86 | 1.1 | 0.38 | 0.97 | 0.88 | 0.39 | 0.49 | 0.37 | 0.13 |
| 95% CI of OR Quart3 | 14 | 71 | 7.6 | 11 | 21 | 7.8 | 15 | 12 | 7.4 |
| OR Quart 4 | 6.8 | 5.2 | 3.4 | 3.2 | 2.6 | 2.5 | 2.6 | 1.5 | 1.5 |
| | 0.0046 | 0.14 | 0.082 | 0.062 | 0.27 | 0.20 | 0.26 | 0.66 | 0.67 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | 1.8 | 0.59 | 0.85 | 0.94 | 0.48 | 0.61 | 0.48 | 0.24 | 0.24 |
| 95% CI of OR Quart4 | 25 | 45 | 13 | 11 | 14 | 10 | 14 | 9.2 | 9.4 |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 150 | 147 | 150 | 148 | 150 | 142 |
| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Average | 156 | 163 | 156 | 161 | 156 | 148 |
| Stdev | 51.8 | 74.3 | 51.8 | 77.4 | 51.8 | 46.6 |
| p(t-test) | | 0.57 | | 0.69 | | 0.55 |
| Min | 38.4 | 46.0 | 38.4 | 46.0 | 38.4 | 76.6 |
| Max | 311 | 444 | 311 | 444 | 311 | 241 |
| n (Samp) | 145 | 28 | 145 | 27 | 145 | 17 |
| n (Patient) | 145 | 28 | 145 | 27 | 145 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 151 | 170 | 151 | 170 | 151 | 156 |
| Average | 160 | 166 | 160 | 162 | 160 | 152 |
| Stdev | 62.6 | 52.1 | 62.6 | 56.8 | 62.6 | 53.9 |
| p(t-test) | | 0.69 | | 0.89 | | 0.67 |
| Min | 38.4 | 102 | 38.4 | 76.6 | 38.4 | 76.6 |
| Max | 444 | 265 | 444 | 265 | 444 | 241 |
| n (Samp) | 284 | 15 | 284 | 15 | 284 | 12 |
| n (Patient) | 284 | 15 | 284 | 15 | 284 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 153 | 144 | 153 | 142 | 153 | 127 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 156 | 153 | 156 | 154 | 156 | 139 |
| Stdev | 52.4 | 87.0 | 52.4 | 89.7 | 52.4 | 46.9 |
| p(t-test) | | 0.87 | | 0.90 | | 0.33 |
| Min | 38.4 | 46.0 | 38.4 | 46.0 | 38.4 | 82.1 |
| Max | 311 | 444 | 311 | 444 | 311 | 241 |
| n (Samp) | 149 | 18 | 149 | 17 | 149 | 10 |
| n (Patient) | 149 | 18 | 149 | 17 | 149 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.55 | 0.44 | 0.50 | 0.52 | 0.44 | 0.45 | 0.48 | 0.40 |
| SE | 0.060 | 0.078 | 0.074 | 0.061 | 0.078 | 0.076 | 0.076 | 0.086 | 0.098 |
| p | 0.86 | 0.52 | 0.43 | 0.97 | 0.75 | 0.44 | 0.54 | 0.80 | 0.30 |
| nCohort 1 | 145 | 284 | 149 | 145 | 284 | 149 | 145 | 284 | 149 |
| nCohort 2 | 28 | 15 | 18 | 27 | 15 | 17 | 17 | 12 | 10 |
| Cutoff 1 | 131 | 128 | 110 | 128 | 118 | 110 | 119 | 105 | 119 |
| Sens 1 | 71% | 73% | 72% | 70% | 73% | 71% | 71% | 75% | 70% |
| Spec 1 | 36% | 35% | 22% | 32% | 29% | 22% | 25% | 22% | 28% |
| Cutoff 2 | 110 | 118 | 102 | 105 | 105 | 102 | 105 | 104 | 105 |
| Sens 2 | 82% | 80% | 83% | 81% | 80% | 82% | 82% | 83% | 80% |
| Spec 2 | 20% | 29% | 15% | 18% | 22% | 15% | 18% | 21% | 21% |
| Cutoff 3 | 102 | 104 | 46.0 | 74.0 | 102 | 46.0 | 94.6 | 94.8 | 94.6 |
| Sens 3 | 93% | 93% | 94% | 93% | 93% | 94% | 94% | 92% | 90% |
| Spec 3 | 13% | 21% | 1% | 3% | 19% | 1% | 10% | 14% | 12% |
| Cutoff 4 | 178 | 186 | 183 | 178 | 186 | 183 | 178 | 186 | 183 |
| Sens 4 | 32% | 27% | 17% | 33% | 27% | 18% | 24% | 17% | 10% |
| Spec 4 | 70% | 70% | 70% | 70% | 70% | 70% | 70% | 70% | 70% |
| Cutoff 5 | 194 | 212 | 198 | 194 | 212 | 198 | 194 | 212 | 198 |
| Sens 5 | 14% | 20% | 11% | 15% | 20% | 12% | 12% | 17% | 10% |
| Spec 5 | 80% | 80% | 81% | 80% | 80% | 81% | 80% | 80% | 81% |
| Cutoff 6 | 220 | 247 | 220 | 220 | 247 | 220 | 220 | 247 | 220 |
| Sens 6 | 14% | 7% | 11% | 15% | 7% | 12% | 12% | 0% | 10% |
| Spec 6 | 90% | 90% | 91% | 90% | 90% | 91% | 90% | 90% | 91% |
| OR Quart 2 | 1.2 | 1.3 | 2.7 | 1.7 | 0.73 | 2.8 | 2.8 | 2.1 | 2.1 |
| | 0.78 | 0.71 | 0.25 | 0.37 | 0.69 | 0.24 | 0.24 | 0.41 | 0.56 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value 95% CI of OR Quart2 | 0.39 | 0.29 | 0.49 | 0.52 | 0.16 | 0.51 | 0.51 | 0.37 | 0.18 |
| | 3.6 | 6.2 | 15 | 5.8 | 3.4 | 15 | 15 | 12 | 24 |
| OR Quart 3 | 1.0 | 1.7 | 2.7 | 1.5 | 1.2 | 2.1 | 3.3 | 1.0 | 4.3 |
| | 1.0 | 0.48 | 0.25 | 0.54 | 0.75 | 0.41 | 0.16 | 1.0 | 0.20 |
| p Value 95% CI of OR Quart3 | 0.32 | 0.39 | 0.49 | 0.43 | 0.32 | 0.36 | 0.63 | 0.14 | 0.46 |
| | 3.1 | 7.3 | 15 | 5.1 | 4.9 | 12 | 18 | 7.3 | 41 |
| OR Quart 4 | 0.81 | 0.99 | 3.4 | 1.5 | 0.73 | 3.4 | 2.2 | 2.1 | 3.2 |
| | 0.73 | 0.99 | 0.15 | 0.54 | 0.69 | 0.15 | 0.39 | 0.41 | 0.32 |
| p Value 95% CI of OR Quart4 | 0.25 | 0.19 | 0.65 | 0.43 | 0.16 | 0.65 | 0.37 | 0.37 | 0.32 |
| | 2.6 | 5.1 | 18 | 5.1 | 3.4 | 18 | 13 | 12 | 33 |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1570 | 3440 | 1570 | 2270 | 1570 | 1780 |
| Average | 2500 | 8940 | 2500 | 8470 | 2500 | 6780 |
| Stdev | 3600 | 17000 | 3600 | 17500 | 3600 | 16800 |
| p(t-test) | | 2.1E-6 | | 1.8E-5 | | 0.0023 |
| Min | 33.0 | 90.7 | 33.0 | 90.7 | 33.0 | 201 |
| Max | 33000 | 80100 | 33000 | 80100 | 33000 | 80100 |
| n (Samp) | 203 | 36 | 203 | 34 | 203 | 22 |
| n (Patient) | 203 | 36 | 203 | 34 | 203 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1810 | 2100 | 1810 | 1980 | 1810 | 1940 |
| Average | 3180 | 7650 | 3180 | 7290 | 3180 | 8250 |
| Stdev | 5450 | 17500 | 5450 | 17400 | 5450 | 20200 |
| p(t-test) | | 0.0029 | | 0.0061 | | 0.0033 |
| Min | 0.0376 | 90.7 | 0.0376 | 90.7 | 0.0376 | 890 |
| Max | 68100 | 80100 | 68100 | 80100 | 68100 | 80100 |
| n (Samp) | 395 | 20 | 395 | 20 | 395 | 15 |
| n (Patient) | 395 | 20 | 395 | 20 | 395 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1720 | 2890 | 1720 | 1950 | 1720 | 1250 |
| Average | 2700 | 8160 | 2700 | 7650 | 2700 | 2540 |
| Stdev | 3680 | 14700 | 3680 | 15500 | 3680 | 3590 |
| p(t-test) | | 2.1E-5 | | 2.3E-4 | | 0.88 |
| Min | 33.0 | 201 | 33.0 | 201 | 33.0 | 201 |
| Max | 33000 | 68100 | 33000 | 68100 | 33000 | 12300 |
| n (Samp) | 215 | 22 | 215 | 20 | 215 | 13 |
| n (Patient) | 215 | 22 | 215 | 20 | 215 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.64 | 0.63 | 0.66 | 0.61 | 0.58 | 0.60 | 0.58 | 0.43 |
| SE | 0.051 | 0.068 | 0.066 | 0.054 | 0.068 | 0.069 | 0.067 | 0.078 | 0.085 |
| p | 5.2E-5 | 0.045 | 0.043 | 0.0023 | 0.10 | 0.24 | 0.15 | 0.33 | 0.42 |
| nCohort 1 | 203 | 395 | 215 | 203 | 395 | 215 | 203 | 395 | 215 |
| nCohort 2 | 36 | 20 | 22 | 34 | 20 | 20 | 22 | 15 | 13 |
| Cutoff 1 | 1760 | 1810 | 1220 | 1460 | 1570 | 1220 | 1170 | 1240 | 813 |
| Sens 1 | 72% | 70% | 73% | 71% | 70% | 70% | 73% | 73% | 77% |
| Spec 1 | 56% | 50% | 39% | 47% | 43% | 39% | 42% | 36% | 27% |
| Cutoff 2 | 1270 | 1660 | 952 | 1190 | 1450 | 952 | 952 | 1170 | 525 |
| Sens 2 | 81% | 80% | 82% | 82% | 80% | 80% | 82% | 80% | 85% |
| Spec 2 | 45% | 46% | 30% | 44% | 41% | 30% | 34% | 34% | 16% |
| Cutoff 3 | 813 | 1240 | 525 | 813 | 1020 | 525 | 813 | 952 | 277 |
| Sens 3 | 92% | 90% | 91% | 91% | 90% | 90% | 91% | 93% | 92% |
| Spec 3 | 31% | 36% | 16% | 31% | 29% | 16% | 31% | 28% | 10% |
| Cutoff 4 | 2360 | 2910 | 2690 | 2360 | 2910 | 2690 | 2360 | 2910 | 2690 |
| Sens 4 | 56% | 45% | 55% | 47% | 45% | 40% | 36% | 33% | 23% |
| Spec 4 | 70% | 70% | 70% | 70% | 70% | 70% | 70% | 70% | 70% |
| Cutoff 5 | 3330 | 4190 | 3610 | 3330 | 4190 | 3610 | 3330 | 4190 | 3610 |
| Sens 5 | 50% | 40% | 45% | 44% | 40% | 35% | 32% | 27% | 15% |
| Spec 5 | 80% | 80% | 80% | 80% | 80% | 80% | 80% | 80% | 80% |
| Cutoff 6 | 4930 | 6560 | 5440 | 4930 | 6560 | 5440 | 4930 | 6560 | 5440 |
| Sens 6 | 44% | 15% | 41% | 35% | 15% | 25% | 27% | 13% | 15% |
| Spec 6 | 90% | 90% | 90% | 90% | 90% | 90% | 90% | 90% | 90% |
| OR Quart 2 | 2.5 | 6.2 | 2.5 | 3.4 | 8.5 | 2.5 | 3.9 | >7.4 | 1.0 |
| | 0.21 | 0.093 | 0.20 | 0.081 | 0.046 | 0.21 | 0.10 | <0.063 | 1.0 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value 95% CI of OR Quart2 | 0.61 | 0.74 | 0.62 | 0.86 | 1.0 | 0.61 | 0.76 | >0.90 | 0.14 |
|  | 10 | 53 | 10 | 13 | 69 | 10 | 19 | na | 7.4 |
| OR Quart 3 | 2.9 | 4.1 | 0.65 | 2.5 | 2.0 | 0.98 | 3.2 | >3.1 | 3.2 |
|  | 0.13 | 0.21 | 0.65 | 0.20 | 0.57 | 0.98 | 0.16 | <0.33 | 0.16 |
| p Value 95% CI of OR Quart3 | 0.72 | 0.45 | 0.11 | 0.62 | 0.18 | 0.19 | 0.62 | >0.32 | 0.62 |
|  | 11 | 37 | 4.1 | 10 | 22 | 5.1 | 17 | na | 17 |
| OR Quart 4 | 8.0 | 9.7 | 3.7 | 6.2 | 9.7 | 2.5 | 3.8 | >5.2 | 1.5 |
|  | 0.0015 | 0.033 | 0.055 | 0.0059 | 0.033 | 0.21 | 0.11 | <0.14 | 0.65 |
| p Value 95% CI ot OR Quart4 | 2.2 | 1.2 | 0.97 | 1.7 | 1.2 | 0.61 | 0.75 | >0.60 | 0.25 |
|  | 29 | 78 | 14 | 23 | 78 | 10 | 19 | na | 9.5 |

**Tenascin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 13.9 | 20.6 | 13.9 | 20.6 | 13.9 | 18.2 |
| Average | 21.5 | 298 | 21.5 | 304 | 21.5 | 20.3 |
| Stdev | 43.9 | 1420 | 43.9 | 1450 | 43.9 | 19.3 |
| p(t-test) |  | 0.018 |  | 0.017 |  | 0.92 |
| Min | 0.0190 | 0.00398 | 0.0190 | 0.00398 | 0.0190 | 0.0190 |
| Max | 470 | 7540 | 470 | 7540 | 470 | 69.1 |
| n (Samp) | 148 | 28 | 148 | 27 | 148 | 17 |
| n (Patient) | 148 | 28 | 148 | 27 | 148 | 17 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 16.2 | 20.6 | 16.2 | 20.6 | 16.2 | 18.2 |
| Average | 54.1 | 23.7 | 54.1 | 23.7 | 54.1 | 20.7 |
| Stdev | 447 | 19.8 | 447 | 19.8 | 447 | 19.8 |
| p(t-test) |  | 0.79 |  | 0.79 |  | 0.80 |
| Min | 0.00398 | 0.338 | 0.00398 | 0.338 | 0.00398 | 0.338 |
| Max | 7540 | 69.1 | 7540 | 69.1 | 7540 | 69.1 |
| n (Samp) | 288 | 15 | 288 | 15 | 288 | 12 |
| n (Patient) | 288 | 15 | 288 | 15 | 288 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 15.2 | 20.6 | 15.2 | 20.6 | 15.2 | 13.0 |
| Average | 28.8 | 448 | 28.8 | 466 | 28.8 | 17.5 |
| Stdev | 86.4 | 1770 | 86.4 | 1820 | 86.4 | 17.8 |
| p(t-test) | | 0.0035 | | 0.0031 | | 0.68 |
| Min | 0.0190 | 0.00398 | 0.0190 | 0.00398 | 0.0190 | 0.0190 |
| Max | 945 | 7540 | 945 | 7540 | 945 | 46.6 |
| n (Samp) | 152 | 18 | 152 | 17 | 152 | 10 |
| n (Patient) | 152 | 18 | 152 | 17 | 152 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.55 | 0.59 | 0.62 | 0.55 | 0.57 | 0.55 | 0.50 | 0.47 |
| SE | 0.060 | 0.078 | 0.074 | 0.062 | 0.078 | 0.076 | 0.076 | 0.085 | 0.096 |
| p | 0.025 | 0.55 | 0.21 | 0.046 | 0.55 | 0.35 | 0.51 | 0.98 | 0.73 |
| nCohort 1 | 148 | 288 | 152 | 148 | 288 | 152 | 148 | 288 | 152 |
| nCohort 2 | 28 | 15 | 18 | 27 | 15 | 17 | 17 | 12 | 10 |
| Cutoff 1 Sens 1 Spec 1 | 14.8 | 8.27 | 10.0 | 14.8 | 8.27 | 10.0 | 6.41 | 7.00 | 6.24 |
| | 71% | 73% | 72% | 70% | 73% | 71% | 71% | 75% | 70% |
| | 55% | 27% | 36% | 55% | 27% | 36% | 33% | 26% | 30% |
| Cutoff 2 Sens 2 Spec 2 | 6.24 | 7.00 | 0.103 | 6.24 | 7.00 | 0.103 | 0.103 | 6.36 | 0.103 |
| | 82% | 80% | 89% | 81% | 80% | 88% | 88% | 83% | 80% |
| | 33% | 26% | 11% | 33% | 26% | 11% | 12% | 26% | 11% |
| Cutoff 3 Sens 3 Spec 3 | 0.103 | 0.103 | 0.0840 | 0.103 | 0.103 | 0.0840 | 0.0840 | 0.103 | 0.0840 |
| | 93% | 100% | 94% | 93% | 100% | 94% | 94% | 100% | 90% |
| | 12% | 10% | 10% | 12% | 10% | 10% | 10% | 10% | 10% |
| Cutoff 4 Sens 4 Spec 4 | 19.8 | 26.2 | 22.0 | 19.8 | 26.2 | 22.0 | 19.8 | 26.2 | 22.0 |
| | 61% | 33% | 44% | 59% | 33% | 41% | 47% | 25% | 30% |
| | 70% | 70% | 70% | 70% | 70% | 70% | 70% | 70% | 70% |
| Cutoff 5 Sens 5 Spec 5 | 28.1 | 36.6 | 28.1 | 28.1 | 36.6 | 28.1 | 28.1 | 36.6 | 28.1 |
| | 36% | 20% | 39% | 33% | 20% | 35% | 24% | 17% | 30% |
| | 83% | 80% | 80% | 83% | 80% | 80% | 83% | 80% | 80% |
| Cutoff 6 Sens 6 Spec 6 | 44.7 | 57.5 | 45.0 | 44.7 | 57.5 | 45.0 | 44.7 | 57.5 | 45.0 |
| | 21% | 7% | 22% | 19% | 7% | 18% | 12% | 8% | 10% |
| | 91% | 90% | 90% | 91% | 90% | 90% | 91% | 90% | 90% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 2 p Value 95% CI of OR Quart2 | 0.57 | 2.0 | 0.71 | 0.56 | 2.0 | 0.73 | 0.73 | 2.1 | 0.67 |
|  | 0.46 | 0.42 | 0.67 | 0.44 | 0.42 | 0.69 | 0.69 | 0.41 | 0.67 |
|  | 0.13 | 0.36 | 0.15 | 0.12 | 0.36 | 0.15 | 0.15 | 0.37 | 0.11 |
|  | 2.6 | 11 | 3.4 | 2.5 | 11 | 3.5 | 3.5 | 12 | 4.2 |
| OR Quart 3 | 1.7 | 2.6 | 0.73 | 1.4 | 2.6 | 0.73 | 1.0 | 2.1 | 0.65 |
|  | 0.37 | 0.27 | 0.69 | 0.56 | 0.27 | 0.69 | 1.0 | 0.41 | 0.65 |
| p Value 95% CI of OR Quart3 | 0.52 | 0.48 | 0.15 | 0.42 | 0.48 | 0.15 | 0.23 | 0.37 | 0.10 |
|  | 5.8 | 14 | 3.5 | 4.9 | 14 | 3.5 | 4.3 | 12 | 4.1 |
| OR Quart 4 p Value 95% CI of OR Quart4 | 2.9 | 2.0 | 2.2 | 2.8 | 2.0 | 1.8 | 1.5 | 1.0 | 1.0 |
|  | 0.066 | 0.42 | 0.24 | 0.073 | 0.42 | 0.36 | 0.53 | 1.0 | 0.97 |
|  | 0.93 | 0.36 | 0.60 | 0.91 | 0.36 | 0.50 | 0.40 | 0.14 | 0.19 |
|  | 9.2 | 11 | 7.8 | 9.0 | 11 | 6.8 | 5.9 | 7.3 | 5.4 |

Fig. 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

**Angiogenin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 167000 | 198000 | 167000 | 187000 | 167000 | 174000 |
| Average | 186000 | 212000 | 186000 | 183000 | 186000 | 166000 |
| Stdev | 183000 | 103000 | 183000 | 91400 | 183000 | 92300 |
| p(t-test) |  | 0.27 |  | 0.90 |  | 0.67 |
| Min | 20700 | 61200 | 20700 | 37100 | 20700 | 34500 |
| Max | 2240000 | 548000 | 2240000 | 446000 | 2240000 | 378000 |
| n (Samp) | 158 | 73 | 158 | 56 | 158 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 175000 | 202000 | 175000 | 192000 | 175000 | 245000 |
| Average | 193000 | 200000 | 193000 | 182000 | 193000 | 197000 |
| Stdev | 144000 | 73800 | 144000 | 65100 | 144000 | 103000 |
| p(t-test) |  | 0.82 |  | 0.80 |  | 0.95 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 10800 | 35700 | 10800 | 61200 | 10800 | 31500 |
| Max | 2240000 | 410000 | 2240000 | 292000 | 2240000 | 308000 |
| n (Samp) | 380 | 20 | 380 | 11 | 380 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 174000 | 195000 | 174000 | 182000 | 174000 | 173000 |
| Average | 193000 | 212000 | 193000 | 186000 | 193000 | 164000 |
| Stdev | 175000 | 110000 | 175000 | 106000 | 175000 | 99200 |
| p(t-test) | | 0.42 | | 0.78 | | 0.49 |
| Min | 20700 | 23900 | 20700 | 37100 | 20700 | 34500 |
| Max | 2240000 | 548000 | 2240000 | 552000 | 2240000 | 378000 |
| n (Samp) | 184 | 64 | 184 | 62 | 184 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.58 | 0.57 | 0.52 | 0.52 | 0.49 | 0.46 | 0.56 | 0.43 |
| SE | 0.041 | 0.068 | 0.042 | 0.045 | 0.089 | 0.043 | 0.080 | 0.11 | 0.073 |
| p | 0.0094 | 0.23 | 0.093 | 0.61 | 0.84 | 0.85 | 0.64 | 0.58 | 0.31 |
| nCohort 1 | 158 | 380 | 184 | 158 | 380 | 184 | 158 | 380 | 184 |
| nCohort 2 | 73 | 20 | 64 | 56 | 11 | 62 | 15 | 7 | 18 |
| Cutoff 1 Sens 1 Spec 1 | 149000 | 179000 | 143000 | 109000 | 174000 | 109000 | 104000 | 174000 | 99900 |
| | 71% | 70% | 70% | 71% | 73% | 71% | 73% | 71% | 72% |
| | 42% | 52% | 38% | 20% | 49% | 20% | 20% | 49% | 16% |
| Cutoff 2 Sens 2 Spec 2 | 124000 | 166000 | 123000 | 92900 | 124000 | 91600 | 100000 | 88200 | 60300 |
| | 81% | 80% | 81% | 80% | 82% | 81% | 80% | 86% | 83% |
| | 30% | 47% | 27% | 12% | 29% | 12% | 16% | 12% | 6% |
| Cutoff 3 Sens 3 Spec 3 | 109000 | 123000 | 96600 | 81100 | 104000 | 81100 | 44000 | 24100 | 44000 |
| | 90% | 90% | 91% | 91% | 91% | 90% | 93% | 100% | 94% |
| | 20% | 29% | 14% | 10% | 19% | 10% | 4% | 1% | 4% |
| Cutoff 4 Sens 4 Spec 4 | 207000 | 225000 | 217000 | 207000 | 225000 | 217000 | 207000 | 225000 | 217000 |
| | 45% | 30% | 42% | 41% | 27% | 35% | 13% | 57% | 17% |
| | 70% | 70% | 70% | 70% | 70% | 70% | 70% | 70% | 70% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 5 Sens 5 Spec 5 | 227000 | 253000 | 247000 | 227000 | 253000 | 247000 | 227000 | 253000 | 247000 |
| | 33% | 15% | 27% | 30% | 9% | 21% | 13% | 43% | 17% |
| | 80% | 80% | 80% | 80% | 80% | 80% | 80% | 80% | 80% |
| Cutoff 6 Sens 6 Spec 6 | 278000 | 326000 | 293000 | 278000 | 326000 | 293000 | 278000 | 326000 | 293000 |
| | 15% | 5% | 20% | 16% | 0% | 18% | 13% | 0% | 17% |
| | 91% | 90% | 90% | 91% | 90% | 90% | 91% | 90% | 90% |
| OR Quart 2 p Value 95% CI of OR Quart2 | 1.6 | 5.2 | 1.3 | 0.37 | 0.99 | 0.67 | 4.1 | 0.49 | 2.2 |
| | 0.31 | 0.14 | 0.51 | 0.039 | 0.99 | 0.34 | 0.091 | 0.56 | 0.29 |
| | 0.66 | 0.60 | 0.56 | 0.14 | 0.14 | 0.30 | 0.80 | 0.044 | 0.51 |
| | 3.6 | 45 | 3.1 | 0.95 | 7.2 | 1.5 | 21 | 5.5 | 9.3 |
| OR Quart 3 p Value 95% CI of OR Quart3 | 1.8 | 9.8 | 1.6 | 0.69 | 2.6 | 0.38 | 0.50 | 0 | 0.65 |
| | 0.16 | 0.032 | 0.29 | 0.39 | 0.27 | 0.035 | 0.58 | na | 0.65 |
| | 0.79 | 1.2 | 0.68 | 0.29 | 0.48 | 0.16 | 0.044 | na | 0.10 |
| | 4.2 | 79 | 3.7 | 1.6 | 13 | 0.94 | 5.7 | na | 4.1 |
| OR Quart 4 p Value 95% CI of OR Quart4 | 2.8 | 5.2 | 2.0 | 1.1 | 0.99 | 1.1 | 2.8 | 2.0 | 2.6 |
| | 0.013 | 0.14 | 0.10 | 0.89 | 0.99 | 0.80 | 0.24 | 0.42 | 0.18 |
| | 1.2 | 0.60 | 0.87 | 0.47 | 0.14 | 0.52 | 0.51 | 0.36 | 0.63 |
| | 6.5 | 45 | 4.5 | 2.4 | 7.2 | 2.4 | 15 | 11 | 11 |

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.9 | 37.7 | 24.9 | 36.3 | 24.9 | 18.2 |
| Average | 53.1 | 48.0 | 53.1 | 54.1 | 53.1 | 40.0 |
| Stdev | 160 | 42.6 | 160 | 56.2 | 160 | 49.0 |
| p(t-test) | | 0.79 | | 0.96 | | 0.75 |
| Min | 1.77 | 6.18 | 1.77 | 4.89 | 1.77 | 5.00 |
| Max | 1900 | 243 | 1900 | 339 | 1900 | 176 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.1 | 52.3 | 31.1 | 51.8 | 31.1 | 43.8 |
| Average | 47.7 | 60.3 | 47.7 | 60.2 | 47.7 | 37.7 |
| Stdev | 108 | 44.6 | 108 | 34.1 | 108 | 30.4 |
| p(t-test) | | 0.60 | | 0.70 | | 0.81 |
| Min | 1.77 | 8.18 | 1.77 | 19.4 | 1.77 | 8.28 |
| Max | 1900 | 192 | 1900 | 127 | 1900 | 94.2 |
| n (Samp) | 379 | 20 | 379 | 11 | 379 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.8 | 36.3 | 31.8 | 36.3 | 31.8 | 26.3 |
| Average | 61.4 | 48.0 | 61.4 | 53.4 | 61.4 | 45.6 |
| Stdev | 152 | 43.1 | 152 | 55.5 | 152 | 45.6 |
| p(t-test) | | 0.49 | | 0.69 | | 0.66 |
| Min | 2.68 | 6.18 | 2.68 | 4.89 | 2.68 | 5.00 |
| Max | 1900 | 243 | 1900 | 339 | 1900 | 176 |
| n (Samp) | 183 | 64 | 183 | 62 | 183 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.68 | 0.54 | 0.63 | 0.71 | 0.55 | 0.44 | 0.49 | 0.46 |
| SE | 0.041 | 0.067 | 0.042 | 0.045 | 0.089 | 0.043 | 0.080 | 0.11 | 0.073 |
| p | 0.0019 | 0.0060 | 0.32 | 0.0040 | 0.016 | 0.25 | 0.45 | 0.95 | 0.62 |
| nCohort 1 | 157 | 379 | 183 | 157 | 379 | 183 | 157 | 379 | 183 |
| nCohort 2 | 73 | 20 | 64 | 56 | 11 | 62 | 15 | 7 | .18 |
| Cutoff 1 Sens 1 Spec 1 | 26.3 | 40.8 | 25.8 | 23.2 | 37.1 | 22.8 | 10.7 | 15.3 | 17.6 |
| | 71% | 70% | 70% | 71% | 73% | 71% | 73% | 71% | 72% |
| | 52% | 68% | 40% | 48% | 62% | 36% | 18% | 21% | 25% |
| Cutoff 2 Sens 2 Spec 2 | 21.8 | 33.9 | 20.7 | 19.4 | 32.5 | 19.4 | 10.4 | 9.30 | 10.7 |
| | 81% | 80% | 81% | 80% | 82% | 81% | 80% | 86% | 83% |
| | 44% | 56% | 31% | 38% | 54% | 28% | 17% | 8% | 13% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 Sens 3 Spec 3 | 13.4 | 22.2 | 14.3 | 13.5 | 28.7 | 14.6 | 8.28 | 8.28 | 9.30 |
| | 90% | 90% | 91% | 91% | 91% | 90% | 93% | 100% | 94% |
| | 24% | 35% | 19% | 24% | 45% | 19% | 10% | 7% | 8% |
| Cutoff 4 Sens 4 Spec 4 | 37.7 | 43.0 | 50.4 | 37.7 | 43.0 | 50.4 | 37.7 | 43.0 | 50.4 |
| | 51% | 60% | 31% | 48% | 64% | 34% | 33% | 57% | 39% |
| | 70% | 70% | 70% | 70% | 70% | 70% | 70% | 70% | 70% |
| Cutoff 5 Sens 5 Spec 5 | 51.6 | 57.5 | 71.1 | 51.6 | 57.5 | 71.1 | 51.6 | 57.5 | 71.1 |
| | 27% | 50% | 19% | 34% | 36% | 16% | 33% | 14% | 22% |
| | 80% | 80% | 80% | 80% | 80% | 80% | 80% | 80% | 80% |
| Cutoff 6 Sens 6 Spec 6 | 88.5 | 87.8 | 105 | 88.5 | 87.8 | 105 | 88.5 | 87.8 | 105 |
| | 10% | 15% | 5% | 14% | 27% | 11% | 13% | 14% | 11% |
| | 90% | 90% | 90% | 90% | 90% | 90% | 90% | 90% | 90% |
| OR Quart 2 p Value 95% CI OR Quart2 | 1.6 | 0.99 | 2.1 | 1.3 | >2.0 | 1.4 | 0 | 3.1 | 0.80 |
| | 0.28 | 0.99 | 0.098 | 0.62 | <0.57 | 0.50 | na | 0.33 | 0.75 |
| | 0.67 | 0.14 | 0.87 | 0.48 | >0.18 | 0.56 | na | 0.32 | 0.20 |
| | 4.0 | 7.2 | 5.0 | 3.4 | na | 3.3 | na | 30 | 3.2 |
| OR Quart 3 p Value 95% CI of OR Quart3 | 3.9 | 3.1 | 2.4 | 2.3 | >3.1 | 2.4 | 0.78 | 0 | 0.80 |
| | 0.0017 | 0.17 | 0.044 | 0.075 | <0.33 | 0.042 | 0.73 | na | 0.75 |
| | 1.7 | 0.61 | 1.0 | 0.92 | >0.32 | 1.0 | 0.19 | na | 0.20 |
| | 9.3 | 16 | 5.8 | 5.8 | na | 5.5 | 3.1 | na | 3.2 |
| OR Quart 4 p Value 95% CI of OR Quart4 | 2.9 | 5.4 | 1.8 | 2.7 | >6.3 | 1.6 | 1.2 | 3.1 | 1.0 |
| | 0.017 | 0.033 | 0.20 | 0.035 | <0.091 | 0.30 | 0.75 | 0.33 | 0.97 |
| | 1.2 | 1.1 | 0.73 | 1.1 | >0.75 | 0.67 | 0.35 | 0.32 | 0.28 |
| | 6.8 | 25 | 4.3 | 6.6 | na | 3.8 | 4.4 | 30 | 3.8 |

**Angiopoietin-related protein 6**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 63.6 | 70.1 | 63.6 | 63.3 | 63.6 | 48.2 |
| Average | 73.5 | 76.7 | 73.5 | 74.2 | 73.5 | 53.6 |
| Stdev | 46.5 | 38.7 | 46.5 | 41.0 | 46.5 | 33.3 |
| p(t-test) | | 0.62 | | 0.93 | | 0.11 |
| Min | 7.54 | 28.3 | 7.54 | 3.36 | 7.54 | 4.29 |
| Max | 240 | 250 | 240 | 212 | 240 | 133 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 64.7 | 79.7 | 64.7 | 64.8 | 64.7 | 64.8 |
| Average | 71.8 | 89.9 | 71.8 | 83.3 | 71.8 | 72.3 |
| Stdev | 41.0 | 56.4 | 41.0 | 52.5 | 41.0 | 31.3 |
| p(t-test) | | 0.060 | | 0.36 | | 0.97 |
| Min | 3.36 | 36.7 | 3.36 | 38.7 | 3.36 | 47.8 |
| Max | 288 | 250 | 288 | 212 | 288 | 138 |
| n (Samp) | 379 | 20 | 379 | 11 | 379 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72.3 | 68.0 | 72.3 | 61.4 | 72.3 | 49.1 |
| Average | 82.2 | 72.7 | 82.2 | 73.4 | 82.2 | 60.5 |
| Stdev | 53.1 | 29.6 | 53.1 | 43.1 | 53.1 | 36.4 |
| p(t-test) | | 0.18 | | 0.24 | | 0.093 |
| Min | 7.54 | 28.3 | 7.54 | 3.36 | 7.54 | 4.29 |
| Max | 283 | 154 | 283 | 250 | 283 | 133 |
| n (Samp) | 183 | 64 | 183 | 62 | 183 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.59 | 0.49 | 0.53 | 0.55 | 0.47 | 0.37 | 0.53 | 0.38 |
| SE | 0.041 | 0.068 | 0.042 | 0.045 | 0.091 | 0.043 | 0.080 | 0.11 | 0.073 |
| p | 0.22 | 0.19 | 0.78 | 0.56 | 0.58 | 0.45 | 0.12 | 0.80 | 0.10 |
| nCohort 1 | 157 | 379 | 183 | 157 | 379 | 183 | 157 | 379 | 183 |
| nCohort 2 | 73 | 20 | 64 | 56 | 11 | 62 | 15 | 7 | 18 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 1 Sens 1 Spec 1 | 52.7 | 48.3 | 53.7 | 49.6 | 53.7 | 47.6 | 40.3 | 53.7 | 41.8 |
| | 71% | 70% | 70% | 71% | 73% | 71% | 73% | 71% | 72% |
| | 42% | 32% | 36% | 37% | 40% | 30% | 27% | 40% | 23% |
| Cutoff 2 Sens 2 | 43.4 | 46.7 | 43.4 | 43.4 | 52.1 | 42.9 | 36.5 | 48.2 | 36.5 |
| | 81% | 80% | 81% | 80% | 82% | 81% | 80% | 86% | 83% |
| Spec 2 | 31% | 29% | 26% | 31% | 37% | 25% | 22% | 32% | 17% |
| Cutoff 3 Sens 3 Spec 3 | 38.1 | 43.4 | 38.1 | 36.5 | 44.0 | 36.5 | 22.6 | 47.7 | 21.7 |
| | 90% | 90% | 91% | 91% | 91% | 90% | 93% | 100% | 94% |
| | 23% | 27% | 18% | 22% | 28% | 17% | 8% | 31% | 5% |
| Cutoff 4 Sens 4 Spec 4 | 89.6 | 87.7 | 92.9 | 89.6 | 87.7 | 92.9 | 89.6 | 87.7 | 92.9 |
| | 29% | 35% | 22% | 25% | 18% | 26% | 13% | 14% | 22% |
| | 70% | 70% | 70% | 70% | 70% | 70% | 70% | 70% | 70% |
| Cutoff 5 Sens 5 Spec 5 | 102 | 96.5 | 114 | 102 | 96.5 | 114 | 102 | 96.5 | 114 |
| | 16% | 25% | 9% | 12% | 18% | 10% | 13% | 14% | 17% |
| | 80% | 80% | 80% | 80% | 80% | 80% | 80% | 80% | 80% |
| Cutoff 6 Sens 6 Spec 6 | 135 | 121 | 151 | 135 | 121 | 151 | 135 | 121 | 151 |
| | 7% | 20% | 2% | 9% | 18% | 8% | 0% | 14% | 0% |
| | 90% | 90% | 90% | 90% | 90% | 90% | 90% | 90% | 90% |
| OR Quart2 p Value 95% CI of OR Quart2 | 2.7 | 2.6 | 2.0 | 3.4 | 4.1 | 1.2 | 1.0 | >3.1 | 0.67 |
| | 0.019 | 0.27 | 0.097 | 0.010 | 0.21 | 0.63 | 1.0 | <0.34 | 0.66 |
| | 1.2 | 0.48 | 0.88 | 1.3 | 0.45 | 0.52 | 0.13 | >0.31 | 0.11 |
| | 6.4 | 13 | 4.8 | 8.7 | 37 | 3.0 | 7.4 | na | 4.2 |
| OR Quart3 p Value 95% CI of OR Quart3 | 2.6 | 4.2 | 2.4 | 2.0 | 4.1 | 2.4 | 4.0 | >3.1 | 2.6 |
| | 0.025 | 0.073 | 0.043 | 0.16 | 0.21 | 0.041 | 0.097 | <0.33 | 0.18 |
| | 1.1 | 0.87 | 1.0 | 0.77 | 0.45 | 1.0 | 0.78 | >0.32 | 0.63 |
| | 6.1 | 20 | 5.5 | 5.3 | 38 | 5.3 | 20 | na | 11 |
| OR Quart4 p Value 95% CI OR Quart4 | 1.7 | 2.6 | 1.3 | 2.0 | 2.0 | 1.2 | 2.1 | >1.0 | 2.2 |
| | 0.21 | 0.27 | 0.62 | 0.17 | 0.57 | 0.63 | 0.41 | <1.0 | 0.29 |
| | 0.73 | 0.48 | 0.51 | 0.75 | 0.18 | 0.52 | 0.36 | >0.062 | 0.51 |
| | 4.1 | 13 | 3.1 | 5.2 | 22 | 3.0 | 12 | na | 9.3 |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 20.4 | 20.9 | 20.4 | 25.2 | 20.4 | 17.7 |
| Average | 25.5 | 24.6 | 25.5 | 33.1 | 25.5 | 33.0 |
| Stdev | 17.7 | 16.7 | 17.7 | 25.5 | 17.7 | 31.9 |
| p(t-test) | | 0.70 | | 0.019 | | 0.16 |
| Min | 0.00246 | 0.00246 | 0.00246 | 5.07 | 0.00246 | 6.76 |
| Max | 121 | 72.6 | 121 | 133 | 121 | 113 |
| n (Samp) | 156 | 71 | 156 | 53 | 156 | 15 |
| n (Patient) | 87 | 71 | 87 | 53 | 87 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.3 | 20.3 | 21.3 | 31.4 | 21.3 | 43.9 |
| Average | 27.2 | 26.7 | 27.2 | 33.1 | 27.2 | 41.8 |
| Stdev | 21.5 | 17.9 | 21.5 | 14.4 | 21.5 | 29.6 |
| p(t-test) | | 0.92 | | 0.37 | | 0.079 |
| Min | 0.00246 | 7.08 | 0.00246 | 12.3 | 0.00246 | 14.3 |
| Max | 178 | 72.6 | 178 | 61.8 | 178 | 98.8 |
| n (Samp) | 372 | 19 | 372 | 11 | 372 | 7 |
| n (Patient) | 173 | 19 | 173 | 11 | 173 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.1 | 20.9 | 21.1 | 24.4 | 21.1 | 19.5 |
| Average | 26.6 | 24.6 | 26.6 | 34.1 | 26.6 | 31.7 |
| Stdev | 21.4 | 16.8 | 21.4 | 29.9 | 21.4 | 30.2 |
| p(t-test) | | 0.51 | | 0.035 | | 0.36 |
| Min | 0.00246 | 0.00246 | 0.00246 | 5.07 | 0.00246 | 4.60 |
| Max | 198 | 72.6 | 198 | 162 | 198 | 113 |
| n (Samp) | 181 | 63 | 181 | 59 | 181 | 18 |
| n (Patient) | 88 | 63 | 88 | 59 | 88 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | 0.50 | 0.47 | 0.58 | 0.66 | 0.56 | 0.50 | 0.67 | 0.49 |
| SE | 0.042 | 0.068 | 0.043 | 0.046 | 0.091 | 0.044 | 0.078 | 0.11 | 0.072 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.65 | 0.98 | 0.55 | 0.095 | 0.071 | 0.16 | 0.97 | 0.14 | 0.90 |
| nCohort 1 | 156 | 372 | 181 | 156 | 372 | 181 | 156 | 372 | 181 |
| nCohort 2 | 71 | 19 | 63 | 53 | 11 | 59 | 15 | 7 | 18 |
| Cutoff 1 | 13.6 | 14.3 | 13.6 | 16.8 | 25.6 | 16.8 | 14.7 | 21.1 | 14.7 |
| Sens 1 | 72% | 74% | 71% | 72% | 73% | 71% | 73% | 71% | 72% |
| Spec 1 | 24% | 27% | 23% | 39% | 61% | 37% | 29% | 50% | 28% |
| Cutoff 2 | 11.4 | 11.7 | 10.3 | 13.5 | 21.8 | 13.5 | 13.9 | 16.1 | 12.5 |
| Sens 2 | 80% | 84% | 81% | 81% | 82% | 81% | 80% | 86% | 83% |
| Spec 2 | 17% | 17% | 13% | 24% | 51% | 22% | 26% | 33% | 18% |
| Cutoff 3 | 8.58 | 8.58 | 8.57 | 11.0 | 18.1 | 11.0 | 6.93 | 14.3 | 6.01 |
| Sens 3 | 90% | 95% | 90% | 91% | 91% | 92% | 93% | 100% | 94% |
| Spec 3 | 12% | 10% | 10% | 15% | 41% | 14% | 6% | 26% | 4% |
| Cutoff 4 | 30.9 | 30.4 | 30.9 | 30.9 | 30.4 | 30.9 | 30.9 | 30.4 | 30.9 |
| Sens 4 | 25% | 32% | 25% | 38% | 55% | 34% | 27% | 57% | 28% |
| Spec 4 | 71% | 70% | 70% | 71% | 70% | 70% | 71% | 70% | 70% |
| Cutoff 5 | 35.6 | 37.3 | 37.0 | 35.6 | 37.3 | 37.0 | 35.6 | 37.3 | 37.0 |
| Sens 5 | 18% | 21% | 16% | 32% | 27% | 29% | 27% | 57% | 22% |
| Spec 5 | 80% | 80% | 81% | 80% | 80% | 81% | 80% | 80% | 81% |
| Cutoff 6 | 47.0 | 52.4 | 45.5 | 47.0 | 52.4 | 45.5 | 47.0 | 52.4 | 45.5 |
| Sens 6 | 11% | 11% | 11% | 23% | 9% | 22% | 20% | 29% | 22% |
| Spec 6 | 90% | 90% | 90% | 90% | 90% | 90% | 90% | 90% | 90% |
| OR Quart 2 | 1.4 | 0.99 | 1.3 | 0.79 | 0.99 | 0.74 | 1.7 | >3.1 | 1.3 |
| | 0.42 | 0.99 | 0.53 | 0.63 | 0.99 | 0.50 | 0.48 | <0.34 | 0.73 |
| p Value 95% CI of OR Quart2 | 0.63 | 0.28 | 0.57 | 0.31 | 0.061 | 0.30 | 0.38 | >0.31 | 0.32 |
| | 3.1 | 3.5 | 2.9 | 2.0 | 16 | 1.8 | 7.7 | na | 5.1 |
| OR Quart 3 | 0.92 | 0.78 | 1.0 | 1.2 | 5.2 | 1.2 | 0.98 | >0 | 1.3 |
| | 0.83 | 0.72 | 1.0 | 0.65 | 0.14 | 0.67 | 0.98 | <na | 0.73 |
| p Value 95% CI of OR Quart3 | 0.40 | 0.20 | 0.43 | 0.50 | 0.59 | 0.52 | 0.19 | >na | 0.32 |
| | 2.1 | 3.0 | 2.3 | 3.0 | 45 | 2.7 | 5.1 | na | 5.1 |
| OR Quart 4 | 1.4 | 0.99 | 1.4 | 1.6 | 4.1 | 1.4 | 1.3 | >4.1 | 1.0 |
| | 0.38 | 0.99 | 0.41 | 0.30 | 0.21 | 0.41 | 0.72 | <0.21 | 0.98 |
| p Value 95% CI ot OR Quart4 | 0.64 | 0.28 | 0.62 | 0.66 | 0.45 | 0.62 | 0.28 | >0.45 | 0.24 |
| | 3.2 | 3.5 | 3.2 | 3.7 | 37 | 3.2 | 6.4 | na | 4.3 |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.02 | 3.84 | 4.02 | 4.08 | 4.02 | 3.93 |
| Average | 4.29 | 4.55 | 4.29 | 5.49 | 4.29 | 5.61 |
| Stdev | 2.94 | 6.28 | 2.94 | 5.39 | 2.94 | 3.80 |
| p(t-test) | | 0.67 | | 0.043 | | 0.12 |
| Min | 0.00226 | 0.00274 | 0.00226 | 0.00282 | 0.00226 | 1.92 |
| Max | 15.0 | 50.2 | 15.0 | 32.2 | 15.0 | 13.9 |
| n (Samp) | 155 | 72 | 155 | 54 | 155 | 14 |
| n (Patient) | 87 | 72 | 87 | 54 | 87 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.02 | 2.23 | 4.02 | 4.58 | 4.02 | 3.61 |
| Average | 4.43 | 5.58 | 4.43 | 5.03 | 4.43 | 5.58 |
| Stdev | 3.21 | 11.4 | 3.21 | 3.31 | 3.21 | 6.34 |
| p(t-test) | | 0.22 | | 0.54 | | 0.36 |
| Min | 0.00226 | 0.00282 | 0.00226 | 0.00282 | 0.00226 | 0.00274 |
| Max | 21.0 | 50.2 | 21.0 | 11.8 | 21.0 | 17.6 |
| n (Samp) | 372 | 19 | 372 | 11 | 372 | 7 |
| n (Patient) | 174 | 19 | 174 | 11 | 174 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.74 | 4.05 | 3.74 | 4.08 | 3.74 | 4.24 |
| Average | 4.87 | 3.93 | 4.87 | 5.98 | 4.87 | 5.34 |
| Stdev | 7.41 | 2.74 | 7.41 | 7.75 | 7.41 | 3.44 |
| p(t-test) | | 0.32 | | 0.32 | | 0.79 |
| Min | 0.00226 | 0.00274 | 0.00226 | 0.00289 | 0.00226 | 1.92 |
| Max | 60.7 | 12.2 | 60.7 | 50.2 | 60.7 | 13.9 |
| n (Samp) | 180 | 64 | 180 | 60 | 180 | 17 |
| n (Patient) | 88 | 64 | 88 | 60 | 88 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or | sCr only | UO only |
| AUC | 0.47 | 0.38 | 0.50 | 0.54 | 0.55 | 0.56 | 0.57 | 0.50 | 0.59 |
| SE | 0.041 | 0.070 | 0.042 | 0.046 | 0.091 | 0.044 | 0.083 | 0.11 | 0.075 |
| p | 0.45 | 0.077 | 0.93 | 0.41 | 0.55 | 0.19 | 0.40 | 0.98 | 0.24 |
| nCohort 1 | 155 | 372 | 180 | 155 | 372 | 180 | 155 | 372 | 180 |
| nCohort 2 | 72 | 19 | 64 | 54 | 11 | 60 | 14 | 7 | 17 |
| Cutoff 1 | 1.92 | 0.0204 | 2.42 | 2.92 | 3.54 | 2.92 | 3.54 | 1.59 | 3.54 |
| Sens 1 | 71% | 74% | 70% | 70% | 73% | 70% | 71% | 71% | 71% |
| Spec 1 | 23% | 9% | 31% | 34% | 42% | 40% | 41% | 16% | 46% |
| Cutoff 2 | 0.743 | 0.00282 | 1.18 | 2.26 | 2.89 | 1.92 | 2.26 | 0.0204 | 2.65 |
| Sens 2 | 81% | 89% | 83% | 81% | 82% | 80% | 93% | 86% | 82% |
| Spec 2 | 10% | 3% | 16% | 25% | 32% | 27% | 25% | 9% | 37% |
| Cutoff 3 | 0.00282 | 0.00274 | 0.00282 | 0.743 | 2.42 | 0.743 | 2.26 | 0.00226 | 2.26 |
| Sens 3 | 93% | 100% | 95% | 91% | 91% | 90% | 93% | 100% | 94% |
| Spec 3 | 4% | 2% | 8% | 10% | 26% | 14% | 25% | 1% | 31% |
| Cutoff 4 | 5.14 | 5.42 | 5.12 | 5.14 | 5.42 | 5.12 | 5.14 | 5.42 | 5.12 |
| Sens 4 | 31% | 21% | 28% | 37% | 18% | 40% | 29% | 43% | 24% |
| Spec 4 | 70% | 70% | 70% | 70% | 70% | 70% | 70% | 70% | 70% |
| Cutoff 5 | 6.20 | 6.52 | 6.20 | 6.20 | 6.52 | 6.20 | 6.20 | 6.52 | 6.20 |
| Sens 5 | 21% | 21% | 17% | 28% | 18% | 27% | 29% | 43% | 24% |
| Spec 5 | 81% | 80% | 81% | 81% | 80% | 81% | 81% | 80% | 81% |
| Cutoff 6 | 7.89 | 8.40 | 7.89 | 7.89 | 8.40 | 7.89 | 7.89 | 8.40 | 7.89 |
| Sens 6 | 12% | 11% | 9% | 19% | 18% | 17% | 29% | 29% | 24% |
| Spec 6 | 90% | 90% | 90% | 90% | 90% | 90% | 90% | 90% | 90% |
| OR Quart 2 | 0.92 | 0.49 | 1.3 | 1.2 | 3.0 | 1.5 | 1.4 | 0 | 8.0 |
| | 0.84 | 0.42 | 0.54 | 0.65 | 0.34 | 0.39 | 0.69 | na | 0.056 |
| p Value 95% CI of OR Quart2 | 0.42 | 0.088 | 0.58 | 0.50 | 0.31 | 0.62 | 0.29 | na | 0.95 |
| | 2.0 | 2.7 | 2.9 | 3.0 | 30 | 3.4 | 6.5 | na | 68 |
| OR Quart 3 | 0.85 | 0.74 | 0.91 | 1.0 | 5.2 | 1.1 | 1.0 | 0.33 | 5.5 |
| | 0.68 | 0.70 | 0.83 | 1.0 | 0.14 | 0.82 | 1.0 | 0.34 | 0.13 |
| | sCr or UO | sCr only | UO only | sCr or UO | sCr only | UO only | sCr or UO | sCr only | UO only |
| p Value | 0.38 | 0.16 | 0.40 | 0.40 | 0.59 | 0.46 | 0.19 | 0.033 | 0.61 |
| 95% CI of OR Quart3 | 1.9 | 3.4 | 2.1 | 2.5 | 45 | 2.7 | 5.3 | 3.2 | 49 |
| OR Quart 4 | 1.3 | 2.7 | 1.2 | 1.4 | 2.0 | 1.9 | 1.3 | 1.0 | 4.2 |
| | 0.51 | 0.10 | 0.68 | 0.41 | 0.57 | 0.15 | 0.72 | 0.99 | 0.21 |

(continued)

| | sCr or UO | sCr only | UO only | sCr or UO | sCr only | UO only | sCr or UO | sCr only | UO only |
|---|---|---|---|---|---|---|---|---|---|
| p Value 95% CI of OR Quart4 | 0.60 | 0.82 | 0.53 | 0.60 | 0.18 | 0.80 | 0.28 | 0.20 | 0.45 |
| | 2.8 | 8.9 | 2.7 | 3.4 | 22 | 4.3 | 6.4 | 5.1 | 39 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 35400 | 42600 | 35400 | 43100 | 35400 | 48800 |
| Average | 43000 | 57500 | 43000 | 54500 | 43000 | 49500 |
| Stdev | 32700 | 49300 | 32700 | 44800 | 32700 | 36500 |
| p(t-test) | | 0.0085 | | 0.042 | | 0.47 |
| Min | 4620 | 8430 | 4620 | 7440 | 4620 | 7440 |
| Max | 328000 | 275000 | 328000 | 241000 | 328000 | 133000 |
| n (Samp) | 158 | 73 | 158 | 56 | 158 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 38700 | 56400 | 38700 | 69400 | 38700 | 65700 |
| Average | 47300 | 62400 | 47300 | 90000 | 47300 | 74800 |
| Stdev | 34800 | 38900 | 34800 | 58000 | 34800 | 40700 |
| p(t-test) | | 0.062 | | 1.1E-4 | | 0.040 |
| Min | 3600 | 13600 | 3600 | 19800 | 3600 | 11600 |
| Max | 328000 | 165000 | 328000 | 196000 | 328000 | 133000 |
| n (Samp) | 380 | 20 | 380 | 11 | 380 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 37700 | 40000 | 37700 | 36800 | 37700 | 42800 |
| Average | 48100 | 56100 | 48100 | 49300 | 48100 | 69100 |
| Stdev | 36900 | 51500 | 36900 | 40400 | 36900 | 107000 |
| p(t-test) | | 0.18 | | 0.83 | | 0.073 |
| Min | 4620 | 8430 | 4620 | 7440 | 4620 | 7440 |
| Max | 328000 | 275000 | 328000 | 241000 | 328000 | 483000 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 184 | 64 | 184 | 62 | 184 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.63 | 0.52 | 0.57 | 0.74 | 0.50 | 0.55 | 0.73 | 0.51 |
| SE | 0.041 | 0.068 | 0.042 | 0.045 | 0.087 | 0.042 | 0.080 | 0.11 | 0.072 |
| p | 0.034 | 0.054 | 0.59 | 0.13 | 0.0051 | 0.99 | 0.53 | 0.033 | 0.84 |
| nCohort 1 | 158 | 380 | 184 | 158 | 380 | 184 | 158 | 380 | 184 |
| nCohort 2 | 73 | 20 | 64 | 56 | 11 | 62 | 15 | 7 | 18 |
| Cutoff 1 | 30600 | 46100 | 29800 | 30600 | 52400 | 30900 | 23500 | 58300 | 25600 |
| Sens 1 | 71% | 70% | 70% | 71% | 73% | 71% | 73% | 71% | 72% |
| Spec 1 | 40% | 61% | 33% | 40% | 69% | 37% | 23% | 75% | 23% |
| Cutoff 2 | 23500 | 31900 | 22700 | 20400 | 52100 | 20100 | 15200 | 55100 | 15700 |
| Sens 2 | 81% | 80% | 81% | 80% | 82% | 81% | 80% | 86% | 83% |
| Spec 2 | 23% | 35% | 18% | 16% | 68% | 14% | 9% | 72% | 8% |
| Cutoff 3 | 18700 | 20100 | 18700 | 14500 | 29300 | 14300 | 12500 | 11100 | 12500 |
| Sens 3 | 90% | 90% | 91% | 91% | 91% | 90% | 93% | 100% | 94% |
| Spec 3 | 13% | 14% | 11% | 7% | 30% | 6% | 4% | 4% | 4% |
| Cutoff 4 | 46600 | 53000 | 52100 | 46600 | 53000 | 52100 | 46600 | 53000 | 52100 |
| Sens 4 | 45% | 60% | 34% | 48% | 64% | 32% | 53% | 86% | 39% |
| Spec 4 | 70% | 70% | 70% | 70% | 70% | 70% | 70% | 70% | 70% |
| Cutoff 5 | 56200 | 66300 | 65300 | 56200 | 66300 | 65300 | 56200 | 66300 | 65300 |
| Sens 5 | 37% | 30% | 28% | 34% | 55% | 27% | 40% | 43% | 22% |
| Spec 5 | 80% | 80% | 80% | 80% | 80% | 80% | 80% | 80% | 80% |
| Cutoff 6 | 78000 | 84400 | 91700 | 78000 | 84400 | 91700 | 78000 | 84400 | 91700 |
| Sens 6 | 19% | 25% | 12% | 16% | 36% | 8% | 13% | 29% | 11% |
| Spec 6 | 91% | 90% | 90% | 91% | 90% | 90% | 91% | 90% | 90% |
| OR Quart 2 | 1.1 | 0.49 | 0.92 | 0.88 | 0.99 | 0.60 | 0 | 0 | 0.46 |
| | 0.88 | 0.42 | 0.84 | 0.78 | 0.99 | 0.23 | na | na | 0.29 |
| p Value 95% CI of OR Quart2 | 0.47 | 0.088 | 0.41 | 0.36 | 0.061 | 0.26 | na | na | 0.11 |
| | 2.4 | 2.7 | 2.1 | 2.2 | 16 | 1.4 | na | na | 1.9 |
| OR Quart 3 | 0.98 | 1.5 | 0.76 | 0.90 | 3.0 | 0.85 | 0.78 | 2.0 | 0.64 |
| | 0.96 | 0.52 | 0.53 | 0.82 | 0.34 | 0.69 | 0.73 | 0.57 | 0.51 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value 95% CI of OR Quart3 | 0.42 | 0.42 | 0.33 | 0.37 | 0.31 | 0.39 | 0.19 | 0.18 | 0.17 |
|  | 2.2 | 5.6 | 1.8 | 2.2 | 30 | 1.9 | 3.1 | 22 | 2.4 |
| OR Quart 4 | 2.4 | 2.1 | 1.4 | 1.7 | 6.3 | 0.87 | 1.2 | 4.1 | 0.80 |
|  | 0.026 | 0.24 | 0.43 | 0.23 | 0.092 | 0.73 | 0.78 | 0.21 | 0.72 |
| p Value | 1.1 | 0.61 | 0.63 | 0.72 | 0.74 | 0.39 | 0.34 | 0.45 | 0.23 |
| 95% CI of OR Quart4 | 5.3 | 7.2 | 3.0 | 3.9 | 53 | 1.9 | 4.3 | 37 | 2.8 |

**Proepiregulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.392 | 0.374 | 0.392 | 0.399 | 0.392 | 0.329 |
| Average | 0.509 | 1.12 | 0.509 | 1.16 | 0.509 | 0.604 |
| Stdev | 0.628 | 3.14 | 0.628 | 4.21 | 0.628 | 0.563 |
| p(t-test) |  | 0.020 |  | 0.061 |  | 0.59 |
| Min | 0.000152 | 0.000152 | 0.000152 | 0.000152 | 0.000152 | 0.0793 |
| Max | 6.96 | 20.6 | 6.96 | 31.1 | 6.96 | 1.68 |
| n (Samp) | 156 | 72 | 156 | 54 | 156 | 14 |
| n (Patient) | 87 | 72 | 87 | 54 | 87 | 14 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.396 | 0.392 | 0.396 | 0.298 | 0.396 | 0.472 |
| Average | 0.667 | 0.825 | 0.667 | 0.693 | 0.667 | 0.673 |
| Stdev | 1.42 | 1.41 | 1.42 | 1.04 | 1.42 | 0.713 |
| p(t-test) |  | 0.64 |  | 0.95 |  | 0.99 |
| Min | 0.000152 | 0.000152 | 0.000152 | 0.106 | 0.000152 | 0.192 |
| Max | 18.0 | 4.75 | 18.0 | 3.65 | 18.0 | 2.24 |
| n (Samp) | 371 | 19 | 371 | 11 | 371 | 7 |
| n (Patient) | 173 | 19 | 173 | 11 | 173 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.383 | 0.363 | 0.383 | 0.399 | 0.383 | 0.365 |
| Average | 0.756 | 1.14 | 0.756 | 1.06 | 0.756 | 0.550 |
| Stdev | 2.51 | 3.29 | 2.51 | 3.99 | 2.51 | 0.524 |
| p(t-test) | | 0.33 | | 0.49 | | 0.74 |
| Min | 0.000152 | 0.000152 | 0.000152 | 0.000152 | 0.000152 | 0.0793 |
| Max | 32.1 | 20.6 | 32.1 | 31.1 | 32.1 | 1.68 |
| n (Samp) | 181 | 64 | 181 | 60 | 181 | 17 |
| n (Patient) | 88 | 64 | 88 | 60 | 88 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.45 | 0.51 | 0.50 | 0.46 | 0.52 | 0.51 | 0.54 | 0.49 |
| SE | 0.041 | 0.070 | 0.042 | 0.046 | 0.090 | 0.043 | 0.081 | 0.11 | 0.074 |
| p | 1.00 | 0.43 | 0.82 | 1.00 | 0.62 | 0.70 | 0.92 | 0.69 | 0.93 |
| nCohort 1 | 156 | 371 | 181 | 156 | 371 | 181 | 156 | 371 | 181 |
| nCohort 2 | 72 | 19 | 64 | 54 | 11 | 60 | 14 | 7 | 17 |
| Cutoff 1 | 0.188 | 0.134 | 0.192 | 0.274 | 0.188 | 0.274 | 0.238 | 0.286 | 0.238 |
| Sens 1 | 71% | 74% | 73% | 70% | 82% | 70% | 71% | 71% | 71% |
| Spec 1 | 20% | 13% | 24% | 30% | 21% | 33% | 25% | 34% | 29% |
| Cutoff 2 | 0.117 | 0.00127 | 0.126 | 0.134 | 0.188 | 0.179 | 0.139 | 0.278 | 0.139 |
| Sens 2 | 81% | 84% | 83% | 83% | 82% | 80% | 86% | 86% | 82% |
| Spec 2 | 12% | 1% | 15% | 12% | 21% | 21% | 14% | 33% | 18% |
| Cutoff 3 | 0.0201 | 0 | 0.0796 | 0.106 | 0.134 | 0.126 | 0.134 | 0.188 | 0.134 |
| Sens 3 | 90% | 100% | 91% | 91% | 91% | 90% | 93% | 100% | 94% |
| Spec 3 | 6% | 0% | 12% | 10% | 13% | 15% | 12% | 21% | 16% |
| Cutoff 4 | 0.589 | 0.645 | 0.587 | 0.589 | 0.645 | 0.587 | 0.589 | 0.645 | 0.587 |
| Sens 4 | 35% | 32% | 30% | 28% | 18% | 30% | 36% | 29% | 29% |
| Spec 4 | 71% | 70% | 70% | 71% | 70% | 70% | 71% | 70% | 70% |
| Cutoff 5 | 0.695 | 0.799 | 0.717 | 0.695 | 0.799 | 0.717 | 0.695 | 0.799 | 0.717 |
| Sens 5 | 32% | 21% | 27% | 19% | 18% | 20% | 36% | 14% | 29% |
| Spec 5 | 81% | 80% | 80% | 81% | 80% | 80% | 81% | 80% | 80% |
| Cutoff 6 | 0.913 | 1.20 | 0.967 | 0.913 | 1.20 | 0.967 | 0.913 | 1.20 | 0.967 |
| Sens 6 | 24% | 11% | 23% | 17% | 18% | 17% | 21% | 14% | 18% |
| Spec 6 | 90% | 90% | 90% | 90% | 90% | 90% | 90% | 90% | 90% |
| OR Quart 2 | 0.39 | 0.49 | 1.0 | 0.93 | 1.0 | 1.0 | 0.97 | 2.0 | 0.38 |
| | 0.028 | 0.32 | 1.0 | 0.87 | 0.99 | 1.0 | 0.97 | 0.57 | 0.27 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value 95% CI of OR Quart2 | 0.17 | 0.12 | 0.45 | 0.39 | 0.14 | 0.43 | 0.23 | 0.18 | 0.071 |
|  | 0.90 | 2.0 | 2.2 | 2.2 | 7.3 | 2.3 | 4.2 | 22 | 2.1 |
| OR Quart 3 | 0.44 | 0.32 | 0.63 | 0.91 | 1.5 | 1.1 | 0.23 | 2.0 | 1.2 |
|  | 0.046 | 0.17 | 0.29 | 0.82 | 0.65 | 0.83 | 0.20 | 0.57 | 0.75 |
| p Value 95% CI of OR Quart3 | 0.19 | 0.063 | 0.27 | 0.38 | 0.25 | 0.47 | 0.025 | 0.18 | 0.35 |
|  | 0.99 | 1.6 | 1.5 | 2.2 | 9.3 | 2.5 | 2.2 | 23 | 4.3 |
| OR Quart 4 | 1.1 | 1.4 | 1.1 | 1.0 | 2.1 | 1.3 | 1.2 | 2.0 | 0.80 |
|  | 0.85 | 0.57 | 0.89 | 0.95 | 0.41 | 0.57 | 0.75 | 0.57 | 0.75 |
| p Value 95% CI of OR Quart4 | 0.51 | 0.46 | 0.48 | 0.43 | 0.37 | 0.56 | 0.31 | 0.18 | 0.20 |
|  | 2.3 | 4.1 | 2.3 | 2.4 | 12 | 2.9 | 5.0 | 22 | 3.2 |

**Fibroblast growth factor 19**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.170 | 0.194 | 0.170 | 0.119 | 0.170 | 0.0550 |
| Average | 0.199 | 0.224 | 0.199 | 0.162 | 0.199 | 0.107 |
| Stdev | 0.167 | 0.236 | 0.167 | 0.209 | 0.167 | 0.129 |
| p(t-test) |  | 0.36 |  | 0.18 |  | 0.040 |
| Min | 8.23E-5 | 0.000127 | 8.23E-5 | 2.92E-5 | 8.23E-5 | 8.23E-5 |
| Max | 0.948 | 1.41 | 0.948 | 1.20 | 0.948 | 0.347 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.145 | 0.203 | 0.145 | 0.136 | 0.145 | 0.103 |
| Average | 0.193 | 0.293 | 0.193 | 0.210 | 0.193 | 0.113 |
| Stdev | 0.190 | 0.307 | 0.190 | 0.196 | 0.190 | 0.0833 |
| p(t-test) |  | 0.027 |  | 0.76 |  | 0.27 |
| Min | 2.92E-5 | 0.000127 | 2.92E-5 | 8.23E-5 | 2.92E-5 | 8.23E-5 |
| Max | 1.41 | 1.32 | 1.41 | 0.619 | 1.41 | 0.254 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Samp) | 379 | 20 | 379 | 11 | 379 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.176 | 0.168 | 0.176 | 0.129 | 0.176 | 0.0787 |
| Average | 0.219 | 0.209 | 0.219 | 0.158 | 0.219 | 0.148 |
| Stdev | 0.216 | 0.233 | 0.216 | 0.193 | 0.216 | 0.160 |
| p(t-test) | | 0.75 | | 0.048 | | 0.17 |
| Min | 8.23E-5 | 0.000127 | 8.23E-5 | 2.92E-5 | 8.23E-5 | 8.23E-5 |
| Max | 2.09 | 1.41 | 2.09 | 1.20 | 2.09 | 0.522 |
| n (Samp) | 183 | 64 | 183 | 62 | 183 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.62 | 0.48 | 0.39 | 0.52 | 0.37 | 0.31 | 0.38 | 0.36 |
| SE | 0.041 | 0.068 | 0.042 | 0.045 | 0.089 | 0.042 | 0.078 | 0.11 | 0.073 |
| p | 0.50 | 0.080 | 0.57 | 0.014 | 0.82 | 0.0016 | 0.014 | 0.29 | 0.062 |
| nCohort 1 | 157 | 379 | 183 | 157 | 379 | 183 | 157 | 379 | 183 |
| nCohort 2 | 73 | 20 | 64 | 56 | 11 | 62 | 15 | 7 | 18 |
| Cutoff 1 | 0.107 | 0.150 | 0.106 | 0.0421 | 0.0860 | 0.0449 | 0.0123 | 0.0885 | 0.0430 |
| Sens 1 | 71% | 70% | 70% | 71% | 73% | 71% | 73% | 71% | 72% |
| Spec 1 | 34% | 51% | 31% | 17% | 33% | 14% | 11% | 33% | 14% |
| Cutoff 2 | 0.0726 | 0.125 | 0.0718 | 0.0375 | 0.0804 | 0.0375 | 8.23E-5 | 0.0423 | 8.23E-5 |
| Sens 2 | 82% | 80% | 81% | 80% | 82% | 81% | 80% | 86% | 83% |
| Spec 2 | 24% | 45% | 20% | 15% | 31% | 12% | 4% | 18% | 2% |
| Cutoff 3 | 0.0209 | 0.0804 | 0.0209 | 0.00457 | 0.0240 | 0.00457 | 0 | 2.92E-5 | 0 |
| Sens 3 | 90% | 90% | 91% | 91% | 91% | 90% | 100% | 100% | 100% |
| Spec 3 | 12% | 31% | 9% | 10% | 12% | 7% | 0% | 1% | 0% |
| Cutoff 4 | 0.255 | 0.238 | 0.267 | 0.255 | 0.238 | 0.267 | 0.255 | 0.238 | 0.267 |
| Sens 4 | 34% | 40% | 25% | 11% | 27% | 11% | 20% | 14% | 28% |
| Spec 4 | 70% | 70% | 71% | 70% | 70% | 71% | 70% | 70% | 71% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 5 | 0.308 | 0.297 | 0.321 | 0.308 | 0.297 | 0.321 | 0.308 | 0.297 | 0.321 |
| Sens 5 | 16% | 30% | 11% | 11% | 27% | 10% | 13% | 0% | 22% |
| Spec 5 | 80% | 80% | 80% | 80% | 80% | 80% | 80% | 80% | 80% |
| Cutoff 6 | 0.442 | 0.401 | 0.450 | 0.442 | 0.401 | 0.450 | 0.442 | 0.401 | 0.450 |
| Sens 6 | 5% | 15% | 3% | 9% | 18% | 6% | 0% | 0% | 6% |
| Spec 6 | 90% | 90% | 90% | 90% | 90% | 90% | 90% | 90% | 90% |
| OR Quart 2 | 1.5 | 5.2 | 1.0 | 2.9 | 2.0 | 2.6 | 0.32 | >2.1 | 0.49 |
|  | 0.34 | 0.14 | 1.0 | 0.048 | 0.42 | 0.060 | 0.33 | <0.56 | 0.42 |
| p Value 95% CI of OR Quart2 | 0.66 | 0.59 | 0.44 | 1.0 | 0.36 | 0.96 | 0.032 | >0.18 | 0.086 |
|  | 3.3 | 45 | 2.3 | 8.2 | 11 | 6.8 | 3.2 | na | 2.8 |
| OR Quart 3 | 1.4 | 6.3 | 1.3 | 3.2 | 1.0 | 3.0 | 0.65 | >3.1 | 0.75 |
|  | 0.41 | 0.092 | 0.54 | 0.030 | 1.0 | 0.024 | 0.65 | <0.33 | 0.72 |
| p Value 95% CI ot OR Quart3 | 0.62 | 0.74 | 0.58 | 1.1 | 0.14 | 1.2 | 0.10 | >0.32 | 0.16 |
|  | 3.1 | 53 | 2.9 | 8.9 | 7.2 | 8.0 | 4.1 | na | 3.5 |
| OR Quart 4 | 1.4 | 8.5 | 1.1 | 5.2 | 1.5 | 4.8 | 3.5 | >2.1 | 2.6 |
|  | 0.45 | 0.045 | 0.79 | 0.0013 | 0.66 | 0.0012 | 0.074 | <0.56 | 0.14 |
| p Value 95% CI of OR Quart4 | 0.61 | 1.0 | 0.49 | 1.9 | 0.25 | 1.9 | 0.88 | >0.18 | 0.74 |
|  | 3.1 | 69 | 2.5 | 14 | 9.2 | 12 | 14 | na | 9.0 |

**Fibroblast growth factor 21**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.0454 | 0.0567 | 0.0454 | 0.0514 | 0.0454 | 0.00987 |
| Average | 0.191 | 0.198 | 0.191 | 0.179 | 0.191 | 0.0861 |
| Stdev | 0.446 | 0.496 | 0.446 | 0.296 | 0.446 | 0.134 |
| p(t-test) |  | 0.91 |  | 0.85 |  | 0.37 |
| Min | 9.65E-6 | 7.01E-5 | 9.65E-6 | 4.60E-6 | 9.65E-6 | 0.00288 |
| Max | 3.21 | 3.36 | 3.21 | 1.29 | 3.21 | 0.438 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0532 | 0.0603 | 0.0532 | 0.105 | 0.0532 | 0.0913 |
| Average | 0.224 | 0.186 | 0.224 | 0.309 | 0.224 | 0.300 |
| Stdev | 0.578 | 0.355 | 0.578 | 0.413 | 0.578 | 0.421 |
| p(t-test) | | 0.77 | | 0.63 | | 0.73 |
| Min | 4.60E-6 | 0.00263 | 4.60E-6 | 0.0202 | 4.60E-6 | 0.00631 |
| Max | 5.72 | 1.39 | 5.72 | 1.25 | 5.72 | 1.15 |
| n (Samp) | 379 | 20 | 379 | 11 | 379 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0535 | 0.0831 | 0.0535 | 0.0496 | 0.0535 | 0.0281 |
| Average | 0.213 | 0.208 | 0.213 | 0.229 | 0.213 | 0.246 |
| Stdev | 0.445 | 0.504 | 0.445 | 0.423 | 0.445 | 0.519 |
| p(t-test) | | 0.94 | | 0.80 | | 0.76 |
| Min | 9.65E-6 | 7.01E-5 | 9.65E-6 | 4.60E-6 | 9.65E-6 | 0.00288 |
| Max | 3.21 | 3.36 | 3.21 | 2.11 | 3.21 | 2.05 |
| n (Samp) | 183 | 64 | 183 | 62 | 183 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.53 | 0.51 | 0.51 | 0.66 | 0.48 | 0.38 | 0.60 | 0.41 |
| SE | 0.041 | 0.067 | 0.042 | 0.045 | 0.091 | 0.043 | 0.080 | 0.11 | 0.073 |
| p | 0.51 | 0.61 | 0.75 | 0.82 | 0.074 | 0.67 | 0.14 | 0.40 | 0.22 |
| nCohort 1 | 157 | 379 | 183 | 157 | 379 | 183 | 157 | 379 | 183 |
| nCohort 2 | 73 | 20 | 64 | 56 | 11 | 62 | 15 | 7 | 18 |
| Cutoff 1 | 0.0172 | 0.0301 | 0.0209 | 0.0138 | 0.0787 | 0.0139 | 0.00459 | 0.0541 | 0.00472 |
| Sens 1 | 71% | 70% | 70% | 71% | 73% | 71% | 73% | 71% | 72% |
| Spec 1 | 24% | 37% | 23% | 22% | 61% | 15% | 10% | 51% | 7% |
| Cutoff 2 | 0.0111 | 0.0164 | 0.0109 | 0.00901 | 0.0288 | 0.00901 | 0.00440 | 0.00953 | 0.00440 |
| Sens 2 | 81% | 80% | 81% | 80% | 82% | 81% | 80% | 86% | 83% |
| Spec 2 | 20% | 28% | 13% | 18% | 37% | 11% | 10% | 21% | 5% |
| Cutoff 3 | 0.00481 | 0.0157 | 0.00481 | 0.00323 | 0.0241 | 0.00323 | 0.00301 | 0.00624 | 0.00288 |
| Sens 3 | 90% | 90% | 91% | 91% | 91% | 90% | 93% | 100% | 94% |
| Spec 3 | 12% | 27% | 7% | 8% | 34% | 3% | 8% | 15% | 3% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 0.0859 | 0.121 | 0.120 | 0.0859 | 0.121 | 0.120 | 0.0859 | 0.121 | 0.120 |
| Sens 4 | 44% | 30% | 38% | 43% | 45% | 37% | 27% | 43% | 33% |
| Spec 4 | 70% | 70% | 70% | 70% | 70% | 70% | 70% | 70% | 70% |
| Cutoff 5 | 0.133 | 0.224 | 0.182 | 0.133 | 0.224 | 0.182 | 0.133 | 0.224 | 0.182 |
| Sens 5 | 29% | 10% | 31% | 34% | 27% | 31% | 27% | 29% | 28% |
| Spec 5 | 80% | 80% | 80% | 80% | 80% | 80% | 80% | 80% | 80% |
| Cutoff 6 | 0.546 | 0.500 | 0.584 | 0.546 | 0.500 | 0.584 | 0.546 | 0.500 | 0.584 |
| Sens 6 | 5% | 10% | 5% | 9% | 27% | 8% | 0% | 29% | 11% |
| Spec 6 | 90% | 90% | 90% | 90% | 90% | 90% | 90% | 90% | 90% |
| OR Quart 2 | 0.53 | 4.2 | 0.46 | 0.45 | >3.1 | 0.34 | 0.23 | 0 | 0.15 |
| | 0.14 | 0.073 | 0.080 | 0.081 | <0.34 | 0.016 | 0.20 | na | 0.088 |
| p Value | 0.23 | 0.87 | 0.19 | 0.19 | >0.31 | 0.14 | 0.025 | na | 0.018 |
| 95% CI of OR Quart2 | 1.2 | 20 | 1.1 | 1.1 | na | 0.82 | 2.2 | na | 1.3 |
| OR Quart 3 | 0.79 | 2.6 | 0.76 | 0.35 | >4.2 | 0.43 | 0.48 | 1.0 | 0.48 |
| | 0.55 | 0.27 | 0.51 | 0.027 | <0.21 | 0.048 | 0.41 | 1.0 | 0.32 |
| p Value | 0.36 | 0.48 | 0.34 | 0.13 | >0.46 | 0.19 | 0.082 | 0.14 | 0.11 |
| 95% CI of OR Quart3 | 1.7 | 13 | 1.7 | 0.89 | na | 0.99 | 2.7 | 7.2 | 2.0 |
| OR Quart 4 | 1.2 | 2.6 | 1.2 | 1.1 | >4.1 | 1.0 | 2.2 | 1.5 | 1.4 |
| | 0.61 | 0.27 | 0.60 | 0.74 | <0.21 | 0.95 | 0.22 | 0.66 | 0.54 |
| p Value | 0.57 | 0.48 | 0.57 | 0.52 | >0.45 | 0.49 | 0.62 | 0.25 | 0.46 |
| 95% CI of OR Quart4 | 2.6 | 13 | 2.6 | 2.5 | na | 2.2 | 8.0 | 9.2 | 4.5 |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14.8 | 15.5 | 14.8 | 18.1 | 14.8 | 19.0 |
| Average | 17.6 | 20.5 | 17.6 | 26.0 | 17.6 | 22.0 |
| Stdev | 11.1 | 16.7 | 11.1 | 23.4 | 11.1 | 16.8 |
| p(t-test) | | 0.12 | | 5.2E-4 | | 0.17 |
| Min | 5.52 | 7.53 | 5.52 | 5.96 | 5.52 | 5.20 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 98.9 | 109 | 98.9 | 113 | 98.9 | 69.4 |
| n (Samp) | 156 | 70 | 156 | 54 | 156 | 14 |
| n (Patient) | 87 | 70 | 87 | 54 | 87 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 15.6 | 18.1 | 15.6 | 18.3 | 15.6 | 25.9 |
| Average | 18.6 | 24.9 | 18.6 | 23.3 | 18.6 | 33.9 |
| Stdev | 12.4 | 24.3 | 12.4 | 14.1 | 12.4 | 19.4 |
| p(t-test) | | 0.042 | | 0.24 | | 0.0016 |
| Min | 5.20 | 9.73 | 5.20 | 8.57 | 5.20 | 9.67 |
| Max | 113 | 109 | 113 | 50.8 | 113 | 61.5 |
| n (Samp) | 371 | 19 | 371 | 10 | 371 | 7 |
| n (Patient) | 173 | 19 | 173 | 10 | 173 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 15.5 | 15.5 | 15.5 | 18.1 | 15.5 | 21.1 |
| Average | 20.2 | 19.5 | 20.2 | 26.9 | 20.2 | 21.5 |
| Stdev | 19.3 | 13.6 | 19.3 | 24.7 | 19.3 | 14.6 |
| p(t-test) | | 0.81 | | 0.031 | | 0.79 |
| Min | 5.52 | 7.53 | 5.52 | 5.96 | 5.52 | 5.20 |
| Max | 186 | 73.8 | 186 | 113 | 186 | 69.4 |
| n (Samp) | 180 | 62 | 180 | 60 | 180 | 17 |
| n (Patient) | 88 | 62 | 88 | 60 | 88 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | 0.60 | 0.52 | 0.62 | 0.60 | 0.61 | 0.57 | 0.78 | 0.58 |
| SE | 0.042 | 0.070 | 0.043 | 0.046 | 0.096 | 0.043 | 0.083 | 0.10 | 0.075 |
| p | 0.14 | 0.14 | 0.60 | 0.0082 | 0.29 | 0.014 | 0.41 | 0.0073 | 0.28 |
| nCohort 1 | 156 | 371 | 180 | 156 | 371 | 180 | 156 | 371 | 180 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 2 | 70 | 19 | 62 | 54 | 10 | 60 | 14 | 7 | 17 |
| Cutoff 1 | 13.6 | 14.7 | 13.2 | 15.8 | 17.4 | 15.9 | 11.5 | 22.5 | 17.1 |
| Sens 1 | 70% | 74% | 71% | 70% | 70% | 70% | 71% | 71% | 71% |
| Spec 1 | 45% | 45% | 39% | 54% | 61% | 51% | 26% | 80% | 57% |
| Cutoff 2 | 12.8 | 13.8 | 12.4 | 12.6 | 12.1 | 13.7 | 8.14 | 21.3 | 11.2 |
| Sens 2 | 80% | 84% | 81% | 81% | 80% | 80% | 86% | 86% | 82% |
| Spec 2 | 38% | 39% | 33% | 38% | 26% | 42% | 6% | 78% | 21% |
| Cutoff 3 | 10.4 | 11.5 | 10.3 | 10.2 | 8.69 | 10.4 | 5.52 | 9.66 | 5.52 |
| Sens 3 | 90% | 95% | 90% | 91% | 90% | 90% | 93% | 100% | 94% |
| Spec 3 | 18% | 22% | 16% | 17% | 7% | 17% | 1% | 10% | 1% |
| Cutoff 4 | 19.4 | 19.5 | 20.2 | 19.4 | 19.5 | 20.2 | 19.4 | 19.5 | 20.2 |
| Sens 4 | 27% | 37% | 26% | 37% | 40% | 33% | 50% | 86% | 59% |
| Spec 4 | 71% | 70% | 70% | 71% | 70% | 70% | 71% | 70% | 70% |
| Cutoff 5 | 21.3 | 22.5 | 22.8 | 21.3 | 22.5 | 22.8 | 21.3 | 22.5 | 22.8 |
| Sens 5 | 21% | 16% | 18% | 31% | 40% | 27% | 36% | 71% | 41% |
| Spec 5 | 80% | 80% | 80% | 80% | 80% | 80% | 80% | 80% | 80% |
| Cutoff 6 | 26.6 | 29.0 | 31.2 | 26.6 | 29.0 | 31.2 | 26.6 | 29.0 | 31.2 |
| Sens 6 | 13% | 11% | 6% | 24% | 30% | 23% | 21% | 43% | 12% |
| Spec 6 | 90% | 90% | 90% | 90% | 90% | 90% | 90% | 90% | 90% |
| OR Quart 2 | 3.1 | 2.6 | 2.2 | 1.4 | 0.49 | 2.0 | 0.23 | 0 | 0 |
| | 0.010 | 0.27 | 0.072 | 0.48 | 0.57 | 0.16 | 0.19 | na | na |
| p Value 95% CI of OR Quart2 | 1.3 | 0.48 | 0.93 | 0.53 | 0.044 | 0.76 | 0.024 | na | na |
| | 7.4 | 13 | 5.1 | 3.9 | 5.5 | 5.1 | 2.1 | na | na |
| OR Quart 3 | 2.4 | 3.1 | 1.8 | 2.9 | 1.5 | 3.2 | 0.73 | 0 | 1.0 |
| | 0.055 | 0.17 | 0.20 | 0.027 | 0.65 | 0.012 | 0.69 | na | 1.0 |
| p Value 95% CI of OR Quart3 | 0.98 | 0.62 | 0.74 | 1.1 | 0.25 | 1.3 | 0.15 | na | 0.27 |
| | 5.7 | 16 | 4.2 | 7.5 | 9.3 | 8.1 | 3.5 | na | 3.7 |
| OR Quart 4 p Value 95% CI of OR Quart4 | 2.1 | 3.1 | 1.3 | 2.6 | 2.0 | 2.8 | 1.5 | 6.3 | 1.4 |
| | 0.095 | 0.17 | 0.53 | 0.049 | 0.42 | 0.030 | 0.53 | 0.092 | 0.56 |
| | 0.88 | 0.61 | 0.55 | 1.0 | 0.36 | 1.1 | 0.40 | 0.74 | 0.42 |
| | 5.1 | 16 | 3.2 | 6.7 | 11 | 7.0 | 5.9 | 53 | 4.9 |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 477 | 610 | 477 | 714 | 477 | 860 |
| Average | 830 | 1060 | 830 | 1140 | 830 | 938 |
| Stdev | 1330 | 1520 | 1330 | 1500 | 1330 | 817 |
| p(t-test) | | 0.24 | | 0.15 | | 0.76 |
| Min | 94.2 | 86.2 | 94.2 | 160 | 94.2 | 143 |
| Max | 13100 | 9040 | 13100 | 9500 | 13100 | 3300 |
| n (Samp) | 158 | 73 | 158 | 55 | 158 | 15 |
| n (Patient) | 88 | 73 | 88 | 55 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 564 | 756 | 564 | 1140 | 564 | 642 |
| Average | 1010 | 1070 | 1010 | 1470 | 1010 | 664 |
| Stdev | 1680 | 983 | 1680 | 1470 | 1680 | 226 |
| p(t-test) | | 0.89 | | 0.37 | | 0.58 |
| Min | 43.9 | 205 | 43.9 | 180 | 43.9 | 385 |
| Max | 18400 | 4250 | 18400 | 5760 | 18400 | 1030 |
| n (Samp) | 378 | 20 | 378 | 11 | 378 | 7 |
| n (Patient) | 175 | 20 | 175 | 11 | 175 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 527 | 668 | 527 | 684 | 527 | 852 |
| Average | 1330 | 1100 | 1330 | 1050 | 1330 | 942 |
| Stdev | 2900 | 1570 | 2900 | 1320 | 2900 | 738 |
| p(t-test) | | 0.55 | | 0.46 | | 0.57 |
| Min | 63.0 | 86.2 | 63.0 | 160 | 63.0 | 143 |
| Max | 26100 | 9040 | 26100 | 9500 | 26100 | 3300 |
| n (Samp) | 184 | 64 | 184 | 61 | 184 | 18 |
| n (Patient) | 89 | 64 | 89 | 61 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.59 | 0.56 | 0.60 | 0.73 | 0.55 | 0.61 | 0.55 | 0.59 |
| SE | 0.041 | 0.068 | 0.042 | 0.046 | 0.088 | 0.043 | 0.080 | 0.11 | 0.073 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p | 0.019 | 0.18 | 0.18 | 0.027 | 0.0098 | 0.22 | 0.15 | 0.67 | 0.21 |
| nCohort 1 | 158 | 378 | 184 | 158 | 378 | 184 | 158 | 378 | 184 |
| nCohort 2 | 73 | 20 | 64 | 55 | 11 | 61 | 15 | 7 | 18 |
| Cutoff 1 | 417 | 486 | 434 | 357 | 1030 | 394 | 411 | 624 | 565 |
| Sens 1 | 71% | 70% | 70% | 71% | 73% | 70% | 73% | 71% | 72% |
| Spec 1 | 41% | 44% | 40% | 37% | 76% | 37% | 41% | 56% | 55% |
| Cutoff 2 | 371 | 450 | 379 | 256 | 901 | 265 | 379 | 417 | 411 |
| Sens 2 | 81% | 80% | 81% | 80% | 82% | 80% | 80% | 86% | 83% |
| Spec 2 | 40% | 39% | 36% | 18% | 70% | 20% | 41% | 35% | 38% |
| Cutoff 3 | 221 | 257 | 221 | 228 | 720 | 240 | 162 | 382 | 162 |
| Sens 3 | 90% | 90% | 91% | 91% | 91% | 90% | 93% | 100% | 94% |
| Spec 3 | 15% | 16% | 15% | 16% | 62% | 16% | 7% | 32% | 7% |
| Cutoff 4 | 678 | 901 | 886 | 678 | 901 | 886 | 678 | 901 | 886 |
| Sens 4 | 47% | 40% | 34% | 55% | 82% | 43% | 60% | 14% | 39% |
| Spec 4 | 70% | 70% | 70% | 70% | 70% | 70% | 70% | 70% | 70% |
| Cutoff 5 | 895 | 1180 | 1200 | 895 | 1180 | 1200 | 895 | 1180 | 1200 |
| Sens 5 | 34% | 35% | 23% | 45% | 45% | 31% | 40% | 0% | 17% |
| Spec 5 | 80% | 80% | 80% | 80% | 80% | 80% | 80% | 80% | 80% |
| Cutoff 6 | 1840 | 2000 | 2700 | 1840 | 2000 | 2700 | 1840 | 2000 | 2700 |
| Sens 6 | 10% | 10% | 8% | 13% | 9% | 5% | 13% | 0% | 6% |
| Spec 6 | 91% | 90% | 90% | 91% | 90% | 90% | 91% | 90% | 90% |
| OR Quart 2 | 1.4 | 1.3 | 1.5 | 0.50 | 0 | 0.82 | 0.65 | >2.0 | 0.64 |
|  | 0.43 | 0.71 | 0.38 | 0.16 | na | 0.66 | 0.65 | <0.56 | 0.63 |
| p Value 95% Clot OR Quart2 | 0.61 | 0.29 | 0.62 | 0.19 | na | 0.34 | 0.10 | >0.18 | 0.10 |
|  | 3.2 | 6.1 | 3.5 | 1.3 | na | 2.0 | 4.1 | na | 4.0 |
| OR Quart 3 | 1.5 | 2.1 | 2.2 | 0.65 | 2.0 | 1.0 | 1.0 | >4.2 | 3.0 |
|  | 0.32 | 0.32 | 0.065 | 0.36 | 0.57 | 1.0 | 1.0 | <0.21 | 0.12 |
| p Value 95% CI of OR Quart3 | 0.66 | 0.50 | 0.95 | 0.26 | 0.18 | 0.43 | 0.19 | >0.46 | 0.74 |
|  | 3.5 | 8.5 | 5.1 | 1.6 | 23 | 2.3 | 5.3 | na | 12 |
| OR Quart 4 | 2.6 | 2.4 | 1.9 | 2.1 | 8.5 | 1.7 | 2.5 | >1.0 | 1.7 |
|  | 0.022 | 0.21 | 0.14 | 0.081 | 0.045 | 0.18 | 0.20 | <1.0 | 0.48 |
| p Value | 1.1 | 0.60 | 0.81 | 0.92 | 1.0 | 0.78 | 0.61 | >0.062 | 0.38 |
| 95% CI of OR Quart4 | 5.8 | 9.6 | 4.4 | 4.7 | 70 | 3.8 | 10 | na | 7.5 |

Fig. 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.6 | 39.3 | 31.6 | 42.7 | 31.6 | 47.1 |
| Average | 48.9 | 60.6 | 48.9 | 69.8 | 48.9 | 58.5 |
| Stdev | 111 | 63.3 | 111 | 69.1 | 111 | 48.0 |
| p(t-test) | | 0.58 | | 0.28 | | 0.69 |
| Min | 1.77 | 9.35 | 1.77 | 12.8 | 1.77 | 8.79 |
| Max | 1900 | 243 | 1900 | 339 | 1900 | 179 |
| n (Samp) | 356 | 28 | 356 | 34 | 356 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 32.7 | 39.3 | 32.7 | 44.3 | 32.7 | 49.6 |
| Average | 50.3 | 60.2 | 50.3 | 69.5 | 50.3 | 61.2 |
| Stdev | 112 | 63.1 | 112 | 68.2 | 112 | 49.3 |
| p(t-test) | | 0.64 | | 0.32 | | 0.67 |
| Min | 2.68 | 9.35 | 2.68 | 12.8 | 2.68 | 8.79 |
| Max | 1900 | 243 | 1900 | 339 | 1900 | 179 |
| n (Samp) | 347 | 28 | 347 | 35 | 347 | 20 |
| n (Patient) | 161 | 28 | 161 | 35 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | nd | 0.58 | 0.65 | nd | 0.64 | 0.63 | nd | 0.63 |
| SE | 0.058 | nd | 0.058 | 0.053 | nd | 0.052 | 0.065 | nd | 0.069 |
| p | 0.11 | nd | 0.18 | 0.0043 | nd | 0.0068 | 0.050 | nd | 0.054 |
| nCohort 1 | 356 | nd | 347 | 356 | nd | 347 | 356 | nd | 347 |
| nCohort 2 | 28 | nd | 28 | 34 | nd | 35 | 22 | nd | 20 |
| Cutoff 1 | 29.1 | nd | 29.1 | 30.3 | nd | 30.3 | 25.8 | nd | 28.9 |
| Sens 1 | 71% | nd | 71% | 71% | nd | 71% | 73% | nd | 70% |
| Spec 1 | 47% | nd | 45% | 48% | nd | 46% | 42% | nd | 44% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 19.0 | nd | 19.0 | 25.0 | nd | 27.0 | 22.1 | nd | 24.9 |
| Sens 2 | 82% | nd | 82% | 82% | nd | 80% | 82% | nd | 80% |
| Spec 2 | 30% | nd | 27% | 41% | nd | 41% | 37% | nd | 38% |
| Cutoff 3 | 13.6 | nd | 13.6 | 22.1 | nd | 22.1 | 15.3 | nd | 17.6 |
| Sens 3 | 93% | nd | 93% | 91% | nd | 91% | 91% | nd | 90% |
| Spec 3 | 19% | nd | 16% | 37% | nd | 34% | 22% | nd | 24% |
| Cutoff 4 | 44.8 | nd | 45.5 | 44.8 | nd | 45.5 | 44.8 | nd | 45.5 |
| Sens 4 | 36% | nd | 36% | 47% | nd | 46% | 64% | nd | 65% |
| Spec 4 | 70% | nd | 70% | 70% | nd | 70% | 70% | nd | 70% |
| Cutoff 5 | 61.9 | nd | 62.7 | 61.9 | nd | 62.7 | 61.9 | nd | 62.7 |
| Sens 5 | 25% | nd | 25% | 32% | nd | 31% | 23% | nd | 25% |
| Spec 5 | 80% | nd | 80% | 80% | nd | 80% | 80% | nd | 80% |
| Cutoff 6 | 87.8 | nd | 88.3 | 87.8 | nd | 88.3 | 87.8 | nd | 88.3 |
| Sens 6 | 21% | nd | 18% | 24% | nd | 23% | 18% | nd | 20% |
| Spec 6 | 90% | nd | 90% | 90% | nd | 90% | 90% | nd | 90% |
| OR Quart 2 | 1.2 | nd | 1.4 | 5.4 | nd | 6.6 | 1.7 | nd | 1.3 |
| | 0.76 | nd | 0.57 | 0.033 | nd | 0.015 | 0.48 | nd | 0.71 |
| p Value 95% CI of OR Quart2 | 0.36 | nd | 0.43 | 1.2 | nd | 1.4 | 0.39 | nd | 0.29 |
| | 4.1 | nd | 4.6 | 25 | nd | 31 | 7.3 | nd | 6.1 |
| OR Quart 3 | 1.7 | nd | 1.6 | 5.5 | nd | 4.3 | 1.7 | nd | 1.7 |
| | 0.39 | nd | 0.40 | 0.031 | nd | 0.071 | 0.47 | nd | 0.48 |
| p Value 95% CI of OR Quart3 | 0.52 | nd | 0.52 | 1.2 | nd | 0.88 | 0.40 | nd | 0.39 |
| | 5.3 | nd | 5.2 | 26 | nd | 21 | 7.3 | nd | 7.3 |
| OR Quart 4 | 1.9 | nd | 1.6 | 6.6 | nd | 7.3 | 3.2 | nd | 2.8 |
| | 0.27 | nd | 0.40 | 0.015 | nd | 0.010 | 0.091 | nd | 0.14 |
| p Value 95% CI of OR Quart4 | 0.61 | nd | 0.52 | 1.4 | nd | 1.6 | 0.83 | nd | 0.72 |
| | 5.8 | nd | 5.2 | 30 | nd | 33 | 12 | nd | 11 |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22.0 | 26.7 | 22.0 | 26.2 | 22.0 | 23.7 |
| Average | 27.4 | 29.5 | 27.4 | 33.1 | 27.4 | 41.5 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 21.5 | 20.0 | 21.5 | 24.5 | 21.5 | 41.2 |
| p(t-test) | | 0.61 | | 0.16 | | 0.0056 |
| Min | 0.00246 | 0.00246 | 0.00246 | 4.44 | 0.00246 | 2.76 |
| Max | 198 | 93.0 | 198 | 133 | 198 | 162 |
| n (Samp) | 349 | 28 | 349 | 32 | 349 | 22 |
| n (Patient) | 172 | 28 | 172 | 32 | 172 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.7 | 26.7 | 21.7 | 25.1 | 21.7 | 23.7 |
| Average | 27.1 | 29.8 | 27.1 | 32.8 | 27.1 | 40.0 |
| Stdev | 21.5 | 20.0 | 21.5 | 24.2 | 21.5 | 40.9 |
| p(t-test) | | 0.53 | | 0.16 | | 0.016 |
| Min | 0.00246 | 0.00246 | 0.00246 | 4.44 | 0.00246 | 2.76 |
| Max | 198 | 93.0 | 198 | 133 | 198 | 162 |
| n (Samp) | 340 | 28 | 340 | 33 | 340 | 20 |
| n (Patient) | 158 | 28 | 158 | 33 | 158 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | nd | 0.56 | 0.58 | nd | 0.58 | 0.55 | nd | 0.55 |
| SE | 0.058 | nd | 0.058 | 0.055 | nd | 0.054 | 0.065 | nd | 0.068 |
| p | 0.38 | nd | 0.31 | 0.13 | nd | 0.12 | 0.40 | nd | 0.49 |
| nCohort 1 | 349 | nd | 340 | 349 | nd | 340 | 349 | nd | 340 |
| nCohort 2 | 28 | nd | 28 | 32 | nd | 33 | 22 | nd | 20 |
| Cutoff 1 | 17.0 | nd | 17.0 | 18.8 | nd | 18.8 | 14.3 | nd | 14.3 |
| Sens 1 | 71% | nd | 71% | 72% | nd | 73% | 73% | nd | 70% |
| Spec 1 | 36% | nd | 37% | 41% | nd | 42% | 27% | nd | 27% |
| Cutoff 2 | 12.3 | nd | 12.3 | 17.0 | nd | 17.0 | 11.7 | nd | 11.7 |
| Sens 2 | 82% | nd | 82% | 81% | nd | 82% | 82% | nd | 80% |
| Spec 2 | 18% | nd | 18% | 36% | nd | 37% | 17% | nd | 17% |
| Cutoff 3 | 8.57 | nd | 8.57 | 12.3 | nd | 12.3 | 11.0 | nd | 11.0 |
| Sens 3 | 93% | nd | 93% | 91% | nd | 91% | 91% | nd | 90% |
| Spec 3 | 10% | nd | 10% | 18% | nd | 18% | 15% | nd | 15% |
| Cutoff 4 | 30.9 | nd | 30.4 | 30.9 | nd | 30.4 | 30.9 | nd | 30.4 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 39% | nd | 43% | 47% | nd | 45% | 36% | nd | 35% |
| Spec 4 | 71% | nd | 70% | 71% | nd | 70% | 71% | nd | 70% |
| Cutoff 5 | 39.0 | nd | 37.0 | 39.0 | nd | 37.0 | 39.0 | nd | 37.0 |
| Sens 5 | 25% | nd | 29% | 25% | nd | 36% | 36% | nd | 35% |
| Spec 5 | 81% | nd | 80% | 81% | nd | 80% | 81% | nd | 80% |
| Cutoff 6 | 52.6 | nd | 50.7 | 52.6 | nd | 50.7 | 52.6 | nd | 50.7 |
| Sens 6 | 11% | nd | 11% | 16% | nd | 18% | 27% | nd | 25% |
| Spec 6 | 90% | nd | 90% | 90% | nd | 90% | 90% | nd | 90% |
| OR Quart 2 | 0.55 | nd | 0.55 | 1.9 | nd | 2.1 | 0.99 | nd | 0.79 |
| | 0.36 | nd | 0.36 | 0.27 | nd | 0.19 | 0.99 | nd | 0.73 |
| p Value | 0.16 | nd | 0.16 | 0.61 | nd | 0.70 | 0.28 | nd | 0.21 |
| 95% CI of OR Quart2 | 2.0 | nd | 2.0 | 5.8 | nd | 6.5 | 3.5 | nd | 3.0 |
| OR Quart 3 | 1.2 | nd | 1.2 | 1.2 | nd | 1.0 | 0.78 | nd | 0.79 |
| | 0.79 | nd | 0.79 | 0.76 | nd | 1.0 | 0.72 | nd | 0.73 |
| p Value 95% CI of OR Quart3 | 0.40 | nd | 0.40 | 0.36 | nd | 0.28 | 0.20 | nd | 0.21 |
| | 3.3 | nd | 3.3 | 4.1 | nd | 3.6 | 3.0 | nd | 3.0 |
| OR Quart 4 | 1.3 | nd | 1.3 | 2.6 | nd | 2.8 | 1.6 | nd | 1.4 |
| | 0.62 | nd | 0.60 | 0.088 | nd | 0.058 | 0.40 | nd | 0.55 |
| p Value 95% CI of OR Quart4 | 0.46 | nd | 0.47 | 0.87 | nd | 0.96 | 0.52 | nd | 0.44 |
| | 3.6 | nd | 3.7 | 7.6 | nd | 8.3 | 5.2 | nd | 4.7 |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.86 | 3.89 | 3.86 | 4.40 | 3.86 | 4.54 |
| Average | 4.76 | 4.02 | 4.76 | 6.09 | 4.76 | 6.45 |
| Stdev | 5.85 | 2.57 | 5.85 | 7.48 | 5.85 | 6.05 |
| p(t-test) | | 0.50 | | 0.22 | | 0.19 |
| Min | 0.00226 | 0.00282 | 0.00226 | 0.00289 | 0.00226 | 0.00274 |
| Max | 60.7 | 10.0 | 60.7 | 43.4 | 60.7 | 24.5 |
| n (Samp) | 349 | 28 | 349 | 33 | 349 | 22 |
| n (Patient) | 173 | 28 | 173 | 33 | 173 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.73 | 3.63 | 3.73 | 4.36 | 3.73 | 4.54 |
| Average | 4.62 | 3.87 | 4.62 | 6.00 | 4.62 | 6.21 |
| Stdev | 5.86 | 2.68 | 5.86 | 7.39 | 5.86 | 5.63 |
| p(t-test) | | 0.50 | | 0.20 | | 0.24 |
| Min | 0.00226 | 0.00282 | 0.00226 | 0.00289 | 0.00226 | 0.700 |
| Max | 60.7 | 10.0 | 60.7 | 43.4 | 60.7 | 24.5 |
| n (Samp) | 340 | 28 | 340 | 34 | 340 | 20 |
| n (Patient) | 159 | 28 | 159 | 34 | 159 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | nd | 0.49 | 0.57 | nd | 0.57 | 0.58 | nd | 0.60 |
| SE | 0.057 | nd | 0.057 | 0.054 | nd | 0.053 | 0.065 | nd | 0.069 |
| p | 0.97 | nd | 0.90 | 0.23 | nd | 0.18 | 0.24 | nd | 0.16 |
| nCohort 1 | 349 | nd | 340 | 349 | nd | 340 | 349 | nd | 340 |
| nCohort 2 | 28 | nd | 28 | 33 | nd | 34 | 22 | nd | 20 |
| Cutoff 1 | 3.11 | nd | 2.65 | 3.09 | nd | 3.09 | 2.89 | nd | 3.54 |
| Sens 1 | 71% | nd | 71% | 73% | nd | 71% | 73% | nd | 70% |
| Spec 1 | 38% | nd | 35% | 38% | nd | 39% | 35% | nd | 46% |
| Cutoff 2 | 1.34 | nd | 0.549 | 2.62 | nd | 2.62 | 2.26 | nd | 2.65 |
| Sens 2 | 82% | nd | 82% | 82% | nd | 82% | 82% | nd | 80% |
| Spec 2 | 16% | nd | 14% | 32% | nd | 34% | 26% | nd | 35% |
| Cutoff 3 | 0.00282 | nd | 0.00282 | 1.78 | nd | 1.78 | 0.743 | nd | 1.92 |
| Sens 3 | 96% | nd | 96% | 91% | nd | 91% | 91% | nd | 90% |
| Spec 3 | 5% | nd | 6% | 23% | nd | 25% | 13% | nd | 26% |
| Cutoff 4 | 5.42 | nd | 5.30 | 5.42 | nd | 5.30 | 5.42 | nd | 5.30 |
| Sens 4 | 25% | nd | 32% | 33% | nd | 32% | 36% | nd | 35% |
| Spec 4 | 70% | nd | 71% | 70% | nd | 71% | 70% | nd | 71% |
| Cutoff 5 | 6.65 | nd | 6.31 | 6.65 | nd | 6.31 | 6.65 | nd | 6.31 |
| Sens 5 | 14% | nd | 18% | 24% | nd | 26% | 32% | nd | 30% |
| Spec 5 | 80% | nd | 80% | 80% | nd | 80% | 80% | nd | 80% |
| Cutoff 6 | 8.60 | nd | 8.27 | 8.60 | nd | 8.27 | 8.60 | nd | 8.27 |
| Sens 6 | 4% | nd | 4% | 15% | nd | 18% | 23% | nd | 20% |
| Spec 6 | 90% | nd | 90% | 90% | nd | 90% | 90% | nd | 90% |
| OR Quart 2 | 1.4 | nd | 1.2 | 1.6 | nd | 1.6 | 1.2 | nd | 2.6 |
| | 0.57 | nd | 0.77 | 0.40 | nd | 0.40 | 0.75 | nd | 0.26 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value 95% CI of OR Quart2 | 0.46 | nd | 0.38 | 0.52 | nd | 0.52 | 0.32 | nd | 0.49 |
| | 4.1 | nd | 3.7 | 5.2 | nd | 5.2 | 4.8 | nd | 14 |
| OR Quart 3 | 1.4 | nd | 1.4 | 2.1 | nd | 2.4 | 1.5 | nd | 3.7 |
| | 0.57 | nd | 0.58 | 0.19 | nd | 0.13 | 0.53 | nd | 0.11 |
| p Value 95% CI of OR Quart3 | 0.46 | nd | 0.45 | 0.70 | nd | 0.79 | 0.41 | nd | 0.75 |
| | 4.1 | nd | 4.1 | 6.4 | nd | 7.1 | 5.6 | nd | 18 |
| OR Quart 4 | 1.0 | nd | 1.2 | 2.1 | nd | 2.1 | 1.8 | nd | 3.1 |
| | 0.98 | nd | 0.77 | 0.19 | nd | 0.19 | 0.37 | nd | 0.17 |
| p Value 95% CI of OR Quart4 | 0.31 | nd | 0.38 | 0.69 | nd | 0.69 | 0.51 | nd | 0.62 |
| | 3.3 | nd | 3.7 | 6.4 | nd | 6.4 | 6.3 | nd | 16 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 39200 | 48000 | 39200 | 33300 | 39200 | 7800 |
| Average | 49200 | 62300 | 49200 | 53400 | 49200 | 46300 |
| Stdev | 41200 | 42600 | 41200 | 51700 | 41200 | 25400 |
| p(t-test) | | 0.11 | | 0.59 | | 0.74 |
| Min | 3600 | 13300 | 3600 | 10400 | 3600 | 12800 |
| Max | 483000 | 180000 | 483000 | 239000 | 483000 | 104000 |
| n (Samp) | 357 | 28 | 357 | 34 | 357 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 40400 | 48000 | 40400 | 34000 | 40400 | 41700 |
| Average | 49800 | 59800 | 49800 | 55800 | 49800 | 44900 |
| Stdev | 41400 | 38100 | 41400 | 52800 | 41400 | 26200 |
| p(t-test) | | 0.22 | | 0.43 | | 0.60 |
| Min | 3600 | 13300 | 3600 | 10400 | 3600 | 12800 |
| Max | 483000 | 180000 | 483000 | 239000 | 483000 | 104000 |
| n (Samp) | 348 | 28 | 348 | 35 | 348 | 20 |
| n (Patient) | 161 | 28 | 161 | 35 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.59 | 0.47 | nd | 0.48 | 0.52 | nd | 0.48 |
| SE | 0.058 | nd | 0.058 | 0.053 | nd | 0.052 | 0.064 | nd | 0.067 |
| p | 0.094 | nd | 0.13 | 0.62 | nd | 0.74 | 0.81 | nd | 0.81 |
| nCohort 1 | 357 | nd | 348 | 357 | nd | 348 | 357 | nd | 348 |
| nCohort 2 | 28 | nd | 28 | 34 | nd | 35 | 22 | nd | 20 |
| Cutoff 1 | 33300 | nd | 33300 | 25600 | nd | 25600 | 31800 | nd | 31800 |
| Sens 1 | 71% | nd | 71% | 71% | nd | 71% | 73% | nd | 70% |
| Spec 1 | 39% | nd | 38% | 22% | nd | 22% | 34% | nd | 33% |
| Cutoff 2 | 30600 | nd | 30600 | 18900 | nd | 21000 | 18300 | nd | 18300 |
| Sens 2 | 82% | nd | 82% | 85% | nd | 80% | 82% | nd | 80% |
| Spec 2 | 32% | nd | 31% | 12% | nd | 14% | 11% | nd | 10% |
| Cutoff 3 | 19600 | nd | 19600 | 13600 | nd | 13600 | 14200 | nd | 14200 |
| Sens 3 | 93% | nd | 93% | 91% | nd | 91% | 91% | nd | 90% |
| Spec 3 | 13% | nd | 12% | 6% | nd | 5% | 6% | nd | 5% |
| Cutoff 4 | 53100 | nd | 53500 | 53100 | nd | 53500 | 53100 | nd | 53500 |
| Sens 4 | 46% | nd | 46% | 32% | nd | 34% | 41% | nd | 35% |
| Spec 4 | 70% | nd | 70% | 70% | nd | 70% | 70% | nd | 70% |
| Cutoff 5 | 65300 | nd | 65900 | 65300 | nd | 65900 | 65300 | nd | 65900 |
| Sens 5 | 39% | nd | 39% | 29% | nd | 29% | 27% | nd | 25% |
| Spec 5 | 80% | nd | 80% | 80% | nd | 80% | 80% | nd | 80% |
| Cutoff 6 | 88800 | nd | 88800 | 88800 | nd | 88800 | 88800 | nd | 88800 |
| Sens 6 | 21% | nd | 21% | 12% | nd | 14% | 5% | nd | 5% |
| Spec 6 | 90% | nd | 90% | 90% | nd | 90% | 90% | nd | 90% |
| OR Quart 2 | 1.4 | nd | 1.7 | 0.34 | nd | 0.30 | 0.64 | nd | 0.65 |
| | 0.55 | nd | 0.39 | 0.071 | nd | 0.046 | 0.51 | nd | 0.52 |
| p Value 95% CI OR Quart2 | 0.44 | nd | 0.52 | 0.10 | nd | 0.094 | 0.18 | nd | 0.18 |
| | 4.7 | nd | 5.3 | 1.1 | nd | 0.98 | 2.4 | nd | 2.4 |
| OR Quart 3 | 0.59 | nd | 0.39 | 0.61 | nd | 0.55 | 0.81 | nd | 0.65 |
| | 0.47 | nd | 0.26 | 0.33 | nd | 0.23 | 0.74 | nd | 0.52 |
| p Value 95% CI of OR Quart3 | 0.14 | nd | 0.073 | 0.23 | nd | 0.21 | 0.24 | nd | 0.18 |
| | 2.5 | nd | 2.0 | 1.6 | nd | 1.5 | 2.8 | nd | 2.4 |
| OR Quart 4 | 2.8 | nd | 2.9 | 1.1 | nd | 1.0 | 1.2 | nd | 1.0 |
| | 0.059 | nd | 0.056 | 0.80 | nd | 0.98 | 0.79 | nd | 1.0 |
| p Value 95% CI of OR Quart4 | 0.96 | nd | 0.98 | 0.47 | nd | 0.43 | 0.38 | nd | 0.31 |
| | 8.2 | nd | 8.4 | 2.7 | nd | 2.4 | 3.6 | nd | 3.2 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.383 | 0.437 | 0.383 | 0.406 | 0.383 | 0.360 |
| Average | 0.636 | 2.03 | 0.636 | 1.81 | 0.636 | 0.789 |
| Stdev | 1.83 | 4.87 | 1.83 | 5.61 | 1.83 | 0.797 |
| p(t-test) | | 0.0014 | | 0.0072 | | 0.70 |
| Min | 0.000152 | 0.0957 | 0.000152 | 0.0249 | 0.000152 | 0.0995 |
| Max | 32.1 | 20.6 | 32.1 | 31.1 | 32.1 | 2.56 |
| n (Samp) | 349 | 28 | 349 | 33 | 349 | 22 |
| n (Patient) | 172 | 28 | 172 | 33 | 172 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.383 | 0.437 | 0.383 | 0.399 | 0.383 | 0.360 |
| Average | 0.637 | 2.04 | 0.637 | 1.77 | 0.637 | 0.741 |
| Stdev | 1.86 | 4.87 | 1.86 | 5.53 | 1.86 | 0.759 |
| p(t-test) | | 0.0015 | | 0.0097 | | 0.80 |
| Min | 0.000152 | 0.0957 | 0.000152 | 0.0249 | 0.000152 | 0.0995 |
| Max | 32.1 | 20.6 | 32.1 | 31.1 | 32.1 | 2.56 |
| n (Samp) | 340 | 28 | 340 | 34 | 340 | 20 |
| n (Patient) | 158 | 28 | 158 | 34 | 158 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | nd | 0.60 | 0.57 | nd | 0.56 | 0.56 | nd | 0.55 |
| SE | 0.058 | nd | 0.059 | 0.054 | nd | 0.053 | 0.065 | nd | 0.068 |
| p | 0.16 | nd | 0.098 | 0.22 | nd | 0.23 | 0.33 | nd | 0.43 |
| nCohort 1 | 349 | nd | 340 | 349 | nd | 340 | 349 | nd | 340 |
| nCohort 2 | 28 | nd | 28 | 33 | nd | 34 | 22 | nd | 20 |
| Cutoff 1 | 0.321 | nd | 0.356 | 0.293 | nd | 0.293 | 0.200 | nd | 0.200 |
| Sens 1 | 71% | nd | 71% | 73% | nd | 71% | 73% | nd | 70% |
| Spec 1 | 41% | nd | 46% | 38% | nd | 39% | 28% | nd | 28% |
| Cutoff 2 | 0.224 | nd | 0.232 | 0.259 | nd | 0.259 | 0.179 | nd | 0.179 |
| Sens 2 | 82% | nd | 82% | 82% | nd | 82% | 82% | nd | 80% |
| Spec 2 | 30% | nd | 31% | 32% | nd | 32% | 21% | nd | 21% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 3 | 0.134 | nd | 0.188 | 0.188 | nd | 0.188 | 0.134 | nd | 0.134 |
| Sens 3 | 96% | nd | 93% | 91% | nd | 91% | 91% | nd | 90% |
| Spec 3 | 16% | nd | 23% | 23% | nd | 23% | 16% | nd | 17% |
| Cutoff 4 | 0.589 | nd | 0.587 | 0.589 | nd | 0.587 | 0.589 | nd | 0.587 |
| Sens 4 | 36% | nd | 36% | 36% | nd | 35% | 45% | nd | 45% |
| Spec 4 | 70% | nd | 70% | 70% | nd | 70% | 70% | nd | 70% |
| Cutoff 5 | 0.734 | nd | 0.734 | 0.734 | nd | 0.734 | 0.734 | nd | 0.734 |
| Sens 5 | 29% | nd | 29% | 27% | nd | 26% | 36% | nd | 35% |
| Spec 5 | 80% | nd | 80% | 80% | nd | 80% | 80% | nd | 80% |
| Cutoff 6 | 1.09 | nd | 1.07 | 1.09 | nd | 1.07 | 1.09 | nd | 1.07 |
| Sens 6 | 21% | nd | 21% | 18% | nd | 18% | 23% | nd | 20% |
| Spec 6 | 90% | nd | 90% | 90% | nd | 90% | 90% | nd | 90% |
| OR Quart 2 | 1.8 | nd | 2.8 | 1.5 | nd | 2.3 | 0.99 | nd | 1.2 |
| | 0.36 | nd | 0.13 | 0.43 | nd | 0.13 | 0.98 | nd | 0.76 |
| p Value 95% CI of OR Quart2 | 0.51 | nd | 0.73 | 0.52 | nd | 0.78 | 0.31 | nd | 0.36 |
| | 6.4 | nd | 11 | 4.5 | nd | 7.0 | 3.2 | nd | 4.1 |
| OR Quart 3 | 2.4 | nd | 3.2 | 1.6 | nd | 1.9 | 0.16 | nd | 0.19 |
| | 0.16 | nd | 0.088 | 0.42 | nd | 0.27 | 0.088 | nd | 0.13 |
| p Value 95% CI of OR Quart3 | 0.71 | nd | 0.84 | 0.53 | nd | 0.61 | 0.018 | nd | 0.022 |
| | 8.0 | nd | 12 | 4.5 | nd | 5.9 | 1.3 | nd | 1.7 |
| OR Quart 4 | 2.1 | nd | 2.8 | 1.5 | nd | 1.9 | 1.5 | nd | 1.7 |
| | 0.25 | nd | 0.13 | 0.43 | nd | 0.28 | 0.43 | nd | 0.39 |
| p Value 95% CI of OR Quart4 | 0.60 | nd | 0.73 | 0.52 | nd | 0.60 | 0.52 | nd | 0.52 |
| | 7.1 | nd | 11 | 4.5 | nd | 5.8 | 4.5 | nd | 5.3 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.163 | 0.226 | 0.163 | 0.172 | 0.163 | 0.106 |
| Average | 0.200 | 0.261 | 0.200 | 0.221 | 0.200 | 0.130 |
| Stdev | 0.207 | 0.213 | 0.207 | 0.211 | 0.207 | 0.105 |
| p(t-test) | | 0.14 | | 0.59 | | 0.12 |
| Min | 2.92E-5 | 0.0472 | 2.92E-5 | 2.92E-5 | 2.92E-5 | 0.000127 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 2.09 | 1.08 | 2.09 | 0.971 | 2.09 | 0.427 |
| n (Samp) | 356 | 28 | 356 | 34 | 356 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.168 | 0.226 | 0.168 | 0.172 | 0.168 | 0.0967 |
| Average | 0.202 | 0.260 | 0.202 | 0.226 | 0.202 | 0.122 |
| Stdev | 0.207 | 0.213 | 0.207 | 0.210 | 0.207 | 0.105 |
| p(t-test) | | 0.16 | | 0.53 | | 0.086 |
| Min | 2.92E-5 | 0.0472 | 2.92E-5 | 2.92E-5 | 2.92E-5 | 0.000127 |
| Max | 2.09 | 1.08 | 2.09 | 0.971 | 2.09 | 0.427 |
| n (Samp) | 347 | 28 | 347 | 35 | 347 | 20 |
| n (Patient) | 161 | 28 | 161 | 35 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | nd | 0.61 | 0.53 | nd | 0.53 | 0.40 | nd | 0.37 |
| SE | 0.058 | nd | 0.058 | 0.053 | nd | 0.052 | 0.066 | nd | 0.068 |
| p | 0.050 | nd | 0.067 | 0.63 | nd | 0.55 | 0.11 | nd | 0.049 |
| nCohort 1 | 356 | nd | 347 | 356 | nd | 347 | 356 | nd | 347 |
| nCohort 2 | 28 | nd | 28 | 34 | nd | 35 | 22 | nd | 20 |
| Cutoff 1 | 0.128 | nd | 0.128 | 0.0839 | nd | 0.0820 | 0.0584 | nd | 0.0584 |
| Sens 1 | 71% | nd | 71% | 71% | nd | 71% | 73% | nd | 70% |
| Spec 1 | 43% | nd | 41% | 29% | nd | 28% | 22% | nd | 21% |
| Cutoff 2 | 0.0791 | nd | 0.0791 | 0.0536 | nd | 0.0564 | 0.0521 | nd | 0.0521 |
| Sens 2 | 82% | nd | 82% | 82% | nd | 80% | 82% | nd | 80% |
| Spec 2 | 27% | nd | 27% | 20% | nd | 20% | 20% | nd | 19% |
| Cutoff 3 | 0.0511 | nd | 0.0511 | 0.0120 | nd | 0.0120 | 0.0240 | nd | 0.0240 |
| Sens 3 | 93% | nd | 93% | 91% | nd | 91% | 91% | nd | 90% |
| Spec 3 | 20% | nd | 19% | 10% | nd | 9% | 12% | nd | 12% |
| Cutoff 4 | 0.243 | nd | 0.248 | 0.243 | nd | 0.248 | 0.243 | nd | 0.248 |
| Sens 4 | 39% | nd | 39% | 32% | nd | 34% | 18% | nd | 10% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 4 | 70% | nd | 70% | 70% | nd | 70% | 70% | nd | 70% |
| Cutoff 5 | 0.300 | nd | 0.302 | 0.300 | nd | 0.302 | 0.300 | nd | 0.302 |
| Sens 5 | 39% | nd | 36% | 26% | nd | 29% | 5% | nd | 5% |
| Spec 5 | 80% | nd | 80% | 80% | nd | 80% | 80% | nd | 80% |
| Cutoff 6 | 0.390 | nd | 0.390 | 0.390 | nd | 0.390 | 0.390 | nd | 0.390 |
| Sens 6 | 18% | nd | 18% | 18% | nd | 20% | 5% | nd | 5% |
| Spec 6 | 90% | nd | 90% | 90% | nd | 90% | 90% | nd | 90% |
| OR Quart 2 | 1.5 | nd | 1.5 | 0.75 | nd | 0.75 | 2.6 | nd | 1.5 |
| | 0.52 | nd | 0.53 | 0.59 | nd | 0.59 | 0.26 | nd | 0.65 |
| p Value 95% CI of OR Quart2 | 0.42 | nd | 0.41 | 0.27 | nd | 0.27 | 0.49 | nd | 0.25 |
| | 5.6 | nd | 5.6 | 2.1 | nd | 2.1 | 14 | nd | 9.3 |
| OR Quart 3 | 1.8 | nd | 2.1 | 0.88 | nd | 0.88 | 3.7 | nd | 3.7 |
| | 0.36 | nd | 0.25 | 0.80 | nd | 0.80 | 0.11 | nd | 0.11 |
| p Value 95% CI of OR Quart3 | 0.51 | nd | 0.60 | 0.32 | nd | 0.32 | 0.75 | nd | 0.75 |
| | 6.4 | nd | 7.1 | 2.4 | nd | 2.4 | 18 | nd | 18 |
| OR Quart 4 | 3.0 | nd | 2.6 | 1.1 | nd | 1.2 | 4.3 | nd | 4.3 |
| | 0.070 | nd | 0.11 | 0.83 | nd | 0.65 | 0.069 | nd | 0.068 |
| p Value 95% CI of OR Quart4 | 0.91 | nd | 0.80 | 0.43 | nd | 0.49 | 0.89 | nd | 0.90 |
| | 9.7 | nd | 8.8 | 2.9 | nd | 3.1 | 21 | nd | 21 |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0459 | 0.0866 | 0.0459 | 0.0750 | 0.0459 | 0.0792 |
| Average | 0.210 | 0.234 | 0.210 | 0.279 | 0.210 | 0.401 |
| Stdev | 0.518 | 0.444 | 0.518 | 0.969 | 0.518 | 0.598 |
| p(t-test) | | 0.82 | | 0.50 | | 0.097 |
| Min | 4.60E-6 | 0.00381 | 4.60E-6 | 9.65E-6 | 4.60E-6 | 0.00226 |
| Max | 4.46 | 2.26 | 4.46 | 5.72 | 4.46 | 2.11 |
| n (Samp) | 356 | 28 | 356 | 34 | 356 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0524 | 0.0866 | 0.0524 | 0.0712 | 0.0524 | 0.0664 |
| Average | 0.224 | 0.235 | 0.224 | 0.272 | 0.224 | 0.426 |
| Stdev | 0.529 | 0.444 | 0.529 | 0.956 | 0.529 | 0.623 |
| p(t-test) | | 0.91 | | 0.64 | | 0.10 |
| Min | 4.60E-6 | 0.00381 | 4.60E-6 | 9.65E-6 | 4.60E-6 | 0.00226 |
| Max | 4.46 | 2.26 | 4.46 | 5.72 | 4.46 | 2.11 |
| n (Samp) | 347 | 28 | 347 | 35 | 347 | 20 |
| n (Patient) | 161 | 28 | 161 | 35 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | nd | 0.56 | 0.57 | nd | 0.54 | 0.61 | nd | 0.58 |
| SE | 0.058 | nd | 0.058 | 0.053 | nd | 0.052 | 0.066 | nd | 0.068 |
| p | 0.18 | nd | 0.30 | 0.22 | nd | 0.44 | 0.10 | nd | 0.25 |
| nCohort 1 | 356 | nd | 347 | 356 | nd | 347 | 356 | nd | 347 |
| nCohort 2 | 28 | nd | 28 | 34 | nd | 35 | 22 | nd | 20 |
| Cutoff 1 | 0.0322 | nd | 0.0322 | 0.0325 | nd | 0.0325 | 0.0301 | nd | 0.0301 |
| Sens 1 | 71% | nd | 71% | 71% | nd | 71% | 73% | nd | 70% |
| Spec 1 | 40% | nd | 37% | 41% | nd | 38% | 38% | nd | 36% |
| Cutoff 2 | 0.0126 | nd | 0.0126 | 0.0209 | nd | 0.0252 | 0.0202 | nd | 0.0202 |
| Sens 2 | 82% | nd | 82% | 82% | nd | 80% | 82% | nd | 80% |
| Spec 2 | 24% | nd | 21% | 32% | nd | 33% | 31% | nd | 29% |
| Cutoff 3 | 0.00659 | nd | 0.00654 | 0.0141 | nd | 0.0141 | 0.00901 | nd | 0.00901 |
| Sens 3 | 93% | nd | 93% | 91% | nd | 91% | 91% | nd | 90% |
| Spec 3 | 16% | nd | 13% | 25% | nd | 22% | 20% | nd | 17% |
| Cutoff 4 | 0.122 | nd | 0.133 | 0.122 | nd | 0.133 | 0.122 | nd | 0.133 |
| Sens 4 | 36% | nd | 36% | 32% | nd | 29% | 45% | nd | 45% |
| Spec 4 | 70% | nd | 70% | 70% | nd | 70% | 70% | nd | 70% |
| Cutoff 5 | 0.214 | nd | 0.224 | 0.214 | nd | 0.224 | 0.214 | nd | 0.224 |
| Sens 5 | 21% | nd | 25% | 18% | nd | 17% | 41% | nd | 35% |
| Spec 5 | 80% | nd | 80% | 80% | nd | 80% | 80% | nd | 80% |
| Cutoff 6 | 0.474 | nd | 0.495 | 0.474 | nd | 0.495 | 0.474 | nd | 0.495 |
| Sens 6 | 18% | nd | 18% | 3% | nd | 3% | 27% | nd | 30% |
| Spec 6 | 90% | nd | 90% | 90% | nd | 90% | 90% | nd | 90% |
| OR Quart 2 | 0.41 | nd | 0.41 | 2.4 | nd | 1.5 | 0.99 | nd | 0.99 |
| | 0.21 | nd | 0.20 | 0.17 | nd | 0.43 | 0.99 | nd | 0.99 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| p Value | 0.10 | nd | 0.10 | 0.70 | nd | 0.52 | 0.24 | nd | 0.24 |
| 95% CI of OR Quart2 | 1.6 | nd | 1.6 | 7.9 | nd | 4.5 | 4.1 | nd | 4.1 |
| OR Quart 3 | 1.3 | nd | 1.5 | 3.3 | nd | 1.9 | 1.3 | nd | 0.99 |
|  | 0.60 | nd | 0.46 | 0.046 | nd | 0.21 | 0.73 | nd | 0.99 |
| p Value 95% CI of OR Quart3 | 0.47 | nd | 0.53 | 1.0 | nd | 0.69 | 0.33 | nd | 0.24 |
|  | 3.7 | nd | 4.0 | 11 | nd | 5.5 | 4.9 | nd | 4.1 |
| OR Quart 4 | 1.3 | nd | 1.1 | 2.4 | nd | 1.5 | 2.4 | nd | 2.1 |
|  | 0.60 | nd | 0.80 | 0.17 | nd | 0.43 | 0.17 | nd | 0.25 |
| p Value 95% CI of OR Quart4 | 0.47 | nd | 0.40 | 0.70 | nd | 0.52 | 0.70 | nd | 0.60 |
|  | 3.7 | nd | 3.3 | 7.9 | nd | 4.5 | 7.9 | nd | 7.1 |

**Tenascin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 562 | 799 | 562 | 664 | 562 | 949 |
| Average | 1170 | 1140 | 1170 | 982 | 1170 | 1330 |
| Stdev | 2460 | 1190 | 2460 | 741 | 2460 | 1050 |
| p(t-test) |  | 0.94 |  | 0.67 |  | 0.76 |
| Min | 43.9 | 146 | 43.9 | 167 | 43.9 | 200 |
| Max | 26100 | 5700 | 26100 | 3000 | 26100 | 4290 |
| n (Samp) | 357 | 28 | 357 | 32 | 357 | 22 |
| n (Patient) | 175 | 28 | 175 | 32 | 175 | 22 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 564 | 799 | 564 | 728 | 564 | 949 |
| Average | 1190 | 1130 | 1190 | 978 | 1190 | 1370 |
| Stdev | 2490 | 1190 | 2490 | 729 | 2490 | 1090 |
| p(t-test) |  | 0.91 |  | 0.63 |  | 0.74 |
| Min | 43.9 | 146 | 43.9 | 167 | 43.9 | 200 |
| Max | 26100 | 5700 | 26100 | 3000 | 26100 | 4290 |
| n (Samp) | 348 | 28 | 348 | 33 | 348 | 20 |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 161 | 28 | 161 | 33 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.59 | 0.58 | nd | 0.58 | 0.71 | nd | 0.70 |
| SE | 0.058 | nd | 0.058 | 0.055 | nd | 0.054 | 0.063 | nd | 0.067 |
| p | 0.10 | nd | 0.13 | 0.14 | nd | 0.14 | 9.3E-4 | nd | 0.0023 |
| nCohort 1 | 357 | nd | 348 | 357 | nd | 348 | 357 | nd | 348 |
| nCohort 2 | 28 | nd | 28 | 32 | nd | 33 | 22 | nd | 20 |
| Cutoff 1 | 482 | nd | 482 | 426 | nd | 426 | 817 | nd | 817 |
| Sens 1 | 71% | nd | 71% | 72% | nd | 73% | 73% | nd | 70% |
| Spec 1 | 45% | nd | 44% | 38% | nd | 38% | 68% | nd | 67% |
| Cutoff 2 | 389 | nd | 389 | 358 | nd | 358 | 704 | nd | 704 |
| Sens 2 | 82% | nd | 82% | 81% | nd | 82% | 82% | nd | 80% |
| Spec 2 | 33% | nd | 33% | 29% | nd | 29% | 63% | nd | 62% |
| Cutoff 3 | 223 | nd | 223 | 310 | nd | 310 | 486 | nd | 486 |
| Sens 3 | 93% | nd | 93% | 91% | nd | 91% | 91% | nd | 90% |
| Spec 3 | 14% | nd | 13% | 24% | nd | 24% | 45% | nd | 44% |
| Cutoff 4 | 875 | nd | 895 | 875 | nd | 895 | 875 | nd | 895 |
| Sens 4 | 43% | nd | 43% | 44% | nd | 42% | 64% | nd | 65% |
| Spec 4 | 70% | nd | 70% | 70% | nd | 70% | 70% | nd | 70% |
| Cutoff 5 | 1160 | nd | 1170 | 1160 | nd | 1170 | 1160 | nd | 1170 |
| Sens 5 | 32% | nd | 29% | 34% | nd | 30% | 32% | nd | 35% |
| Spec 5 | 80% | nd | 80% | 80% | nd | 80% | 80% | nd | 80% |
| Cutoff 6 | 1950 | nd | 1950 | 1950 | nd | 1950 | 1950 | nd | 1950 |
| Sens 6 | 18% | nd | 18% | 12% | nd | 12% | 18% | nd | 20% |
| Spec 6 | 90% | nd | 90% | 90% | nd | 90% | 90% | nd | 90% |
| OR Quart 2 | 1.0 | nd | 1.0 | 2.7 | nd | 1.9 | 3.0 | nd | 3.1 |
| | 1.0 | nd | 1.0 | 0.11 | nd | 0.27 | 0.34 | nd | 0.34 |
| p Value 95% CI of OR Quart2 | 0.28 | nd | 0.28 | 0.81 | nd | 0.61 | 0.31 | nd | 0.31 |
| | 3.6 | nd | 3.6 | 8.8 | nd | 5.8 | 30 | nd | 30 |
| OR Quart 3 | 1.7 | nd | 1.7 | 1.0 | nd | 1.0 | 8.6 | nd | 7.5 |
| | 0.39 | nd | 0.39 | 1.0 | nd | 1.0 | 0.045 | nd | 0.062 |
| p Value 95% CI of OR Quart3 | 0.52 | nd | 0.52 | 0.24 | nd | 0.28 | 1.0 | nd | 0.90 |
| | 5.3 | nd | 5.3 | 4.1 | nd | 3.6 | 70 | nd | 62 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 4 | 2.1 | nd | 2.1 | 3.9 | nd | 3.1 | 11 | nd | 9.9 |
|  | 0.19 | nd | 0.19 | 0.021 | nd | 0.039 | 0.024 | nd | 0.032 |
| p Value 95% CI of OR Quart4 | 0.69 | nd | 0.70 | 1.2 | nd | 1.1 | 1.4 | nd | 1.2 |
|  | 6.4 | nd | 6.5 | 12 | nd | 8.9 | 87 | nd | 80 |

Fig. 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).

**Angiopoietin-related protein 4**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 37.7 | 31.0 | 52.4 | 47.4 | 35.1 | 30.5 |
| Average | 46.3 | 50.4 | 68.0 | 64.4 | 38.8 | 49.2 |
| Stdev | 43.5 | 44.4 | 65.2 | 59.7 | 22.9 | 41.0 |
| p(t-test) |  | 0.67 |  | 0.90 |  | 0.16 |
| Min | 2.71 | 8.79 | 8.18 | 20.5 | 2.71 | 8.79 |
| Max | 317 | 192 | 317 | 179 | 101 | 192 |
| n (Samp) | 65 | 31 | 26 | 6 | 50 | 26 |
| n (Patient) | 65 | 31 | 26 | 6 | 50 | 26 |

|  | At Enrollment | | |
|---|---|---|---|
|  | sCr or UO | sCr only | UO only |
| AUC | 0.50 | 0.45 | 0.53 |
| SE | 0.063 | 0.13 | 0.071 |
| p | 0.98 | 0.70 | 0.65 |
| nCohort 1 | 65 | 26 | 50 |
| nCohort 2 | 31 | 6 | 26 |
| Cutoff 1 | 24.5 | 22.3 | 25.1 |
| Sens 1 | 71% | 83% | 73% |
| Spec 1 | 28% | 15% | 32% |
| Cutoff 2 | 22.1 | 22.3 | 22.1 |
| Sens 2 | 81% | 83% | 81% |
| Spec 2 | 23% | 15% | 26% |
| Cutoff 3 | 15.1 | 15.1 | 14.5 |

(continued)

|  | At Enrollment | | |
|---|---|---|---|
|  | sCr or UO | sCr only | UO only |
| Sens 3 | 90% | 100% | 92% |
| Spec 3 | 14% | 12% | 12% |
| Cutoff 4 | 45.5 | 72.2 | 43.0 |
| Sens 4 | 42% | 17% | 42% |
| Spec 4 | 71% | 73% | 70% |
| Cutoff 5 | 63.2 | 78.2 | 51.6 |
| Sens 5 | 19% | 17% | 38% |
| Spec 5 | 80% | 81% | 80% |
| Cutoff 6 | 82.3 | 154 | 75.6 |
| Sens 6 | 13% | 17% | 19% |
| Spec 6 | 91% | 92% | 90% |
| OR Quart 2 | 1.0 | 2.3 | 2.0 |
| p Value | 1.0 | 0.53 | 0.32 |
| 95% CI of | 0.29 | 0.17 | 0.52 |
| OR Quart2 | 3.5 | 33 | 7.3 |
| OR Quart 3 | 1.2 | 1.0 | 0.25 |
| p Value | 0.76 | 1.0 | 0.13 |
| 95% CI of | 0.36 | 0.052 | 0.044 |
| OR Quart3 | 4.1 | 19 | 1.5 |
| OR Quart 4 | 1.5 | 2.3 | 2.0 |
| p Value | 0.54 | 0.53 | 0.32 |
| 95% CI of | 0.44 | 0.17 | 0.52 |
| OR Quart4 | 4.9 | 33 | 7.3 |

**Amphiregulin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 22.0 | 22.0 | 24.1 | 22.4 | 20.9 | 21.1 |
| Average | 31.8 | 30.9 | 39.2 | 46.8 | 27.0 | 26.9 |
| Stdev | 34.4 | 30.7 | 44.4 | 57.8 | 24.3 | 18.6 |
| p(t-test) |  | 0.90 |  | 0.72 |  | 0.98 |
| Min | 2.70 | 0.00246 | 4.60 | 13.6 | 2.70 | 0.00246 |
| Max | 198 | 162 | 198 | 162 | 145 | 72.6 |
| n (Samp) | 63 | 30 | 25 | 6 | 49 | 25 |
| n (Patient) | 63 | 30 | 25 | 6 | 49 | 25 |

(continued)

| | At Enrollment | | |
|---|---|---|---|
| | sCr or UO | sCr only | UO only |
| AUC | 0.51 | 0.51 | 0.53 |
| SE | 0.064 | 0.13 | 0.072 |
| p | 0.91 | 0.92 | 0.66 |
| nCohort 1 | 63 | 25 | 49 |
| nCohort 2 | 30 | 6 | 25 |
| Cutoff 1 | 16.1 | 13.9 | 17.4 |
| Sens 1 | 70% | 83% | 72% |
| Spec 1 | 32% | 24% | 41% |
| Cutoff 2 | 13.5 | 13.9 | 13.9 |
| Sens 2 | 80% | 83% | 80% |
| Spec 2 | 27% | 24% | 35% |
| Cutoff 3 | 9.97 | 11.9 | 8.92 |
| Sens 3 | 90% | 100% | 92% |
| Spec 3 | 16% | 20% | 12% |
| Cutoff 4 | 30.4 | 40.6 | 27.6 |
| Sens 4 | 30% | 33% | 28% |
| Spec 4 | 71% | 72% | 71% |
| Cutoff 5 | 40.6 | 51.0 | 36.0 |
| Sens 5 | 20% | 17% | 20% |
| Spec 5 | 81% | 80% | 82% |
| Cutoff 6 | 60.5 | 72.6 | 60.5 |
| Sens 6 | 10% | 17% | 8% |
| Spec 6 | 90% | 92% | 92% |
| OR Quart 2 | 1.8 | 2.0 | 1.5 |
| p Value | 0.35 | 0.61 | 0.56 |
| 95% CI of | 0.52 | 0.14 | 0.38 |
| OR Quart2 | 6.4 | 28 | 6.1 |
| OR Quart 3 | 1.0 | 0.86 | 1.3 |
| p Value | 1.0 | 0.92 | 0.72 |
| 95% CI of | 0.27 | 0.044 | 0.31 |
| OR Quart3 | 3.7 | 17 | 5.4 |
| OR Quart 4 | 1.7 | 2.0 | 1.5 |
| p Value | 0.40 | 0.61 | 0.56 |
| 95% CI of | 0.49 | 0.14 | 0.38 |
| OR Quart4 | 5.9 | 28 | 6.1 |

**Endostatin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 44200 | 37400 | 58300 | 34700 | 41500 | 34100 |
| Average | 59000 | 57100 | 77700 | 41500 | 48000 | 62200 |
| Stdev | 62600 | 61300 | 88700 | 32600 | 30200 | 69700 |
| p(t-test) |  | 0.89 |  | 0.34 |  | 0.22 |
| Min | 6940 | 9280 | 13600 | 13800 | 6940 | 9280 |
| Max | 483000 | 275000 | 483000 | 102000 | 146000 | 275000 |
| n (Samp) | 65 | 31 | 26 | 6 | 50 | 26 |
| n (Patient) | 65 | 31 | 26 | 6 | 50 | 26 |

|  | At Enrollment | | |
|---|---|---|---|
|  | sCr or UO | sCr only | UO only |
| AUC | 0.46 | 0.28 | 0.48 |
| SE | 0.064 | 0.13 | 0.070 |
| p | 0.49 | 0.077 | 0.80 |
| nCohort 1 | 65 | 26 | 50 |
| nCohort 2 | 31 | 6 | 26 |
| Cutoff 1 Sens 1 Spec 1 | 29900 | 13800 | 26300 |
|  | 71% | 83% | 73% |
|  | 28% | 4% | 24% |
| Cutoff 2 Sens 2 Spec 2 | 26000 | 13800 | 23600 |
|  | 81% | 83% | 81% |
|  | 20% | 4% | 22% |
| Cutoff 3 Sens 3 Spec 3 | 17000 | 13600 | 17000 |
|  | 90% | 100% | 92% |
|  | 11% | 4% | 12% |
| Cutoff 4 Sens 4 Spec 4 | 61800 | 73100 | 53400 |
|  | 29% | 17% | 31% |
|  | 71% | 73% | 70% |
| Cutoff 5 Sens 5 Spec 5 | 69800 | 93800 | 66900 |
|  | 23% | 17% | 27% |
|  | 80% | 81% | 80% |
| Cutoff 6 Sens 6 Spec 6 | 115000 | 119000 | 80400 |
|  | 6% | 0% | 19% |
|  | 91% | 92% | 90% |

(continued)

|  | At Enrollment | | |
|---|---|---|---|
|  | sCr or UO | sCr only | UO only |
| OR Quart 2 p Value 95% CI of OR Quart2 | 0.53 | 0 | 0.61 |
|  | 0.33 | na | 0.49 |
|  | 0.14 | na | 0.15 |
|  | 1.9 | na | 2.4 |
| OR Quart 3 p Value 95% CI of OR Quart3 | 1.4 | 4.2 | 1.0 |
|  | 0.55 | 0.27 | 1.0 |
|  | 0.44 | 0.33 | 0.27 |
|  | 4.6 | 53 | 3.7 |
| OR Quart 4 p Value 95% CI of OR Quart4 | 1.0 | 2.3 | 1.0 |
|  | 1.0 | 0.53 | 1.0 |
|  | 0.30 | 0.17 | 0.27 |
|  | 3.3 | 33 | 3.7 |

**Fibroblast growth factor 19**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.164 | 0.149 | 0.223 | 0.104 | 0.140 | 0.150 |
| Average | 0.209 | 0.236 | 0.295 | 0.149 | 0.182 | 0.240 |
| Stdev | 0.210 | 0.287 | 0.279 | 0.181 | 0.205 | 0.297 |
| p(t-test) |  | 0.60 |  | 0.23 |  | 0.32 |
| Min | 0.000127 | 8.23E-5 | 0.000127 | 8.23E-5 | 0.000127 | 0.00974 |
| Max | 1.41 | 1.32 | 1.32 | 0.482 | 1.41 | 1.32 |
| n (Samp) | 65 | 31 | 26 | 6 | 50 | 26 |
| n (Patient) | 65 | 31 | 26 | 6 | 50 | 26 |

|  | At Enrollment | | |
|---|---|---|---|
|  | sCr or UO | sCr only | UO only |
| AUC | 0.48 | 0.28 | 0.54 |
| SE | 0.064 | 0.13 | 0.071 |
| p | 0.71 | 0.082 | 0.61 |
| nCohort 1 | 65 | 26 | 50 |
| nCohort 2 | 31 | 6 | 26 |
| Cutoff 1 | 0.0804 | 8.23E-5 | 0.0816 |
| Sens 1 | 71% | 83% | 73% |

(continued)

| | At Enrollment | | |
|---|---|---|---|
| | sCr or UO | sCr only | UO only |
| Spec 1 | 23% | 0% | 28% |
| Cutoff 2 | 0.0659 | 8.23E-5 | 0.0676 |
| Sens 2 | 81% | 83% | 81% |
| Spec 2 | 22% | 0% | 24% |
| Cutoff 3 | 0.0209 | 0 | 0.0616 |
| Sens 3 | 90% | 100% | 92% |
| Spec 3 | 11% | 0% | 22% |
| Cutoff 4 | 0.265 | 0.326 | 0.215 |
| Sens 4 | 32% | 17% | 31% |
| Spec 4 | 71% | 73% | 70% |
| Cutoff 5 | 0.303 | 0.357 | 0.265 |
| Sens 5 | 23% | 17% | 31% |
| Spec 5 | 80% | 81% | 80% |
| Cutoff 6 | 0.346 | 0.543 | 0.304 |
| Sens 6 | 16% | 0% | 19% |
| Spec 6 | 91% | 92% | 90% |
| OR Quart 2 | 0.67 | 0 | 0.61 |
| p Value | 0.53 | na | 0.49 |
| 95% CI of | 0.19 | na | 0.15 |
| OR Quart2 | 2.3 | na | 2.4 |
| OR Quart 3 | 1.0 | 2.3 | 0.79 |
| p Value | 1.0 | 0.53 | 0.73 |
| 95% CI of | 0.30 | 0.17 | 0.21 |
| OR Quart3 | 3.3 | 33 | 3.0 |
| At | At Enrollment | | |
| | sCr or UO | sCr only | UO only |
| OR Quart 4 | 1.2 | 4.2 | 1.2 |
| p Value | 0.76 | 0.27 | 0.74 |
| 95% CI of | 0.37 | 0.33 | 0.34 |
| OR Quart4 | 3.9 | 53 | 4.6 |

**Fibroblast growth factor 21**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 0.0451 | 0.0670 | 0.0773 | 0.288 | 0.0610 | 0.0618 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Average | 0.212 | 0.402 | 0.257 | 0.634 | 0.191 | 0.340 |
| Stdev | 0.512 | 1.08 | 0.489 | 0.803 | 0.485 | 1.11 |
| p(t-test) |  | 0.24 |  | 0.14 |  | 0.42 |
| Min | 7.01E-5 | 0.00323 | 0.00263 | 0.0306 | 7.01E-5 | 0.00323 |
| Max | 3.36 | 5.72 | 2.05 | 2.11 | 3.36 | 5.72 |
| n (Samp) | 65 | 31 | 26 | 6 | 50 | 26 |
| n (Patient) | 65 | 31 | 26 | 6 | 50 | 26 |

|  | At Enrollment | | |
|---|---|---|---|
|  | sCr or UO | sCr only | UO only |
| AUC | 0.58 | 0.71 | 0.52 |
| SE | 0.064 | 0.13 | 0.071 |
| p | 0.21 | 0.099 | 0.73 |
| nCohort 1 | 65 | 26 | 50 |
| nCohort 2 | 31 | 6 | 26 |
| Cutoff 1 | 0.0322 | 0.0938 | 0.0231 |
| Sens 1 | 71% | 83% | 73% |
| Spec 1 | 43% | 58% | 38% |
| Cutoff 2 | 0.0231 | 0.0938 | 0.0189 |
| Sens 2 | 81% | 83% | 81% |
| Spec 2 | 40% | 58% | 32% |
| Cutoff 3 | 0.0126 | 0.0217 | 0.00762 |
| Sens 3 | 90% | 100% | 92% |
| Spec 3 | 22% | 27% | 20% |
| Cutoff 4 | 0.129 | 0.181 | 0.170 |
| Sens 4 | 35% | 50% | 31% |
| Spec 4 | 71% | 73% | 70% |
| Cutoff 5 | 0.253 | 0.206 | 0.252 |
| Sens 5 | 19% | 50% | 12% |
| Spec 5 | 80% | 81% | 80% |
| Cutoff 6 | 0.430 | 1.05 | 0.312 |
| Sens 6 | 13% | 17% | 12% |
| Spec 6 | 91% | 92% | 90% |
| OR Quart 2 | 3.0 | 0 | 3.4 |
| p Value | 0.11 | na | 0.094 |
| 95% CI of | 0.77 | na | 0.81 |

(continued)

|  | At Enrollment | | |
|---|---|---|---|
|  | sCr or UO | sCr only | UO only |
| OR Quart2 | 12 | na | 14 |
| OR Quart 3 | 3.6 | 2.3 | 2.2 |
| p Value | 0.064 | 0.53 | 0.29 |
| 95% CI of | 0.93 | 0.17 | 0.52 |
| OR Quart3 | 14 | 33 | 9.3 |
| OR Quart 4 | 2.5 | 4.2 | 1.7 |
| p Value | 0.19 | 0.27 | 0.46 |
| 95% CI of | 0.64 | 0.33 | 0.40 |
| OR Quart4 | 9.8 | 53 | 7.5 |

**Thrombospondin-2**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 41900 | 21700 | 59900 | 18300 | 40400 | 24200 |
| Average | 66700 | 67900 | 89800 | 87900 | 65600 | 52300 |
| Stdev | 75700 | 119000 | 102000 | 169000 | 81200 | 83000 |
| p(t-test) |  | 0.95 |  | 0.97 |  | 0.50 |
| Min | 1240 | 1620 | 1400 | 4470 | 1240 | 1620 |
| Max | 434000 | 433000 | 418000 | 433000 | 434000 | 351000 |
| n (Samp) | 66 | 31 | 26 | 6 | 51 | 26 |
| n (Patient) | 66 | 31 | 26 | 6 | 51 | 26 |

|  | At Enrollment | | |
|---|---|---|---|
|  | sCr or UO | sCr only | UO only |
| AUC | 0.39 | 0.34 | 0.41 |
| SE | 0.063 | 0.13 | 0.070 |
| p | 0.076 | 0.23 | 0.18 |
| nCohort 1 | 66 | 26 | 51 |
| nCohort 2 | 31 | 6 | 26 |
| Cutoff 1 | 15500 | 9090 | 16500 |
| Sens 1 | 71% | 83% | 73% |
| Spec 1 | 26% | 15% | 27% |
| Cutoff 2 | 8620 | 9090 | 13100 |
| Sens 2 | 81% | 83% | 81% |

(continued)

|  | At Enrollment | | |
|---|---|---|---|
|  | sCr or UO | sCr only | UO only |
| Spec 2 | 14% | 15% | 20% |
| Cutoff 3 | 4470 | 2860 | 3360 |
| Sens 3 | 90% | 100% | 92% |
| Spec 3 | 6% | 8% | 6% |
| Cutoff 4 | 67700 | 106000 | 55600 |
| Sens 4 | 16% | 17% | 15% |
| Spec 4 | 71% | 73% | 71% |
| Cutoff 5 | 112000 | 123000 | 112000 |
| Sens 5 | 13% | 17% | 12% |
| Spec 5 | 80% | 81% | 80% |
| Cutoff 6 | 152000 | 237000 | 160000 |
| Sens 6 | 13% | 17% | 12% |
| Spec 6 | 91% | 92% | 90% |
| OR Quart 2 | 1.1 | 0 | 1.4 |
| p Value | 0.94 | na | 0.64 |
| 95% CI of | 0.26 | na | 0.32 |
| OR Quart2 | 4.2 | na | 6.4 |
| OR Quart 3 | 4,7 | 2.3 | 5.5 |
| p Value | 0.016 | 0.53 | 0.019 |
| 95% CI of | 1.3 | 0.17 | 1.3 |
| OR Quart3 | 17 | 33 | 23 |
| OR Quart 4 | 2.0 | 4.2 | 1.8 |
| p Value | 0.29 | 0.27 | 0.41 |
| 95% CI of | 0.55 | 0.33 | 0.43 |
| OR Quart4 | 7.3 | 53 | 8.0 |

Fig. 8: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

**Angiogenin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 179000 | 204000 | 179000 | 193000 | 179000 | 250000 |
| Average | 209000 | 206000 | 209000 | 197000 | 209000 | 251000 |
| Stdev | 234000 | 104000 | 234000 | 109000 | 234000 | 96600 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.97 | | 0.87 | | 0.64 |
| Min | 33100 | 48000 | 33100 | 48000 | 33100 | 88800 |
| Max | 2240000 | 345000 | 2240000 | 345000 | 2240000 | 345000 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 198000 | 141000 | 198000 | 140000 |
| Average | 221000 | 170000 | 221000 | 156000 |
| Stdev | 235000 | 104000 | 235000 | 106000 |
| p(t-test) | | 0.57 | | 0.47 |
| Min | 33100 | 48000 | 33100 | 48000 |
| Max | 2240000 | 345000 | 2240000 | 345000 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.55 | 0.40 | 0.52 | 0.35 | 0.70 |
| SE | 0.095 | 0.12 | 0.093 | 0.12 | 0.11 |
| p | 0.61 | 0.40 | 0.87 | 0.21 | 0.083 |
| nCohort 1 | 88 | 89 | 88 | 89 | 88 |
| nCohort 2 | 11 | 7 | 11 | 7 | 7 |
| Cutoff 1 | 140000 | 109000 | 140000 | 87400 | 202000 |
| Sens 1 | 73% | 71% | 73% | 71% | 71% |
| Spec 1 | 35% | 17% | 35% | 9% | 60% |
| Cutoff 2 | 109000 | 87400 | 87400 | 76300 | 191000 |
| Sens 2 | 82% | 86% | 82% | 86% | 86% |
| Spec 2 | 17% | 9% | 9% | 6% | 56% |
| Cutoff 3 | 87400 | 33100 | 76300 | 33100 | 87400 |
| Sens 3 | 91% | 100% | 91% | 100% | 100% |
| Spec 3 | 9% | 1% | 7% | 1% | 9% |
| Cutoff 4 | 225000 | 245000 | 225000 | 245000 | 225000 |
| Sens 4 | 36% | 29% | 36% | 29% | 57% |
| Spec 4 | 70% | 71% | 70% | 71% | 70% |

(continued)

|  | 0hr prior to AKI stage | | 24hr prior to AKI stage | | 48hr prior to AKI stage |
|---|---|---|---|---|---|
|  | sCr or UO | UO only | sCr or UO | UO only | sCr or UO |
| Cutoff 5 | 259000 | 271000 | 259000 | 271000 | 259000 |
| Sens 5 | 27% | 14% | 27% | 14% | 43% |
| Spec 5 | 81% | 81% | 81% | 81% | 81% |
| Cutoff 6 | 285000 | 330000 | 285000 | 330000 | 285000 |
| Sens 6 | 27% | 14% | 27% | 14% | 43% |
| Spec 6 | 91% | 91% | 91% | 91% | 91% |
| OR Quart 2 | 0.29 | 2.1 | 0.61 | 1.0 | 0 |
|  | 0.30 | 0.56 | 0.61 | 1.0 | na |
| p Value 95% CI of OR Quart2 | 0.028 | 0.18 | 0.092 | 0.059 | na |
|  | 3.0 | 25 | 4.0 | 17 | na |
| OR Quart 3 | 0.95 | 1.0 | 0.61 | 2.1 | 2.0 |
|  | 0.96 | 1.0 | 0.61 | 0.56 | 0.58 |
| p Value 95% CI of OR Quart3 | 0.17 | 0.059 | 0.092 | 0.18 | 0.17 |
|  | 5.3 | 17 | 4.0 | 25 | 24 |
| OR Quart 4 | 1.3 | 3.3 | 1.3 | 3.3 | 4.4 |
|  | 0.73 | 0.32 | 0.73 | 0.32 | 0.20 |
| p Value 95% CI of OR Quart4 | 0.27 | 0.32 | 0.27 | 0.32 | 0.45 |
|  | 6.7 | 34 | 6.7 | 34 | 43 |

**Angiopoietin-related protein 4**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 23.2 | 94.8 | 23.2 | 94.8 | 23.2 | 61.9 |
| Average | 65.5 | 136 | 65.5 | 122 | 65.5 | 78.2 |
| Stdev | 212 | 101 | 212 | 89.2 | 212 | 48.4 |
| p(t-test) |  | 0.28 |  | 0.39 |  | 0.88 |
| Min | 1.77 | 34.0 | 1.77 | 34.0 | 1.77 | 34.0 |
| Max | 1900 | 339 | 1900 | 339 | 1900 | 179 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.8 | 179 | 31.8 | 133 | nd | nd |
| Average | 78.1 | 185 | 78.1 | 163 | nd | nd |
| Stdev | 213 | 95.1 | 213 | 88.7 | nd | nd |
| p(t-test) | | 0.19 | | 0.30 | nd | nd |
| Min | 2.68 | 71.1 | 2.68 | 71.1 | nd | nd |
| Max | 1900 | 339 | 1900 | 339 | nd | nd |
| n (Samp) | 89 | 7 | 89 | 7 | nd | nd |
| n (Patient) | 89 | 7 | 89 | 7 | nd | nd |

| | 0hr prior to AKI stage | | 24hr prior to AKI stage | | 48hr prior to AKI stage |
|---|---|---|---|---|---|
| | sCr or UO | UO only | sCr or UO | sCr or UO | UO only |
| AUC | 0.88 | 0.90 | 0.87 | 0.90 | 0.83 |
| SE | 0.069 | 0.078 | 0.070 | 0.080 | 0.098 |
| p | 6.1E-8 | 2.8E-7 | 1.3E-7 | 8.7E-7 | 7.5E-4 |
| nCohort 1 | 88 | 89 | 88 | 89 | 88 |
| nCohort 2 | 11 | 7 | 11 | 7 | 7 |
| Cutoff 1 | 55.0 | 127 | 55.0 | 127 | 51.6 |
| Sens 1 | 73% | 71% | 73% | 71% | 71% |
| Spec 1 | 82% | 91% | 82% | 91% | 81% |
| Cutoff 2 | 51.6 | 93.9 | 51.6 | 93.9 | 47.5 |
| Sens 2 | 82% | 86% | 82% | 86% | 86% |
| Spec 2 | 81% | 85% | 81% | 85% | 80% |
| Cutoff 3 | 47.5 | 69.3 | 47.5 | 69.3 | 33.7 |
| Sens 3 | 91% | 100% | 91% | 100% | 100% |
| Spec 3 | 80% | 76% | 80% | 76% | 66% |
| Cutoff 4 | 36.9 | 51.6 | 36.9 | 51.6 | 36.9 |
| Sens 4 | 91% | 100% | 91% | 100% | 86% |
| Spec 4 | 70% | 71% | 70% | 71% | 70% |
| Cutoff 5 | 51.6 | 83.6 | 51.6 | 83.6 | 51.6 |
| Sens 5 | 82% | 86% | 82% | 86% | 71% |
| Spec 5 | 81% | 81% | 81% | 81% | 81% |
| Cutoff 6 | 93.9 | 127 | 93.9 | 127 | 93.9 |
| Sens 6 | 55% | 71% | 55% | 71% | 29% |
| Spec 6 | 91% | 91% | 91% | 91% | 91% |
| OR Quart 2 | >0 | >0 | >0 | >0 | >0 |
| | <na | <na | <na | <na | <na |

(continued)

|  | 0hr prior to AKI stage | | 24hr prior to AKI stage | | 48hr prior to AKI stage |
|---|---|---|---|---|---|
|  | sCr or UO | UO only | sCr or UO | sCr or UO | UO only |
| p Value 95% CI of OR Quart2 | >na | >na | >na | >na | >na |
|  | na | na | na | na | na |
| OR Quart 3 | >3.3 | >1.0 | >3.3 | >1.0 | >1.0 |
|  | <0.32 | <0.98 | <0.32 | <0.98 | <1.0 |
| p Value 95% CI of OR Quart3 | >0.32 | >0.062 | >0.32 | >0.062 | >0.059 |
|  | na | na | na | na | na |
| OR Quart 4 | >11 | >8.0 | >11 | >8.0 | >7.7 |
|  | <0.029 | <0.064 | <0.029 | <0.064 | <0.070 |
| p Value 95% CI of OR Quart4 | >1.3 | >0.88 | >1.3 | >0.88 | >0.85 |
|  | na | na | na | na | na |

**Angiopoietin-related protein 6**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 65.2 | 95.0 | 65.2 | 85.1 | 65.2 | 96.4 |
| Average | 76.4 | 104 | 76.4 | 98.6 | 76.4 | 122 |
| Stdev | 50.5 | 69.2 | 50.5 | 70.8 | 50.5 | 80.1 |
| p(t-test) |  | 0.10 |  | 0.19 |  | 0.030 |
| Min | 7.54 | 21.5 | 7.54 | 21.5 | 7.54 | 21.5 |
| Max | 240 | 250 | 240 | 250 | 240 | 250 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
|  | Cohort 1 | Cohort 2 | Cohort 1 | Cohort 2 |
| Median | 75.7 | 82.6 | 75.7 | 51.5 |
| Average | 87.4 | 88.8 | 87.4 | 79.7 |
| Stdev | 57.4 | 63.1 | 57.4 | 63.2 |
| p(t-test) |  | 0.95 |  | 0.74 |
| Min | 7.54 | 21.5 | 7.54 | 21.5 |
| Max | 283 | 211 | 283 | 211 |
| n (Samp) | 89 | 7 | 89 | 7 |
| n (Patient) | 89 | 7 | 89 | 7 |

|  | 0hr prior to AKI stage | | 24hr prior to AKI stage | | 48hr prior to AKI stage |
|  | sCr or UO | UO only | sCr or UO | UO only | sCr or UO |
|---|---|---|---|---|---|
| AUC | 0.64 | 0.51 | 0.60 | 0.44 | 0.71 |
| SE | 0.094 | 0.11 | 0.095 | 0.12 | 0.11 |
| p | 0.13 | 0.92 | 0.29 | 0.61 | 0.065 |
| nCohort 1 | 88 | 89 | 88 | 89 | 88 |
| nCohort 2 | 11 | 7 | 11 | 7 | 7 |
| Cutoff 1 | 69.6 | 48.9 | 50.4 | 48.9 | 92.9 |
| Sens 1 | 73% | 71% | 73% | 71% | 71% |
| Spec 1 | 55% | 29% | 35% | 29% | 77% |
| Cutoff 2 | 49.9 | 42.2 | 49.9 | 42.2 | 69.6 |
| Sens 2 | 82% | 86% | 82% | 86% | 86% |
| Spec 2 | 35% | 22% | 35% | 22% | 55% |
| Cutoff 3 | 42.2 | 18.9 | 42.2 | 18.9 | 18.9 |
| Sens 3 | 91% | 100% | 91% | 100% | 100% |
| Spec 3 | 27% | 3% | 27% | 3% | 6% |
| Cutoff 4 | 89.6 | 92.9 | 89.6 | 92.9 | 89.6 |
| Sens 4 | 55% | 43% | 45% | 29% | 71% |
| Spec 4 | 70% | 71% | 70% | 71% | 70% |
| Cutoff 5 | 96.5 | 117 | 96.5 | 117 | 96.5 |
| Sens 5 | 36% | 14% | 27% | 14% | 43% |
| Spec 5 | 81% | 81% | 81% | 81% | 81% |
| Cutoff 6 | 142 | 186 | 142 | 186 | 142 |
| Sens 6 | 18% | 14% | 18% | 14% | 29% |
| Spec 6 | 91% | 91% | 91% | 91% | 91% |
| OR Quart 2 | 2.0 | 0.48 | 3.1 | 2.1 | 0 |
|  | 0.58 | 0.56 | 0.34 | 0.56 | na |
| p Value | 0.17 | 0.040 | 0.30 | 0.18 | na |
| 95% CI of OR Quart2 | 24 | 5.7 | 32 | 25 | na |
| OR Quart 3 | 3.1 | 1.0 | 2.0 | 2.1 | 2.0 |
|  | 0.34 | 1.0 | 0.58 | 0.56 | 0.58 |
| p Value 95% CI of OR Quart3 | 0.30 | 0.13 | 0.17 | 0.18 | 0.17 |
|  | 32 | 7.7 | 24 | 25 | 24 |
| OR Quart 4 | 5.8 | 1.0 | 5.8 | 2.1 | 4.4 |
|  | 0.12 | 1.0 | 0.12 | 0.56 | 0.20 |
| p Value 95% CI of OR Quart4 | 0.62 | 0.13 | 0.62 | 0.18 | 0.45 |
|  | 53 | 7.7 | 53 | 25 | 43 |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22.9 | 37.9 | 22.9 | 37.9 | 22.9 | 26.0 |
| Average | 28.0 | 51.7 | 28.0 | 49.9 | 28.0 | 46.3 |
| Stdev | 20.9 | 42.8 | 20.9 | 41.2 | 20.9 | 52.2 |
| p(t-test) | | 0.0028 | | 0.0051 | | 0.058 |
| Min | 0.672 | 16.1 | 0.672 | 16.1 | 0.672 | 16.1 |
| Max | 121 | 162 | 121 | 162 | 121 | 162 |
| n (Samp) | 87 | 11 | 87 | 11 | 87 | 7 |
| n (Patient) | 87 | 11 | 87 | 11 | 87 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 24.2 | 47.0 | 24.2 | 47.0 |
| Average | 31.0 | 65.5 | 31.0 | 62.6 |
| Stdev | 27.1 | 48.3 | 27.1 | 47.0 |
| p(t-test) | | 0.0030 | | 0.0062 |
| Min | 0.672 | 25.1 | 0.672 | 25.1 |
| Max | 198 | 162 | 198 | 162 |
| n (Samp) | 88 | 7 | 88 | 7 |
| n (Patient) | 88 | 7 | 88 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.73 | 0.82 | 0.73 | 0.81 | 0.63 |
| SE | 0.090 | 0.100 | 0.090 | 0.10 | 0.12 |
| p | 0.0095 | 0.0015 | 0.010 | 0.0018 | 0.27 |
| nCohort 1 | 87 | 88 | 87 | 88 | 87 |
| nCohort 2 | 11 | 7 | 11 | 7 | 7 |
| Cutoff 1 | 25.5 | 37.0 | 25.5 | 37.0 | 24.8 |
| Sens 1 | 73% | 71% | 73% | 71% | 71% |
| Spec 1 | 59% | 75% | 59% | 75% | 57% |
| Cutoff 2 | 24.8 | 32.6 | 24.8 | 32.6 | 18.3 |
| Sens 2 | 82% | 86% | 82% | 86% | 86% |
| Spec 2 | 57% | 68% | 57% | 68% | 43% |

(continued)

|  | 0hr prior to AKI stage | | 24hr prior to AKI stage | | 48hr prior to AKI stage |
|  | sCr or UO | UO only | sCr or UO | UO only | sCr or UO |
|---|---|---|---|---|---|
| Cutoff 3 | 18.3 | 24.8 | 18.3 | 24.8 | 16.0 |
| Sens 3 | 91% | 100% | 91% | 100% | 100% |
| Spec 3 | 43% | 53% | 43% | 53% | 33% |
| Cutoff 4 | 32.2 | 34.0 | 32.2 | 34.0 | 32.2 |
| Sens 4 | 64% | 71% | 64% | 71% | 29% |
| Spec 4 | 70% | 70% | 70% | 70% | 70% |
| Cutoff 5 | 41.4 | 42.2 | 41.4 | 42.2 | 41.4 |
| Sens 5 | 45% | 57% | 45% | 57% | 29% |
| Spec 5 | 80% | 82% | 80% | 82% | 80% |
| Cutoff 6 | 55.7 | 56.8 | 55.7 | 56.8 | 55.7 |
| Sens 6 | 27% | 43% | 27% | 43% | 14% |
| Spec 6 | 91% | 91% | 91% | 91% | 91% |
| OR Quart 2 | >2.1 | >0 | >2.1 | >0 | >2.1 |
|  | <0.56 | <na | <0.56 | <na | <0.56 |
| p Value 95% CI of OR Quart2 | >0.18 | >na | >0.18 | >na | >0.18 |
|  | na | na | na | na | na |
| OR Quart 3 | >3.4 | >3.3 | >3.4 | >3.3 | >3.4 |
|  | <0.30 | <0.32 | <0.30 | <0.32 | <0.30 |
| p Value 95% CI of OR Quart3 | >0.33 | >0.32 | >0.33 | >0.32 | >0.33 |
|  | na | na | na | na | na |
| OR Quart 4 | >7.6 | >4.6 | >7.6 | >4.6 | >2.1 |
|  | <0.071 | <0.19 | <0.071 | <0.19 | <0.56 |
| p Value 95% CI of OR Quart4 | >0.84 | >0.47 | >0.84 | >0.47 | >0.18 |
|  | na | na | na | na | na |

**Betacellulin**

| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
|---|---|---|---|---|---|---|
| Median | 4.68 | 4.10 | 4.68 | 4.10 | 4.68 | 5.12 |
| Average | 5.23 | 11.0 | 5.23 | 11.0 | 5.23 | 15.1 |
| Stdev | 3.00 | 15.7 | 3.00 | 15.7 | 3.00 | 18.7 |
| p(t-test) |  | 0.0025 |  | 0.0025 |  | 2.2E-5 |
| Min | 0.0147 | 0.00289 | 0.0147 | 0.00289 | 0.0147 | 2.42 |
| Max | 15.0 | 50.2 | 15.0 | 50.2 | 15.0 | 50.2 |
| n (Samp) | 87 | 11 | 87 | 11 | 87 | 7 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 87 | 11 | 87 | 11 | 87 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 4.80 | 3.40 | 4.80 | 3.40 |
| Average | 5.62 | 7.76 | 5.62 | 7.76 |
| Stdev | 6.59 | 11.1 | 6.59 | 11.1 |
| p(t-test) | | 0.44 | | 0.44 |
| Min | 0.00274 | 0.00289 | 0.00274 | 0.00289 |
| Max | 60.7 | 32.2 | 60.7 | 32.2 |
| n (Samp) | 88 | 7 | 88 | 7 |
| n (Patient) | 88 | 7 | 88 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.51 | 0.43 | 0.51 | 0.43 | 0.60 |
| SE | 0.093 | 0.12 | 0.093 | 0.12 | 0.12 |
| p | 0.94 | 0.52 | 0.94 | 0.52 | 0.39 |
| nCohort 1 | 87 | 88 | 87 | 88 | 87 |
| nCohort 2 | 11 | 7 | 11 | 7 | 7 |
| Cutoff 1 | 3.32 | 3.09 | 3.32 | 3.09 | 4.08 |
| Sens 1 | 73% | 71% | 73% | 71% | 71% |
| Spec 1 | 24% | 24% | 24% | 24% | 39% |
| Cutoff 2 | 3.09 | 1.92 | 3.09 | 1.92 | 3.40 |
| Sens 2 | 82% | 86% | 82% | 86% | 86% |
| Spec 2 | 22% | 15% | 22% | 15% | 25% |
| Cutoff 3 | 1.92 | 0.00282 | 1.92 | 0.00282 | 1.92 |
| Sens 3 | 91% | 100% | 91% | 100% | 100% |
| Spec 3 | 11% | 2% | 11% | 2% | 11% |
| Cutoff 4 | 6.11 | 6.11 | 6.11 | 6.11 | 6.11 |
| Sens 4 | 36% | 29% | 36% | 29% | 43% |
| Spec 4 | 70% | 70% | 70% | 70% | 70% |
| Cutoff 5 | 7.64 | 7.64 | 7.64 | 7.64 | 7.64 |
| Sens 5 | 36% | 29% | 36% | 29% | 43% |
| Spec 5 | 80% | 81% | 80% | 81% | 80% |
| Cutoff 6 | 8.88 | 8.84 | 8.88 | 8.84 | 8.88 |

(continued)

|  | 0hr prior to AKI stage | | 24hr prior to AKI stage | | 48hr prior to AKI stage |
|---|---|---|---|---|---|
|  | sCr or UO | UO only | sCr or UO | UO only | sCr or UO |
| Sens 6 | 27% | 29% | 27% | 29% | 29% |
| Spec 6 | 91% | 91% | 91% | 91% | 91% |
| OR Quart 2 | 0.95 | 0 | 0.95 | 0 | 2.0 |
|  | 0.96 | na | 0.96 | na | 0.58 |
| p Value 95% CI of OR Quart2 | 0.17 | na | 0.17 | na | 0.17 |
|  | 5.3 | na | 5.3 | na | 24 |
| OR Quart 3 | 0.30 | 1.6 | 0.30 | 1.6 | 1.0 |
|  | 0.32 | 0.64 | 0.32 | 0.64 | 1.0 |
| p Value 95% CI of OR Quart3 | 0.029 | 0.24 | 0.029 | 0.24 | 0.059 |
|  | 3.2 | 10 | 3.2 | 10 | 17 |
| OR Quart 4 | 1.3 | 1.0 | 1.3 | 1.0 | 3.1 |
|  | 0.73 | 0.96 | 0.73 | 0.96 | 0.34 |
| p Value 95% CI of OR Quart4 | 0.27 | 0.13 | 0.27 | 0.13 | 0.30 |
|  | 6.7 | 8.1 | 6.7 | 8.1 | 33 |

**Endostatin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 38800 | 65700 | 38800 | 65700 | 38800 | 58900 |
| Average | 49600 | 90000 | 49600 | 89000 | 49600 | 96500 |
| Stdev | 39800 | 75300 | 39800 | 75500 | 39800 | 91900 |
| p(t-test) |  | 0.0058 |  | 0.0071 |  | 0.0094 |
| Min | 10500 | 14300 | 10500 | 14300 | 10500 | 14300 |
| Max | 328000 | 275000 | 328000 | 275000 | 328000 | 275000 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
|  | Cohort 1 | Cohort 2 | Cohort 1 | Cohort 2 |
| Median | 44700 | 83700 | 44700 | 73100 |
| Average | 56000 | 110000 | 56000 | 108000 |
| Stdev | 43600 | 89900 | 43600 | 90500 |
| p(t-test) |  | 0.0053 |  | 0.0067 |
| Min | 10500 | 14300 | 10500 | 14300 |

(continued)

| UO only | 0hr prior to AKI stage | | 24hr prior to AKI stage | |
|---|---|---|---|---|
| | Cohort 1 | Cohort 2 | Cohort 1 | Cohort 2 |
| Max | 328000 | 275000 | 328000 | 275000 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.70 | 0.69 | 0.69 | 0.68 | 0.68 |
| SE | 0.092 | 0.11 | 0.092 | 0.12 | 0.12 |
| p | 0.030 | 0.094 | 0.036 | 0.11 | 0.13 |
| nCohort 1 | 88 | 89 | 88 | 89 | 88 |
| nCohort 2 | 11 | 7 | 11 | 7 | 7 |
| Cutoff 1 | 51400 | 65300 | 51400 | 65300 | 51400 |
| Sens 1 | 73% | 71% | 73% | 71% | 71% |
| Spec 1 | 68% | 73% | 68% | 73% | 68% |
| Cutoff 2 | 37100 | 32400 | 37100 | 32400 | 37100 |
| Sens 2 | 82% | 86% | 82% | 86% | 86% |
| Spec 2 | 47% | 28% | 47% | 28% | 47% |
| Cutoff 3 | 32400 | 13300 | 32400 | 13300 | 13300 |
| Sens 3 | 91% | 100% | 91% | 100% | 100% |
| Spec 3 | 33% | 4% | 33% | 4% | 6% |
| Cutoff 4 | 54400 | 62700 | 54400 | 62700 | 54400 |
| Sens 4 | 64% | 71% | 64% | 71% | 57% |
| Spec 4 | 70% | 71% | 70% | 71% | 70% |
| Cutoff 5 | 67500 | 75500 | 67500 | 75500 | 67500 |
| Sens 5 | 45% | 57% | 45% | 43% | 43% |
| Spec 5 | 81% | 81% | 81% | 81% | 81% |
| Cutoff 6 | 84900 | 112000 | 84900 | 112000 | 84900 |
| Sens 6 | 27% | 43% | 27% | 43% | 29% |
| Spec 6 | 91% | 91% | 91% | 91% | 91% |
| OR Quart 2 | 2.0 | 1.0 | 2.0 | 1.0 | 0.96 |
| | 0.58 | 1.0 | 0.58 | 1.0 | 0.98 |
| p Value 95% CI of OR Quart2 | 0.17 | 0.059 | 0.17 | 0.059 | 0.056 |
| | 24 | 17 | 24 | 17 | 16 |
| OR Quart 3 | 3.1 | 1.0 | 3.1 | 1.0 | 2.0 |
| | 0.34 | 1.0 | 0.34 | 1.0 | 0.58 |

(continued)

|  | 0hr prior to AKI stage | | 24hr prior to AKI stage | | 48hr prior to AKI stage |
|---|---|---|---|---|---|
|  | sCr or UO | UO only | sCr or UO | UO only | sCr or UO |
| p Value 95% CI of OR Quart3 | 0.30 | 0.059 | 0.30 | 0.059 | 0.17 |
|  | 32 | 17 | 32 | 17 | 24 |
| OR Quart 4 | 5.8 | 4.6 | 5.8 | 4.6 | 3.1 |
|  | 0.12 | 0.19 | 0.12 | 0.19 | 0.34 |
| p Value | 0.62 | 0.47 | 0.62 | 0.47 | 0.30 |
| 95% CI of OR Quart4 | 53 | 45 | 53 | 45 | 33 |

**Proepiregulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 0.416 | 1.00 | 0.416 | 0.626 | 0.416 | 0.561 |
| Average | 0.599 | 3.17 | 0.599 | 1.67 | 0.599 | 0.714 |
| Stdev | 0.808 | 5.62 | 0.808 | 3.24 | 0.808 | 0.508 |
| p(t-test) |  | 9.6E-5 |  | 0.011 |  | 0.71 |
| Min | 0.000896 | 0.0249 | 0.000896 | 0.0249 | 0.000896 | 0.188 |
| Max | 6.96 | 17.1 | 6.96 | 11.3 | 6.96 | 1.47 |
| n (Samp) | 87 | 11 | 87 | 11 | 87 | 7 |
| n (Patient) | 87 | 11 | 87 | 11 | 87 | 7 |

| UO only | 0hr prior to AKI stage | | 24hr prior to AKI stage | |
|---|---|---|---|---|
|  | Cohort 1 | Cohort 2 | Cohort 1 | Cohort 2 |
| Median | 0.472 | 1.19 | 0.472 | 0.626 |
| Average | 1.04 | 4.50 | 1.04 | 2.15 |
| Stdev | 3.51 | 6.85 | 3.51 | 4.07 |
| p(t-test) |  | 0.023 |  | 0.43 |
| Min | 0.000896 | 0.0249 | 0.000896 | 0.0249 |
| Max | 32.1 | 17.1 | 32.1 | 11.3 |
| n (Samp) | 88 | 7 | 88 | 7 |
| n (Patient) | 88 | 7 | 88 | 7 |

|  | 0hr prior to AKI stage | | 24hr prior to AKI stage | | 48hr prior to AKI stage |
|---|---|---|---|---|---|
|  | sCr or UO | UO only | sCr or UO | UO only | sCr or UO |
| AUC | 0.67 | 0.64 | 0.64 | 0.59 | 0.61 |
| SE | 0.094 | 0.12 | 0.095 | 0.12 | 0.12 |

(continued)

|  | 0hr prior to AKI stage | | 24hr prior to AKI stage | | 48hr prior to AKI stage |
|---|---|---|---|---|---|
|  | sCr or UO | UO only | sCr or UO | UO only | sCr or UO |
| p | 0.067 | 0.23 | 0.15 | 0.45 | 0.36 |
| nCohort 1 | 87 | 88 | 87 | 88 | 87 |
| nCohort 2 | 11 | 7 | 11 | 7 | 7 |
| Cutoff 1 Sens 1 Spec 1 | 0.356 | 0.356 | 0.356 | 0.356 | 0.286 |
|  | 73% | 71% | 73% | 71% | 71% |
|  | 43% | 38% | 43% | 38% | 31% |
| Cutoff 2 Sens 2 Spec 2 | 0.261 | 0.179 | 0.261 | 0.179 | 0.261 |
|  | 82% | 86% | 82% | 86% | 86% |
|  | 31% | 20% | 31% | 20% | 31% |
| Cutoff 3 | 0.179 | 0.0201 | 0.179 | 0.0201 | 0.179 |
| Sens 3 | 91% | 100% | 91% | 100% | 100% |
| Spec 3 | 23% | 5% | 23% | 5% | 23% |
| Cutoff 4 | 0.695 | 0.717 | 0.695 | 0.717 | 0.695 |
| Sens 4 | 55% | 57% | 45% | 43% | 43% |
| Spec 4 | 71% | 70% | 71% | 70% | 71% |
| Cutoff 5 | 0.848 | 0.893 | 0.848 | 0.893 | 0.848 |
| Sens 5 | 55% | 57% | 45% | 43% | 43% |
| Spec 5 | 82% | 81% | 82% | 81% | 82% |
| Cutoff 6 | 1.00 | 1.07 | 1.00 | 1.07 | 1.00 |
| Sens 6 | 55% | 57% | 45% | 43% | 43% |
| Spec 6 | 91% | 91% | 91% | 91% | 91% |
| OR Quart 2 | 0.96 | 0.46 | 0.96 | 0.46 | 2.0 |
|  | 0.97 | 0.53 | 0.97 | 0.53 | 0.58 |
| p Value 95% CI of OR Quart2 | 0.12 | 0.039 | 0.12 | 0.039 | 0.17 |
|  | 7.4 | 5.4 | 7.4 | 5.4 | 24 |
| OR Quart 3 | 0.48 | 0 | 1.0 | 0.46 | 1.0 |
|  | 0.56 | na | 1.0 | 0.53 | 1.0 |
| p Value 95% CI of: OR Quart3 | 0.040 | na | 0.13 | 0.039 | 0.059 |
|  | 5.7 | na | 7.7 | 5.4 | 17 |
| OR Quart 4 | 3.5 | 2.1 | 2.8 | 1.5 | 3.1 |
|  | 0.15 | 0.42 | 0.26 | 0.67 | 0.34 |
| p Value 95% Clot OR Quart4 | 0.63 | 0.35 | 0.48 | 0.23 | 0.30 |
|  | 19 | 13 | 16 | 9.9 | 33 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.197 | 0.274 | 0.197 | 0.274 | 0.197 | 0.274 |
| Average | 0.208 | 0.379 | 0.208 | 0.379 | 0.208 | 0.275 |
| Stdev | 0.188 | 0.380 | 0.188 | 0.381 | 0.188 | 0.167 |
| p(t-test) | | 0.015 | | 0.015 | | 0.36 |
| Min | 8.23E-5 | 0.000127 | 8.23E-5 | 0.000127 | 8.23E-5 | 0.000127 |
| Max | 0.948 | 1.32 | 0.948 | 1.32 | 0.948 | 0.482 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 0.213 | 0.385 | 0.213 | 0.385 |
| Average | 0.255 | 0.433 | 0.255 | 0.433 |
| Stdev | 0.272 | 0.470 | 0.272 | 0.471 |
| p(t-test) | | 0.12 | | 0.12 |
| Min | 8.23E-5 | 0.000127 | 8.23E-5 | 0.000127 |
| Max | 2.09 | 1.32 | 2.09 | 1.32 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.66 | 0.60 | 0.66 | 0.60 | 0.65 |
| SE | 0.094 | 0.12 | 0.094 | 0.12 | 0.12 |
| p | 0.099 | 0.41 | 0.099 | 0.41 | 0.19 |
| nCohort 1 | 88 | 89 | 88 | 89 | 88 |
| nCohort 2 | 11 | 7 | 11 | 7 | 7 |
| Cutoff 1 | 0.125 | 0.124 | 0.125 | 0.124 | 0.260 |
| Sens 1 | 73% | 71% | 73% | 71% | 71% |
| Spec 1 | 42% | 31% | 42% | 31% | 70% |
| Cutoff 2 | 0.124 | 0.0430 | 0.124 | 0.0430 | 0.124 |
| Sens 2 | 82% | 86% | 82% | 86% | 86% |
| Spec 2 | 42% | 19% | 42% | 19% | 42% |
| Cutoff 3 | 0.0421 | 8.23E-5 | 0.0421 | 8.23E-5 | 8.23E-5 |
| Sens 3 | 91% | 100% | 91% | 100% | 100% |
| Spec 3 | 24% | 2% | 24% | 2% | 7% |

(continued)

| | 0hr prior to AKI stage | | 24hr prior to AKI stage | | 48hr prior to AKI stage |
| --- | --- | --- | --- | --- | --- |
| | sCr or UO | UO only | sCr or UO | UO only | sCr or UO |
| Cutoff 4 | 0.260 | 0.285 | 0.260 | 0.285 | 0.260 |
| Sens 4 | 64% | 57% | 64% | 57% | 71% |
| Spec 4 | 70% | 71% | 70% | 71% | 70% |
| Cutoff 5 | 0.321 | 0.368 | 0.321 | 0.368 | 0.321 |
| Sens 5 | 45% | 57% | 45% | 57% | 43% |
| Spec 5 | 81% | 81% | 81% | 81% | 81% |
| Cutoff 6 | 0.485 | 0.508 | 0.485 | 0.508 | 0.485 |
| Sens 6 | 18% | 29% | 18% | 29% | 0% |
| Spec 6 | 91% | 91% | 91% | 91% | 91% |
| OR Quart 2 | 0.96 | 0.48 | 0.96 | 0.48 | 0.96 |
| | 0.97 | 0.56 | 0.97 | 0.56 | 0.98 |
| p Value 95% CI of OR Quart2 | 0.12 | 0.040 | 0.12 | 0.040 | 0.056 |
| | 7.4 | 5.7 | 7.4 | 5.7 | 16 |
| OR Quart 3 | 0.96 | 0 | 0.96 | 0 | 2.0 |
| | 0.97 | na | 0.97 | na | 0.58 |
| p Value | 0.12 | na | 0.12 | na | 0.17 |
| 95% CI of OR Quart3 | 7.4 | na | 7.4 | na | 24 |
| OR Quart 4 | 2.8 | 2.2 | 2.8 | 2.2 | 3.1 |
| | 0.26 | 0.39 | 0.26 | 0.39 | 0.34 |
| p Value | 0.48 | 0.36 | 0.48 | 0.36 | 0.30 |
| 95% CI of OR Quart4 | 16 | 13 | 16 | 13 | 33 |

**Fibroblast growth factor 21**

| sCr or UO | 0hr prior to AKI stage | | 24hr prior to AKI stage | | 48hr prior to AKI stage | |
| --- | --- | --- | --- | --- | --- | --- |
| | Cohort 1 | Cohort 2 | Cohort 1 | Cohort 2 | Cohort 1 | Cohort 2 |
| Median | 0.0451 | 0.418 | 0.0451 | 0.311 | 0.0451 | 0.295 |
| Average | 0.230 | 0.798 | 0.230 | 0.647 | 0.230 | 0.670 |
| Stdev | 0.539 | 0.794 | 0.539 | 0.655 | 0.539 | 0.799 |
| p(t-test) | | 0.0025 | | 0.020 | | 0.048 |
| Min | 9.65E-6 | 0.0658 | 9.65E-6 | 0.0658 | 9.65E-6 | 0.0658 |
| Max | 3.21 | 2.26 | 3.21 | 2.11 | 3.21 | 2.11 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 0.0674 | 0.605 | 0.0674 | 0.418 |
| Average | 0.284 | 0.955 | 0.284 | 0.718 |
| Stdev | 0.564 | 0.869 | 0.564 | 0.694 |
| p(t-test) | | 0.0046 | | 0.057 |
| Min | 9.65E-6 | 0.159 | 9.65E-6 | 0.159 |
| Max | 3.21 | 2.26 | 3.21 | 2.11 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.87 | 0.87 | 0.86 | 0.84 | 0.84 |
| SE | 0.071 | 0.087 | 0.073 | 0.095 | 0.095 |
| p | 1.9E-7 | 1.8E-5 | 1.2E-6 | 3.3E-4 | 3.0E-4 |
| nCohort 1 | 88 | 89 | 88 | 89 | 88 |
| nCohort 2 | 11 | 7 | 11 | 7 | 7 |
| Cutoff 1 | 0.277 | 0.387 | 0.198 | 0.281 | 0.194 |
| Sens 1 | 73% | 71% | 73% | 71% | 71% |
| Spec 1 | 86% | 83% | 83% | 82% | 83% |
| Cutoff 2 | 0.186 | 0.281 | 0.186 | 0.186 | 0.186 |
| Sens 2 | 82% | 86% | 82% | 86% | 86% |
| Spec 2 | 83% | 82% | 83% | 74% | 83% |
| Cutoff 3 | 0.143 | 0.143 | 0.143 | 0.143 | 0.0619 |
| Sens 3 | 91% | 100% | 91% | 100% | 100% |
| Spec 3 | 81% | 72% | 81% | 72% | 59% |
| Cutoff 4 | 0.0949 | 0.134 | 0.0949 | 0.134 | 0.0949 |
| Sens 4 | 91% | 100% | 91% | 100% | 86% |
| Spec 4 | 70% | 71% | 70% | 71% | 70% |
| Cutoff 5 | 0.143 | 0.267 | 0.143 | 0.267 | 0.143 |
| Sens 5 | 91% | 86% | 91% | 71% | 86% |
| Spec 5 | 81% | 81% | 81% | 81% | 81% |
| Cutoff 6 | 0.687 | 1.23 | 0.687 | 1.23 | 0.687 |
| Sens 6 | 36% | 29% | 36% | 14% | 29% |
| Spec 6 | 91% | 91% | 91% | 91% | 91% |
| OR Quart | >0 | >0 | >0 | >0 | >0 |
| 2 | <na | <na | <na | <na | <na |

(continued)

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| p Value | >na | >na | >na | >na | >na |
| 95% CI of OR Quart2 | na | na | na | na | na |
| OR Quart | >2.1 | >1.0 | >2.1 | >2.2 | >1.0 |
| 3 | <0.56 | <0.98 | <0.56 | <0.54 | <1.0 |
| p Value | >0.18 | >0.062 | >0.18 | >0.18 | >0.059 |
| 95% CI of OR Quart3 | na | na | na | na | na |
| OR Quart | >14 | >8.0 | >14 | >6.3 | >7.7 |
| 4 | <0.018 | <0.064 | <0.018 | <0.11 | <0.070 |
| p Value | >1.6 | >0.88 | >1.6 | >0.68 | >0.85 |
| 95% CI of OR Quart4 | na | na | na | na | na |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 17.3 | 36.4 | 17.3 | 31.2 | 17.3 | 22.6 |
| Average | 20.5 | 62.5 | 20.5 | 57.2 | 20.5 | 62.8 |
| Stdev | 13.5 | 61.2 | 13.5 | 61.5 | 13.5 | 77.9 |
| p(t-test) | 2.2E-7 | 2.2E-7 | | 4.7E-6 | | 1.8E-5 |
| Min | 7.16 | 11.6 | 7.16 | 11.6 | 7.16 | 11.6 |
| Max | 98.9 | 222 | 98.9 | 222 | 98.9 | 222 |
| n (Samp) | 87 | 11 | 87 | 11 | 87 | 7 |
| n (Patient) | 87 | 11 | 87 | 11 | 87 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 18.6 | 44.7 | 18.6 | 36.4 |
| Average | 23.6 | 58.7 | 23.6 | 50.3 |
| Stdev | 22.4 | 31.2 | 22.4 | 30.7 |
| p(t-test) | | 2.0E-4 | | 0.0039 |
| Min | 7.16 | 29.4 | 7.16 | 26.9 |
| Max | 186 | 110 | 186 | 110 |
| n (Samp) | 88 | 7 | 88 | 7 |
| n (Patient) | 88 | 7 | 88 | 7 |

|  | 0hr prior to AKI stage | | 24hr prior to AKI stage | | 48hr prior to AKI stage |
|---|---|---|---|---|---|
|  | sCr or UO | UO only | sCr or UO | UO only | sCr or UO |
| AUC | 0.84 | 0.91 | 0.83 | 0.88 | 0.75 |
| SE | 0.076 | 0.075 | 0.079 | 0.085 | 0.11 |
| p | 8.6E-6 | 4.9E-8 | 3.4E-5 | 6.8E-6 | 0.025 |
| nCohort 1 | 87 | 88 | 87 | 88 | 87 |
| nCohort 2 | 11 | 7 | 11 | 7 | 7 |
| Cutoff 1 | 29.0 | 33.3 | 26.6 | 31.2 | 21.2 |
| Sens 1 | 73% | 71% | 73% | 71% | 71% |
| Spec 1 | 87% | 86% | 84% | 84% | 71% |
| Cutoff 2 | 21.8 | 31.2 | 21.8 | 29.0 | 20.9 |
| Sens 2 | 82% | 86% | 82% | 86% | 86% |
| Spec 2 | 74% | 84% | 74% | 81% | 69% |
| Cutoff 3 | 20.9 | 29.0 | 20.9 | 25.3 | 11.5 |
| Sens 3 | 91% | 100% | 91% | 100% | 100% |
| Spec 3 | 69% | 81% | 69% | 77% | 18% |
| Cutoff 4 | 21.1 | 21.8 | 21.1 | 21.8 | 21.1 |
| Sens 4 | 82% | 100% | 82% | 100% | 71% |
| Spec 4 | 70% | 70% | 70% | 70% | 70% |
| Cutoff 5 | 24.6 | 29.0 | 24.6 | 29.0 | 24.6 |
| Sens 5 | 73% | 100% | 73% | 86% | 43% |
| Spec 5 | 80% | 81% | 80% | 81% | 80% |
| Cutoff 6 | 31.2 | 40.1 | 31.2 | 40.1 | 31.2 |
| Sens 6 | 64% | 57% | 55% | 43% | 43% |
| Spec 6 | 91% | 91% | 91% | 91% | 91% |
| OR Quart | 0 | >0 | 0 | >0 | 0 |
| 2 | na | <na | na | <na | na |
| p Value | na | >na | na | >na | na |
| 95% CI of OR Quart2 | na | na | na | na | na |
| OR Quart | 2.1 | >0 | 2.1 | >1.0 | 3.3 |
| 3 | 0.56 | <na | 0.56 | <1.0 | 0.32 |
| p Value | 0.18 | >na | 0.18 | >0.059 | 0.32 |
| 95% CI of OR Quart3 | 25 | na | 25 | na | 34 |
| OR Quart | 11 | >9.5 | 11 | >7.7 | 3.1 |
| 4 | 0.032 | <0.044 | 0.032 | <0.070 | 0.34 |
| p Value | 1.2 | >1.1 | 1.2 | >0.85 | 0.30 |
| 95% CI of OR Quart4 | 95 | na | 95 | na | 33 |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 40300 | 95600 | 40300 | 95600 | 40300 | 72500 |
| Average | 68700 | 147000 | 68700 | 134000 | 68700 | 111000 |
| Stdev | 81100 | 159000 | 81100 | 143000 | 81100 | 146000 |
| p(t-test) | | 0.0093 | | 0.024 | | 0.22 |
| Min | 1030 | 15000 | 1030 | 14100 | 1030 | 15000 |
| Max | 591000 | 455000 | 591000 | 433000 | 591000 | 433000 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 50300 | 95800 | 50300 | 95800 |
| Average | 75600 | 199000 | 75600 | 179000 |
| Stdev | 82100 | 180000 | 82100 | 164000 |
| p(t-test) | | 9.2E-4 | | 0.0043 |
| Min | 2770 | 24000 | 2770 | 14100 |
| Max | 591000 | 455000 | 591000 | 433000 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.67 | 0.74 | 0.63 | 0.69 | 0.58 |
| SE | 0.094 | 0.11 | 0.095 | 0.11 | 0.12 |
| p | 0.067 | 0.034 | 0.16 | 0.10 | 0.49 |
| nCohort 1 | 88 | 89 | 88 | 89 | 88 |
| nCohort 2 | 11 | 7 | 11 | 7 | 7 |
| Cutoff 1 | 50600 | 91600 | 27700 | 91600 | 27700 |
| Sens 1 | 73% | 71% | 73% | 71% | 71% |
| Spec 1 | 60% | 69% | 35% | 69% | 35% |
| Cutoff 2 | 27700 | 50500 | 23400 | 23400 | 23400 |
| Sens 2 | 82% | 86% | 82% | 86% | 86% |
| Spec 2 | 35% | 52% | 32% | 31% | 32% |
| Cutoff 3 | 23400 | 23400 | 14100 | 14100 | 13900 |

(continued)

| | 0hr prior to AKI stage | | 24hr prior to AKI stage | | 48hr prior to AKI stage |
| | sCr or UO | UO only | sCr or UO | UO only | sCr or UO |
|---|---|---|---|---|---|
| Sens 3 | 91% | 100% | 91% | 100% | 100% |
| Spec 3 | 32% | 31% | 17% | 19% | 17% |
| Cutoff 4 | 77700 | 100000 | 77700 | 100000 | 77700 |
| Sens 4 | 55% | 43% | 55% | 43% | 43% |
| Spec 4 | 70% | 71% | 70% | 71% | 70% |
| Cutoff 5 | 110000 | 126000 | 110000 | 126000 | 110000 |
| Sens 5 | 36% | 43% | 36% | 43% | 29% |
| Spec 5 | 81% | 81% | 81% | 81% | 81% |
| Cutoff 6 | 159000 | 171 000 | 159000 | 171000 | 159000 |
| Sens 6 | 27% | 43% | 27% | 43% | 14% |
| Spec 6 | 91% | 91% | 91% | 91% | 91% |
| OR Quart 2 | 2.0 | >2.2 | 0.96 | 1.0 | 2.0 |
| | 0.58 | <0.54 | 0.97 | 1.0 | 0.58 |
| p Value 95% CI of OR Quart2 | 0.17 | >0.18 | 0.12 | 0.059 | 0.17 |
| | 24 | na | 7.4 | 17 | 24 |
| OR Quart 3 | 4.4 | >2.2 | 1.5 | 2.1 | 0.96 |
| | 0.20 | <0.54 | 0.67 | 0.56 | 0.98 |
| p Value 95% CI of OR Quart3 | 0.45 | >0.18 | 0.23 | 0.18 | 0.056 |
| | 42 | na | 9.9 | 25 | 16 |
| OR Quart 4 | 4.4 | >3.4 | 2.1 | 3.3 | 3.1 |
| | 0.20 | <0.30 | 0.42 | 0.32 | 0.34 |
| p Value 95% CI of OR Quart4 | 0.45 | >0.33 | 0.35 | 0.32 | 0.30 |
| | 42 | na | 13 | 34 | 33 |

**Tenascin**

| sCr or UO | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
|---|---|---|---|---|---|---|
| Median | 483 | 1240 | 483 | 1240 | 483 | 1180 |
| Average | 912 | 1670 | 912 | 1410 | 912 | 1150 |
| Stdev | 1550 | 1380 | 1550 | 584 | 1550 | 333 |
| p(t-test) | | 0.13 | | 0.30 | | 0.69 |
| Min | 94.2 | 815 | 94.2 | 815 | 94.2 | 815 |
| Max | 13100 | 5700 | 13100 | 2830 | 13100 | 1780 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 527 | 1260 | 527 | 1260 |
| Average | 1410 | 1960 | 1410 | 1550 |
| Stdev | 3250 | 1670 | 3250 | 632 |
| p(t-test) | | 0.66 | | 0.91 |
| Min | 63.0 | 959 | 63.0 | 959 |
| Max | 26100 | 5700 | 26100 | 2830 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.86 | 0.82 | 0.85 | 0.82 | 0.83 |
| SE | 0.074 | 0.099 | 0.074 | 0.100 | 0.098 |
| p | 1.4E-6 | 0.0010 | 2.3E-6 | 0.0015 | 8.7E-4 |
| nCohort 1 | 88 | 89 | 88 | 89 | 88 |
| nCohort 2 | 11 | 7 | 11 | 7 | 7 |
| Cutoff 1 | 1140 | 1220 | 1140 | 1220 | 866 |
| Sens 1 | 73% | 71% | 73% | 71% | 71% |
| Spec 1 | 85% | 80% | 85% | 80% | 80% |
| Cutoff 2 | 866 | 1200 | 866 | 1200 | 815 |
| Sens 2 | 82% | 86% | 82% | 86% | 86% |
| Spec 2 | 80% | 80% | 80% | 80% | 76% |
| Cutoff 3 | 815 | 905 | 815 | 905 | 792 |
| Sens 3 | 91% | 100% | 91% | 100% | 100% |
| Spec 3 | 76% | 72% | 76% | 72% | 76% |
| Cutoff 4 | 697 | 866 | 697 | 866 | 697 |
| Sens 4 | 100% | 100% | 100% | 100% | 100% |
| Spec 4 | 70% | 71% | 70% | 71% | 70% |
| Cutoff 5 | 969 | 1250 | 969 | 1250 | 969 |
| Sens 5 | 73% | 57% | 73% | 57% | 57% |
| Spec 5 | 81% | 81% | 81% | 81% | 81% |
| Cutoff 6 | 2080 | 2750 | 2080 | 2750 | 2080 |
| Sens 6 | 9% | 14% | 9% | 14% | 0% |
| Spec 6 | 91% | 91% | 91% | 91% | 91% |
| OR Quart 2 | >0 | >0 | >0 | >0 | >0 |
| | <na | <na | <na | <na | <na |

(continued)

|  | 0hr prior to AKI stage | | 24hr prior to AKI stage | | 48hr prior to AKI stage |
|---|---|---|---|---|---|
|  | sCr or UO | UO only | sCr or UO | UO only | sCr or UO |
| p Value 95% CI of OR Quart2 | >na | >na | >na | >na | >na |
|  | na | na | na | na | na |
| OR Quart 3 | >3.3 | >1.0 | >3.3 | >1.0 | >2.1 |
|  | <0.32 | <0.98 | <0.32 | <0.98 | <0.56 |
| p Value 95% CI ot OR Quart3 | >0.32 | >0.062 | >0.32 | >0.062 | >0.18 |
|  | na | na | na | na | na |
| OR Quart 4 | >11 | >8.0 | >11 | >8.0 | >6.1 |
|  | <0.029 | <0.064 | <0.029 | <0.064 | <0.11 |
| p Value 95% CI of OR Quart4 | >1.3 | >0.88 | >1.3 | >0.88 | >0.65 |
|  | na | na | na | na | na |

Fig. 9: Comparison of marker levels in urine samples collected from Cohort 1 (patients that did not progress beyond RIFLE stage 0, R, or I) and in urine samples collected from Cohort 2 (subjects who progress to RIFLE stage F) at 0, 24 hours, and 48 hours prior to the subject reaching RIFLE stage I.

**Angiogenin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 6050 | 14100 | 6050 | 10600 | 6050 | 5430 |
| Average | 9790 | 13000 | 9790 | 12400 | 9790 | 8880 |
| Stdev | 8750 | 9340 | 8750 | 9330 | 8750 | 9610 |
| p(t-test) |  | 0.080 |  | 0.15 |  | 0.70 |
| Min | 0.00873 | 647 | 0.00873 | 849 | 0.00873 | 54.6 |
| Max | 30600 | 30600 | 30600 | 30600 | 30600 | 30600 |
| n (Samp) | 1483 | 23 | 1483 | 24 | 1483 | 14 |
| n (Patient) | 493 | 23 | 493 | 24 | 493 | 14 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 6110 | 14100 | 6110 | 20000 | 6110 | 11200 |
| Average | 9850 | 15100 | 9850 | 15400 | 9850 | 13100 |
| Stdev | 8800 | 10300 | 8800 | 9770 | 8800 | 9260 |
| p(t-test) |  | 0.076 |  | 0.036 |  | 0.23 |
| Min | 0.00873 | 647 | 0.00873 | 1440 | 0.00873 | 854 |
| Max | 30600 | 30600 | 30600 | 30600 | 30600 | 30600 |
| n (Samp) | 1540 | 9 | 1540 | 11 | 1540 | 11 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 509 | 9 | 509 | 11 | 509 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6360 | 20200 | 6360 | 9890 | nd | nd |
| Average | 10000 | 14600 | 10000 | 11000 | nd | nd |
| Stdev | 8840 | 9830 | 8840 | 9090 | nd | nd |
| p(t-test) | | 0.060 | | 0.62 | nd | nd |
| Min | 0.00873 | 903 | 0.00873 | 849 | nd | nd |
| Max | 30600 | 28600 | 30600 | 30600 | nd | nd |
| n (Samp) | 1513 | 13 | 1513 | 18 | nd | nd |
| n (Patient) | 472 | 13 | 472 | 18 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.66 | 0.61 | 0.59 | 0.67 | 0.54 | 0.44 | 0.62 | nd |
| SE | 0.063 | 0.100 | 0.084 | 0.062 | 0.090 | 0.070 | 0.080 | 0.091 | nd |
| p | 0.11 | 0.11 | 0.18 | 0.14 | 0.052 | 0.55 | 0.44 | 0.19 | nd |
| nCohort 1 | 1483 | 1540 | 1513 | 1483 | 1540 | 1513 | 1483 | 1540 | nd |
| nCohort 2 | 23 | 9 | 13 | 24 | 11 | 18 | 14 | 11 | nd |
| Cutoff 1 | 5510 | 8720 | 2120 | 4960 | 6090 | 4140 | 1620 | 6520 | nd |
| Sens 1 | 74% | 78% | 77% | 71% | 73% | 72% | 71% | 73% | nd |
| Spec 1 | 47% | 58% | 20% | 44% | 50% | 37% | 15% | 51% | nd |
| Cutoff 2 | 2120 | 4780 | 1750 | 2580 | 4960 | 2480 | 844 | 5660 | nd |
| Sens 2 | 83% | 89% | 85% | 83% | 82% | 83% | 86% | 82% | nd |
| Spec 2 | 21% | 43% | 16% | 25% | 44% | 24% | 7% | 48% | nd |
| Cutoff 3 | 964 | 645 | 964 | 2260 | 3510 | 1050 | 409 | 3240 | nd |
| Sens 3 | 91% | 100% | 92% | 92% | 91% | 94% | 93% | 91% | nd |
| Spec 3 | 9% | 5% | 8% | 22% | 33% | 9% | 3% | 31% | nd |
| Cutoff 4 | 14400 | 14600 | 14900 | 14400 | 14600 | 14900 | 14400 | 14600 | nd |
| Sens 4 | 48% | 44% | 54% | 42% | 64% | 33% | 29% | 45% | nd |
| Spec 4 | 70% | 70% | 70% | 70% | 70% | 70% | 70% | 70% | nd |
| Cutoff 5 | 19800 | 20000 | 20300 | 19800 | 20000 | 20300 | 19800 | 20000 | nd |
| Sens 5 | 39% | 33% | 46% | 29% | 55% | 22% | 21% | 36% | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 5 | 80% | 80% | 80% | 80% | 80% | 80% | 80% | 80% | nd |
| Cutoff 6 | 22700 | 22700 | 23400 | 22700 | 22700 | 23400 | 22700 | 22700 | nd |
| Sens 6 | 13% | 33% | 15% | 12% | 18% | 11% | 7% | 9% | nd |
| Spec 6 | 90% | 90% | 90% | 90% | 90% | 90% | 90% | 90% | nd |
| OR Quart 2 | 0.60 | 1.0 | 0 | 1.00 | 3.0 | 0.40 | 1.0 | 2.0 | nd |
| | 0.48 | 1.0 | na | 1.00 | 0.34 | 0.27 | 1.00 | 0.57 | nd |
| p Value 95% CI of OR Quart2 | 0.14 | 0.062 | na | 0.25 | 0.31 | 0.076 | 0.20 | 0.18 | nd |
| | 2.5 | 16 | na | 4.0 | 29 | 2.1 | 5.0 | 22 | nd |
| OR Quart 3 | 1.2 | 4.0 | 0.50 | 2.0 | 1.00 | 1.2 | 0.67 | 4.0 | nd |
| | 0.76 | 0.21 | 0.42 | 0.26 | 1.00 | 0.76 | 0.66 | 0.21 | nd |
| p Value 95% CI of OR Quart3 | 0.36 | 0.45 | 0.091 | 0.60 | 0.062 | 0.36 | 0.11 | 0.45 | nd |
| | 4.0 | 36 | 2.7 | 6.8 | 16 | 4.0 | 4.0 | 36 | nd |
| OR Quart 4 | 1.8 | 3.0 | 1.8 | 2.0 | 6.1 | 1.00 | 2.0 | 4.0 | nd |
| | 0.29 | 0.34 | 0.37 | 0.26 | 0.096 | 1.00 | 0.32 | 0.21 | nd |
| p Value 95% CI of OR Quart4 | 0.60 | 0.31 | 0.51 | 0.60 | 0.73 | 0.29 | 0.50 | 0.45 | nd |
| | 5.5 | 29 | 6.1 | 6.8 | 51 | 3.5 | 8.1 | 36 | nd |

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.5 | 52.3 | 12.5 | 17.2 | 12.5 | 8.12 |
| Average | 46.9 | 120 | 46.9 | 98.0 | 46.9 | 135 |
| Stdev | 108 | 178 | 108 | 141 | 108 | 289 |
| p(t-test) | | 0.0066 | | 0.033 | | 0.013 |
| Min | 0.000466 | 3.67 | 0.000466 | 2.98 | 0.000466 | 0.794 |
| Max | 1370 | 647 | 1370 | 400 | 1370 | 878 |
| n (Samp) | 1276 | 17 | 1276 | 21 | 1276 | 10 |
| n (Patient) | 400 | 17 | 400 | 21 | 400 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.6 | 191 | 12.6 | 18.2 | 12.6 | 17.4 |
| Average | 47.7 | 205 | 47.7 | 103 | 47.7 | 169 |

(continued)

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 108 | 221 | 108 | 147 | 108 | 317 |
| p(t-test) | | 1.3E-4 | | 0.092 | | 0.0019 |
| Min | 0.000466 | 18.6 | 0.000466 | 2.98 | 0.000466 | 2.66 |
| Max | 1370 | 647 | 1370 | 413 | 1370 | 878 |
| n (Samp) | 1324 | 7 | 1324 | 11 | 1324 | 8 |
| n (Patient) | 414 | 7 | 414 | 11 | 414 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.7 | 16.8 | 12.7 | 12.9 | nd | nd |
| Average | 48.9 | 33.5 | 48.9 | 91.1 | nd | nd |
| Stdev | 111 | 28.2 | 111 | 150 | nd | nd |
| p(t-test) | | 0.68 | | 0.15 | nd | nd |
| Min | 0.000466 | 3.16 | 0.000466 | 0.000466 | nd | nd |
| Max | 1370 | 83.3 | 1370 | 400 | nd | nd |
| n (Samp) | 1331 | 9 | 1331 | 15 | nd | nd |
| n (Patient) | 397 | 9 | 397 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.85 | 0.60 | 0.63 | 0.66 | 0.52 | 0.44 | 0.57 | nd |
| SE | 0.072 | 0.091 | 0.10 | 0.066 | 0.090 | 0.076 | 0.095 | 0.11 | nd |
| p | 0.013 | 1.3E-4 | 0.32 | 0.048 | 0.072 | 0.74 | 0.50 | 0.51 | nd |
| nCohort 1 | 1276 | 1324 | 1331 | 1276 | 1324 | 1331 | 1276 | 1324 | nd |
| nCohort 2 | 17 | 7 | 9 | 21 | 11 | 15 | 10 | 8 | nd |
| Cutoff 1 | 16.2 | 56.8 | 11.2 | 14.0 | 17.2 | 7.57 | 5.34 | 9.32 | nd |
| Sens 1 | 71% | 71% | 78% | 71% | 73% | 73% | 70% | 75% | nd |
| Spec 1 | 59% | 83% | 44% | 53% | 60% | 31% | 21% | 39% | nd |
| Cutoff 2 | 8.64 | 23.3 | 8.64 | 11.2 | 14.1 | 5.81 | 4.48 | 6.86 | nd |
| Sens 2 | 82% | 86% | 89% | 81% | 82% | 80% | 80% | 88% | nd |
| Spec 2 | 37% | 67% | 36% | 45% | 53% | 23% | 17% | 28% | nd |
| Cutoff 3 | 6.65 | 18.5 | 3.15 | 5.81 | 14.0 | 2.97 | 2.29 | 2.62 | nd |
| Sens 3 | 94% | 100% | 100% | 90% | 91% | 93% | 90% | 100% | nd |
| Spec 3 | 27% | 62% | 9% | 23% | 53% | 9% | 6% | 7% | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 4 | 24.6 | 25.5 | 24.7 | 24.6 | 25.5 | 24.7 | 24.6 | 25.5 | nd |
| Sens 4 | 53% | 71% | 44% | 38% | 36% | 33% | 20% | 25% | nd |
| Spec 4 | 70% | 70% | 70% | 70% | 70% | 70% | 70% | 70% | nd |
| Cutoff 5 | 44.7 | 47.0 | 46.0 | 44.7 | 47.0 | 46.0 | 44.7 | 47.0 | nd |
| Sens 5 | 53% | 71% | 44% | 33% | 36% | 27% | 20% | 25% | nd |
| Spec 5 | 80% | 80% | 80% | 80% | 80% | 80% | 80% | 80% | nd |
| Cutoff 6 | 118 | 125 | 130 | 118 | 125 | 130 | 118 | 125 | nd |
| Sens 6 | 29% | 57% | 0% | 24% | 27% | 20% | 20% | 25% | nd |
| Spec 6 | 90% | 90% | 90% | 90% | 90% | 90% | 90% | 90% | nd |
| OR Quart 2 | 4.0 | >0 | 2.0 | 0.66 | 0 | 0.75 | 1.0 | 2.0 | nd |
| | 0.21 | <na | 0.57 | 0.66 | na | 0.70 | 1.00 | 0.57 | nd |
| p Value 95% CI of OR Quart2 | 0.45 | >na | 0.18 | 0.11 | na | 0.17 | 0.14 | 0.18 | nd |
| | 36 | na | 22 | 4.0 | na | 3.4 | 7.2 | 22 | nd |
| OR Quart 3 | 3.0 | >2.0 | 2.0 | 2.7 | 6.1 | 0.75 | 1.0 | 3.0 | nd |
| | 0.34 | <0.57 | 0.57 | 0.14 | 0.096 | 0.70 | 1.0 | 0.34 | nd |
| p Value 95% CI of OR Quart3 | 0.31 | >0.18 | 0.18 | 0.71 | 0.73 | 0.17 | 0.14 | 0.31 | nd |
| | 29 | na | 22 | 10 | 51 | 3.4 | 7.1 | 29 | nd |
| OR Quart 4 p Value 95% CI of OR Quart4 | 9.2 | >5.1 | 4.0 | 2.7 | 4.0 | 1.2 | 2.0 | 2.0 | nd |
| | 0.036 | <0.14 | 0.21 | 0.15 | 0.21 | 0.74 | 0.42 | 0.57 | nd |
| | 1.2 | >0.59 | 0.45 | 0.71 | 0.45 | 0.33 | 0.37 | 0.18 | nd |
| | 73 | na | 36 | 10 | 36 | 4.7 | 11 | 22 | nd |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.5 | 61.0 | 24.5 | 49.9 | 24.5 | 40.5 |
| Average | 55.8 | 106 | 55.8 | 473 | 55.8 | 45.4 |
| Stdev | 142 | 112 | 142 | 969 | 142 | 38.3 |
| p(t-test) | | 0.16 | | 9.9E-19 | | 0.82 |
| Min | 0.00131 | 9.94 | 0.00131 | 17.6 | 0.00131 | 5.13 |
| Max | 1650 | 364 | 1650 | 3480 | 1650 | 124 |
| n (Samp) | 884 | 16 | 884 | 18 | 884 | 10 |
| n (Patient) | 367 | 16 | 367 | 18 | 367 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 25.1 | 44.5 | 25.1 | 72.7 |
| Average | nd | nd | 63.2 | 286 | 63.2 | 104 |
| Stdev | nd | nd | 195 | 579 | 195 | 71.9 |
| p(t-test) | nd | nd | | 0.0018 | | 0.58 |
| Min | nd | nd | 0.00131 | 29.8 | 0.00131 | 25.9 |
| Max | nd | nd | 3480 | 1710 | 3480 | 246 |
| n (Samp) | nd | nd | 916 | 8 | 916 | 7 |
| n (Patient) | nd | nd | 380 | 8 | 380 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 25.6 | 36.4 | 25.6 | 45.7 | nd | nd |
| Average | 56.5 | 70.0 | 56.5 | 468 | nd | nd |
| Stdev | 145 | 71.3 | 145 | 1040 | nd | nd |
| p(t-test) | | 0.77 | | 3.4E-15 | nd | nd |
| Min | 0.00131 | 10.0 | 0.00131 | 10.1 | nd | nd |
| Max | 1710 | 229 | 1710 | 3480 | nd | nd |
| n (Samp) | 879 | 10 | 879 | 14 | nd | nd |
| n (Patient) | 342 | 10 | 342 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | nd | 0.63 | 0.74 | 0.76 | 0.68 | 0.56 | 0.83 | nd |
| SE | 0.074 | nd | 0.095 | 0.068 | 0.099 | 0.080 | 0.095 | 0.095 | nd |
| p | 0.010 | nd | 0.17 | 4.5E-4 | 0.0075 | 0.022 | 0.50 | 4.8E-4 | nd |
| nCohort 1 | 884 | nd | 879 | 884 | 916 | 879 | 884 | 916 | nd |
| nCohort 2 | 16 | nd | 10 | 18 | 8 | 14 | 10 | 7 | nd |
| Cutoff 1 | 20.2 | nd | 20.2 | 33.8 | 40.8 | 23.2 | 19.3 | 67.3 | nd |
| Sens 1 | 75% | nd | 70% | 72% | 75% | 71% | 70% | 71% | nd |
| Spec 1 | 44% | nd | 42% | 62% | 66% | 46% | 41% | 83% | nd |
| Cutoff 2 | 19.3 | nd | 19.3 | 23.2 | 33.8 | 20.9 | 10.6 | 65.4 | nd |
| Sens 2 | 81% | nd | 80% | 83% | 88% | 86% | 80% | 86% | nd |
| Spec 2 | 41% | nd | 40% | 48% | 61% | 43% | 21% | 82% | nd |
| Cutoff 3 | 12.6 | nd | 16.5 | 20.9 | 29.8 | 18.9 | 7.58 | 25.8 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 94% | nd | 90% | 94% | 100% | 93% | 90% | 100% | nd |
| Spec 3 | 26% | nd | 35% | 45% | 56% | 39% | 14% | 51% | nd |
| Cutoff 4 | 43.9 | nd | 45.3 | 43.9 | 45.6 | 45.3 | 43.9 | 45.6 | nd |
| Sens 4 | 56% | nd | 40% | 56% | 50% | 50% | 50% | 86% | nd |
| Spec 4 | 70% | nd | 70% | 70% | 70% | 70% | 70% | 70% | nd |
| Cutoff 5 | 57.2 | nd | 57.7 | 57.2 | 58.7 | 57.7 | 57.2 | 58.7 | nd |
| Sens 5 | 50% | nd | 40% | 44% | 38% | 43% | 40% | 86% | nd |
| Spec 5 | 80% | nd | 80% | 80% | 80% | 80% | 80% | 80% | nd |
| Cutoff 6 | 96.9 | nd | 96.9 | 96.9 | 104 | 96.9 | 96.9 | 104 | nd |
| Sens 6 | 31% | nd | 20% | 33% | 38% | 29% | 10% | 43% | nd |
| Spec 6 | 90% | nd | 90% | 90% | 90% | 90% | 90% | 90% | nd |
| OR Quart 2 | 4.1 | nd | 3.0 | >5.1 | >0 | 4.1 | 0.33 | >0 | nd |
| | 0.21 | nd | 0.34 | <0.14 | <na | 0.21 | 0.34 | <na | nd |
| p Value 95% CI of OR Quart2 | 0.45 | nd | 0.31 | >0.59 | >na | 0.45 | 0.034 | >na | nd |
| | 37 | nd | 29 | na | na | 37 | 3.2 | na | nd |
| OR Quart 3 | 3.0 | nd | 2.0 | >4.1 | >5.1 | 2.0 | 0.33 | >1.0 | nd |
| | 0.34 | nd | 0.57 | <0.21 | <0.14 | 0.57 | 0.34 | <1.0 | nd |
| p Value 95% CI of; OR Quart3 | 0.31 | nd | 0.18 | >0.45 | >0.59 | 0.18 | 0.034 | >0.062 | nd |
| | 29 | nd | 22 | na | na | 22 | 3.2 | na | nd |
| OR Quart 4 | 8.3 | nd | 4.0 | >9.3 | >3.0 | 7.2 | 1.7 | >6.1 | nd |
| | 0.047 | nd | 0.21 | <0.035 | <0.34 | 0.067 | 0.48 | <0.094 | nd |
| p Value | 1.0 | nd | 0.45 | >1.2 | >0.31 | 0.87 | 0.40 | >0.73 | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart4 | 67 | nd | 36 | na | na | 59 | 7.1 | na | nd |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.761 | 2.00 | 0.761 | 1.56 | 0.761 | 1.47 |
| Average | 1.38 | 2.22 | 1.38 | 1.91 | 1.38 | 1.12 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 2.23 | 1.03 | 2.23 | 2.67 | 2.23 | 0.804 |
| p(t-test) | | 0.13 | | 0.32 | | 0.72 |
| Min | 0.00179 | 0.00230 | 0.00179 | 0.00240 | 0.00179 | 0.00240 |
| Max | 28.3 | 3.96 | 28.3 | 11.6 | 28.3 | 1.94 |
| n (Samp) | 884 | 16 | 884 | 18 | 884 | 10 |
| n (Patient) | 367 | 16 | 367 | 18 | 367 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.761 | 1.57 | 0.761 | 1.43 |
| Average | nd | nd | 1.41 | 1.73 | 1.41 | 1.33 |
| Stdev | nd | nd | 2.25 | 1.06 | 2.25 | 1.59 |
| p(t-test) | nd | nd | | 0.70 | | 0.93 |
| Min | nd | nd | 0.00179 | 0.0742 | 0.00179 | 0.00246 |
| Max | nd | nd | 28.3 | 3.65 | 28.3 | 4.42 |
| n (Samp) | nd | nd | 916 | 8 | 916 | 7 |
| n (Patient) | nd | nd | 380 | 8 | 380 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.761 | 2.09 | 0.761 | 1.56 | nd | nd |
| Average | 1.37 | 2.33 | 1.37 | 2.05 | nd | nd |
| Stdev | 2.12 | 0.816 | 2.12 | 3.05 | nd | nd |
| p(t-test) | | 0.15 | | 0.23 | nd | nd |
| Min | 0.00179 | 1.30 | 0.00179 | 0.00240 | nd | nd |
| Max | 28.3 | 3.96 | 28.3 | 11.6 | nd | nd |
| n (Samp) | 879 | 10 | 879 | 14 | nd | nd |
| n (Patient) | 342 | 10 | 342 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.72 | nd | 0.77 | 0.59 | 0.65 | 0.57 | 0.47 | 0.47 | nd |
| SE | 0.073 | nd | 0.088 | 0.071 | 0.11 | 0.080 | 0.093 | 0.11 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p | 0.0021 | nd | 0.0024 | 0.20 | 0.16 | 0.35 | 0.79 | 0.79 | nd |
| nCohort 1 | 884 | nd | 879 | 884 | 916 | 879 | 884 | 916 | nd |
| nCohort 2 | 16 | nd | 10 | 18 | 8 | 14 | 10 | 7 | nd |
| Cutoff 1 Sens 1 Spec 1 | 1.74 | nd | 1.94 | 0.135 | 1.51 | 0.108 | 1.10 | 0.0209 | nd |
| | 75% | nd | 70% | 72% | 75% | 71% | 70% | 71% | nd |
| | 69% | nd | 71% | 41% | 60% | 40% | 55% | 25% | nd |
| Cutoff 2 Sens 2 Spec 2 | 1.59 | nd | 1.74 | 0.0522 | 0.900 | 0.0209 | 0.00240 | 0.00240 | nd |
| | 81% | nd | 80% | 89% | 88% | 86% | 90% | 100% | nd |
| | 66% | nd | 69% | 34% | 51% | 27% | 7% | 7% | nd |
| Cutoff 3 Sens 3 Spec 3 | 1.10 | nd | 1.59 | 0.0209 | 0.0522 | 0.00342 | 0.00240 | 0.00240 | nd |
| | 94% | nd | 90% | 94% | 100% | 93% | 90% | 100% | nd |
| | 55% | nd | 66% | 26% | 33% | 20% | 7% | 7% | nd |
| Cutoff 4 Sens 4 Spec 4 | 1.87 | nd | 1.87 | 1.87 | 1.87 | 1.87 | 1.87 | 1.87 | nd |
| | 62% | nd | 70% | 33% | 38% | 43% | 10% | 14% | nd |
| | 71% | nd | 71% | 71% | 70% | 71% | 71% | 70% | nd |
| Cutoff 5 Sens 5 Spec 5 | 2.41 | nd | 2.41 | 2.41 | 2.42 | 2.41 | 2.41 | 2.42 | nd |
| | 38% | nd | 40% | 22% | 25% | 29% | 0% | 14% | nd |
| | 80% | nd | 80% | 80% | 81% | 80% | 80% | 81% | nd |
| Cutoff 6 Sens 6 Spec 6 | 3.36 | nd | 3.36 | 3.36 | 3.36 | 3.36 | 3.36 | 3.36 | nd |
| | 12% | nd | 10% | 11% | 12% | 14% | 0% | 14% | nd |
| | 91% | nd | 91% | 91% | 91% | 91% | 91% | 91% | nd |
| OR Quart 2 | 0 | nd | >0 | 5.1 | >1.0 | 2.0 | >7.3 | 3.0 | nd |
| | na | nd | <na | 0.14 | <1.00 | 0.42 | <0.065 | 0.34 | nd |
| p Value 95% CI of OR Quart2 | na | nd | >na | 0.59 | >0.062 | 0.37 | >0.89 | 0.31 | nd |
| | na | nd | na | 44 | na | 11 | na | 29 | nd |
| OR Quart 3 | 9.3 | nd | >5.1 | 8.3 | >5.1 | 2.0 | >0 | 1.0 | nd |
| | 0.035 | nd | <0.14 | 0.047 | <0.14 | 0.42 | <na | 1.0 | nd |
| p Value 95% CI of OR Quart3 | 1.2 | nd | >0.59 | 1.0 | >0.59 | 0.37 | >na | 0.062 | nd |
| | 74 | nd | na | 67 | na | 11 | na | 16 | nd |
| OR Quart 4 | 6.1 | nd | >5.1 | 4.0 | >2.0 | 2.0 | >3.1 | 2.0 | nd |
| | 0.094 | nd | <0.14 | 0.21 | <0.57 | 0.42 | <0.34 | 0.57 | nd |
| p Value 95% CI of OR Quart4 | 0.73 | nd | >0.59 | 0.45 | >0.18 | 0.36 | >0.32 | 0.18 | nd |
| | 51 | nd | na | 36 | na | 11 | na | 22 | nd |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5660 | 17000 | 5660 | 9540 | 5660 | 3750 |
| Average | 17000 | 44500 | 17000 | 25900 | 17000 | 20200 |
| Stdev | 29300 | 65600 | 29300 | 34200 | 29300 | 39600 |
| p(t-test) | | 1.5E-5 | | 0.14 | | 0.69 |
| Min | 0.0130 | 932 | 0.0130 | 1110 | 0.0130 | 261 |
| Max | 238000 | 227000 | 238000 | 148000 | 238000 | 149000 |
| n (Samp) | 1483 | 23 | 1483 | 24 | 1483 | 14 |
| n (Patient) | 493 | 23 | 493 | 24 | 493 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5760 | 17000 | 5760 | 8130 | 5760 | 19300 |
| Average | 17200 | 56500 | 17200 | 27500 | 17200 | 36000 |
| Stdev | 29600 | 81800 | 29600 | 35200 | 29600 | 47400 |
| p(t-test) | | 9.7E-5 | | 0.25 | | 0.037 |
| Min | 0.0130 | 1980 | 0.0130 | 2050 | 0.0130 | 1790 |
| Max | 238000 | 227000 | 238000 | 110000 | 238000 | 149000 |
| n (Samp) | 1540 | 9 | 1540 | 11 | 1540 | 11 |
| n (Patient) | 509 | 9 | 509 | 11 | 509 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5760 | 16700 | 5760 | 9520 | nd | nd |
| Average | 17500 | 42400 | 17500 | 24900 | nd | nd |
| Stdev | 30400 | 63700 | 30400 | 37100 | nd | nd |
| p(t-test) | | 0.0038 | | 0.31 | nd | nd |
| Min | 0.0130 | 932 | 0.0130 | 1110 | nd | nd |
| Max | 267000 | 190000 | 267000 | 148000 | nd | nd |
| n (Samp) | 1513 | 13 | 1513 | 18 | nd | nd |
| n (Patient) | 472 | 13 | 472 | 18 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.62 | 0.68 | 0.61 | 0.61 | 0.61 | 0.57 | 0.46 | 0.66 | nd |
| SE | 0.063 | 0.099 | 0.084 | 0.062 | 0.091 | 0.071 | 0.079 | 0.090 | nd |
| p | 0.050 | 0.078 | 0.17 | 0.063 | 0.22 | 0.30 | 0.59 | 0.069 | nd |
| nCohort 1 | 1483 | 1540 | 1513 | 1483 | 1540 | 1513 | 1483 | 1540 | nd |
| nCohort 2 | 23 | 9 | 13 | 24 | 11 | 18 | 14 | 11 | nd |
| Cutoff 1 | 4710 | 4940 | 4710 | 4640 | 4410 | 4410 | 3180 | 9560 | nd |
| Sens 1 | 74% | 78% | 77% | 71% | 73% | 72% | 71% | 73% | nd |
| Spec 1 | 43% | 45% | 43% | 43% | 41% | 41% | 29% | 66% | nd |
| Cutoff 2 | 2720 | 3120 | 2150 | 3560 | 4190 | 2970 | 1420 | 3250 | nd |
| Sens 2 | 83% | 89% | 85% | 83% | 82% | 83% | 86% | 82% | nd |
| Spec 2 | 25% | 28% | 17% | 33% | 39% | 26% | 9% | 30% | nd |
| Cutoff 3 | 2010 | 1960 | 2010 | 2950 | 2970 | 1410 | 1290 | 3180 | nd |
| Sens 3 | 91% | 100% | 92% | 92% | 91% | 94% | 93% | 91% | nd |
| Spec 3 | 16% | 16% | 15% | 27% | 27% | 8% | 8% | 29% | nd |
| Cutoff 4 | 11600 | 12000 | 12000 | 11600 | 12000 | 12000 | 11600 | 12000 | nd |
| Sens 4 | 52% | 67% | 54% | 42% | 36% | 39% | 29% | 55% | nd |
| Spec 4 | 70% | 70% | 70% | 70% | 70% | 70% | 70% | 70% | nd |
| Cutoff 5 | 22600 | 22900 | 23700 | 22600 | 22900 | 23700 | 22600 | 22900 | nd |
| Sens 5 | 43% | 44% | 38% | 33% | 36% | 28% | 21% | 36% | nd |
| Spec 5 | 80% | 80% | 80% | 80% | 80% | 80% | 80% | 80% | nd |
| Cutoff 6 | 47200 | 47900 | 48800 | 47200 | 47900 | 48800 | 47200 | 47900 | nd |
| Sens 6 | 26% | 33% | 23% | 25% | 27% | 22% | 14% | 27% | nd |
| Spec 6 | 90% | 90% | 90% | 90% | 90% | 90% | 90% | 90% | nd |
| OR Quart 2 | 0.80 | 2.0 | 0.33 | 4.1 | 4.0 | 1.7 | 0.50 | 2.0 | nd |
|  | 0.73 | 0.57 | 0.34 | 0.078 | 0.21 | 0.48 | 0.42 | 0.57 | nd |
| p Value 95% CI of OR Quart2 | 0.21 | 0.18 | 0.034 | 0.86 | 0.45 | 0.40 | 0.091 | 0.18 | nd |
|  | 3.0 | 22 | 3.2 | 19 | 36 | 7.0 | 2.7 | 22 | nd |
| OR Quart 3 | 0.40 | 1.0 | 0.66 | 2.5 | 2.0 | 1.3 | 1.0 | 2.0 | nd |
|  | 0.27 | 1.0 | 0.66 | 0.27 | 0.57 | 0.71 | 1.00 | 0.57 | nd |
| p Value 95% CI of. OR Quart3 | 0.076 | 0.062 | 0.11 | 0.48 | 0.18 | 0.30 | 0.25 | 0.18 | nd |
|  | 2.1 | 16 | 4.0 | 13 | 22 | 6.0 | 4.0 | 22 | nd |
| OR Quart 4 | 2.4 | 5.0 | 2.4 | 4.6 | 4.0 | 2.0 | 1.0 | 6.1 | nd |
|  | 0.097 | 0.14 | 0.22 | 0.053 | 0.21 | 0.33 | 1.00 | 0.096 | nd |
| p Value 95% CI of OR Quart4 | 0.85 | 0.59 | 0.60 | 0.98 | 0.45 | 0.50 | 0.25 | 0.73 | nd |
|  | 7.0 | 43 | 9.2 | 21 | 36 | 8.1 | 4.0 | 51 | nd |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.39 | 4.55 | 3.39 | 6.87 | 3.39 | 3.30 |
| Average | 6.14 | 9.42 | 6.14 | 14.7 | 6.14 | 4.98 |
| Stdev | 12.6 | 12.5 | 12.6 | 21.8 | 12.6 | 5.03 |
| p(t-test) | | 0.30 | | 0.0069 | | 0.77 |
| Min | 0.000104 | 1.42 | 0.000104 | 0.758 | 0.000104 | 0.376 |
| Max | 279 | 49.4 | 279 | 81.9 | 279 | 15.2 |
| n (Samp) | 854 | 16 | 854 | 17 | 854 | 10 |
| n (Patient) | 364 | 16 | 364 | 17 | 364 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 3.46 | 6.47 | 3.46 | 6.79 |
| Average | nd | nd | 6.30 | 13.5 | 6.30 | 7.21 |
| Stdev | nd | nd | 12.8 | 17.7 | 12.8 | 4.19 |
| p(t-test) | nd | nd | | 0.11 | | 0.85 |
| Min | nd | nd | 0.000104 | 5.17 | 0.000104 | 2.96 |
| Max | nd | nd | 279 | 57.1 | 279 | 15.2 |
| n (Samp) | nd | nd | 885 | 8 | 885 | 7 |
| n (Patient) | nd | nd | 377 | 8 | 377 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.52 | 3.45 | 3.52 | 7.39 | nd | nd |
| Average | 6.37 | 5.92 | 6.37 | 13.2 | nd | nd |
| Stdev | 12.8 | 7.42 | 12.8 | 21.6 | nd | nd |
| p(t-test) | | 0.91 | | 0.061 | nd | nd |
| Min | 0.000104 | 1.79 | 0.000104 | 0.758 | nd | nd |
| Max | 279 | 26.4 | 279 | 81.9 | nd | nd |
| n (Samp) | 849 | 10 | 849 | 13 | nd | nd |
| n (Patient) | 339 | 10 | 339 | 13 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | nd | 0.52 | 0.67 | 0.77 | 0.61 | 0.47 | 0.70 | nd |
| SE | 0.076 | nd | 0.093 | 0.073 | 0.098 | 0.084 | 0.094 | 0.11 | nd |
| p | 0.099 | nd | 0.79 | 0.017 | 0.0050 | 0.20 | 0.73 | 0.078 | nd |
| nCohort 1 | 854 | nd | 849 | 854 | 885 | 849 | 854 | 885 | nd |
| nCohort 2 | 16 | nd | 10 | 17 | 8 | 13 | 10 | 7 | nd |
| Cutoff 1 | 3.18 | nd | 2.37 | 5.16 | 5.96 | 1.95 | 1.37 | 4.73 | nd |
| Sens 1 | 75% | nd | 70% | 71% | 75% | 77% | 70% | 71% | nd |
| Spec 1 | 47% | nd | 35% | 66% | 70% | 30% | 21% | 63% | nd |
| Cutoff 2 | 2.22 | nd | 2.22 | 1.95 | 5.42 | 1.45 | 0.806 | 3.74 | nd |
| Sens 2 | 81% | nd | 80% | 82% | 88% | 85% | 80% | 86% | nd |
| Spec 2 | 35% | nd | 33% | 32% | 67% | 21% | 12% | 55% | nd |
| Cutoff 3 | 1.78 | nd | 2.05 | 0.944 | 5.16 | 0.944 | 0.493 | 2.95 | nd |
| Sens 3 | 94% | nd | 90% | 94% | 100% | 92% | 90% | 100% | nd |
| Spec 3 | 29% | nd | 31% | 14% | 66% | 12% | 6% | 44% | nd |
| Cutoff 4 | 5.74 | nd | 6.02 | 5.74 | 5.90 | 6.02 | 5.74 | 5.90 | nd |
| Sens 4 | 44% | nd | 30% | 59% | 75% | 54% | 40% | 57% | nd |
| Spec 4 | 70% | nd | 70% | 70% | 70% | 70% | 70% | 70% | nd |
| Cutoff 5 | 8.36 | nd | 8.53 | 8.36 | 8.47 | 8.53 | 8.36 | 8.47 | nd |
| Sens 5 | 25% | nd | 10% | 41% | 38% | 46% | 20% | 29% | nd |
| Spec 5 | 80% | nd | 80% | 80% | 80% | 80% | 80% | 80% | nd |
| Cutoff 6 | 13.0 | nd | 13.4 | 13.0 | 13.4 | 13.4 | 13.0 | 13.4 | nd |
| Sens 6 | 19% | nd | 10% | 24% | 12% | 23% | 10% | 14% | nd |
| Spec 6 | 90% | nd | 90% | 90% | 90% | 90% | 90% | 90% | nd |
| OR Quart 2 | 4.0 | nd | >6.1 | 0.33 | >0 | 0.66 | 0.66 | >1.0 | nd |
| | 0.21 | nd | <0.094 | 0.34 | <na | 0.65 | 0.66 | <1.00 | nd |
| p Value 95% CI of OR Quart2 | 0.45 | nd | >0.73 | 0.034 | >na | 0.11 | 0.11 | >0.062 | nd |
| | 36 | nd | na | 3.2 | na | 4.0 | 4.0 | na | nd |
| OR Quart 3 | 6.1 | nd | >2.0 | 1.7 | >5.1 | 0.33 | 0.33 | >3.0 | nd |
| | 0.094 | nd | <0.57 | 0.48 | <0.14 | 0.34 | 0.34 | <0.34 | nd |
| p Value 95% CI of OR Quart3 | 0.73 | nd | >0.18 | 0.40 | >0.59 | 0.034 | 0.034 | >0.31 | nd |
| | 51 | nd | na | 7.1 | na | 3.2 | 3.2 | na | nd |
| OR Quart 4 | 5.1 | nd | >2.0 | 2.7 | >3.0 | 2.4 | 1.3 | >3.0 | nd |
| | 0.14 | nd | <0.57 | 0.14 | <0.34 | 0.22 | 0.70 | <0.34 | nd |
| p Value 95% CI of OR Quart4 | 0.59 | nd | >0.18 | 0.71 | >0.31 | 0.60 | 0.30 | >0.31 | nd |
| | 44 | nd | na | 10 | na | 9.3 | 6.1 | na | nd |

**Heparin-binding growth factor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 848 | 289 | 848 | 549 | 848 | 429 |
| Average | 1200 | 488 | 1200 | 967 | 1200 | 582 |
| Stdev | 1420 | 551 | 1420 | 1200 | 1420 | 595 |
| p(t-test) | | 0.016 | | 0.43 | | 0.10 |
| Min | 0.00328 | 55.5 | 0.00328 | 8.50 | 0.00328 | 35.1 |
| Max | 16700 | 2430 | 16700 | 5020 | 16700 | 1910 |
| n (Samp) | 1482 | 23 | 1482 | 23 | 1482 | 14 |
| n (Patient) | 493 | 23 | 493 | 23 | 493 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 834 | 189 | 834 | 494 | 834 | 194 |
| Average | 1180 | 335 | 1180 | 624 | 1180 | 433 |
| Stdev | 1400 | 447 | 1400 | 504 | 1400 | 506 |
| p(t-test) | | 0.070 | | 0.21 | | 0.076 |
| Min | 0.00328 | 55.5 | 0.00328 | 90.5 | 0.00328 | 32.9 |
| Max | 16700 | 1470 | 16700 | 1650 | 16700 | 1500 |
| n (Samp) | 1539 | 9 | 1539 | 10 | 1539 | 11 |
| n (Patient) | 509 | 9 | 509 | 10 | 509 | 11 |
| UO only | 0hr prior to AKI stage | | 24hr prior to AKI stage | | 48hr prior to AKI stage | |
| | Cohort 1 | Cohort 2 | Cohort 1 | Cohort 2 | Cohort 1 | Cohort 2 |
| Median | 842 | 320 | 842 | 546 | nd | nd |
| Average | 1200 | 444 | 1200 | 827 | nd | nd |
| Stdev | 1430 | 350 | 1430 | 889 | nd | nd |
| p(t-test) | | 0.056 | | 0.27 | nd | nd |
| Min | 0.00328 | 70.1 | 0.00328 | 8.50 | nd | nd |
| Max | 16700 | 1140 | 16700 | 3420 | nd | nd |
| n (Samp) | 1511 | 13 | 1511 | 18 | nd | nd |
| n (Patient) | 472 | 13 | 472 | 18 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.28 | 0.20 | 0.28 | 0.42 | 0.37 | 0.40 | 0.32 | 0.26 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.061 | 0.089 | 0.081 | 0.063 | 0.095 | 0.071 | 0.080 | 0.086 | nd |
| p | 2.1E-4 | 9.2E-4 | 0.0059 | 0.19 | 0.16 | 0.17 | 0.026 | 0.0045 | nd |
| nCohort 1 | 1482 | 1539 | 1511 | 1482 | 1539 | 1511 | 1482 | 1539 | nd |
| nCohort 2 | 23 | 9 | 13 | 23 | 10 | 18 | 14 | 11 | nd |
| Cutoff 1 | 155 | 110 | 155 | 364 | 449 | 364 | 125 | 77.7 | nd |
| Sens 1 | 74% | 78% | 77% | 74% | 70% | 72% | 71% | 73% | nd |
| Spec 1 | 11% | 8% | 11% | 24% | 30% | 25% | 9% | 6% | nd |
| Cutoff 2 | 145 | 69.8 | 145 | 119 | 247 | 119 | 61.5 | 61.5 | nd |
| Sens 2 | 83% | 89% | 85% | 83% | 80% | 83% | 86% | 82% | nd |
| Spec 2 | 11% | 5% | 11% | 9% | 17% | 9% | 5% | 5% | nd |
| Cutoff 3 | 110 | 55.2 | 119 | 89.7 | 91.2 | 75.4 | 51.2 | 34.6 | nd |
| Sens 3 | 91% | 100% | 92% | 91% | 90% | 94% | 93% | 91% | nd |
| Spec 3 | 8% | 5% | 9% | 7% | 7% | 6% | 4% | 3% | nd |
| Cutoff 4 | 1380 | 1340 | 1360 | 1380 | 1340 | 1360 | 1380 | 1340 | nd |
| Sens 4 | 9% | 11% | 0% | 13% | 10% | 11% | 14% | 9% | nd |
| Spec 4 | 70% | 70% | 70% | 70% | 70% | 70% | 70% | 70% | nd |
| Cutoff 5 | 1730 | 1710 | 1730 | 1730 | 1710 | 1730 | 1730 | 1710 | nd |
| Sens 5 | 4% | 0% | 0% | 13% | 0% | 11% | 7% | 0% | nd |
| Spec 5 | 80% | 80% | 80% | 80% | 80% | 80% | 80% | 80% | nd |
| Cutoff 6 | 2450 | 2430 | 2460 | 2450 | 2430 | 2460 | 2450 | 2430 | nd |
| Sens 6 | 0% | 0% | 0% | 13% | 0% | 11% | 0% | 0% | nd |
| Spec 6 | 90% | 90% | 90% | 90% | 90% | 90% | 90% | 90% | nd |
| OR Quart 2 | 3.0 | >1.0 | >3.0 | 2.0 | 2.0 | 2.5 | 3.0 | >2.0 | nd |
| | 0.34 | <1.00 | <0.34 | 0.32 | 0.57 | 0.27 | 0.34 | <0.57 | nd |
| p Value 95% CI of OR Quart2 | 0.31 | >0.062 | >0.31 | 0.50 | 0.18 | 0.49 | 0.31 | >0.18 | nd |
| | 29 | na | na | 8.1 | 22 | 13 | 29 | na | nd |
| OR Quart 3 | 5.1 | >1.0 | >2.0 | 2.4 | 4.0 | 2.5 | 3.0 | >2.0 | nd |
| | 0.14 | <1.00 | <0.57 | 0.21 | 0.21 | 0.27 | 0.34 | <0.57 | nd |
| p Value 95% CI ol44 OR Quart3 | 0.59 | >0.062 | >0.18 | 0.61 | 0.45 | 0.49 | 0.31 | >0.18 | nd |
| | 44 | na | na | 9.2 | 36 | 13 | 29 | na | nd |
| OR Quart 4 | 15 | >7.1 | >8.2 | 2.4 | 3.0 | 3.0 | 7.1 | >7.1 | nd |
| | 0.0099 | <0.067 | <0.048 | 0.21 | 0.34 | 0.17 | 0.067 | <0.066 | nd |
| p Value 95% CI of OR Quart4 | 1.9 | >0.87 | >1.0 | 0.61 | 0.31 | 0.61 | 0.87 | >0.88 | nd |
| | 110 | na | na | 9.2 | 29 | 15 | 58 | na | nd |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.83E-5 | 2.83E-5 | 0.000168 | 2.83E-5 | 2.63E-5 |
| Average | 0.00494 | 0.0778 | 0.00494 | 0.0504 | 0.00494 | 0.00105 |
| Stdev | 0.0336 | 0.249 | 0.0336 | 0.155 | 0.0336 | 0.00323 |
| p(t-test) | | 9.9E-12 | | 9.4E-8 | | 0.71 |
| Min | 5.62E-6 | 5.62E-6 | 5.62E-6 | 1.66E-5 | 5.62E-6 | 2.17E-5 |
| Max | 0.634 | 1.02 | 0.634 | 0.560 | 0.634 | 0.0102 |
| n (Samp) | 1276 | 17 | 1276 | 21 | 1276 | 10 |
| n (Patient) | 400 | 17 | 400 | 21 | 400 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 0.00600 | 2.83E-5 | 0.000197 | 2.83E-5 | 2.55E-5 |
| Average | 0.00524 | 0.188 | 0.00524 | 0.0539 | 0.00524 | 2.62E-5 |
| Stdev | 0.0354 | 0.377 | 0.0354 | 0.168 | 0.0354 | 3.91E-6 |
| p(t-test) | | 2.0E-27 | | 2.7E-5 | | 0.68 |
| Min | 5.62E-6 | 7.53E-6 | 5.62E-6 | 1.88E-5 | 5.62E-6 | 2.17E-5 |
| Max | 0.634 | 1.02 | 0.634 | 0.560 | 0.634 | 3.21E-5 |
| n (Samp) | 1324 | 7 | 1324 | 11 | 1324 | 8 |
| n (Patient) | 414 | 7 | 414 | 11 | 414 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.83E-5 | 2.83E-5 | 4.39E-5 | nd | nd |
| Average | 0.00839 | 0.113 | 0.00839 | 0.0349 | nd | nd |
| Stdev | 0.0779 | 0.340 | 0.0779 | 0.121 | nd | nd |
| p(t-test) | | 1.3E-4 | | 0.19 | nd | nd |
| Min | 5.62E-6 | 1.88E-5 | 5.62E-6 | 1.66E-5 | nd | nd |
| Max | 2.28 | 1.02 | 2.28 | 0.470 | nd | nd |
| n (Samp) | 1331 | 9 | 1331 | 15 | nd | nd |
| n (Patient) | 397 | 9 | 397 | 15 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.55 | 0.61 | 0.54 | 0.66 | 0.66 | 0.62 | 0.47 | 0.41 | nd |
| SE | 0.072 | 0.11 | 0.099 | 0.066 | 0.090 | 0.078 | 0.093 | 0.11 | nd |
| p | 0.52 | 0.34 | 0.69 | 0.015 | 0.075 | 0.12 | 0.77 | 0.37 | nd |
| nCohort 1 | 1276 | 1324 | 1331 | 1276 | 1324 | 1331 | 1276 | 1324 | nd |
| nCohort 2 | 17 | 7 | 9 | 21 | 11 | 15 | 10 | 8 | nd |
| Cutoff 1 | 1.92E-5 | 1.82E-5 | 2.17E-5 | 2.53E-5 | 2.17E-5 | 2.46E-5 | 2.38E-5 | 2.17E-5 | nd |
| Sens 1 | 71% | 71% | 78% | 76% | 73% | 73% | 70% | 75% | nd |
| Spec 1 | 26% | 18% | 30% | 39% | 29% | 36% | 34% | 29% | nd |
| Cutoff 2 | 1.82E-5 | 1.34E-5 | 1.88E-5 | 1.92E-5 | 1.88E-5 | 1.92E-5 | 2.17E-5 | 1.92E-5 | nd |
| Sens 2 | 88% | 86% | 89% | 81% | 82% | 80% | 80% | 100% | nd |
| Spec 2 | 18% | 10% | 22% | 26% | 22% | 26% | 29% | 26% | nd |
| Cutoff 3 | 1.34E-5 | 5.62E-6 | 1.82E-5 | 1.82E-5 | 1.82E-5 | 1.82E-5 | 1.92E-5 | 1.92E-5 | nd |
| Sens 3 | 94% | 100% | 100% | 95% | 100% | 93% | 100% | 100% | nd |
| Spec 3 | 10% | 4% | 18% | 18% | 18% | 18% | 26% | 26% | nd |
| Cutoff 4 | 4.39E-5 | 4.39E-5 | 4.39E-5 | 4.39E-5 | 4.39E-5 | 4.39E-5 | 4.39E-5 | 4.39E-5 | nd |
| Sens 4 | 35% | 57% | 11% | 57% | 64% | 47% | 10% | 0% | nd |
| Spec 4 | 72% | 73% | 73% | 72% | 73% | 73% | 72% | 73% | nd |
| Cutoff 5 | 0.000114 | 0.000114 | 0.000114 | 0.000114 | 0.000114 | 0.00011 4 | 0.000114 | 0.000114 | nd |
| Sens 5 | 35% | 57% | 11% | 52% | 55% | 47% | 10% | 0% | nd |
| Spec 5 | 81% | 81% | 81% | 81% | 81% | 81% | 81% | 81% | nd |
| Cutoff 6 | 0.00364 | 0.00364 | 0.00364 | 0.00364 | 0.00364 | 0.00364 | 0.00364 | 0.00364 | nd |
| Sens 6 | 29% | 57% | 11% | 19% | 36% | 20% | 10% | 0% | nd |
| Spec 6 | 90% | 90% | 90% | 90% | 90% | 90% | 90% | 90% | nd |
| OR Quart 2 | 0.60 | 0 | 1.0 | 0.75 | 0.33 | 1.00 | 3.0 | >3.0 | nd |
|  | 0.48 | na | 1.0 | 0.70 | 0.34 | 1.00 | 0.34 | <0.34 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value 95% CI of OR Quart2 | 0.14 | na | 0.14 | 0.17 | 0.034 | 0.20 | 0.31 | >0.31 | nd |
| | 2.5 | na | 7.1 | 3.4 | 3.2 | 5.0 | 29 | na | nd |
| OR Quart 3 | 0.60 | 0 | 2.0 | 0.50 | 0 | 0.66 | 6.1 | >5.1 | nd |
| | 0.48 | na | 0.42 | 0.42 | na | 0.66 | 0.095 | <0.14 | nd |
| p Value 95% CI of OR Quart3 | 0.14 | na | 0.37 | 0.090 | na | 0.11 | 0.73 | >0.59 | nd |
| | 2.5 | na | 11 | 2.7 | na | 4.0 | 51 | na | nd |
| OR Quart 4 | 1.2 | 1.3 | 0.50 | 3.1 | 2.4 | 2.4 | 0 | >0 | nd |
| | 0.77 | 0.71 | 0.57 | 0.054 | 0.22 | 0.22 | na | <na | nd |
| p Value | 0.36 | 0.30 | 0.045 | 0.98 | 0.60 | 0.60 | na | >na | nd |
| 95% CI of OR Quart4 | 4.0 | 6.0 | 5.5 | 9.6 | 9.2 | 9.2 | na | na | nd |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0140 | 0.0567 | 0.0140 | 0.0466 | 0.0140 | 0.00852 |
| Average | 0.168 | 0.249 | 0.168 | 0.214 | 0.168 | 0.0619 |
| Stdev | 0.559 | 0.393 | 0.559 | 0.393 | 0.559 | 0.149 |
| p(t-test) | | 0.55 | | 0.71 | | 0.55 |
| Min | 1.14E-9 | 0.00290 | 1.14E-9 | 7.54E-8 | 1.14E-9 | 0.000662 |
| Max | 8.92 | 1.53 | 8.92 | 1.59 | 8.92 | 0.482 |
| n (Samp) | 1276 | 17 | 1276 | 21 | 1276 | 10 |
| n (Patient) | 400 | 17 | 400 | 21 | 400 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0145 | 0.318 | 0.0145 | 0.0466 | 0.0145 | 0.0115 |
| Average | 0.174 | 0.434 | 0.174 | 0.0590 | 0.174 | 0.110 |
| Stdev | 0.558 | 0.553 | 0.558 | 0.0659 | 0.558 | 0.185 |
| p(t-test) | | 0.22 | | 0.50 | | 0.75 |
| Min | 1.14E-9 | 0.00620 | 1.14E-9 | 7.54E-8 | 1.14E-9 | 0.00255 |
| Max | 8.92 | 1.53 | 8.92 | 0.206 | 8.92 | 0.482 |
| n (Samp) | 1324 | 7 | 1324 | 11 | 1324 | 8 |
| n (Patient) | 414 | 7 | 414 | 11 | 414 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0142 | 0.0809 | 0.0142 | 0.0598 | nd | nd |
| Average | 0.175 | 0.176 | 0.175 | 0.287 | nd | nd |
| Stdev | 0.590 | 0.183 | 0.590 | 0.449 | nd | nd |
| p(t-test) | | 1.00 | | 0.46 | nd | nd |
| Min | 1.14E-9 | 0.00163 | 1.14E-9 | 7.54E-8 | nd | nd |
| Max | 8.92 | 0.442 | 8.92 | 1.59 | nd | nd |
| n (Samp) | 1331 | 9 | 1331 | 15 | nd | nd |
| n (Patient) | 397 | 9 | 397 | 15 | nd | nd |

|  | 0hr prior to AKI stage | | | 24hr prior to AKI stage | | | 48hr prior to AKI stage | | |
| --- | --- | --- | --- | --- | --- | --- | --- | --- | --- |
|  | sCr or UO | sCr only | UO only | sCr or UO | sCr only | UO only | sCr or UO | sCr only | UO only |
| AUC | 0.66 | 0.71 | 0.66 | 0.59 | 0.56 | 0.60 | 0.42 | 0.50 | nd |
| SE | 0.073 | 0.11 | 0.100 | 0.066 | 0.090 | 0.078 | 0.095 | 0.10 | nd |
| p | 0.029 | 3.052 | 0.10 | 0.20 | 0.50 | 0.18 | 0.37 | 1.00 | nd |
| nCohort 1 | 1276 | 1324 | 1331 | 1276 | 1324 | 1331 | 1276 | 1324 | nd |
| nCohort 2 | 17 | 7 | 9 | 21 | 11 | 15 | 10 | 8 | nd |
| Cutoff 1 | 0.0171 | 0.0191 | 0.0172 | 0.00670 | 0.0116 | 0.00670 | 0.00324 | 0.00583 | nd |
| Sens 1 | 71% | 71% | 78% | 71% | 73% | 73% | 70% | 75% | nd |
| Spec 1 | 56% | 57% | 56% | 32% | 43% | 32% | 18% | 28% | nd |
| Cutoff 2 | 0.00618 | 0.0115 | 0.00289 | 0.00514 | 0.00484 | 0.00544 | 0.00254 | 0.00324 | nd |
| Sens 2 | 82% | 86% | 89% | 81% | 82% | 80% | 80% | 88% | nd |
| Spec 2 | 30% | 43% | 17% | 26% | 25% | 27% | 15% | 18% | nd |
| Cutoff 3 | 0.00293 | 0.00618 | 0.00162 | 0.00228 | 0.00108 | 0.00228 | 0.00105 | 0.00254 | nd |
| Sens 3 | 94% | 100% | 100% | 90% | 91% | 93% | 90% | 100% | nd |
| Spec 3 | 17% | 30% | 10% | 14% | 8% | 14% | 8% | 15% | nd |
| Cutoff 4 | 0.0347 | 0.0374 | 0.0353 | 0.0347 | 0.0374 | 0.0353 | 0.0347 | 0.0374 | nd |
| Sens 4 | 59% | 57% | 67% | 52% | 55% | 53% | 30% | 38% | nd |
| Spec 4 | 70% | 70% | 70% | 70% | 70% | 70% | 70% | 70% | nd |
| Cutoff 5 | 0.0850 | 0.0996 | 0.0863 | 0.0850 | 0.0996 | 0.0863 | 0.0850 | 0.0996 | nd |
| Sens 5 | 41% | 57% | 44% | 33% | 18% | 40% | 10% | 25% | nd |
| Spec 5 | 80% | 80% | 80% | 80% | 80% | 80% | 80% | 80% | nd |
| Cutoff 6 | 0.374 | 0.400 | 0.374 | 0.374 | 0.400 | 0.374 | 0.374 | 0.400 | nd |
| Sens 6 | 24% | 43% | 22% | 19% | 0% | 27% | 10% | 12% | nd |
| Spec 6 | 90% | 90% | 90% | 90% | 90% | 90% | 90% | 90% | nd |
| OR Quart 2 | 0.33 | >2.0 | 0 | 1.7 | 0.33 | 2.0 | 1.0 | 1.0 | nd |
|  | 0.34 | <0.57 | na | 0.48 | 0.34 | 0.42 | 1.00 | 1.0 | nd |
| p Value | 0.034 | >0.18 | na | 0.40 | 0.034 | 0.36 | 0.14 | 0.14 | nd |
| 95% CI of: OR Quart2 | 3.2 | na | na | 7.1 | 3.2 | 11 | 7.2 | 7.1 | nd |
| OR Quart 3 | 1.3 | >1.0 | 0.50 | 1.0 | 0.66 | 0.50 | 1.0 | 1.0 | nd |
|  | 0.70 | <1.0 | 0.57 | 1.0 | 0.65 | 0.57 | 1.0 | 1.0 | nd |
| p Value | 0.30 | >0.062 | 0.045 | 0.20 | 0.11 | 0.045 | 0.14 | 0.14 | nd |
| 95% CI of OR Quart3 | 6.0 | na | 5.5 | 5.0 | 4.0 | 5.5 | 7.1 | 7.1 | nd |
| OR Quart 4 | 3.0 | >4.0 | 3.0 | 3.4 | 1.7 | 4.1 | 2.0 | 1.0 | nd |
|  | 0.097 | <0.21 | 0.18 | 0.065 | 0.48 | 0.078 | 0.42 | 1.0 | nd |
| p Value | 0.82 | >0.45 | 0.61 | 0.93 | 0.40 | 0.86 | 0.37 | 0.14 | nd |
| 95% CI of OR Quart4 | 11 | na | 15 | 12 | 7.1 | 19 | 11 | 7.1 | nd |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 131 | 135 | 131 | 157 | 131 | 136 |
| Average | 140 | 141 | 140 | 163 | 140 | 129 |
| Stdev | 58.5 | 43.3 | 58.5 | 90.7 | 58.5 | 38.1 |
| p(t-test) | | 0.94 | | 0.14 | | 0.56 |
| Min | 31.0 | 83.4 | 31.0 | 53.4 | 31.0 | 69.9 |
| Max | 360 | 234 | 360 | 444 | 360 | 177 |
| n (Samp) | 816 | 16 | 816 | 16 | 816 | 10 |
| n (Patient) | 362 | 16 | 362 | 16 | 362 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 130 | 151 | 130 | 170 |
| Average | nd | nd | 140 | 152 | 140 | 160 |
| Stdev | nd | nd | 58.9 | 61.3 | 58.9 | 42.7 |
| p(t-test) | nd | nd | | 0.57 | | 0.37 |
| Min | nd | nd | 31.0 | 74.6 | 31.0 | 76.6 |
| Max | nd | nd | 444 | 265 | 444 | 217 |
| n (Samp) | nd | nd | 847 | 8 | 847 | 7 |
| n (Patient) | nd | nd | 375 | 8 | 375 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 130 | 116 | 130 | 124 | nd | nd |
| Average | 140 | 129 | 140 | 149 | nd | nd |
| Stdev | 58.8 | 48.9 | 58.8 | 96.8 | nd | nd |
| p(t-test) | | 0.58 | | 0.56 | nd | nd |
| Min | 31.0 | 77.1 | 31.0 | 53.4 | nd | nd |
| Max | 360 | 234 | 360 | 444 | nd | nd |
| n (Samp) | 811 | 10 | 811 | 13 | nd | nd |
| n (Patient) | 338 | 10 | 338 | 13 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.53 | nd | 0.46 | 0.56 | 0.56 | 0.49 | 0.47 | 0.64 | nd |
| SE | 0.074 | nd | 0.094 | 0.075 | 0.11 | 0.081 | 0.093 | 0.11 | nd |
| p | 0.72 | nd | 0.63 | 0.39 | 0.56 | 0.91 | 0.76 | 0.22 | nd |
| nCohort 1 | 816 | nd | 811 | 816 | 847 | 811 | 816 | 847 | nd |
| nCohort 2 | 16 | nd | 10 | 16 | 8 | 13 | 10 | 7 | nd |
| Cutoff 1 | 118 | nd | 100 | 110 | 102 | 100 | 120 | 164 | nd |
| Sens 1 | 75% | nd | 70% | 75% | 75% | 77% | 70% | 71% | nd |
| Spec 1 | 42% | nd | 31% | 36% | 31% | 31% | 42% | 70% | nd |
| Cutoff 2 | 107 | nd | 92.4 | 102 | 98.4 | 99.1 | 96.4 | 143 | nd |
| Sens 2 | 81% | nd | 80% | 81% | 88% | 85% | 80% | 86% | nd |
| Spec 2 | 34% | nd | 25% | 31% | 29% | 30% | 27% | 58% | nd |
| Cutoff 3 | 84.8 | nd | 84.8 | 74.4 | 74.4 | 81.8 | 76.5 | 76.5 | nd |
| Sens 3 | 94% | nd | 90% | 94% | 100% | 92% | 90% | 100% | nd |
| Spec 3 | 17% | nd | 18% | 10% | 11% | 16% | 12% | 12% | nd |
| Cutoff 4 | 166 | nd | 166 | 166 | 165 | 166 | 166 | 165 | nd |
| Sens 4 | 25% | nd | 20% | 31% | 38% | 23% | 20% | 57% | nd |
| Spec 4 | 70% | nd | 70% | 70% | 70% | 70% | 70% | 70% | nd |
| Cutoff 5 | 192 | nd | 192 | 192 | 190 | 192 | 192 | 190 | nd |
| Sens 5 | 12% | nd | 10% | 12% | 12% | 8% | 0% | 14% | nd |
| Spec 5 | 80% | nd | 80% | 80% | 80% | 80% | 80% | 80% | nd |
| Cutoff 6 | 222 | nd | 220 | 222 | 220 | 220 | 222 | 220 | nd |
| Sens 6 | 6% | nd | 10% | 12% | 12% | 8% | 0% | 0% | nd |
| Spec 6 | 90% | nd | 90% | 90% | 90% | 90% | 90% | 90% | nd |
| OR Quart 2 | 3.1 | nd | 1.0 | 2.0 | 2.0 | 2.0 | >6.2 | 0 | nd |
|  | 0.17 | nd | 1.00 | 0.42 | 0.57 | 0.42 | <0.092 | na | nd |
| p Value 95% CI of OR Quart2 | 0.61 | nd | 0.14 | 0.37 | 0.18 | 0.37 | >0.74 | na | nd |
|  | 15 | nd | 7.2 | 11 | 22 | 11 | na | na | nd |
| OR Quart 3 | 2.0 | nd | 2.0 | 3.1 | 2.0 | 2.5 | >2.0 | 4.1 | nd |
|  | 0.42 | nd | 0.42 | 0.17 | 0.57 | 0.27 | <0.57 | 0.21 | nd |
| p Value 95% CI of OR Quart3 | 0.37 | nd | 0.37 | 0.61 | 0.18 | 0.49 | >0.18 | 0.45 | nd |
|  | 11 | nd | 11 | 15 | 22 | 13 | na | 37 | nd |
| OR Quart 4 | 2.0 | nd | 1.0 | 2.0 | 3.0 | 1.0 | >2.0 | 2.0 | nd |
|  | 0.42 | nd | 1.00 | 0.42 | 0.34 | 1.0 | <0.56 | 0.57 | nd |
| p Value 95% CI of OR Quart4 | 0.37 | nd | 0.14 | 0.37 | 0.31 | 0.14 | >0.18 | 0.18 | nd |
|  | 11 | nd | 7.2 | 11 | 29 | 7.2 | na | 22 | nd |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1300 | 2180 | 1300 | 1520 | 1300 | 1450 |
| Average | 2160 | 3460 | 2160 | 8940 | 2160 | 1480 |
| Stdev | 2890 | 3590 | 2890 | 20600 | 2890 | 1120 |
| p(t-test) | | 0.032 | | 2.1E-17 | | 0.38 |
| Min | 0.0376 | 90.7 | 0.0376 | 113 | 0.0376 | 14.6 |
| Max | 45800 | 14600 | 45800 | 80100 | 45800 | 3740 |
| n (Samp) | 1483 | 23 | 1483 | 24 | 1483 | 14 |
| n (Patient) | 493 | 23 | 493 | 24 | 493 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1340 | 1920 | 1340 | 1840 | 1340 | 1590 |
| Average | 2320 | 3280 | 2320 | 9720 | 2320 | 1770 |
| Stdev | 3500 | 4440 | 3500 | 23400 | 3500 | 1070 |
| p(t-test) | | 0.41 | | 9.0E-10 | | 0.60 |
| Min | 0.0376 | 90.7 | 0.0376 | 679 | 0.0376 | 419 |
| Max | 68100 | 14600 | 68100 | 80100 | 68100 | 3740 |
| n (Samp) | 1540 | 9 | 1540 | 11 | 1540 | 11 |
| n (Patient) | 509 | 9 | 509 | 11 | 509 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1370 | 1670 | 1370 | 1250 | nd | nd |
| Average | 2280 | 2790 | 2280 | 6600 | nd | nd |
| Stdev | 3540 | 2840 | 3540 | 16200 | nd | nd |
| p(t-test) | | 0.60 | | 3.5E-6 | nd | nd |
| Min | 0.0376 | 150 | 0.0376 | 113 | nd | nd |
| Max | 80100 | 8180 | 80100 | 68100 | nd | nd |
| n (Samp) | 1513 | 13 | 1513 | 18 | nd | nd |
| n (Patient) | 472 | 13 | 472 | 18 | nd | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.60 | 0.58 | 0.53 | 0.57 | 0.66 | 0.48 | 0.46 | 0.54 | nd |
| SE | 0.063 | 0.100 | 0.082 | 0.061 | 0.090 | 0.069 | 0.079 | 0.089 | nd |
| p | 0.12 | 0.44 | 0.71 | 0.27 | 0.071 | 0.80 | 0.61 | 0.66 | nd |
| nCohort 1 | 1483 | 1540 | 1513 | 1483 | 1540 | 1513 | 1483 | 1540 | nd |
| nCohort 2 | 23 | 9 | 13 | 24 | 11 | 18 | 14 | 11 | nd |
| Cutoff 1 | 831 | 878 | 481 | 1020 | 1460 | 560 | 813 | 1250 | nd |
| Sens 1 | 74% | 78% | 77% | 71% | 73% | 72% | 71% | 73% | nd |
| Spec 1 | 33% | 34% | 17% | 40% | 54% | 21% | 32% | 47% | nd |
| Cutoff 2 | 404 | 831 | 333 | 560 | 1330 | 360 | 276 | 1020 | nd |
| Sens 2 | 83% | 89% | 85% | 83% | 82% | 83% | 86% | 82% | nd |
| Spec 2 | 16% | 33% | 11% | 22% | 50% | 12% | 9% | 40% | nd |
| Cutoff 3 | 163 | 90.6 | 163 | 291 | 1020 | 167 | 72.4 | 619 | nd |
| Sens 3 | 91% | 100% | 92% | 92% | 91% | 94% | 93% | 91% | nd |
| Spec 3 | 4% | 1% | 4% | 10% | 40% | 4% | 1% | 24% | nd |
| Cutoff 4 | 2250 | 2330 | 2330 | 2250 | 2330 | 2330 | 2250 | 2330 | nd |
| Sens 4 | 48% | 33% | 46% | 38% | 36% | 28% | 21% | 18% | nd |
| Spec 4 | 70% | 70% | 70% | 70% | 70% | 70% | 70% | 70% | nd |
| Cutoff 5 | 3140 | 3250 | 3240 | 3140 | 3250 | 3240 | 3140 | 3250 | nd |
| Sens 5 | 39% | 22% | 31% | 29% | 36% | 22% | 7% | 18% | nd |
| Spec 5 | 80% | 80% | 80% | 80% | 80% | 80% | 80% | 80% | nd |
| Cutoff 6 | 4640 | 5100 | 4800 | 4640 | 5100 | 4800 | 4640 | 5100 | nd |
| sCr | sCr or UO | sCr only | UO only | sCr or UO | sCr only | UO only | sCr or UO | sCr only | UO only |
| Sens 6 Spec 6 | 35% | 11% | 31% | 21% | 27% | 17% | 0% | 0% | nd |
|  | 90% | 90% | 90% | 90% | 90% | 90% | 90% | 90% | nd |
| OR Quart 2 | 0.80 | 2.0 | 0.50 | 1.00 | >3.0 | 0.60 | 1.7 | 1.5 | nd |
|  | 0.73 | 0.57 | 0.42 | 1.00 | <0.34 | 0.48 | 0.48 | 0.66 | nd |
| p Value 95% CI of OR Quart2 | 0.21 | 0.18 | 0.090 | 0.29 | >0.31 | 0.14 | 0.40 | 0.25 | nd |
|  | 3.0 | 22 | 2.7 | 3.5 | na | 2.5 | 7.1 | 9.0 | nd |
| OR Quart 3 | 0.60 | 4.0 | 0.50 | 1.2 | >4.0 | 0.80 | 0.67 | 2.0 | nd |
|  | 0.48 | 0.21 | 0.42 | 0.77 | <0.21 | 0.74 | 0.66 | 0.42 | nd |
| p Value 95% CI of OR Quart3 | 0.14 | 0.45 | 0.091 | 0.36 | >0.45 | 0.21 | 0.11 | 0.37 | nd |
|  | 2.5 | 36 | 2.7 | 4.0 | na | 3.0 | 4.0 | 11 | nd |
| OR Quart 4 | 2.2 | 2.0 | 1.2 | 1.6 | >4.0 | 1.2 | 1.3 | 1.00 | nd |
|  | 0.14 | 0.57 | 0.74 | 0.41 | <0.21 | 0.76 | 0.70 | 1.00 | nd |

(continued)

| sCr | sCr or UO | sCr only | UO only | sCr or UO | sCr only | UO only | sCr or UO | sCr only | UO only |
|---|---|---|---|---|---|---|---|---|---|
| p Value 95% CI ot OR Quart4 | 0.77 | 0.18 | 0.33 | 0.52 | >0.45 | 0.37 | 0.30 | 0.14 | nd |
| | 6.5 | 22 | 4.7 | 5.0 | na | 4.0 | 6.0 | 7.1 | nd |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.6 | 9.54 | 10.6 | 15.6 | 10.6 | 0.221 |
| Average | 19.1 | 19.1 | 19.1 | 437 | 19.1 | 10.9 |
| Stdev | 50.4 | 34.4 | 50.4 | 1770 | 50.4 | 20.2 |
| p(t-test) | | 1.00 | | 3.5E-12 | | 0.61 |
| Min | 0.00398 | 0.0190 | 0.00398 | 0.0190 | 0.00398 | 0.0184 |
| Max | 945 | 141 | 945 | 7540 | 945 | 50.9 |
| n (Samp) | 883 | 16 | 883 | 18 | 883 | 10 |
| n (Patient) | 367 | 16 | 367 | 18 | 367 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 10.6 | 20.6 | 10.6 | 0.338 |
| Average | nd | nd | 27.3 | 23.6 | 27.3 | 9.59 |
| Stdev | nd | nd | 254 | 9.89 | 254 | 19.1 |
| p(t-test) | nd | nd | | 0.97 | | 0.85 |
| Min | nd | nd | 0.00398 | 9.36 | 0.00398 | 0.0184 |
| Max | nd | nd | 7540 | 39.5 | 7540 | 50.9 |
| n (Samp) | nd | nd | 915 | 8 | 915 | 7 |
| n (Patient) | nd | nd | 380 | 8 | 380 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.7 | 11.4 | 10.7 | 4.85 | nd | nd |
| Average | 19.4 | 26.0 | 19.4 | 552 | nd | nd |
| Stdev | 50.4 | 42.2 | 50.4 | 2010 | nd | nd |
| p(t-test) | | 0.68 | | 4.9E-15 | nd | nd |
| Min | 0.00398 | 0.0208 | 0.00398 | 0.00398 | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 945 | 141 | 945 | 7540 | nd | nd |
| n (Samp) | 878 | 10 | 878 | 14 | nd | nd |
| n (Patient) | 342 | 10 | 342 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | nd | 0.56 | 0.58 | 0.73 | 0.45 | 0.35 | 0.34 | nd |
| SE | 0.073 | nd | 0.095 | 0.071 | 0.10 | 0.080 | 0.095 | 0.11 | nd |
| p | 0.91 | nd | 0.54 | 0.28 | 0.026 | 0.52 | 0.12 | 0.17 | nd |
| nCohort 1 | 883 | nd | 878 | 883 | 915 | 878 | 883 | 915 | nd |
| nCohort 2 | 16 | nd | 10 | 18 | 8 | 14 | 10 | 7 | nd |
| Cutoff 1 | 0.338 | nd | 7.28 | 8.63 | 20.5 | 0.0748 | 0.0187 | 0.0187 | nd |
| Sens 1 | 75% | nd | 70% | 72% | 75% | 79% | 70% | 71% | nd |
| Spec 1 | 28% | nd | 43% | 46% | 72% | 18% | 11% | 11% | nd |
| Cutoff 2 | 0.315 | nd | 0.338 | 0.0840 | 15.6 | 0.0187 | 0.0184 | 0.0184 | nd |
| Sens 2 | 88% | nd | 80% | 83% | 88% | 93% | 80% | 86% | nd |
| Spec 2 | 26% | nd | 28% | 20% | 63% | 11% | 9% | 9% | nd |
| Cutoff 3 | 0.0190 | nd | 0.315 | 0.0748 | 8.63 | 0.0187 | 0.0179 | 0.0179 | nd |
| Sens 3 | 94% | nd | 90% | 94% | 100% | 93% | 100% | 100% | nd |
| Spec 3 | 13% | nd | 26% | 17% | 46% | 11% | 8% | 8% | nd |
| Cutoff 4 | 19.5 | nd | 19.8 | 19.5 | 19.5 | 19.8 | 19.5 | 19.5 | nd |
| Sens 4 | 31% | nd | 40% | 39% | 75% | 29% | 20% | 14% | nd |
| Spec 4 | 70% | nd | 70% | 70% | 70% | 70% | 70% | 70% | nd |
| Cutoff 5 | 27.8 | nd | 28.1 | 27.8 | 27.8 | 28.1 | 27.8 | 27.8 | nd |
| Sens 5 | 19% | nd | 30% | 22% | 38% | 14% | 20% | 14% | nd |
| Spec 5 | 80% | nd | 81% | 80% | 80% | 81% | 80% | 80% | nd |
| Cutoff 6 | 40.0 | nd | 41.6 | 40.0 | 40,0 | 41.6 | 40.0 | 40.0 | nd |
| Sens 6 | 6% | nd | 10% | 11% | 0% | 14% | 20% | 14% | nd |
| Spec 6 | 90% | nd | 90% | 90% | 90% | 90% | 90% | 90% | nd |
| OR Quart 2 | 4.1 | nd | 4.1 | 0.75 | >1.0 | 2.0 | 0.50 | 1.0 | nd |
| | 0.077 | nd | 0.21 | 0.70 | <1.0 | 0.42 | 0.57 | 1.00 | nd |
| p Value 95% CI of OR Quart2 | 0.86 | nd | 0.45 | 0.17 | >0.062 | 0.37 | 0.045 | 0.062 | nd |
| | 19 | nd | 37 | 3.4 | na | 11 | 5.6 | 16 | nd |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart 3 | 1.00 | nd | 1.0 | 1.8 | >4.1 | 1.0 | 1.0 | 2.0 | nd |
|  | 1.00 | nd | 1.0 | 0.37 | <0.21 | 1.0 | 1.00 | 0.57 | nd |
| p Value 95% CI of OR Quart3 | 0.14 | nd | 0.062 | 0.51 | >0.45 | 0.14 | 0.14 | 0.18 | nd |
|  | 7.1 | nd | 16 | 6.1 | na | 7.2 | 7.2 | 22 | nd |
| OR Quart 4 | 2.0 | nd | 4.1 | 1.00 | >3.0 | 3.1 | 2.5 | 3.0 | nd |
|  | 0.42 | nd | 0.21 | 0.99 | <0.34 | 0.17 | 0.27 | 0.34 | nd |
| p Value 95% CI of OR Quart4 | 0.36 | nd | 0.45 | 0.25 | >0.31 | 0.61 | 0.49 | 0.31 | nd |
|  | 11 | nd | 37 | 4.0 | na | 15 | 13 | 29 | nd |

Fig. 10: Comparison of marker levels in EDTA samples collected from Cohort 1 (patients that did not progress beyond RIFLE stage 0, R, or I) and in EDTA samples collected from Cohort 2 (subjects who progress to RIFLE stage F) at 0, 24 hours, and 48 hours prior to the subject reaching RIFLE stage I.

**Angiopoietin-related protein 4**

| sCr or UO | 24hr prior to AKI stage | |
|---|---|---|
|  | Cohort 1 | Cohort 2 |
| Median | 32.9 | 96.2 |
| Average | 49.1 | 127 |
| Stdev | 97.1 | 115 |
| p(t-test) |  | 0.051 |
| Min | 1.77 | 25.0 |
| Max | 1900 | 339 |
| n (Samp) | 487 | 6 |
| n (Patient) | 218 | 6 |

|  | 24hr prior to AKI stage | | |
|---|---|---|---|
|  | sCr or UO | sCr only | UO only |
| AUC | 0.80 | nd | nd |
| SE | 0.11 | nd | nd |
| p | 0.0049 | nd | nd |
| nCohort 1 | 487 | nd | nd |
| nCohort 2 | 6 | nd | nd |

(continued)

| | 24hr prior to AKI stage | | |
|---|---|---|---|
| | sCr or UO | sCr only | UO only |
| Cutoff 1 Sens 1 | 52.4 | nd | nd |
| | 83% | nd | nd |
| Spec 1 | 74% | nd | nd |
| Cutoff 2 Sens 2 Spec 2 | 52.4 | nd | nd |
| | 83% | nd | nd |
| | 74% | nd | nd |
| Cutoff 3 Sens 3 Spec 3 | 25.0 | nd | nd |
| | 100% | nd | nd |
| | 37% | nd | nd |
| Cutoff 4 Sens 4 Spec 4 | 47.5 | nd | nd |
| | 83% | nd | nd |
| | 70% | nd | nd |
| Cutoff 5 Sens 5 Spec 5 | 61.9 | nd | nd |
| | 50% | nd | nd |
| | 80% | nd | nd |
| Cutoff 6 Sens 6 Spec 6 | 92.2 | nd | nd |
| | 50% | nd | nd |
| | 90% | nd | nd |
| OR Quart 2 | >1.0 | nd | nd |
| | <1.00 | nd | nd |
| p Value 95% CI of OR Quart2 | >0.062 | nd | nd |
| | na | nd | nd |
| OR Quart 3 | >1.0 | nd | nd |
| | <1.00 | nd | nd |
| p Value 95% CI of OR Quart3 | >0.062 | nd | nd |
| | na | nd | nd |
| OR Quart 4 | >4.1 | nd | nd |
| | <0.21 | nd | nd |
| p Value 95% CI of OR Quart4 | >0.45 | nd | nd |
| | na | nd | nd |

**Amphiregulin**

| sCr or UO | 24hr prior to AKI stage | |
|---|---|---|
| | Cohort 1 | Cohort 2 |
| Median | 22.4 | 41.7 |

(continued)

| sCr or UO | 24hr prior to AKI stage | |
|---|---|---|
| | Cohort 1 | Cohort 2 |
| Average | 28.3 | 40.8 |
| Stdev | 22.9 | 16.4 |
| p(t-test) | | 0.18 |
| Min | 0.00246 | 19.8 |
| Max | 198 | 60.8 |
| n (Samp) | 478 | 6 |
| n (Patient) | 214 | 6 |

| | 24hr prior to AKI stage | | |
|---|---|---|---|
| | sCr or UO | sCr only | UO only |
| AUC | 0.75 | nd | nd |
| SE | 0.12 | nd | nd |
| p | 0.034 | nd | nd |
| nCohort 1 | 478 | nd | nd |
| nCohort 2 | 6 | nd | nd |
| Cutoff 1 | 24.8 | nd | nd |
| Sens 1 | 83% | nd | nd |
| Spec 1 | 56% | nd | nd |
| Cutoff 2 | 24.8 | nd | nd |
| Sens 2 | 83% | nd | nd |
| Spec 2 | 56% | nd | nd |
| Cutoff 3 | 19.6 | nd | nd |
| Sens 3 | 100% | nd | nd |
| Spec 3 | 43% | nd | nd |
| Cutoff 4 | 32.2 | nd | nd |
| Sens 4 | 67% | nd | nd |
| Spec 4 | 70% | nd | nd |
| Cutoff 5 | 39.9 | nd | nd |
| Sens 5 | 50% | nd | nd |
| Spec 5 | 80% | nd | nd |
| Cutoff 6 | 54.0 | nd | nd |
| Sens 6 | 33% | nd | nd |
| Spec 6 | 90% | nd | nd |
| OR | >1.0 | nd | nd |
| Quart 2 | <1.00 | nd | nd |
| p Value | >0.062 | nd | nd |

(continued)

|  | 24hr prior to AKI stage | | |
|  | sCr or UO | sCr only | UO only |
| --- | --- | --- | --- |
| 95% CI of OR Quart2 | na | nd | nd |
| OR Quart 3 | >1.0 | nd | nd |
|  | <1.00 | nd | nd |
| p Value 95% CI of OR Quart3 | >0.062 | nd | nd |
|  | na | nd | nd |
| OR Quart 4 | >4.1 | nd | nd |
|  | <0.21 | nd | nd |
| p Value 95% CI of OR Quart4 | >0.46 | nd | nd |
|  | na | nd | nd |

**Betacellulin**

| sCr or UO | 24hr prior to AKI stage | |
|  | Cohort 1 | Cohort 2 |
| --- | --- | --- |
| Median | 4.02 | 6.55 |
| Average | 4.75 | 12.8 |
| Stdev | 5.32 | 16.1 |
| p(t-test) |  | 4.7E-4 |
| Min | 0.00226 | 0.00289 |
| Max | 60.7 | 43.4 |
| n (Samp) | 479 | 6 |
| n (Patient) | 216 | 6 |

|  | 24hr prior to AKI stage | | |
|  | sCr or UO | sCr only | UO only |
| --- | --- | --- | --- |
| AUC | 0.64 | nd | nd |
| SE | 0.12 | nd | nd |
| p | 0.25 | nd | nd |
| nCohort 1 | 479 | nd | nd |
| nCohort 2 | 6 | nd | nd |
| Cutoff 1 | 3.09 | nd | nd |
| Sens 1 | 83% | nd | nd |
| Spec 1 | 36% | nd | nd |
| Cutoff 2 | 3.09 | nd | nd |
| Sens 2 | 83% | nd | nd |

(continued)

| | 24hr prior to AKI stage | | |
|---|---|---|---|
| | sCr or UO | sCr only | UO only |
| Spec 2 | 36% | nd | nd |
| Cutoff 3 | 0.00282 | nd | nd |
| Sens 3 | 100% | nd | nd |
| Spec 3 | 5% | nd | nd |
| Cutoff 4 | 5.42 | nd | nd |
| Sens 4 | 50% | nd | nd |
| Spec 4 | 70% | nd | nd |
| Cutoff 5 | 6.66 | nd | nd |
| Sens 5 | 50% | nd | nd |
| Spec 5 | 80% | nd | nd |
| Cutoff 6 | 8.60 | nd | nd |
| Sens 6 | 50% | nd | nd |
| Spec 6 | 90% | nd | nd |
| OR Quart 2 | 1.0 | nd | nd |
| | 1.0 | nd | nd |
| p Value 95% CI of OR Quart2 | 0.062 | nd | nd |
| | 16 | nd | nd |
| OR Quart 3 | 1.0 | nd | nd |
| | 1.0 | nd | nd |
| p Value 95% CI of OR Quart3 | 0.062 | nd | nd |
| | 16 | nd | nd |
| OR Quart 4 | 3.0 | nd | nd |
| | 0.34 | nd | nd |
| p Value | 0.31 | nd | nd |
| 95% CI of OR Quart4 | 29 | nd | nd |

**Proepiregulin**

| sCr or UO | 24hr prior to AKI stage | |
|---|---|---|
| | Cohort 1 | Cohort 2 |
| Median | 0.392 | 1.07 |
| Average | 0.771 | 2.58 |
| Stdev | 2.33 | 4.32 |
| p(t-test) | | 0.063 |
| Min | 0.000152 | 0.0249 |
| Max | 32.1 | 11.3 |

(continued)

| sCr or UO | 24hr prior to AKI stage | |
|---|---|---|
| | Cohort 1 | Cohort 2 |
| n (Samp) | 478 | 6 |
| n (Patient) | 215 | 6 |

| | 24hr prior to AKI stage | | |
|---|---|---|---|
| | sCr or UO | sCr only | UO only |
| AUC | 0.72 | nd | nd |
| SE | 0.12 | nd | nd |
| p | 0.067 | nd | nd |
| nCohort 1 | 478 | nd | nd |
| nCohort 2 | 6 | nd | nd |
| Cutoff 1 | 0.513 | nd | nd |
| Sens 1 | 83% | nd | nd |
| Spec 1 | 61% | nd | nd |
| Cutoff 2 | 0.513 | nd | nd |
| Sens 2 | 83% | nd | nd |
| Spec 2 | 61% | nd | nd |
| Cutoff 3 | 0.0201 | nd | nd |
| Sens 3 | 100% | nd | nd |
| Spec 3 | 5% | nd | nd |
| Cutoff 4 | 0.645 | nd | nd |
| Sens 4 | 67% | nd | nd |
| Spec 4 | 70% | nd | nd |
| Cutoff 5 | 0.806 | nd | nd |
| Sens 5 | 67% | nd | nd |
| Spec 5 | 81% | nd | nd |
| Cutoff 6 | 1.24 | nd | nd |
| Sens 6 | 50% | nd | nd |
| Spec 6 | 90% | nd | nd |
| OR Quart 2 | 0 | nd | nd |
| | 11a | nd | nd |
| p Value 95% CI of OR Quart2 | na | nd | nd |
| | na | nd | nd |
| OR Quart 3 | 1.0 | nd | nd |
| | 1.0 | nd | nd |
| p Value 95% CI of OR Quart3 | 0.062 | nd | nd |
| | 16 | nd | nd |

(continued)

|  | 24hr prior to AKI stage | | |
|---|---|---|---|
|  | sCr or UO | sCr only | UO only |
| OR Quart 4 | 4.1 | nd | nd |
|  | 0.21 | nd | nd |
| p Value 95% CI of OR Quart4 | 0.45 | nd | nd |
|  | 37 | nd | nd |

**Fibroblast growth factor 19**

| sCr or UO | 24hr prior to AKI stage | |
|---|---|---|
|  | Cohort 1 | Cohort 2 |
| Median | 0.157 | 0.271 |
| Average | 0.198 | 0.414 |
| Stdev | 0.198 | 0.369 |
| p(t-test) |  | 0.0090 |
| Min | 2.92E-5 | 0.0449 |
| Max | 2.09 | 0.971 |
| n (Samp) | 487 | 6 |
| n (Patient) | 218 | 6 |

|  | 24hr prior to AKI stage | | |
|---|---|---|---|
|  | sCr or UO | sCr only | UO only |
| AUC | 0.68 | nd | nd |
| SE | 0.12 | nd | nd |
| p | 0.14 | nd | nd |
| nCohort 1 | 487 | nd | nd |
| nCohort 2 | 6 | nd | nd |
| Cutoff 1 | 0.172 | nd | nd |
| Sens 1 | 83% | nd | nd |
| Spec 1 | 54% | nd | nd |
| Cutoff 2 | 0.172 | nd | nd |
| Sens 2 | 83% | nd | nd |
| Spec 2 | 54% | nd | nd |
| Cutoff 3 | 0.0430 | nd | nd |
| Sens 3 | 100% | nd | nd |
| Spec 3 | 16% | nd | nd |
| Cutoff 4 | 0.233 | nd | nd |

(continued)

|  | 24hr prior to AKI stage | | |
|---|---|---|---|
|  | sCr or UO | sCr only | UO only |
| Sens 4 | 50% | nd | nd |
| Spec 4 | 70% | nd | nd |
| Cutoff 5 | 0.300 | nd | nd |
| Sens 5 | 50% | nd | nd |
| Spec 5 | 80% | nd | nd |
| Cutoff 6 | 0.395 | nd | nd |
| Sens 6 | 33% | nd | nd |
| Spec 6 | 90% | nd | nd |
| OR Quart 2 | 0 | nd | nd |
|  | na | nd | nd |
| p Value 95% CI of OR Quart2 | na | nd | nd |
|  | na | nd | nd |
| OR Quart 3 | 2.0 | nd | nd |
|  | 0.57 | nd | nd |
| p Value | 0.18 | nd | nd |
| 95% CI of OR Quart3 | 23 | nd | nd |
| OR Quart 4 | 3.0 | nd | nd |
|  | 0.34 | nd | nd |
| p Value 95% CI of OR Quart4 | 0.31 | nd | nd |
|  | 29 | nd | nd |

**Fibroblast growth factor 21**

| sCr or UO | 24hr prior to AKI stage | |
|---|---|---|
|  | Cohort 1 | Cohort 2 |
| Median | 0.0538 | 0.103 |
| Average | 0.214 | 0.141 |
| Stdev | 0.529 | 0.145 |
| p(t-test) |  | 0.74 |
| Min | 4.60E-6 | 0.0212 |
| Max | 5.72 | 0.418 |
| n (Samp) | 487 | 6 |
| n (Patient) | 218 | 6 |

|  | 24hr prior to AKI stage | | |
|---|---|---|---|
|  | sCr or UO | sCr only | UO only |
| AUC | 0.61 | nd | nd |
| SE | 0.12 | nd | nd |
| p | 0.39 | nd | nd |
| nCohort 1 | 487 | nd | nd |
| nCohort 2 | 6 | nd | nd |
| Cutoff 1 | 0.0388 | nd | nd |
| Sens 1 | 83% | nd | nd |
| Spec 1 | 42% | nd | nd |
| Cutoff 2 | 0.0388 | nd | nd |
| Sens 2 | 83% | nd | nd |
| Spec 2 | 42% | nd | nd |
| Cutoff 3 | 0.0209 | nd | nd |
| Sens 3 | 100% | nd | nd |
| Spec 3 | 29% | nd | nd |
| Cutoff 4 | 0.129 | nd | nd |
| Sens 4 | 33% | nd | nd |
| Spec 4 | 70% | nd | nd |
| Cutoff 5 | 0.224 | nd | nd |
| Sens 5 | 17% | nd | nd |
| Spec 5 | 80% | nd | nd |
| Cutoff 6 | 0.495 | nd | nd |
| Sens 6 | 0% | nd | nd |
| Spec 6 | 90% | nd | nd |
| OR Quart 2 | >2.0 | nd | nd |
|  | <0.56 | nd | nd |
| p Value 95% CI of OR Quart2 | >0.18 | nd | nd |
|  | na | nd | nd |
| OR Quart 3 | >3.1 | nd | nd |
|  | <0.33 | nd | nd |
| p Value 95% CI of OR Quart3 | >0.32 | nd | nd |
|  | na | nd | nd |
| OR Quart 4 | >1.0 | nd | nd |
|  | <1.0 | nd | nd |
| p Value 95% CI of OR Quart4 | >0.062 | nd | nd |
|  | na | nd | nd |

**Heparin-binding EGF-like growth factor**

| sCr or UO | 24hr prior to AKI stage | |
|---|---|---|
| | Cohort 1 | Cohort 2 |
| Median | 16.3 | 46.9 |
| Average | 20.0 | 74.3 |
| Stdev | 15.8 | 74.4 |
| p(t-test) | | 2.1E-13 |
| Min | 5.20 | 21.0 |
| Max | 186 | 222 |
| n (Samp) | 476 | 6 |
| n (Patient) | 215 | 6 |

| | 24hr prior to AKI stage | | |
|---|---|---|---|
| | sCr or UO | sCr only | UO only |
| AUC | 0.92 | nd | nd |
| SE | 0.076 | nd | nd |
| p | 2.4E-8 | nd | nd |
| nCohort 1 | 476 | nd | nd |
| nCohort 2 | 6 | nd | nd |
| Cutoff 1 | 35.8 | nd | nd |
| Sens 1 | 83% | nd | nd |
| Spec 1 | 92% | nd | nd |
| Cutoff 2 | 35.8 | nd | nd |
| Sens 2 | 83% | nd | nd |
| Spec 2 | 92% | nd | nd |
| Cutoff 3 | 20.9 | nd | nd |
| Sens 3 | 100% | nd | nd |
| Spec 3 | 73% | nd | nd |
| Cutoff 4 | 20.2 | nd | nd |
| Sens 4 | 100% | nd | nd |
| Spec 4 | 70% | nd | nd |
| Cutoff 5 | 23.6 | nd | nd |
| Sens 5 | 83% | nd | nd |
| Spec 5 | 80% | nd | nd |
| Cutoff 6 | 31.9 | nd | nd |
| Sens 6 | 83% | nd | nd |
| Spec 6 | 90% | nd | nd |
| OR | >0 | nd | nd |
| Quart 2 | <na | nd | nd |

(continued)

|  | 24hr prior to AKI stage | | |
|  | sCr or UO | sCr only | UO only |
| --- | --- | --- | --- |
| p Value | >na | nd | nd |
| 95% CI of OR Quart2 | na | nd | nd |
| OR Quart 3 | >1.0 | nd | nd |
|  | <1.00 | nd | nd |
| p Value 95% CI of OR Quart3 | >0.062 | nd | nd |
|  | na | nd | nd |
| OR Quart 4 | >5.2 | nd | nd |
|  | <0.14 | nd | nd |
| p Value 95% CI of OR Quart4 | >0.60 | nd | ud |
|  | na | nd | nd |

**Tenascin**

| sCr or UO | 24hr prior to AKI stage | |
|  | Cohort 1 | Cohort 2 |
| --- | --- | --- |
| Median | 593 | 1520 |
| Average | 1170 | 1380 |
| Stdev | 2220 | 718 |
| p(t-test) |  | 0.82 |
| Min | 43.9 | 323 |
| Max | 26100 | 2220 |
| n (Samp) | 486 | 6 |
| n (Patient) | 217 | 6 |

|  | 24hr prior to AKI stage | | |
|  | sCr or UO | sCr only | UO only |
| --- | --- | --- | --- |
| AUC | 0.72 | nd | nd |
| SE | 0.12 | nd | nd |
| p | 0.062 | nd | nd |
| nCohort 1 | 486 | nd | nd |
| nCohort 2 | 6 | nd | nd |
| Cutoff 1 | 814 | nd | nd |
| Sens 1 | 83% | nd | nd |
| Spec 1 | 64% | nd | nd |
| Cutoff 2 | 814 | nd | nd |

(continued)

| | 24hr prior to AKI stage | | |
|---|---|---|---|
| | sCr or UO | sCr only | UO only |
| Sens 2 | 83% | nd | nd |
| Spec 2 | 64% | nd | nd |
| Cutoff 3 | 322 | nd | nd |
| Sens 3 | 100% | nd | nd |
| Spec 3 | 23% | nd | nd |
| Cutoff 4 | 964 | nd | nd |
| Sens 4 | 67% | nd | nd |
| Spec 4 | 70% | nd | nd |
| Cutoff 5 | 1250 | nd | nd |
| Sens 5 | 67% | nd | nd |
| Spec 5 | 80% | nd | nd |
| Cutoff 6 | 2140 | nd | nd |
| Sens 6 | 17% | nd | nd |
| Spec 6 | 90% | nd | nd |
| OR Quart 2 | 0 | nd | nd |
| | na | nd | nd |
| p Value 95% CI of OR Quart2 | na | nd | nd |
| | na | nd | nd |
| OR Quart 3 | 1.0 | nd | nd |
| | 1.0 | nd | nd |
| p Value 95% CI of OR Quart3 | 0.062 | nd | nd |
| | 16 | nd | nd |
| OR Quart 4 | 4.1 | nd | nd |
| | 0.21 | nd | nd |
| p Value | 0.45 | nd | nd |
| 95% CI of OR Quart4 | 37 | nd | nd |

Fig. 11: Comparison of marker levels in enroll urine samples collected from Cohort 1 (patients that did not progress beyond RIFLE stage 0 or R within 48hrs) and in enroll urine samples collected from Cohort 2 (subjects reaching RIFLE stage I or F within 48hrs). Enroll samples from patients already at RIFLE stage I or F were included in Cohort 2.

**Angiogenin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 4420 | 8700 | 5060 | 8890 | 4570 | 8690 |
| Average | 7680 | 11200 | 8320 | 11600 | 7900 | 10900 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Stdev | 7650 | 8940 | 7980 | 10100 | 7910 | 8530 |
| p(t-test) |  | 1.9E-4 |  | 0.092 |  | 0.0030 |
| Min | 0.00873 | 54.6 | 0.00873 | 647 | 0.00873 | 54.6 |
| Max | 30600 | 30600 | 30600 | 30600 | 30600 | 30600 |
| n (Samp) | 352 | 93 | 421 | 18 | 343 | 82 |
| n (Patient) | 352 | 93 | 421 | 18 | 343 | 82 |

|  | At Enrollment | | |
|---|---|---|---|
|  | sCr or UO | sCr only | UO only |
| AUC | 0.62 | 0.58 | 0.61 |
| SE | 0.034 | 0.072 | 0.036 |
| p | 6.5E-4 | 0.28 | 0.0017 |
| nCohort 1 | 352 | 421 | 343 |
| nCohort 2 | 93 | 18 | 82 |
| Cutoff 1 | 4290 | 4330 | 4330 |
| Sens 1 | 71% | 72% | 71% |
| Spec 1 | 50% | 46% | 50% |
| Cutoff 2 | 2200 | 995 | 2580 |
| Sens 2 | 81% | 83% | 80% |
| Spec 2 | 28% | 12% | 33% |
| Cutoff 3 | 985 | 831 | 1050 |
| Sens 3 | 90% | 94% | 90% |
| Spec 3 | 12% | 9% | 13% |
| Cutoff 4 | 9070 | 10600 | 9500 |
| Sens 4 | 49% | 44% | 48% |
| Spec 4 | 70% | 70% | 70% |
| Cutoff 5 | 15600 | 16600 | 15800 |
| Sens 5 | 33% | 33% | 29% |
| Spec 5 | 80% | 80% | 80% |
| Cutoff 6 | 20400 | 20400 | 20400 |
| Sens 6 | 14% | 17% | 12% |
| Spec 6 | 93% | 92% | 92% |
| OR Quart 2 | 0.93 | 0.39 | 1.4 |
| p Value | 0.85 | 0.26 | 0.43 |
| 95% CI of | 0.44 | 0.073 | 0.63 |
| OR Quart2 | 2.0 | 2.0 | 3.0 |

(continued)

|  | At Enrollment | | |
|---|---|---|---|
|  | sCr or UO | sCr only | UO only |
| OR Quart 3 | 1.5 | 0.78 | 2.0 |
| p Value | 0.23 | 0.72 | 0.071 |
| 95% CI of | 0.77 | 0.21 | 0.94 |
| OR Quart3 | 3.0 | 3.0 | 4.2 |
| OR Quart 4 | 2.6 | 1.4 | 2.7 |
| p Value | 0.0038 | 0.57 | 0.0078 |
| 95% CI of | 1.4 | 0.43 | 1.3 |
| OR Quart4 | 5.0 | 4.6 | 5.5 |

**Angiopoietin-related protein 4**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 9.75 | 14.6 | 10.6 | 20.7 | 10.6 | 13.0 |
| Average | 39.3 | 51.6 | 40.1 | 74.6 | 39.8 | 49.3 |
| Stdev | 89.3 | 95.9 | 89.2 | 118 | 89.5 | 96.1 |
| p(t-test) |  | 0.29 |  | 0.13 |  | 0.43 |
| Min | 0.000466 | 0.000466 | 0.000466 | 0.000466 | 0.000466 | 0.000466 |
| Max | 708 | 413 | 708 | 413 | 708 | 413 |
| n (Samp) | 294 | 79 | 351 | 17 | 296 | 69 |
| n (Patient) | 294 | 79 | 351 | 17 | 296 | 69 |

|  | At Enrollment | | |
|---|---|---|---|
|  | sCr or UO | sCr only | UO only |
| AUC | 0.58 | 0.61 | 0.56 |
| SE | 0.037 | 0.074 | 0.039 |
| p | 0.036 | 0.12 | 0.13 |
| nCohort 1 | 294 | 351 | 296 |
| nCohort 2 | 79 | 17 | 69 |
| Cutoff 1 | 7.09 | 8.48 | 7.09 |
| Sens 1 | 71% | 71% | 71% |
| Spec 1 | 38% | 43% | 38% |
| Cutoff 2 | 5.29 | 5.29 | 5.10 |
| Sens 2 | 81% | 82% | 81% |
| Spec 2 | 30% | 28% | 28% |

(continued)

| | At Enrollment | | |
|---|---|---|---|
| | sCr or UO | sCr only | UO only |
| Cutoff 3 | 3.28 | 2.94 | 3.21 |
| Sens 3 | 91% | 94% | 91% |
| Spec 3 | 15% | 13% | 14% |
| Cutoff 4 | 18.1 | 19.6 | 19.4 |
| Sens 4 | 46% | 53% | 39% |
| Spec 4 | 70% | 70% | 70% |
| Cutoff 5 | 36.6 | 36.2 | 37.2 |
| Sens 5 | 25% | 47% | 22% |
| Spec 5 | 80% | 80% | 80% |
| Cutoff 6 | 96.2 | 96.5 | 96.2 |
| Sens 6 | 15% | 24% | 14% |
| Spec 6 | 90% | 90% | 90% |
| OR Quart 2 | 1.5 | 1.3 | 1.4 |
| p Value | 0.26 | 0.70 | 0.43 |
| 95% CI of | 0.73 | 0.29 | 0.63 |
| OR Quart2 | 3.3 | 6.2 | 3.0 |
| OR Quart 3 | 1.4 | 0.66 | 1.4 |
| p Value | 0.44 | 0.65 | 0.43 |
| 95% CI of | 0.63 | 0.11 | 0.63 |
| OR Quart3 | 2.9 | 4.0 | 3.0 |
| OR Quart 4 | 2.3 | 2.8 | 1.9 |
| p Value | 0.026 | 0.13 | 0.10 |
| 95% CI of | 1.1 | 0.73 | 0.88 |
| OR Quart4 | 4.7 | 11 | 4.0 |

**Amphiregulin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 23.5 | 39.4 | 25.0 | 42.8 | 25.2 | 35.9 |
| Average | 72.3 | 177 | 90.2 | 248 | 73.1 | 162 |
| Stdev | 202 | 527 | 298 | 478 | 211 | 522 |
| p(t-test) | | 0.012 | | 0.082 | | 0.043 |
| Min | 0.00131 | 2.63 | 0.00131 | 10.1 | 0.00131 | 2.63 |
| Max | 1640 | 3480 | 3480 | 1710 | 1710 | 3480 |
| n (Samp) | 245 | 68 | 296 | 12 | 232 | 60 |

(continued)

|  | sCr or UO | | sCr only | | UO only | |
| --- | --- | --- | --- | --- | --- | --- |
|  | Cohort 1 | Cohort 2 | Cohort 1 | Cohort 2 | Cohort 1 | Cohort 2 |
| n (Patient) | 245 | 68 | 296 | 12 | 232 | 60 |

|  | At Enrollment | | |
| --- | --- | --- | --- |
|  | sCr or UO | sCr only | UO only |
| AUC | 0.61 | 0.65 | 0.59 |
| SE | 0.040 | 0.088 | 0.042 |
| p | 0.0062 | 0.081 | 0.027 |
| nCohort 1 | 245 | 296 | 232 |
| nCohort 2 | 68 | 12 | 60 |
| Cutoff 1 | 18.9 | 18.9 | 18.9 |
| Sens 1 | 72% | 83% | 70% |
| Spec 1 | 41% | 39% | 39% |
| Cutoff 2 | 15.0 | 18.9 | 15.0 |
| Sens 2 | 81% | 83% | 80% |
| Spec 2 | 35% | 39% | 34% |
| Cutoff 3 | 10.0 | 12.6 | 10.0 |
| Sens 3 | 91% | 92% | 90% |
| Spec 3 | 21% | 26% | 20% |
| Cutoff 4 | 45.7 | 49.1 | 45.9 |
| Sens 4 | 44% | 42% | 45% |
| Spec 4 | 70% | 70% | 70% |
| Cutoff 5 | 59.5 | 68.6 | 59.5 |
| Sens 5 | 34% | 42% | 33% |
| Spec 5 | 80% | 80% | 80% |
| Cutoff 6 | 118 | 133 | 114 |
| Sens 6 | 22% | 42% | 20% |
| Spec 6 | 90% | 90% | 90% |
| OR Quart 2 | 2.0 | 3.1 | 1.9 |
| p Value | 0.099 | 0.33 | 0.14 |
| 95% CI of | 0.87 | 0.31 | 0.81 |
| OR Quart2 | 4.8 | 30 | 4.5 |
| OR Quart 3 | 1.5 | 3.1 | 1.0 |
| p Value | 0.38 | p.33 | 1.0 |
| 95% CI of | 0.62 | 0.31 | 0.39 |
| OR Quart3 | 3.6 | 30 | 2.6 |
| OR Quart 4 | 3.3 | 5.3 | 2.9 |

(continued)

|  | At Enrollment | | |
|---|---|---|---|
|  | sCr or UO | sCr only | UO only |
| p Value | 0.0037 | 3.13 | 0.012 |
| 95% CI of | 1.5 | 0.60 | 1.3 |
| OR Quart4 | 7.5 | 46 | 6.6 |

**Betacellulin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.793 | 1.22 | 0.777 | 1.80 | 0.909 | 0.777 |
| Average | 1.41 | 1.56 | 1.42 | 2.12 | 1.35 | 1.47 |
| Stdev | 2.13 | 1.94 | 2.11 | 1.52 | 1.63 | 1.98 |
| p(t-test) |  | 0.59 |  | 0.25 |  | 0.62 |
| Min | 0.00179 | 0.00240 | 0.00179 | 0.00352 | 0.00179 | 0.00240 |
| Max | 23.1 | 11.6 | 23.1 | 4.57 | 9.19 | 11.6 |
| n (Samp) | 245 | 68 | 296 | 12 | 232 | 60 |
| n (Patient) | 245 | 68 | 296 | 12 | 232 | 60 |

|  | At Enrollment | | |
|---|---|---|---|
|  | sCr or UO | sCr only | UO only |
| AUC | 0.53 | 0.67 | 0.50 |
| SE | 0.040 | 0.087 | 0.042 |
| p | 0.48 | 0.049 | 0.94 |
| nCohort 1 | 245 | 296 | 232 |
| nCohort 2 | 68 | 12 | 60 |
| Cutoff 1 | 0.0407 | 1.51 | 0.0407 |
| Sens 1 | 74% | 75% | 70% |
| Spec 1 | 28% | 59% | 28% |
| Cutoff 2 | 0.00342 | 0.793 | 0.00342 |
| Sens 2 | 82% | 83% | 80% |
| Spec 2 | 18% | 51% | 19% |
| Cutoff 3 | 0.00246 | 0.0522 | 0.00246 |
| Sens 3 | 91% | 92% | 90% |
| Spec 3 | 10% | 31% | 11% |
| Cutoff 4 | 1.76 | 1.87 | 1.78 |
| Sens 4 | 37% | 50% | 33% |

(continued)

|  | At Enrollment | | |
| --- | --- | --- | --- |
|  | sCr or UO | sCr only | UO only |
| Spec 4 | 70% | 70% | 70% |
| Cutoff 5 | 2.41 | 2.42 | 2.41 |
| Sens 5 | 25% | 33% | 23% |
| Spec 5 | 80% | 81% | 80% |
| Cutoff 6 | 3.36 | 3.36 | 3.36 |
| Sens 6 | 15% | 25% | 13% |
| Spec 6 | 91% | 90% | 91% |
| OR Quart 2 | 0.85 | 1.0 | 0.78 |
| p Value | 0.69 | 1.0 | 0.54 |
| 95% CI of | 0.39 | 0.061 | 0.35 |
| OR Quart2 | 1.9 | 16 | 1.7 |
| OR Quart 3 | 1.0 | 5.3 | 0.78 |
| p Value | 1.0 | 0.13 | 0.54 |
| 95% CI of | 0.47 | 0.60 | 0.35 |
| OR Quart3 | 2.1 | 46 | 1.7 |
| OR Quart 4 | 1.1 | 5.3 | 0.85 |
| p Value | 0.74 | 0.13 | 0.69 |
| 95% CI of | 0.54 | 0.60 | 0.39 |
| OR Quart4 | 2.4 | 46 | 1.9 |

**Endostatin**

|  | sCr or UO | | sCr only | | UO only | |
| --- | --- | --- | --- | --- | --- | --- |
|  | Cohort 1 | Cohort 2 | Cohort 1 | Cohort 2 | Cohort 1 | Cohort 2 |
| Median | 4620 | 5530 | 4710 | 9920 | 4690 | 5400 |
| Average | 16600 | 17200 | 16300 | 24300 | 16400 | 16900 |
| Stdev | 32000 | 27800 | 31100 | 28800 | 31000 | 28600 |
| p(t-test) |  | 0.87 |  | 0.28 |  | 0.90 |
| Min | 0.0130 | 261 | 0.0130 | 748 | 0.0130 | 261 |
| Max | 238000 | 148000 | 238000 | 110000 | 238000 | 148000 |
| n (Samp) | 352 | 93 | 421 | 18 | 343 | 82 |
| n (Patient) | 352 | 93 | 421 | 18 | 343 | 82 |

|  | At Enrollment | | |
|---|---|---|---|
|  | sCr or UO | sCr only | UO only |
| AUC | 0.56 | 0.61 | 0.55 |
| SE | 0.034 | 0.072 | 0.036 |
| p | 0.096 | 0.12 | 0.20 |
| nCohort 1 | 352 | 421 | 343 |
| nCohort 2 | 93 | 18 | 82 |
| Cutoff 1 | 3530 | 2970 | 3530 |
| Sens 1 | 71% | 72% | 71% |
| Spec 1 | 42% | 35% | 41% |
| Cutoff 2 | 2540 | 2540 | 2570 |
| Sens 2 | 81% | 83% | 80% |
| Spec 2 | 32% | 30% | 32% |
| Cutoff 3 | 1510 | 1410 | 1510 |
| Sens 3 | 90% | 94% | 90% |
| Spec 3 | 15% | 13% | 15% |
| Cutoff 4 | 9580 | 9440 | 10000 |
| Sens 4 | 33% | 50% | 32% |
| Spec 4 | 70% | 70% | 70% |
| Cutoff 5 | 20800 | 20000 | 21900 |
| Sens 5 | 23% | 44% | 21% |
| Spec 5 | 80% | 80% | 80% |
| Cutoff 6 | 43700 | 43700 | 43700 |
| Sens 6 | 12% | 17% | 11% |
| Spec 6 | 90% | 90% | 90% |
| OR Quart 2 | 1.4 | 0.99 | 1.3 |
| p Value | 0.38 | 0.99 | 0.47 |
| 95% CI of | 0.68 | 0.20 | 0.64 |
| OR Quart2 | 2.7 | 5.0 | 2.7 |
| OR Quart 3 | 1.9 | 1.3 | 1.6 |
| p Value | 0.069 | 0.71 | 0.16 |
| 95% CI of | 0.95 | 0.29 | 0.82 |
| OR Quart3 | 3.6 | 6.1 | 3.3 |
| OR Quart 4 | 1.7 | 2.8 | 1.5 |
| p Value | 0.14 | 0.14 | 0.30 |
| 95% CI of | 0.85 | 0.72 | 0.72 |
| OR Quart4 | 3.3 | 11 | 3.0 |

**Proepiregulin**

|  | sCr or UO |  | sCr only |  | UO only |  |
|---|---|---|---|---|---|---|
|  | Cohort 1 | Cohort 2 | Cohort 1 | Cohort 2 | Cohort 1 | Cohort 2 |
| Median | 3.42 | 5.13 | 3.55 | 3.48 | 3.52 | 5.13 |
| Average | 6.98 | 13.9 | 8.25 | 15.1 | 7.67 | 13.0 |
| Stdev | 19.3 | 22.1 | 20.3 | 20.4 | 20.3 | 21.9 |
| p(t-test) |  | 0.014 |  | 0.27 |  | 0.081 |
| Min | 0.0298 | 0.376 | 0.0298 | 1.42 | 0.0298 | 0.376 |
| Max | 279 | 134 | 279 | 57.1 | 279 | 134 |
| n (Samp) | 237 | 64 | 285 | 11 | 223 | 57 |
| n (Patient) | 237 | 64 | 285 | 11 | 223 | 57 |

|  | At Enrollment |  |  |
|---|---|---|---|
|  | sCr or UO | sCr only | UO only |
| AUC | 0.63 | 0.60 | 0.61 |
| SE | 0.041 | 0.092 | 0.043 |
| p | 0.0020 | 0.29 | 0.015 |
| nCohort 1 | 237 | 285 | 223 |
| nCohort 2 | 64 | 11 | 57 |
| Cutoff 1 | 2.85 | 2.57 | 2.85 |
| Sens 1 | 70% | 73% | 70% |
| Spec 1 | 43% | 38% | 42% |
| Cutoff 2 | 1.81 | 2.10 | 1.81 |
| Sens 2 | 81% | 82% | 81% |
| Spec 2 | 31% | 32% | 30% |
| Cutoff 3 | 0.971 | 1.46 | 0.750 |
| Sens 3 | 91% | 91% | 91% |
| Spec 3 | 16% | 22% | 9% |
| Cutoff 4 | 5.81 | 6.55 | 6.30 |
| Sens 4 | 48% | 45% | 46% |
| Spec 4 | 70% | 70% | 70% |
| Cutoff 5 | 8.47 | 10.2 | 9.47 |
| Sens 5 | 39% | 36% | 37% |
| Spec 5 | 80% | 80% | 80% |
| Cutoff 6 | 13.6 | 17.1 | 14.6 |
| Sens 6 | 27% | 27% | 23% |
| Spec 6 | 90% | 90% | 90% |
| OR Quart 2 | 1.9 | 2.1 | 1.5 |
| p Value | 0.14 | 0.41 | 0.37 |

(continued)

| At | At Enrollment | | |
|---|---|---|---|
| | sCr or UO | sCr only | UO only |
| 95% CI of | 0.81 | 0.37 | 0.62 |
| OR Quart2 | 4.5 | 12 | 3.7 |
| OR Quart 3 | 1.1 | 0.49 | 1.1 |
| p Value | 0.81 | 0.57 | 0.81 |
| 95% CI of | 0.44 | 0.044 | 0.44 |
| OR Quart3 | 2.8 | 5.6 | 2.8 |
| OR Quart 4 | 3.4 | 2.1 | 2.8 |
| p Value | 0.0035 | 0.41 | 0.018 |
| 95% CI of | 1.5 | 0.37 | 1.2 |
| OR Quart4 | 7.7 | 12 | 6.4 |

**Heparin-binding growth factor 1**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 867 | 712 | 869 | 396 | 861 | 820 |
| Average | 1250 | 1160 | 1250 | 768 | 1240 | 1200 |
| Stdev | 1400 | 1340 | 1390 | 1230 | 1420 | 1310 |
| p(t-test) | | 0.58 | | 0.15 | | 0.82 |
| Min | 0.00328 | 2.62 | 0.00328 | 2.62 | 0.00328 | 2.62 |
| Max | 12300 | 6460 | 12300 | 5360 | 12300 | 6460 |
| n (Samp) | 352 | 93 | 421 | 18 | 343 | 82 |
| n (Patient) | 352 | 93 | 421 | 18 | 343 | 82 |

| | At Enrollment | | |
|---|---|---|---|
| | sCr or UO | sCr only | UO only |
| AUC | 0.46 | 0.32 | 0.48 |
| SE | 0.034 | 0.071 | 0.036 |
| p | 0.21 | 0.013 | 0.66 |
| nCohort 1 | 352 | 421 | 343 |
| nCohort 2 | 93 | 18 | 82 |
| Cutoff 1 | 338 | 221 | 417 |
| Sens 1 | 71% | 72% | 71% |
| Spec 1 | 22% | 14% | 29% |
| Cutoff 2 | 226 | 101 | 280 |

(continued)

|  | At Enrollment | | |
|---|---|---|---|
|  | sCr or UO | sCr only | UO only |
| Sens 2 | 81% | 83% | 80% |
| Spec 2 | 13% | 8% | 18% |
| Cutoff 3 | 101 | 49.5 | 119 |
| Sens 3 | 90% | 94% | 90% |
| Spec 3 | 8% | 4% | 9% |
| Cutoff 4 | 1430 | 1420 | 1420 |
| Sens 4 | 26% | 17% | 27% |
| Spec 4 | 70% | 70% | 70% |
| Cutoff 5 | 1850 | 1850 | 1820 |
| Sens 5 | 18% | 6% | 23% |
| Spec 5 | 80% | 80% | 80% |
| Cutoff 6 | 2630 | 2670 | 2670 |
| Sens 6 | 11% | 6% | 11% |
| Spec 6 | 90% | 90% | 90% |
| OR Quart 2 | 1.1 | 4.1 | 1.1 |
| p Value | 0.71 | 0.21 | 0.84 |
| 95% CI of | 0.59 | 0.45 | 0.54 |
| OR Quart2 | 2.2 | 37 | 2.1 |
| OR Quart 3 | 0.95 | 4.1 | 0.95 |
| p Value | 0.89 | 0.21 | 0.88 |
| 95% CI of | 0.48 | 0.45 | 0.47 |
| OR Quart3 | 1.9 | 37 | 1.9 |
| OR Quart 4 | 1.5 | 9.8 | 1.1 |
| p Value | 0.19 | 0.032 | 0.71 |
| 95% CI of | 0.81 | 1.2 | 0.58 |
| OR Quart4 | 2.9 | 79 | 2.2 |

**Thrombospondin-2**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 988 | 1300 | 1050 | 2210 | 1050 | 1300 |
| Average | 1640 | 3290 | 1940 | 3080 | 1740 | 3270 |
| Stdev | 2280 | 7500 | 4070 | 3520 | 2430 | 7860 |
| p(t-test) | 4.1E-4 | 4.1E-4 |  | 0.24 |  | 0.0024 |
| Min | 0.0376 | 14.6 | 0.0376 | 90.7 | 0.0376 | 14.6 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Max | 26900 | 68100 | 68100 | 14600 | 26900 | 68100 |
| n (Samp) | 352 | 93 | 421 | 18 | 343 | 82 |
| n (Patient) | 352 | 93 | 421 | 18 | 343 | 82 |

|  | At Enrollment | | |
|---|---|---|---|
|  | sCr or UO | sCr only | UO only |
| AUC | 0.60 | 0.61 | 0.58 |
| SE | 0.034 | 0.072 | 0.036 |
| p | 0.0038 | 0.14 | 0.019 |
| nCohort 1 | 352 | 421 | 343 |
| nCohort 2 | 93 | 18 | 82 |
| Cutoff 1 | 760 | 679 | 760 |
| Sens 1 | 71% | 72% | 71% |
| Spec 1 | 39% | 34% | 37% |
| Cutoff 2 | 498 | 360 | 502 |
| Sens 2 | 81% | 83% | 80% |
| Spec 2 | 25% | 17% | 25% |
| Cutoff 3 | 332 | 122 | 360 |
| Sens 3 | 90% | 94% | 90% |
| Spec 3 | 16% | 5% | 18% |
| Cutoff 4 | 1770 | 1830 | 1840 |
| Sens 4 | 41% | 56% | 39% |
| Spec 4 | 70% | 70% | 70% |
| Cutoff 5 | 2270 | 2430 | 2350 |
| Sens 5 | 40% | 44% | 37% |
| Spec 5 | 80% | 80% | 80% |
| Cutoff 6 | 3270 | 4010 | 3620 |
| Sens 6 | 28% | 28% | 22% |
| Spec 6 | 90% | 90% | 90% |
| OR Quart 2 | 0.71 | 0.19 | 0.86 |
| p Value | 0.36 | 0.13 | 0.70 |
| 95% CI of | 0.34 | 0.022 | 0.41 |
| OR Quart2 | 1.5 | 1.7 | 1.8 |
| OR Quart 3 | 1.1 | 0.58 | 1.1 |
| p Value | 0.86 | 0.47 | 0.85 |
| 95% CI of | 0.54 | 0.14 | 0.52 |

(continued)

|  | At Enrollment | | |
| --- | --- | --- | --- |
|  | sCr or UO | sCr only | UO only |
| OR Quart3 | 2.1 | 2.5 | 2.2 |
| OR Quart 4 | 2.2 | 1.9 | 2.2 |
| p Value | 0.011 | 0.28 | 0.018 |
| 95% CI of | 1.2 | 0.60 | 1.2 |
| OR Quart4 | 4.2 | 5.7 | 4.3 |

Fig. 12: Comparison of marker levels in enroll EDTA samples collected from Cohort 1 (patients that did not progress beyond RIFLE stage 0 or R within 48hrs) and in enroll EDTA samples collected from Cohort 2 (subjects reaching RIFLE stage I or F within 48hrs). Enroll samples from patients already at stage I or F were included in Cohort 2.

**Angiogenin**

|  | sCr or UO | | UO only | |
| --- | --- | --- | --- | --- |
|  | Cohort 1 | Cohort 2 | Cohort 1 | Cohort 2 |
| Median | 179000 | 117000 | 179000 | 110000 |
| Average | 197000 | 159000 | 197000 | 157000 |
| Stdev | 197000 | 107000 | 201000 | 108000 |
| p(t-test) |  | 0.32 |  | 0.30 |
| Min | 10800 | 24100 | 10800 | 24100 |
| Max | 2240000 | 430000 | 2240000 | 430000 |
| n (Samp) | 141 | 30 | 135 | 29 |
| n (Patient) | 141 | 30 | 135 | 29 |

|  | At Enrollment | |
| --- | --- | --- |
|  | sCr or UO | UO only |
| AUC | 0.40 | 0.39 |
| SE | 0.059 | 0.060 |
| p | 0.079 | 0.056 |
| nCohort 1 | 141 | 135 |
| nCohort 2 | 30 | 29 |
| Cutoff 1 | 90900 | 88800 |
| Sens 1 | 70% | 72% |
| Spec 1 | 15% | 14% |
| Cutoff 2 | 82700 | 80900 |
| Sens 2 | 80% | 83% |
| Spec 2 | 13% | 13% |
| Cutoff 3 | 76300 | 44000 |

(continued)

|  | At Enrollment | |
| --- | --- | --- |
|  | sCr or UO | UO only |
| Sens 3 | 90% | 93% |
| Spec 3 | 12% | 4% |
| Cutoff 4 | 218000 | 215000 |
| Sens 4 | 23% | 24% |
| Spec 4 | 70% | 70% |
| Cutoff 5 | 241000 | 239000 |
| Sens 5 | 20% | 21% |
| Spec 5 | 80% | 80% |
| Cutoff 6 | 314000 | 332000 |
| Sens 6 | 10% | 10% |
| Spec 6 | 90% | 90% |
| OR Quart 2 | 0.81 | 0.63 |
| p Value | 0.75 | 0.50 |
| 95% CI of | 0.23 | 0.16 |
| OR Quart2 | 2.9 | 2.4 |
| OR Quart 3 | 1.2 | 1.2 |
| p Value | 0.76 | 0.76 |
| 95% CI of | 0.37 | 0.37 |
| OR Quart3 | 3.9 | 3.9 |
| OR Quart 4 | 2.5 | 2.4 |
| p Value | 0.11 | 0.12 |
| 95% CI of | 0.83 | 0.81 |
| OR Quart4 | 7.4 | 7.2 |

**Angiopoietin-related protein 4**

|  | sCr or UO | | UO only | |
| --- | --- | --- | --- | --- |
|  | Cohort 1 | Cohort 2 | Cohort 1 | Cohort 2 |
| Median | 34.2 | 47.0 | 35.0 | 47.4 |
| Average | 63.6 | 73.3 | 65.6 | 75.0 |
| Stdev | 166 | 66.6 | 169 | 67.2 |
| p(t-test) |  | 0.75 |  | 0.77 |
| Min | 1.77 | 16.4 | 2.71 | 16.4 |
| Max | 1900 | 339 | 1900 | 339 |
| n (Samp) | 141 | 30 | 135 | 29 |
| n (Patient) | 141 | 30 | 135 | 29 |

|  | At Enrollment | |
| --- | --- | --- |
|  | sCr or UO | UO only |
| AUC | 0.65 | 0.65 |
| SE | 0.058 | 0.060 |
| P | 0.0094 | 0.011 |
| nCohort 1 | 141 | 135 |
| nCohort 2 | 30 | 29 |
| Cutoff 1 | 35.6 | 35.6 |
| Sens 1 | 70% | 72% |
| Spec 1 | 53% | 52% |
| Cutoff 2 | 29.1 | 29.1 |
| Sens 2 | 80% | 83% |
| Spec 2 | 41% | 39% |
| Cutoff 3 | 24.9 | 22.7 |
| Sens 3 | 90% | 93% |
| Spec 3 | 35% | 27% |
| Cutoff 4 | 51.8 | 53.8 |
| Sens 4 | 43% | 45% |
| Spec 4 | 70% | 70% |
| Cutoff 5 | 71.1 | 71.8 |
| Sens 5 | 30% | 31% |
| Spec 5 | 80% | 80% |
| Cutoff 6 | 98.7 | 104 |
| Sens 6 | 27% | 28% |
| Spec 6 | 90% | 90% |
| OR Quart 2 | 4.6 | 2.2 |
| p Value | 0.065 | 0.30 |
| 95% CI of | 0.91 | 0.50 |
| OR Quart2 | 23 | 9.4 |
| OR Quart 3 | 6.1 | 4.1 |
| p Value | 0.026 | 0.045 |
| 95% CI of | 1.2 | 1.0 |
| OR Quart3 | 30 | 16 |
| OR Quart 4 | 6.1 | 4.1 |
| p Value | 0.026 | 0.045 |
| 95% CI of | 1.2 | 1.0 |
| OR Quart4 | 30 | 16 |

**Amphiregulin**

|  | sCr or UO | | UO only | |
|---|---|---|---|---|
|  | Cohort 1 | Cohort 2 | Cohort 1 | Cohort 2 |
| Median | 24.9 | 27.5 | 24.5 | 31.5 |
| Average | 32.9 | 37.1 | 32.6 | 38.1 |
| Stdev | 29.0 | 32.2 | 29.3 | 32.3 |
| p(t-test) |  | 0.50 |  | 0.38 |
| Min | 0.00246 | 2.76 | 0.00246 | 2.76 |
| Max | 198 | 162 | 198 | 162 |
| n (Samp) | 136 | 29 | 130 | 28 |
| n (Patient) | 136 | 29 | 130 | 28 |

|  | At Enrollment | |
|---|---|---|
|  | sCr or UO | UO only |
| AUC | 0.53 | 0.56 |
| SE | 0.060 | 0.061 |
| p | 0.58 | 0.37 |
| nCohort 1 | 136 | 130 |
| nCohort 2 | 29 | 28 |
| Cutoff 1 | 16.8 | 17.0 |
| Sens 1 | 72% | 71% |
| Spec 1 | 29% | 30% |
| Cutoff 2 | 11.1 | 11.1 |
| Sens 2 | 83% | 86% |
| Spec 2 | 12% | 14% |
| Cutoff 3 | 8.21 | 8.93 |
| Sens 3 | 97% | 93% |
| Spec 3 | 9% | 12% |
| Cutoff 4 | 34.0 | 34.0 |
| Sens 4 | 48% | 50% |
| Spec 4 | 71% | 72% |
| Cutoff 5 | 44.7 | 44.1 |
| Sens 5 | 34% | 36% |
| Spec 5 | 80% | 80% |
| Cutoff 6 | 67.0 | 60.3 |
| Sens 6 | 14% | 14% |
| Spec 6 | 90% | 90% |

(continued)

|  | At Enrollment | |
|---|---|---|
|  | sCr or UO | UO only |
| OR Quart 2 | 0.57 | 0.65 |
| p Value | 0.37 | 0.50 |
| 95% CI of | 0,17 | 0.19 |
| OR Quart2 | 1.9 | 2.3 |
| OR Quart 3 | 0.71 | 0.83 |
| p Value | 0.56 | 0.76 |
| 95% CI of | 0.22 | 0.25 |
| OR Quart3 | 2.3 | 2.7 |
| OR Quart 4 | 1.3 | 1.5 |
| p Value | 0.64 | 0.45 |
| 95% CI of | 0.45 | 0.51 |
| OR Quart4 | 3.7 | 4.5 |

**Betacellulin**

|  | sCr or UO | | UO only | |
|---|---|---|---|---|
|  | Cohort 1 | Cohort 2 | Cohort 1 | Cohort 2 |
| Median | 3.93 | 3.11 | 3.73 | 3.11 |
| Average | 5.07 | 5.36 | 4.89 | 5.50 |
| Stdev | 5.50 | 7.04 | 5.49 | 7.12 |
| p(t-test) |  | 0.80 |  | 0.61 |
| Min | 0.00226 | 0.00274 | 0.00226 | 0.00274 |
| Max | 52.9 | 32.2 | 52.9 | 32.2 |
| n (Samp) | 137 | 30 | 131 | 29 |
| n (Patient) | 137 | 30 | 131 | 29 |

|  | At Enrollment | |
|---|---|---|
|  | sCr or UO | UO only |
| AUC | 0.45 | 0.47 |
| SE | 0.059 | 0.060 |
| P | 0.36 | 0.60 |
| nCohort 1 | 137 | 131 |
| nCohort 2 | 30 | 29 |
| Cutoff 1 | 2.26 | 2.26 |
| Sens 1 | 70% | 72% |

(continued)

| | At Enrollment | |
|---|---|---|
| | sCr or UO | UO only |
| Spec 1 | 25% | 27% |
| Cutoff 2 | 1.78 | 1.78 |
| Sens 2 | 80% | 83% |
| Spec 2 | 21% | 23% |
| Cutoff 3 | 0.00282 | 0.00282 |
| Sens 3 | 97% | 97% |
| Spec 3 | 4% | 5% |
| Cutoff 4 | 5.88 | 5.83 |
| Sens 4 | 23% | 24% |
| Spec 4 | 70% | 70% |
| Cutoff 5 | 7.71 | 7.22 |
| Sens 5 | 17% | 17% |
| Spec 5 | 80% | 80% |
| Cutoff 6 | 10.1 | 9.07 |
| Sens 6 | 13% | 14% |
| Spec 6 | 91% | 90% |
| OR Quart 2 | 1.0 | 1.0 |
| p Value | 1.0 | 1.0 |
| 95% CI of | 0.29 | 0.29 |
| OR Quart2 | 3.4 | 3.4 |
| OR Quart 3 | 1.6 | 1.9 |
| p Value | 0.40 | 0.27 |
| 95% CI of | 0.53 | 0.61 |
| OR Quart3 | 5.1 | 5.8 |
| OR Quart 4 | 1.7 | 1.2 |
| p Value | 0.37 | 0.76 |
| 95% CI of | 0.54 | 0.37 |
| OR Quart4 | 5.3 | 4.0 |

**Proepiregulin**

| | sCr or UO | | UO only | |
|---|---|---|---|---|
| | Cohort 1 | Cohort 2 | Cohort 1 | Cohort 2 |
| Median | 0.411 | 0.383 | 0.406 | 0.394 |
| Average | 0.804 | 0.981 | 0.805 | 1.01 |
| Stdev | 2.80 | 2.10 | 2.86 | 2.13 |

(continued)

|  | sCr or UO | | UO only | |
| --- | --- | --- | --- | --- |
|  | Cohort 1 | Cohort 2 | Cohort 1 | Cohort 2 |
| p(t-test) |  | 0.75 |  | 0.72 |
| Min | 0.000152 | 0.0249 | 0.000152 | 0.0249 |
| Max | 32.1 | 11.3 | 32.1 | 11.3 |
| n (Samp) | 136 | 29 | 130 | 28 |
| n (Patient) | 136 | 29 | 130 | 28 |

|  | At Enrollment | |
| --- | --- | --- |
|  | sCr or UO | UO only |
| AUC | 0.51 | 0.54 |
| SE | 0.060 | 0.061 |
| p | 0.84 | 0.56 |
| nCohort 1 | 136 | 130 |
| nCohort 2 | 29 | 28 |
| Cutoff 1 | 0.242 | 0.242 |
| Sens 1 | 72% | 75% |
| Spec 1 | 31% | 32% |
| Cutoff 2 | 0.200 | 0.200 |
| Sens 2 | 83% | 82% |
| Spec 2 | 25% | 27% |
| Cutoff 3 | 0.0796 | 0.117 |
| Sens 3 | 93% | 93% |
| Spec 3 | 12% | 18% |
| Cutoff 4 | 0.647 | 0.589 |
| Sens 4 | 24% | 36% |
| Spec 4 | 72% | 71% |
| Cutoff 5 | 0.806 | 0.750 |
| Sens 5 | 21% | 25% |
| Spec 5 | 80% | 80% |
| Cutoff 6 | 1.24 | 1.20 |
| Sens 6 | 17% | 18% |
| Spec 6 | 90% | 90% |
| OR Quart 2 | 1.9 | 2.0 |
| p Value | 0.27 | 0.27 |
| 95% CI of | 0.61 | 0.60 |
| OR Quart2 | 5.8 | 6.5 |
| OR Quart 3 | 1.0 | 1.5 |

(continued)

| | At Enrollment | |
|---|---|---|
| | sCr or UO | UO only |
| p Value | 1.0 | 0.53 |
| 95% CI of | 0.29 | 0.43 |
| OR Quart3 | 3.4 | 5.2 |
| OR Quart 4 | 1.2 | 1.4 |
| p Value | 0.80 | 0.56 |
| 95% CI of | 0.36 | 0.42 |
| OR Quart4 | 3.8 | 5.0 |

The examples provided herein are representative of preferred embodiments, are exemplary, and are not intended as limitations on the scope of the invention. Modifications therein and other uses will occur to those skilled in the art.

All patents and publications mentioned in the specification are indicative of the levels of those of ordinary skill in the art to which the invention pertains.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Other embodiments are set forth within the following claims.

**Claims**

1.  A method for evaluating renal status in a subject, comprising:

performing one or more assays configured to detect Angiopoietin-related protein 4 in a body fluid sample obtained from the subject to provide an assay result; and

correlating the assay result(s) to the renal status of the subject, wherein said correlating step comprises assigning a likelihood of one or more future changes in renal status to the subject based on the assay result(s).

2. A method according to claim 1, wherein said correlation step comprises correlating the assay result(s) to one or more of risk stratification, diagnosis, staging, prognosis, classifying and monitoring of the renal status of the subject.

3. A method according to claim 1, wherein said one or more future changes in renal status comprise one or more of a future injury to renal function, future reduced renal function, future improvement in renal function, future acute renal failure (ARF) and death.

4. A method according to claim 1, comprising predicting a decreased or increased risk that the subject will recover from an injury to renal function or predicting a decreased or increased risk that a subject will recover from ARF.

5. A method according to one of claims 1-4, wherein a plurality of assay results are combined using a function that converts the plurality of assay results into a single composite result.

6. A method according to claim 1, wherein the likelihood of one or more future changes in renal status is that an event of interest is more or less likely to occur within a period of time selected from the group consisting of 90 days, 30 days, 21 days, 14 days, 7 days, 5 days, 96 hours, 72 hours, 48 hours, 36 hours, 24 hours, and 12 hours of the time at which the body fluid sample is obtained from the subject.

7. A method according to one of claims 1-4, wherein the subject is selected for evaluation of renal status based on the pre-existence in the subject of one or more known risk factors for prerenal, intrinsic renal, or postrenal ARF, or wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

8. A method according to one of claims 1-4, wherein said correlating step comprises assessing whether or not renal function is improving or worsening in a subject who has suffered from an injury to renal function, reduced renal function, or ARF based on the assay result(s).

9. A method according to one of claims 1-4, wherein said method is a method of assigning a risk of the future occurrence or nonoccurrence of reduced renal function in said subject, wherein said method is a method of assigning a risk of the future occurrence or nonoccurrence of acute renal failure in said subject, wherein said method is a method of assigning a risk of the future occurrence or nonoccurrence of a need for renal replacement therapy in said subject, or wherein said method is a method of assigning a risk of the future occurrence or nonoccurrence of a need for renal transplantation in said subject.

10. Use of Angiopoietin-related protein 4 for the evaluation of renal injury in a body fluid sample obtained from a subject, the use comprising performing one or more assays configured to detect Angiopoietin-related protein 4 and correlating the assay result(s) to the renal status of the subject, wherein said correlating step comprises assigning a likelihood of one or more future changes in renal status to the subject based on the assay result(s).

11. The use of claim 10, wherein said correlating step comprises assessing whether or not renal function is improving or worsening in a subject who has suffered from an injury to renal function, reduced renal function, or ARF based on the assay result(s).

**Patentansprüche**

1. Verfahren zum Auswerten des Nierenstatus bei einem Subjekt, Folgendes umfassend:

   Durchführen eines oder mehrerer Assays, die konfiguriert sind zum Nachweisen von Angiopoietin-verwandtem Protein 4 in einer von dem Subjekt erhaltenen Körperflüssigkeitsprobe, um ein Testergebnis bereitzustellen; und Korrelieren des/der Testergebnisse(s) mit dem Nierenstatus des Subjekts, wobei der Korrelationsschritt das Zuweisen einer Wahrscheinlichkeit einer oder mehrerer zukünftiger Änderungen des Nierenstatus bei dem Subjekt basierend auf dem/den Testergebnis(sen) umfasst.

2. Verfahren nach Anspruch 1, wobei der Korrelationsschritt das Korrelieren des/der Testergebnisse(s) mit dem Risikostratifizierung, Diagnose, Staging, Prognose, Klassifizierung und/oder Überwachung des Nierenstatus des Subjekts umfasst.

3. Verfahren nach Anspruch 1, wobei die eine oder die mehreren zukünftigen Änderungen des Nierenstatus eine zukünftige Schädigung der Nierenfunktion, zukünftige verminderte Nierenfunktion, zukünftige Verbesserung der Nierenfunktion, zukünftiges akutes Nierenversagen (ARF) und/oder Tod umfassen.

4. Verfahren nach Anspruch 1, umfassend das Vorhersagen einer erhöhten oder verminderten Wahrscheinlichkeit, dass sich das Subjekt von einer Schädigung der Nierenfunktion erholt, oder das Vorhersagen einer erhöhten oder verminderten Wahrscheinlichkeit, dass sich das Subjekt von ARF erholt.

5. Verfahren nach einem der Ansprüche 1-4, wobei mehrere Testergebnisse unter Einsatz einer Funktion kombiniert werden, die mehrere Assayergebnisse zu einem einzigen zusammengesetzten Ergebnis umwandelt.

6. Verfahren nach Anspruch 1, wobei die Wahrscheinlichkeit eines oder mehrerer zukünftiger Veränderungen des Nierenstatus ist, dass ein Vorkommnis von Interesse wahrscheinlicher oder weniger wahrscheinlich in einem Zeitraum eintritt, ausgewählt aus der Gruppe bestehend aus 90 Tagen, 30 Tagen, 21 Tagen, 14 Tagen, 7 Tagen, 5 Tagen, 96 Stunden, 72 Stunden, 48 Stunden, 36 Stunden, 24 Stunden und 12 Stunden von dem Zeitpunkt, zu dem die Körperflüssigkeitsprobe vom Probanden entnommen wird.

7. Verfahren nach einem der Ansprüche 1-4, wobei das Subjekt zur Bewertung des Nierenstatus auf der Grundlage der Vorexistenz in dem Subjekt von einem oder mehreren bekannten Risikofaktoren für prärenales, intrinsisches renales oder postrenales ARF ausgewählt ist, oder
   wobei das Subjekt zur Bewertung des Nierenstatus ausgewählt wird, basierend auf einer bestehenden Diagnose von Herzinsuffizienz, Präeklampsie, Eklampsie, Diabetes mellitus, Bluthochdruck, koronarer Herzkrankheit, Proteinurie, Niereninsuffizienz, glomerulärer Filtration unterhalb des normalen Bereichs, Zirrhose, Serumkreatinin über dem normalen Bereich, Sepsis, Schädigung der Nierenfunktion, verminderter Nierenfunktion und/oder ARF oder aufgrund einer größeren Gefäßoperation, eines Bypasses der Koronararterie oder einer anderen Herzoperation oder aufgrund einer Exposition gegenüber NSAIDs, Cyclosporinen, Tacrolimus, Aminoglycosiden, Foscarnet, Ethylenglykol, Hämoglobin, Myoglobin, Ifosfamid, Schwermetallen, Methotrexat, röntgendichtem Kontrastmittel oder Streptozotocin.

8. Verfahren nach einem der Ansprüche 1-4, wobei der Korrelationsschritt das Auswerten auf der Grundlage des/der Testergebnisse(s) umfasst, ob sich die Nierenfunktion bei einem Subjekt, der eine Schädigung der Nierenfunktion, eine verminderten Nierenfunktion oder ARF erlitten hat, verbessert oder verschlechtert.

9. Verfahren nach einem der Ansprüche 1-4, wobei das Verfahren ein Verfahren zum Zuordnen eines Risikos des zukünftigen Auftretens oder Nichtauftretens verminderter Nierenfunktion bei dem Subjekt ist,
   wobei das Verfahren ein Verfahren zum Zuordnen eines Risikos des zukünftigen Auftretens oder Nichtauftretens von akutem Nierenversagen bei dem Subjekt ist,
   wobei das Verfahren ein Verfahren zum Zuordnen eines Risikos des zukünftigen Auftretens oder Nichtauftretens des Bedarfs an Nierenersatztherapie bei dem Subjekt ist, oder
   wobei das Verfahren ein Verfahren zum Zuordnen eines Risikos des zukünftigen Auftretens oder Nichtauftretens des Bedarfs an Nierentransplantation bei dem Subjekt ist.

10. Verwendung von Angiopoietin-verwandtem Protein 4 zum Auswerten von Nierenschäden in einer Körperflüssigkeitsprobe, die von einem Subjekt erhalten worden ist, wobei die Verwendung Folgendes umfasst: das Durchführen eines oder mehrerer Assays, die konfiguriert sind zum Nachweisen von Angiopoietin-verwandtem Protein 4, und das Korrelieren des/der Testergebnisse(s) mit dem Nierenstatus des Subjekts, wobei der Korrelationsschritt das Zuweisen einer Wahrscheinlichkeit einer oder mehrerer zukünftiger Änderungen des Nierenstatus bei dem Subjekt basierend auf dem/den Testergebnis(sen) umfasst.

**11.** Verwendung nach Anspruch 10, wobei der Korrelationsschritt das Auswerten auf der Grundlage des/der Testergebnisse(s) umfasst, ob sich die Nierenfunktion bei einem Subjekt, der eine Schädigung der Nierenfunktion, eine verminderten Nierenfunktion oder ARF erlitten hat, verbessert oder verschlechtert.

**Revendications**

**1.** Procédé pour l'évaluation de l'état rénal chez un sujet, comprenant :

la réalisation d'une ou plusieurs analyses conçues pour détecter la protéine 4 associée à l'angiopoïétine dans un échantillon de fluide corporel obtenu du sujet pour fournir un résultat d'analyse ; et
la mise en corrélation du ou des résultat(s) d'analyse avec l'état rénal du sujet, ladite étape de mise en corrélation comprenant l'attribution d'une probabilité d'un ou plusieurs changements futurs de l'état rénal du sujet sur la base du ou des résultat(s) d'analyse.

**2.** Procédé selon la revendication 1, ladite étape de mise en corrélation comprenant la mise en corrélation du ou des résultat(s) d'analyse avec l'un ou plusieurs parmi une stratification du risque, un diagnostic, un échelonnement, un pronostic, une classification et un suivi de l'état rénal du sujet.

**3.** Procédé selon la revendication 1, ledit ou lesdits changements futurs de l'état rénal comprenant l'un ou plusieurs parmi une lésion future de l'état rénal, une fonction rénale future réduite, une amélioration future de la fonction rénale, une défaillance rénale future aiguë (ARF) et la mort.

**4.** Procédé selon la revendication 1, comprenant la prédiction d'un risque diminué ou augmenté pour le sujet de se remettre d'une lésion de la fonction rénale ou la prédiction d'un risque diminué ou augmenté pour un sujet de se remettre d'une ARF.

**5.** Procédé selon l'une quelconque des revendications 1 à 4, une pluralité de résultats d'analyse étant combinés à l'aide d'une fonction qui convertit la pluralité de résultats d'analyse en un unique résultat composite.

**6.** Procédé selon la revendication 1, la probabilité d'un ou plusieurs changements futurs de l'état rénal étant qu'il est plus ou moins probable qu'un événement d'intérêt ait lieu dans une période de temps choisie dans le groupe constitué par 90 jours, 30 jours, 21 jours, 14 jours, 7 jours, 5 jours, 96 heures, 72 heures, 48 heures, 36 heures, 24 heures, et 12 heures à compter du temps auquel l'échantillon de fluide corporel est obtenu du sujet.

**7.** Procédé selon l'une des revendications 1 à 4, le sujet étant choisi pour une évaluation de l'état rénal sur la base de la préexistence chez le sujet d'un ou plusieurs facteurs de risque connus pour une ARF prérénale, rénale intrinsèque, ou postrénale, ou
le sujet étant choisi pour une évaluation de l'état rénal sur la base d'un diagnostic existant d'un ou plusieurs parmi une défaillance cardiaque congestive, une prééclampsie, une éclampsie, un diabète sucré, de l'hypertension, une maladie coronarienne, une protéinurie, une insuffisance rénale, une filtration glomérulaire au-dessous de la plage normale, une cirrhose, une créatinine sérique au-dessus de la plage normale, une septicémie, une lésion de la fonction rénale, une fonction rénale réduite, ou une ARF, ou basé sur le fait qu'il subit ou qu'il a subi une chirurgie vasculaire majeure, un pontage aorto-coronarien, ou une autre chirurgie cardiaque, ou basé sur une exposition à des AINS, à des cyclosporines, au tacrolimus, à des aminoglycosides, au foscarnet, à l'éthylèneglycol, à l'hémoglobine, à la myoglobine, à l'ifosfamide, aux métaux lourds, au méthotrexate, à des agents de contraste radio-opaques, ou à la streptozotocine.

**8.** Procédé selon l'une des revendications 1 à 4, ladite étape de mise en corrélation comprenant l'évaluation du fait que la fonction rénale s'améliore ou s'aggrave, ou pas, chez un sujet qui a souffert d'une lésion de la fonction rénale, d'une fonction rénale réduite, ou d'une ARF sur la base du ou des résultat(s) d'analyse.

**9.** Procédé selon l'une des revendications 1 à 4,

ledit procédé étant un procédé d'attribution d'un risque de l'occurrence ou de la non occurrence future d'une fonction rénale réduite chez ledit sujet,

ledit procédé étant un procédé d'attribution d'un risque de l'occurrence ou de la non occurrence future d'une défaillance rénale aiguë chez ledit sujet,

ledit procédé étant un procédé d'attribution d'un risque de l'occurrence ou de la non occurrence future d'un besoin d'une thérapie de remplacement rénal chez ledit sujet, ou

ledit procédé étant un procédé d'attribution d'un risque de l'occurrence ou de la non occurrence future d'un besoin d'une transplantation rénale chez ledit sujet.

**10.** Utilisation de la protéine 4 associée à l'angiopoïétine pour l'évaluation d'une lésion rénale dans un échantillon de fluide corporel obtenu d'un sujet, l'utilisation comprenant la réalisation d'une ou plusieurs analyses conçues pour détecter la protéine 4 associée à l'angiopoïétine et la mise en corrélation du ou des résultat(s) d'analyse avec l'état rénal du sujet, ladite étape de mise en corrélation comprenant l'attribution d'une probabilité d'un ou plusieurs changements futurs de l'état rénal du sujet sur la base du ou des résultat(s) d'analyse.

**11.** Utilisation selon la revendication 10, ladite étape de mise en corrélation comprenant l'évaluation du fait que la fonction rénale s'améliore ou s'aggrave, ou pas, chez un sujet qui a souffert d'une lésion de la fonction rénale, d'une fonction rénale réduite, ou d'une ARF sur la base du ou des résultat(s) d'analyse.

## REFERENCES CITED IN THE DESCRIPTION

### Patent documents cited in the description

- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A
- US 2012020572 W
- US 61430977 B
- US 61430978 B
- US 61430979 B
- US 61430980 B
- US 61430981 B
- US 61430982 B
- US 61430983 B
- US 61430984 B
- US 61430985 B
- US 61430986 B
- US 61430987 B
- US 61430988 B
- US 61430989 B

### Non-patent literature cited in the description

- Harrison's Principles of Internal Medicine. McGraw Hill, 1741-1830
- Current Medical Diagnosis & Treatment. McGraw Hill, 2008, 785-815
- Merck Manual
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care.,* 2004, vol. 8 (4), R204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 (4), 177-197
- **THAKAR et al.** *J. Am. Soc. Nephrol.,* 2005, vol. 16, 162-68
- **MEHRAN et al.** *J. Am. Coll. Cardiol.,* 2004, vol. 44, 1393-99
- **WIJEYSUNDERA et al.** *JAMA,* 2007, vol. 297, 1801-9
- **GOLDSTEIN ; CHAWLA.** *Clin. J. Am. Soc. Nephrol.,* 2010, vol. 5, 943-49
- **CHAWLA et al.** *Kidney Intl.,* 2005, vol. 68, 2274-80
- The Immunoassay Handbook. Stockton Press, 1994
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed.,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant.,* 2008, vol. 23, 1203-1210
- Merck Manual of Diagnosis and Therapy. Merck Research Laboratories, 1999
- **HANLEY, J. A. ; MCNEIL, B.J.** The meaning and use of the area under a receiver operating characteristic (ROC) curve. *Radiology,* 1982, vol. 143, 29-36